# EUROPÄISCHE PATENTSCHRIFT

(11) **EP 2 501 813 B1**
(45) Veröffentlichungstag und Bekanntmachung des Hinweises auf die Patenterteilung: **21.02.2018**
(21) Anmeldenummer: 10785001.8
(22) Anmeldetag: 19.10.2010
(51) Int. Cl.: C12N 15/10

(54) **ZELLEN, NUKLEINSÄUREN, ENZYME UND DEREN VERWENDUNG SOWIE VERFAHREN ZUR HERSTELLUNG VON SOPHOROLIPIDEN**
CELLS, NUCLEIC ACIDS, ENZYMES, AND USE THEREOF, AND METHODS FOR THE PRODUCTION OF SOPHOROLIPIDS
CELLULES, ACIDES NUCLÉIQUES, ENZYMES ET LEUR UTILISATION, AINSI QUE PROCÉDÉ DE PRODUCTION DE SOPHOROLIPIDES

(30) Priorität: 18.11.2009 DE 102009046799; 12.04.2010 DE 102010014680
(43) Veröffentlichungstag der Anmeldung: 26.09.2012
(73) Patentinhaber: Evonik Degussa GmbH, 45128 Essen (DE)
(72) Erfinder: SCHAFFER, Steffen, 45699 Herten (DE); WESSEL, Mirja, 44799 Bochum (DE); THIESSENHUSEN, Anja, 48147 Münster (DE)
(86) Internationale Anmeldenummer: PCT/EP2010/065713
(87) Internationale Veröffentlichungsnummer: WO 2011/061032

(56) Entgegenhaltungen:
- WO-A1-2011/070113
- WO-A1-2011/154523
- VAN BOGAERT INGE N A ET AL: "Importance of the cytochrome P450 monooxygenase CYP52 family for the sophorolipid-producing yeast Candida bombicola", FEMS YEAST RESEARCH, Bd. 9, Nr. 1, Februar 2009 (2009-02), Seiten 87-94, XP002640993,
- LOTTERMOSER KATRIN ET AL: "Cytochromes P450 of the sophorose lipid-producing yeast Candida apicola: Heterogeneity and polymerase chain reaction-mediated cloning of two genes", YEAST, Bd. 12, Nr. 6, 1996, Seiten 565-575, XP002640994, ISSN: 0749-503X
- VAN BOGAERT INGE N A ET AL: "Knocking out the MFE-2 gene of Candida bombicola leads to improved medium-chain sophorolipid production", FEMS YEAST RESEARCH, WILEY-BLACKWELL PUBLISHING LTD, GB, NL, Bd. 9, Nr. 4, 1. Juni 2009 (2009-06-01), Seiten 610-617, XP009123208, ISSN: 1567-1356, DOI: DOI:10.1111/J.1567-1364.2009.00501.X [gefunden am 2009-03-17]
- INGE N A VAN BOGAERT ET AL: "Microbial production and application of sophorolipids", APPLIED MICROBIOLOGY AND BIOTECHNOLOGY, SPRINGER, BERLIN, DE, Bd. 76, Nr. 1, 3. Mai 2007 (2007-05-03), Seiten 23-34, XP019538805, ISSN: 1432-0614, DOI: DOI:10.1007/S00253-007-0988-7
- VAN BOGAERT INGE N A ET AL: "Development of a transformation and selection system for the glycolipid-producing yeast Candida bombicola", YEAST, JOHN WILEY & SONS LTD, GB, Bd. 25, Nr. 4, 1. April 2008 (2008-04-01), Seiten 273-278, XP009123057, ISSN: 0749-503X, DOI: DOI:10.1002/YEA.1586 [gefunden am 2008-03-10]

## Beschreibung

### Gebiet der Erfindung

Gegenstand der Erfindung sind Nukleinsäuren, Enzyme und Zellen sowie deren Verwendung zur Herstellung von Sophorolipiden als auch Verfahren zur Herstellung von Sophorolipiden.

### Stand der Technik

Surfactants werden heutzutage im Wesentlichen basierend auf Basis petrochemischer Rohstoffe hergestellt. Die Nutzung von Surfactants auf Basis nachwachsender Rohstoffe ist aufgrund der absehbaren Verknappung petrochemischer Rohstoffe und zunehmender Nachfrage nach Produkten, die auf nachwachsenden Rohstoffen basieren bzw. biologisch abbaubar sind, eine entsprechende Alternative.
Sophorolipide besitzen die oberflächenaktiven Eigenschaften, die man zum Einsatz als Surfactant benötigt.
Diese Lipide werden heutzutage mit Wildtyp-Isolaten verschiedener Hefen, insbesondere mit *Candida bombicola,* hergestellt.
Eine Verbesserung von Leistungsparametern der Produktbildung wie Kohlenstoffausbeute, Raum-Zeit-Ausbeute, Produktkonzentration, Produkt-Homogenität (Acetylierungsgrad, Fettsäurespezies, Lacton- vs. offenkettige Form) findet bisher ausschließlich über die Optimierung der Prozessführung (pH-Wert, Sauerstoffversorgung, Medienzusammensetzung, feeding-Strategien, Stickstoffversorgung, Temperatur, Substratwahl, etc.) statt. Einzige Ausnahme ist die genetische Modifikation von *Candida bombicola* dahingehend, dass die β-Oxidation ausgeschaltet wurde, so dass als Substrat zugeführte Triglyceride, Fettsäuren, Fettalkohole, etc., nicht mehr als Kohlenstoffquelle genutzt, also abgebaut werden können (Van Bogaert et al FEMS Yeast Res. 2009 Jun;9(4):610-7). So sollte es möglich sein, durch Wahl des Substrates den Fettsäureanteil der Sophorolipide gezielt zu steuern, um die Produkteigenschaften zu beeinflussen.

Da die Verbesserung von Leistungsparametern der biotechnologischen Herstellung von Sophorolipiden über die Optimierung der Prozessführung nur begrenzt möglich ist, muss auch eine genetische Modifikation der Zellen stattfinden. Dies beinhaltet auf der einen Seite die Verstärkung der Enzyme, die an der Sophorolipid-Synthese beteiligt sind: Cytochrom P450-Monoxygenase, Glycosyltransferase I, Glycosyltransferase II, Acetyltransferase, Sophorolipid-Exporter mit dem Ziel der Verbesserung der Leistungsparameter der Produktbildung wie Kohlenstoffausbeute, Raum-Zeit-Ausbeute, Produktkonzentration, Produkt-Homogenität (Acetylierungsgrad, Fettsäurespezies), etc.. Dies beinhaltet auf der anderen Seite die Abschwächung einiger der Enzyme, die an der Sophorolipid-Synthese beteiligt sind: Glycosyltransferase II, Acetyltransferase mit dem Ziel der Modifikation der Struktur und der Eigenschaften der produzierten Sophorolipide: Glycosyltransferase II: Produktion von Monoglycosyl-Sophorolipiden; Acetyltransferase: Produktion von nicht-acetylierten Sophorolipiden.

Van Bogaert et al. offenbaren in "Importance of the cytochrome P450 monooxygenase CYP52 family for the sophorolipid-producing yeast Candida bombicola", FEMS YEAST RESEARCH, (2009-02), vol. 9, no. 1, pages 87 - 94, die Klonierung und Charakterisierung dreier CYP52 Gene aus C. *bombicola.*

Lottermoser et al. offenbaren in "Cytochromes P450 of the sophorose lipid-producing yeast Candida apicola: Heterogeneity and polymerase chain reaction-mediated cloning of two genes", YEAST, (1996), vol. 12, no. 6, die Klonierung und Charakterisierung zweier CYP52 Gene aus C. *apicola.*

Van Bogaert et al. offenbaren in "Knocking out the MFE-2 gene of Candida bombicola leads to improved medium-chain sophorolipid production", FEMS YEAST RESEARCH, WILEY-BLACKWELL PUBLISHING LTD, GB, NL, vol. 9, no. 4, doi:DOI:10.1111/J.1567-1364.2009.00501.X, ISSN 1567-1356, (20090601), pages 610 - 617, (20090317), in ihrer beta-Oxidation blockierte C. *bombicola* Stämme.

Sophorolipide werden, sollen sie im großen Masse als Surfactants in Reinigungs-, Kosmetik- und anderen Anwendungen eingesetzt werden, in den Wettbewerb mit den heutzutage eingesetzten Surfactants treten müssen. Diese sind Bulk-Chemikalien, die zu sehr niedrigen Kosten hergestellt werden können. Daher müssen Sophorolipide zu möglichst niedrigen Kosten hergestellt werden.

Dies ist durch Optimierung der Leistungsparameter über Prozessoptimierung allein nicht möglich.

Es besteht daher ein steigender Bedarf an effizienten Produktionen von Sophorolipiden mit hohen Produktausbeuten.

Der vorliegenden Erfindung lag daher die Aufgabe zugrunde, Werkzeuge und/oder Verfahren bereitzustellen, mit deren Hilfe sich spezifische Sophorolipide einfach und in großen Mengen darstellen lassen.

### Beschreibung der Erfindung

Überraschenderweise wurde gefunden, dass die im Folgenden beschriebenen Zellen, Nukleinsäuren, Polypeptide und Verfahren in der Lage sind, die vorbenannte Aufgabe zu lösen.

Gegenstand der vorliegenden Erfindung sind daher gentechnisch modifizierte Zellen mit einer veränderten enzymatischen Ausstattung zur Sophorolipid-Synthese.
Ein weiterer Gegenstand der Erfindung sind neue Nukleinsäuren und Vektoren wie in Anspruch 5 und 6 beschrieben.
Noch ein Gegenstand der vorliegenden Erfindung sind neue Enzyme, nützlich in der Sophorolipid-Biosynthese.

Die vorliegende Erfindung hat den Vorteil, dass nicht nur die Leistungsparameter der Sophorolipid-Bildung wie Kohlenstoffausbeute und Raum-Zeit-Ausbeute verbessert sind, sondern auch die Produkthomogenität bezüglich beispielsweise Acetylierungsgrad und Fettsäurespezies verbessert werden können.

Ein Gegenstand der Erfindung ist eine Zelle, die Sophorolipide zu bilden vermag, welche derart gentechnisch verändert wurde, dass sie verglichen zu ihrem Wildtyp eine geänderte, wie jeweils im Folgenden spezifizierte Aktivität mindestens eines der Enzyme ausgewählt aus der Gruppe bestehend aus,
mindestens ein Enzym E₁ mit Polypeptidsequenz Seq ID Nr. 7, Seq ID Nr. 53, Seq ID Nr. 55, Seq ID Nr. 57, Seq ID Nr. 59, Seq ID Nr. 61 oder Seq ID Nr. 63, insbesondere Seq ID Nr. 7, oder mit einer Polypeptidsequenz bei der bis zu 25%, bevorzugt bis zu 20%, besonders bevorzugt bis zu 15% insbesondere bis zu 10, 9, 8, 7, 6, 5, 4, 3, 2, 1 % der Aminosäurereste gegenüber der jeweiligen Bezugssequenz Seq ID Nr. 7, Seq ID Nr. 53, Seq ID Nr. 55, Seq ID Nr. 57, Seq ID Nr. 59, Seq ID Nr. 61 oder Seq ID Nr. 63, insbesondere Seq ID Nr. 7, durch Deletion, Insertion, Substitution oder eine Kombination davon verändert sind und die noch mindestens 50%, bevorzugt 65%, besonders bevorzugt 80%, insbesondere mehr als 90 % der enzymatischen Aktivität des Enzyms mit der jeweiligen Bezugssequenz Seq ID Nr. 7, Seq ID Nr. 53, Seq ID Nr. 55, Seq ID Nr. 57, Seq ID Nr. 59, Seq ID Nr. 61 oder Seq ID Nr. 63 besitzt, wobei unter enzymatischer Aktivität für ein Enzym E₁ die Fähigkeit verstanden wird, Z-9-Octadecensäure zu 17-Hydroxy-Z-9-Octadecensäure umzusetzen,
mindestens ein Enzym E₂ mit Polypeptidsequenz Seq ID Nr. 8 oder Seq ID Nr. 11 oder mit einer Polypeptidsequenz bei der bis zu 60%, bevorzugt bis zu 25%, besonders bevorzugt bis zu 15% insbesondere bis zu 10, 9, 8, 7, 6, 5, 4, 3, 2, 1 % der Aminosäurereste gegenüber Seq ID Nr. 8 oder Seq ID Nr. 11 durch Deletion, Insertion, Substitution oder eine Kombination davon verändert sind und die noch mindestens 50%, bevorzugt 65%, besonders bevorzugt 80%, insbesondere mehr als 90 % der enzymatischen Aktivität des Enzyms mit der jeweiligen Bezugssequenz Seq ID Nr. 8 oder Seq ID Nr. 11 besitzt, wobei unter enzymatischer Aktivität für ein Enzym E₂ die Fähigkeit verstanden wird, UDP-Glukose und 17-Hydroxy-Z-9-Octadecensäure zu 17-(β-D-Glucopyranosyl-Oxy)-Z-9-Octadecensäure umzusetzen,
mindestens ein Enzym E₃ mit Polypeptidsequenz Seq ID Nr. 11 oder mit einer Polypeptidsequenz bei der bis zu 60%, bevorzugt bis zu 25%, besonders bevorzugt bis zu 15% insbesondere bis zu 10, 9, 8, 7, 6, 5, 4, 3, 2, 1 % der Aminosäurereste gegenüber Seq ID Nr. 11 durch Deletion, Insertion, Substitution oder eine Kombination davon verändert sind und die noch mindestens 50%, bevorzugt 65%, besonders bevorzugt 80%, insbesondere mehr als 90 % der enzymatischen Aktivität des Enzyms mit der Bezugssequenz Seq ID Nr. 11 besitzt, wobei unter enzymatischer Aktivität für ein Enzym E₃ die Fähigkeit verstanden wird, 17-(β-D-Glucopyranosyl-Oxy)-Z-9-Octadecensäure und UDP-Glukose zu 17-L-[(2'-*O*-β-D-Glucopyranosyl-β-D-Glucopyranosyl)-Oxy]-Z-9-Octadecensäure umzusetzen,
mindestens ein Enzym E₄ mit Polypeptidsequenz Seq ID Nr. 9 oder mit einer Polypeptidsequenz bei der bis zu 50%, bevorzugt bis zu 25%, besonders bevorzugt bis zu 15% insbesondere bis zu 10, 9, 8, 7, 6, 5, 4, 3, 2, 1 % der Aminosäurereste gegenüber Seq ID Nr. 9 durch Deletion, Insertion, Substitution oder eine Kombination davon verändert sind und die noch mindestens 50%, bevorzugt 65%, besonders bevorzugt 80%, insbesondere mehr als 90 % der enzymatischen Aktivität des Enzyms mit Seq ID Nr. 9 besitzt, wobei unter enzymatischer Aktivität für ein Enzym E₄ die Fähigkeit verstanden wird, 17-L-[(2'-*O*-β-D-Glucopyranosyl-β-D-Glucopyranosyl)-Oxy]-Z-9-Octadecensäure-1',4''-Lacton und Acetyl-Coenzym A zu 17-L-[(2'-*O*-β-D-Glucopyranosyl-β-D-Glucopyranosyl)-Oxy]-Z-9-Octadecensäure-1',4"-Lacton-Monoacetat
oder 17-L-[(2'-*O*-β-D-Glucopyranosyl-β-D-Glucopyranosyl)-Oxy]-Z-9-Octadecensäure-1',4''-Lacton-Monoacetat und Acetyl-Coenzym A zu 17-L-[(2'-*O*-β-D-Glucopyranosyl-β-D-Glucopyranosyl)-Oxy]-Z-9-Octadecensäure-1',4''-Lacton-Diacetat oder 17-L-[(2'-*O*-β-D-Glucopyranosyl-β-D-Glucopyranosyl)-Oxy]-Z-9-Octadecensäure-1',4"-Lacton und Acetyl-Coenzym A zu 17-L-[(2'-*O*-β-D-Glucopyranosyl-β-D-Glucopyranosyl)-Oxy]-Z-9-Octadecensäure-1',4''-Lacton-Diacetat, wobei ersteres bevorzug ist, umzusetzen,
mindestens ein Enzym E₅ mit Polypeptidsequenz Seq ID Nr. 10 oder mit einer Polypeptidsequenz bei der bis zu 45%, bevorzugt bis zu 25%, besonders bevorzugt bis zu 15% insbesondere bis zu 10, 9, 8, 7, 6, 5, 4, 3, 2, 1 % der Aminosäurereste gegenüber Seq ID Nr. 10 durch Deletion, Insertion, Substitution oder eine Kombination davon verändert sind und die noch mindestens 50%, bevorzugt 65%, besonders bevorzugt 80%, insbesondere mehr als 90 % der enzymatischen Aktivität des Enzyms mit Seq ID Nr. 10 besitzt, wobei unter enzymatischer Aktivität für ein Enzym E₅ die Fähigkeit verstanden wird, ein Sophorolipid aus einer Zelle heraus ins umgebende Medium zu transferieren,
dadurch gekennzeichnet, dass
sie gesteigerte Aktivitäten folgender Enzymkombinationen: E₁E₂, E₁E₃, E₁E₄, E₁E₅, E₂E₃, E₂E₄, E₂E₅, E₃E₄, E₃E₅, E₄E₅, E₁E₂E₃, E₁E₂E₄, E₁E₂E₅, E₁E₃E₄, E₁E₃E₅, E₁E₄E₅, E₂E₃E₄, E₂E₄E₅, E₃E₄E₅, E₁E₂E₃E₄, E₂E₃E₄E₅, E₁E₃E₄E₅, E₁E₂E₄E₅, E₁E₂E₃E₅, E₁E₂E₃E₄ und E₁E₂E₃E₄E₅
oder dass sie eine verminderte Aktivität des Enzyms E₃ und gegebenenfalls eine gesteigerte Aktivität folgender Enzymkombinationen:
E₁E₂, E₁E₄, E₁E₅, E₂E₄, E₂E₅, E₄E₅, E₁E₂E₄, E₁E₂E₅, E₁E₄E₅ und E₁E₂E₄E₅
oder dass sie eine verminderte Aktivität des Enzyms E₄ und gegebenenfalls eine gesteigerte Aktivität folgender Enzymkombinationen:
E₁E₂, E₁E₃, E₁E₅, E₂E₃, E₂E₅, E₃E₅, E₁E₂E₃, E₁E₂E₅, E₁E₃E₅ und E₁E₂E₃E₅
oder dass sie eine verminderte Aktivität der Enzyme E₃ und E₄ und gegebenenfalls eine gesteigerte Aktivität folgender Enzymkombinationen:
E₁E₂, E₁E₅, E₂E₅, E₁E₂E₅,
aufweist.

Unter dem Begriff "Sophorolipide" im Zusammenhang mit der vorliegenden Erfindung werden Verbindungen der allgemeinen Formel (Ia) und (Ib) verstanden, 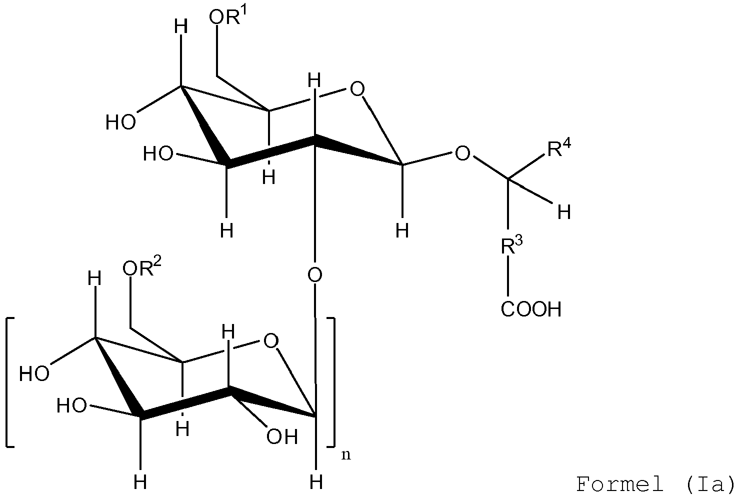 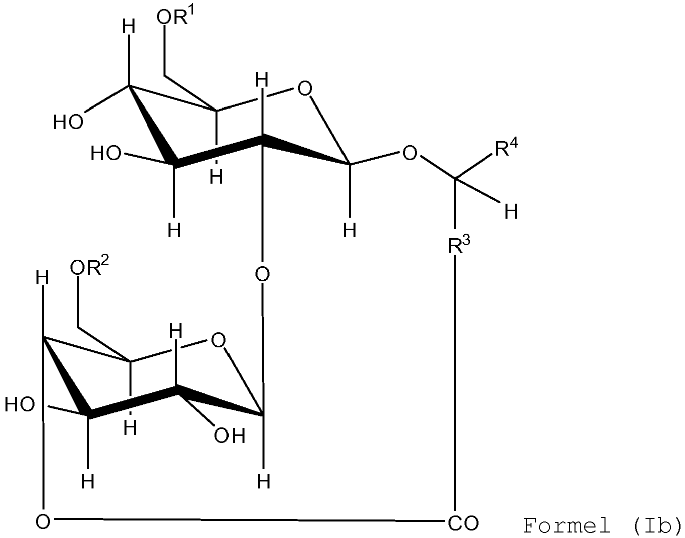 bei denen
- R¹: = H oder CO-CH₃,
- R²: = H oder CO-CH₃,
- R³: = ein unsubstituierter oder mit Hydroxyfunktionen substituierter, unverzweigter, gegebenenfalls ein bis drei Doppel- oder Dreifachbindungen enthaltender, zweibindiger organischer Rest, umfassend 6 bis 32 Kohlenstoffatome,
- R⁴: = H, CH₃ oder ein unsubstituierter oder mit Hydroxyfunktionen substituierter, unverzweigter, gegebenenfalls ein bis drei Doppel- oder Dreifachbindungen enthaltender, einbindiger organischer Rest, umfassend 2 bis 10 Kohlenstoffatome und
- n: = 1 oder 0
ist.

Unter einem "Wildtyp" einer Zelle wird im Zusammenhang mit der vorliegenden Erfindung bevorzugt der Ausgangsstamm verstanden, aus dem die erfindungsgemäße Zelle durch gentechnische Manipulation an den genetischen Elementen, die für die Aktivitäten der Enzyme der genannten Seq ID Nr. verantwortlich sind, hervorgegangen ist.

Der Begriff "geänderte Aktivität eines Enzyms" ist vorzugsweise als geänderte intrazelluläre Aktivität zu verstehen.

Änderungen von Aminosäureresten einer gegebenen Polypeptidsequenz, die zu keinen wesentlichen Änderungen der Eigenschaften und Funktion des gegebenen Polypeptides führen, sind dem Fachmann bekannt. So können beispielsweise sogenannte konservierte Aminosäuren gegeneinander ausgetauscht werden; Beispiele für solche geeigneten Aminosäuresubstitutionen sind: Ala gegen Ser; Arg gegen Lys; Asn gegen Gln oder His; Asp gegen Glu; Cys gegen Ser; Gln gegen Asn; Glu gegen Asp; Gly gegen Pro; His gegen Asn oder Gln; Ile gegen Leu oder Val; Leu gegen Met oder Val; Lys gegen Arg oder Gln oder Glu; Met gegen Leu oder Ile; Phe gegen Met oder Leu oder Tyr; Ser gegen Thr; Thr gegen Ser; Trp gegen Tyr; Tyr gegen Trp oder Phe; Val gegen Ile oder Leu. Ebenso ist bekannt, dass Änderungen besonders am N- oder C-Terminus eines Polypeptides in Form von beispielsweise Aminosäureinsertionen oder -deletionen oft keinen wesentlichen Einfluss auf die Funktion des Polypeptides ausüben.

Die Aktivität eines Enzyms E₁ kann bestimmt werden, indem Zellen, welche diese Aktivität enthalten, auf dem Fachmann bekannte Art und Weise aufgeschlossen werden, beispielsweise mit Hilfe einer Kugelmühle, einer French Press oder eines Ultraschall-Desintegrators und anschließend intakte Zellen, Zellbruchstücke und Aufschluss-Hilfsmittel, wie etwa Glaskugeln durch 10 Minuten Zentrifugation bei 13.000 rpm und 4°C abgetrennt werden. Mit dem resultierenden zellfreien Rohextrakt können dann Enzymassays mit anschließender LC-ESI-MS Detektion der Produkte durchgeführt werden. Alternativ kann das Enzym in dem Fachmann bekannter Art und Weise durch chromatographische Methoden (wie Nickel-Nitrilotriessigsäure-Affinitäts-Chromatographie, Streptavidin-Affinitäts-Chromatographie, Gelfiltrations-Chromatographie oder Ionenaustausch-Chromatographie) angereichert oder auch bis zu Homogenität gereinigt werden. Ein Standardassay kann in einem Gesamtvolumen von 200 µl 200 mM Natriumphosphat-Puffer (pH 7,4), 0,5 mM NADPH, 0,5 mM Dithiothreitol, 3 mM Glukose-6-Phosphat und 0,5 U Glukose-6-Phosphat-Dehydrogenase und 50 µl Proteinrohextrakt (ca. 1 mg Gesamtprotein) oder gereinigtem Protein in Lösung (10 µg gereinigtes Protein) durchgeführt werden. Die Reaktion wird durch die Zugabe von a) 5 µl 10 mM ethanolischer Substratlösung (Z-9-Octadecensäure) oder b) von 5 µl 10 mM Substratlösung (Z-9-Octadecensäure) in 0,1% Triton X-100, welche zuvor durch zwei Ultraschallbehandlungen von je 30 Sekunden vorbehandelt wurde, gestartet und 30 Minuten bei 30°C inkubiert. Anschließend wird die Reaktion mit 200 µl Ethylacetat extrahiert. Ungelöste Bestandteile werden sedimentiert, Phasentrennung durch kurze Zentrifugation (16.100 g, 5 Minuten) herbei geführt und die Ethylacetatphase mittels LC-ESI-MS analysiert. Die Identifizierung der Produkte erfolgt durch Analyse der entsprechenden Massenspuren und der MS²-Spektren.

Die Aktivität eines Enzyms E₂ kann bestimmt werden, indem Zellen, welche diese Aktivität enthalten, auf dem Fachmann bekannte Art und Weise aufgeschlossen werden, beispielsweise mit Hilfe einer Kugelmühle, einer French Press oder eines Ultraschall-Desintegrators und anschließend intakte Zellen, Zellbruchstücke und Aufschluss-Hilfsmittel, wie etwa Glaskugeln durch 10 Minuten Zentrifugation bei 13.000 rpm und 4°C abgetrennt werden. Mit dem resultierenden zellfreien Rohextrakt können dann Enzymassays mit anschließender LC-ESI-MS Detektion der Produkte durchgeführt werden. Alternativ kann das Enzym in dem Fachmann bekannter Art und Weise durch chromatographische Methoden (wie Nickel-Nitrilotriessigsäure-Affinitäts-Chromatographie, Streptavidin-Affinitäts-Chromatographie, Gelfiltrations-Chromatographie oder Ionenaustausch-Chromatographie) angereichert oder auch bis zu Homogenität gereinigt werden. Ein Standardassay kann aus 185 µl 10 mM Tris-HCl (pH 7,5), 10 µl 125 mM UDP-Glukose und 50 µl Proteinrohextrakt (ca. 1 mg Gesamtprotein) oder gereinigtem Protein in Lösung (10 µg gereinigtes Protein) bestehen. Die Reaktion wird durch die Zugabe von a) 5 µl 10 mM ethanolischer Substratlösung (wie etwa 18-Hydroxy-Z-9-Octadecensäure) oder b) von 5 µl 10 mM Substratlösung (wie etwa 18-Hydroxy-Z-9-Octadecensäure) in 0,1% Triton X-100, welche zuvor durch zwei Ultraschallbehandlungen von je 30 Sekunden vorbehandelt wurde, gestartet und 30 Minuten bei 30°C inkubiert. Anschließend wird die Reaktion mit 200 µl Ethylacetat extrahiert. Ungelöste Bestandteile werden sedimentiert, Phasentrennung durch kurze Zentrifugation (16.100 *g*, 5 Minuten) herbei geführt und die Ethylacetatphase mittels LC-ESI-MS analysiert. Die Identifizierung der Produkte erfolgt durch Analyse der entsprechenden Massenspuren und der MS²-Spektren. In diesem Assay wird bevorzugt 18-Hydroxy-Z-9-Octadecensäure als Substrat eingesetzt, da es kommerziell verfügbar ist und bereits an verschiedenen Stellen gezeigt wurde, dass die Enzyme der Sophorolipid-Biosynthese sowohl 18-Hydroxy-Z-9-Octadecensäure, 17-Hydroxy-Z-9-Octadecensäure als auch Hydroxyfettsäuren anderer Kettenlängen (gesättigt oder ungesättigt) und hydroxyliert am ω-oder ω-1-Kohlenstoff, wie auch die im Verlauf der Sophorolipid-Biosynthese daraus entstehenden Mono- und Diglucoside, als Substrate akzeptieren (Asmer, H.J., Lang, S., Wagner, F., Wray, V. (1988). Microbial production, structure elucidation and bioconversion of sophorose lipids. J. Am. Oil Chem. Soc. 65:1460-1466; Nunez, A., Ashby, R., Foglia, T.A. et al. (2001). Analysis and characterization of sophorolipids by liquid chromatography with atmospheric pressure chemical ionization. Chromatographia 53:673-677; Ashby, R.D., Solaiman, D.K., Foglia, T.A. (2008). Property control of sophorolipids: influence of fatty acid substrate and blending. Biotechnology Letters 30:1093-1100).

Die Aktivität eines Enzyms E₃ kann bestimmt werden, indem Zellen, welche diese Aktivität enthalten, auf dem Fachmann bekannte Art und Weise aufgeschlossen werden, beispielsweise mit Hilfe einer Kugelmühle, einer French Press oder eines Ultraschall-Desintegrators und anschließend intakte Zellen, Zellbruchstücke und Aufschluss-Hilfsmittel, wie etwa Glaskugeln durch 10 Minuten Zentrifugation bei 13.000 rpm und 4°C abgetrennt werden. Mit dem resultierenden zellfreien Rohextrakt können dann Enzymassays mit anschließender LC-ESI-MS Detektion der Produkte durchgeführt werden. Alternativ kann das Enzym in dem Fachmann bekannter Art und Weise durch chromatographische Methoden (wie Nickel-Nitrilotriessigsäure-Affinitäts-Chromatographie, Streptavidin-Affinitäts-Chromatographie, Gelfiltrations-Chromatographie oder Ionenaustausch-Chromatographie) angereichert oder auch bis zu Homogenität gereinigt werden. Ein Standardassay kann aus 185 µl 10 mM Tris-HCl (pH 7,5), 10 µl 125 mM UDP-Glukose und 50 µl Proteinrohextrakt (ca. 1 mg Gesamtprotein) oder gereinigtem Protein in Lösung (10 µg gereinigtes Protein) bestehen. Die Reaktion wird durch die Zugabe von a) 5 µl 10 mM ethanolischer Substratlösung (wie etwa 18-(β-D-Glucopyranosyl-Oxy)-Z-9-Octadecensäure) oder b) von 5 µl 10 mM Substratlösung (18-(β-D-Glucopyranosyl-Oxy)-Z-9-Octadecensäure) in 0,1% Triton X-100, welche zuvor durch zwei Ultraschallbehandlungen von je 30 Sekunden vorbehandelt wurde, oder c) durch Zugabe des für die Aktivitätsbestimmung des Enzyms E₂ beschriebenen Reaktionsansatzes gestartet und 30 Minuten bei 30°C inkubiert. Anschließend wird die Reaktion mit 200 µl (Substrat zugegeben, wie in a) und b) beschrieben) oder 400 µl (Substrat zugegeben, wie in c) beschrieben) Ethylacetat extrahiert. Ungelöste Bestandteile werden sedimentiert, Phasentrennung durch kurze Zentrifugation (16.100 g, 5 Minuten) herbei geführt und die Ethylacetatphase mittels LC-ESI-MS analysiert. Die Identifizierung der Produkte erfolgt durch Analyse der entsprechenden Massenspuren und der MS²-Spektren. In diesem Assay wird bevorzugt 18-(β-D-Glucopyranosyl-Oxy)-Z-9-Octadecensäure als Substrat eingesetzt, da dessen Vorläufermolekül 18-Hydroxy-Z-9-Octadecensäure kommerziell verfügbar ist und bereits an verschiedenen Stellen gezeigt wurde, dass die Enzyme der Sophorolipid-Biosynthese sowohl 18-Hydroxy-Z-9-Octadecensäure, 17-Hydroxy-Z-9-Octadecensäure als auch Hydroxyfettsäuren anderer Kettenlängen (gesättigt oder ungesättigt) und hydroxyliert am ω-oder ω-1-Kohlenstoff, wie auch die im Verlauf der Sophorolipid-Biosynthese daraus entstehenden Mono- und Diglucoside, als Substrate akzeptieren.

Die Aktivität eines Enzyms E₄ kann bestimmt werden, indem Zellen, welche diese Aktivität enthalten, auf dem Fachmann bekannte Art und Weise aufgeschlossen werden, beispielsweise mit Hilfe einer Kugelmühle, einer French Press oder eines Ultraschall-Desintegrators und anschließend intakte Zellen, Zellbruchstücke und Aufschluss-Hilfsmittel, wie etwa Glaskugeln durch 10 Minuten Zentrifugation bei 13.000 rpm und 4°C abgetrennt werden. Mit dem resultierenden zellfreien Rohextrakt können dann Enzymassays mit anschließender LC-ESI-MS Detektion der Produkte durchgeführt werden. Alternativ kann das Enzym in dem Fachmann bekannter Art und Weise durch chromatographische Methoden (wie Nickel-Nitrilotriessigsäure-Affinitäts-Chromatographie, Streptavidin-Affinitäts-Chromatographie, Gelfiltrations-Chromatographie oder Ionenaustausch-Chromatographie) angereichert oder auch bis zu Homogenität gereinigt werden. Ein Standardassay kann aus 185 µl 10 mM Tris-HCl (pH 7,5), 2,5 µl 100 mM Acetyl-Coenzym A und 50 µl Proteinrohextrakt (ca. 1 mg Gesamtprotein) oder gereinigtem Protein in Lösung (10 µg gereinigtes Protein) bestehen. Die Reaktion wird durch die Zugabe von a) 5 µl 10 mM ethanolischer Substratlösung (chemisch deacetylierte Sophorolipide) oder b) von 5 µl 10 mM Substratlösung (chemisch deacetylierte Sophorolipide) in 0,1% Triton X-100, welche zuvor durch zwei Ultraschallbehandlungen von je 30 Sekunden vorbehandelt wurde, oder c) durch Zugabe des für die Aktivitätsbestimmung des Enzyms E₃ beschriebenen Reaktionsansatzes (nach der dort unter c) beschriebenen Art der Substratzugabe, gefolgt von Inkubation von 30 Minuten bei 30°C), gestartet und 30 Minuten bei 30°C inkubiert. Anschließend wird die Reaktion mit 200 µl (Substrat zugegeben, wie in a) und b) beschrieben) oder 600 µl (Substrat zugegeben, wie in c) beschrieben) Ethylacetat extrahiert. Ungelöste Bestandteile werden sedimentiert, Phasentrennung durch kurze Zentrifugation (16.100 g, 5 Minuten) herbei geführt und die Ethylacetatphase mittels LC-ESI-MS analysiert. Die Identifizierung der Produkte erfolgt durch Analyse der entsprechenden Massenspuren und der MS²-Spektren. Es ist erfindungsgemäß bevorzugt, dass das Enzym E₄ nicht nur die Lacton-Formen der Sophorolipide, wie hier für die Referenzaktivitäten gewählt, als Substrate akzeptiert, sondern ebenfalls in der Lage ist, die Säureform der Sophorolipide, wie generell in Formel (Ia) mit R¹ und R² = H dargestellt, an entsprechenden Stellen mindestens einfach zu acetylieren.

Die geänderte Aktivität eines Enzyms E₅ im Vergleich zu ihrem Wildtyp kann am einfachsten stellvertretend über die absolute Menge an Enzyme E₅ pro Zelle bestimmt werden, da man davon ausgehen kann, dass ein verstärktes Vorliegen zu einer Aktivitätserhöhung und ein verringertes Vorliegen eine Aktivitätserniedrigung bezogen auf die Zelle bewirkt und diese Zusammenhänge direkt voneinander abhängig sind. Das veränderte Vorliegen des Enzyms E₅ im Vergleich zu dem Wildtyp kann mit herkömmlichen Methoden bestimmt werden. Die Proteinkonzentration kann somit durch Western-Blot-Hybridisierung mit einem für das nachzuweisende Protein spezifischen Antikörper (Sambrook et al., Molecular Cloning: a laboratory manual, 2nd Ed. Cold Spring Harbor Laboratory Press, Cold Spring Harbor, N.Y. USA, 1989) und anschließende optische Auswertung mit entsprechender Software zur Konzentrationsbestimmung (Lohaus und Meyer (1989) Biospektrum, 5: 32-39; Lottspeich (1999), Angewandte Chemie 111: 2630-2647) analysiert werden.

Erfindungsgemäß bevorzugte Zellen sind Mikroorganismen, vorzugsweise Bakterien-, Hefe- oder Pilzzellen, wobei insbesondere Schlauchpilze der Gattungen *Candida und Wickerhamiella,* insbesondere *Candida bombicola, Candida bogoriensis, Candida batistae, Candida apicola* und *Wickerhamiella domericqiae* besonders bevorzugt sind. Insbesondere die Stämme *Candida bombicola* ATCC 22214, *Candida bogoriensis* NRRL Y-5980, *Candida batistae* CBS 8550, *Candida apicola* IMET 42747 und *Wickerhamiella domericqiae* sind besonders geeignete Zellen.

Da die Sophorolipide von erfindungsgemäßer Zelle aus Glukose und Fettsäuren gebildet werden, ist es vorteilhaft, wenn erfindungsgemäße Zellen in ihrer β-Oxidation zumindest teilweise blockiert sind, da dies den Abfluss von Substrat verhindert und somit höhere Produktkonzentrationen und Kohlennstoffausbeuten ermöglicht werden. In der β-Oxidation blockierte *Candida* Zellen werden beispielsweise in der WO03/100013 beschrieben, in der β-Oxidation blockierte *Candida bombicola* Zellen in Van Bogaert et al FEMS Yeast Res. 2009 Jun;9(4):610-7.

Zur Herstellung von Sophorolipiden der allgemeinen Formel (Ia) mit n = 0 sollte möglichst wenig enzymatische Aktivität eines Enzyms E₃ in der Zelle vorliegen. Somit ist in einer bestimmten Ausführungsform der erfindungsgemäßen Zelle die geänderte Aktivität eines Enzyms E₃ eine verminderte.
In diesem Zusammenhang werden erfindungsgemäß Zellen bevorzugt, die eine verminderte Aktivität eines Enzyms E₃ aufweisen und
eine gesteigerte Aktivität folgender Enzymkombinationen:
E₁E₂, E₁E₄, E₁E₅, E₂E₄, E₂E₅, E₄E₅, E₁E₂E₄, E₁E₂E₅, E₁E₄E₅ und E₁E₂E₄E₅
   insbesondere bevorzugt
   E₁E₂, E₁E₄, E₁E₅, E₂E₄, E₂E₅, E₄E₅ und E₁E₂E₄E₅
   aufweisen.
In diesem Zusammenhang handelt es sich bei der erfindungsgemäßen Zelle bevorzugt um eine *Candida bombicola-, Candida bogoriensis-, Candida batistae-, Candida apicola-* oder *Wickerhamiella domericqiae-Zelle.*
Weiterhin bevorzugt sind hier erfindungsgemäße Zellen, bei denen die Verminderung der enzymatischen Aktivität erreicht wird durch Modifikation eines Genes umfassend eine Nukleinsäuresequenz ausgewählt aus der Gruppe bestehend aus Seq ID Nr. 6 und einer Sequenz, die zu mindestens 80 %, besonders bevorzugt zu mindestens 90 %, darüber hinaus bevorzugt zu mindestens 95 % und am meisten bevorzugt zu mindestens 99 % identisch zu der Bezugssequenz Seq ID Nr. 6 ist,
wobei die Modifikation ausgewählt ist aus der Gruppe umfassend, bevorzugt bestehend aus,
Insertion von Fremd-DNA in das Gen, Deletion mindestens von Teilen des Gens, Punktmutationen in der Gensequenz, RNA-Interferenz (siRNA), *antisense*-RNA oder Modifikation (Insertion, Deletion oder Punktmutationen) von regulatorischen Sequenzen, welche das Gen flankieren.
Eine zur Herstellung solcher Zellen geeignete Nukleinsäure ist beispielsweise eine mit Seq ID Nr 16. Zur Herstellung von Sophorolipiden der allgemeinen Formel (Ia) oder (Ib) mit R¹ und R² gleich H sollte möglichst wenig enzymatische Aktivität eines Enzyms E₄ in der Zelle vorliegen. Somit ist in einer bestimmten Ausführungsform der erfindungsgemäßen Zelle die geänderte Aktivität eines Enzymes E₄ eine verminderte.
In diesem Zusammenhang werden erfindungsgemäß Zellen bevorzugt, die eine verminderte Aktivität mindestens eines Enzyms E₄ aufweisen und eine gesteigerte Aktivität folgender Enzymkombinationen:
E₁E₂, E₁E₃, E₁E₅, E₂E₃, E₂E₅, E₃E₅, E₁E₂E₃, E₁E₂E₅, E₁E₃E₅ und E₁E₂E₃E₅ insbesondere bevorzugt
   E₁E₂, E₁E₃, E₁E₅, E₂E₃, E₂E₅, E₃E₅ und E₁E₂E₃E₅ aufweisen.
In diesem Zusammenhang handelt es sich bei der erfindungsgemäßen Zelle bevorzugt um eine *Candida bombicola-, Candida bogoriensis-, Candida batistae-, Candida apicola-* oder *Wickerhamiella domericqiae*-Zelle.
Weiterhin bevorzugt sind hier erfindungsgemäße Zellen, bei denen die Verminderung der enzymatischen Aktivität erreicht wird durch Modifikation eines Genes umfassend eine Nukleinsäuresequenz ausgewählt aus der Gruppe bestehend aus Seq ID Nr. 4 und einer Sequenz, die zu mindestens 80 %, besonders bevorzugt zu mindestens 90 %, darüber hinaus bevorzugt zu mindestens 95 % und am meisten bevorzugt zu mindestens 99 % identisch zu Seq ID Nr. 4 ist,
wobei die Modifikation ausgewählt ist aus der Gruppe umfassend, bevorzugt bestehend aus,
Insertion von Fremd-DNA in das Gen, Deletion mindestens von Teilen des Gens, Punktmutationen in der Gensequenz, RNA-Interferenz (siRNA), *antisense*-RNA oder Modifikation (Insertion, Deletion oder Punktmutationen) von regulatorischen Sequenzen, welche das Gen flankieren.
Eine zur Herstellung solcher Zellen geeignete Nukleinsäure ist beispielsweise eine mit Seq ID Nr. 14.

Zur Herstellung von Sophorolipiden der allgemeinen Formel (Ia) mit n = 0 und R¹ gleich H sollte möglichst wenig enzymatische Aktivität der Enzyme E₃ und E₄ in der Zelle vorliegen. Somit ist in einer bestimmten Ausführungsform der erfindungsgemäßen Zelle die geänderte Aktivität der Enzyme E₃ und E₄ eine verminderte.
In diesem Zusammenhang werden erfindungsgemäß Zellen bevorzugt, die eine verminderte Aktivität jeweils mindestens eines Enzyms E₃ und E₄ aufweisen und gleichzeitig eine gesteigerte Aktivität mindestens einer der Enzyme E₁, E₂ und E₅ aufweisen und insbesondere neben der verminderten Aktivität jeweils mindestens eines Enzyms E₃ und E₄ eine gesteigerte Aktivität folgender Enzymkombinationen:
E₁E₂, E₁E₅, E₂E₅, E₁E₂E₅,
   insbesondere bevorzugt
   E₁E₂, E₁E₅ und E₂E₅
   aufweisen.
In diesem Zusammenhang handelt es sich bei der erfindungsgemäßen Zelle bevorzugt um eine *Candida bombicola-, Candida bogoriensis-*, *Candida batistae-, Candida apicola-* oder *Wickerhamiella domericqiae*-Zelle.
Weiterhin bevorzugt sind hier erfindungsgemäße Zellen, bei denen die Verminderung der enzymatischen Aktivität erreicht wird durch Modifikation eines Genes umfassend eine Nukleinsäuresequenz ausgewählt aus der Gruppe bestehend aus Seq ID Nr. 4 und einer Sequenz, die zu mindestens 80 %, besonders bevorzugt zu mindestens 90 %, darüber hinaus bevorzugt zu mindestens 95 % und am meisten bevorzugt zu mindestens 99 % identisch zu Seq ID Nr. 4 ist
und
eines Genes umfassend eine Nukleinsäuresequenz ausgewählt aus der Gruppe bestehend aus Seq ID Nr. 6 und einer Sequenz, die zu mindestens 80 %, besonders bevorzugt zu mindestens 90 %, darüber hinaus bevorzugt zu mindestens 95 % und am meisten bevorzugt zu mindestens 99 % identisch zu der Bezugssequenz Seq ID Nr. 6 ist, wobei die Modifikation ausgewählt ist aus der Gruppe umfassend, bevorzugt bestehend aus,
Insertion von Fremd-DNA in das Gen, Deletion mindestens von Teilen des Gens, Punktmutationen in der Gensequenz, RNA-Interferenz (siRNA), *antisense*-RNA oder Modifikation (Insertion, Deletion oder Punktmutationen) von regulatorischen Sequenzen, welche die Gene flankieren.
Zur Herstellung solcher Zellen geeignete Nukleinsäuren sind beispielsweise solche mit Seq ID Nr. 14 und 16.

Die nun folgenden Ausführungen zur Steigerung der Enzymaktivität in Zellen gelten sowohl für die Steigerung der Aktivität des Enzyme E₁ bis E₅ als auch für alle nachfolgend genannten Enzyme, deren Aktivität gegebenenfalls erhöht werden kann. Grundsätzlich lässt sich eine Steigerung der enzymatischen Aktivität dadurch erzielen, dass man die Kopienzahl der Gensequenz bzw. der Gensequenzen erhöht, welche für das Enzym kodieren, einen starken Promotor verwendet, die Kodonnutzung des Gens verändert, auf verschiedene Art und Weise die Halbwertszeit der mRNA oder des Enzyms erhöht, die Regulation der Expression des Gens modifiziert oder ein Gen oder Allel nutzt, das für ein entsprechendes Enzym mit einer gesteigerten Aktivität kodiert und gegebenenfalls diese Maßnahmen kombiniert. Erfindungsgemäß gentechnisch veränderte Zellen werden beispielsweise durch Transformation, Transduktion, Konjugation oder eine Kombination dieser Methoden mit einem Vektor erzeugt, der das gewünschte Gen, ein Allel dieses Gens oder Teile davon und einen die Expression des Gens ermöglichenden Promotor enthält. Die heterologe Expression wird insbesondere durch Integration des Gens oder der Allele in das Chromosom der Zelle oder einen extrachromosomal replizierenden Vektor erzielt.
Einen Überblick über die Möglichkeiten zur Erhöhung der Enzym-Aktivität in Zellen am Beispiel der Isocitratlyase gibt EP0839211, die hiermit als Referenz eingeführt wird und deren Offenbarungsgehalt hinsichtlich der Möglichkeiten zur Erhöhung der Enzym-Aktivität in Zellen einen Teil der Offenbarung der vorliegenden Erfindung bildet.

Die Expression der vorstehend und aller nachfolgend genannten Enzyme bzw. Gene ist mit Hilfe von 1- und 2-dimensionaler Proteingelauftrennung und anschließender optischer Identifizierung der Proteinkonzentration mit entsprechender Auswertesoftware im Gel nachweisbar. Wenn die Erhöhung einer Enzymaktivität ausschließlich auf einer Erhöhung der Expression des entsprechenden Gens basiert, so kann die Quantifizierung der Erhöhung der Enzymaktivität in einfacher Weise durch einen Vergleich der 1- oder 2-dimensionalen Proteinauftrennungen zwischen Wildtyp und gentechnisch veränderter Zelle bestimmt werden. Eine gebräuchliche Methode zur Präparation der Proteingele bei coryneformen Bakterien und zur Identifizierung der Proteine ist die von Hermann et al. (Electrophoresis, 22: 1712.23 (2001) beschriebene Vorgehensweise. Die Proteinkonzentration kann ebenfalls durch Western-Blot-Hybridisierung mit einem für das nachzuweisende Protein spezifischen Antikörper (Sambrook et al., Molecular Cloning: a laboratory manual, 2nd Ed. Cold Spring Harbor Laboratory Press, Cold Spring Harbor, N.Y. USA, 1989) und anschließende optische Auswertung mit entsprechender Software zur Konzentrationsbestimmung (Lohaus und Meyer (1989) Biospektrum, 5: 32-39; Lottspeich (1999), Angewandte Chemie 111: 2630-2647) analysiert werden. Die Aktivität von DNA-bindenden Proteinen kann mittels DNA-Band-Shift-Assays (auch als Gelretardation bezeichnet) gemessen werden (Wilson et al. (2001) Journal of Bacteriology, 183: 2151-2155). Die Wirkung von DNA-bindenden Proteinen auf die Expression anderer Gene kann durch verschiedene gut beschriebene Methoden des Reportergen-Assays nachgewiesen werden (Sambrook et al., Molecular Cloning: a laboratory manual, 2nd Ed. Cold Spring Harbor Laboratory Press, Cold Spring Harbor, N.Y. USA, 1989). Die intrazellulären (spezifisch) enzymatischen Aktivitäten können nach verschiedenen beschriebenen Methoden (Donahue et al. (2000) Journal of Bacteriology 182 (19): 5624-5627; Ray et al. (2000) Journal of Bacteriology 182 (8): 2277-2284; Freedberg et al. (1973) Journal of Bacteriology 115 (3): 816-823) bestimmt werden. Sofern in den nachfolgenden Ausführungen keine konkreten Methoden zur Bestimmung der Aktivität eines bestimmten Enzyms angegeben werden, erfolgt die Bestimmung der Steigerung der Enzymaktivität und auch die Bestimmung der Verminderung einer Enzymaktivität vorzugsweise mittels der in Hermann et al., Electrophoresis, 22: 1712-23 (2001), Lohaus et al., Biospektrum 5 32-39 (1998), Lottspeich, Angewandte Chemie 111: 2630-2647 (1999) und Wilson et al., Journal of Bacteriology 183: 2151-2155 (2001) beschriebenen Methoden.

Wird die Erhöhung der Enzymaktivität durch Mutation des endogenen Gens bewerkstelligt, so können derartige Mutationen entweder nach klassischen Methoden ungerichtet erzeugt werden, wie etwa durch UV-Bestrahlung oder durch mutationsauslösende Chemikalien, oder gezielt mittels gentechnologischer Methoden wie Deletion(en), Insertion(en) und/oder Nukleotidaustausch(e). Durch diese Mutationen werden veränderte Zellen erhalten. Besonders bevorzugte Mutanten von Enzymen sind insbesondere auch solche Enzyme, die nicht mehr oder zumindest im Vergleich zum Wildtyp-Enzym vermindert feedback-inhibierbar sind.
Wird die Erhöhung der Enzymaktivität durch Erhöhung der Synthese eines Enzyms bewerkstelligt, so erhöht man beispielsweise die Kopienzahl der entsprechenden Gene oder mutiert die Promotorund Regulationsregion oder die Ribosomenbindungsstelle, die sich stromaufwärts des Strukturgens befindet. In gleicher Weise wirken Expressionskassetten, die stromaufwärts des Strukturgens eingebaut werden. Durch induzierbare Promotoren ist es zusätzlich möglich, die Expression zu jedem beliebigen Zeitpunkt zu steigern. Des Weiteren können dem Enzym-Gen als regulatorische Sequenzen aber auch sogenannte "Enhancer" zugeordnet sein, die über eine verbesserte Wechselwirkung zwischen RNA-Polymerase und DNA ebenfalls eine erhöhte Genexpression bewirken. Durch Maßnahmen zur Verlängerung der Lebensdauer der mRNA wird ebenfalls die Expression verbessert. Weiterhin wird durch Verhinderung des Abbaus des Enzyms ebenfalls die Enzymaktivität verstärkt. Die Gene oder Genkonstrukte liegen dabei entweder in Plasmiden mit unterschiedlicher Kopienzahl vor oder sind im Chromosom integriert und amplifiziert. Alternativ kann weiterhin eine Überexpression der betreffenden Gene durch Veränderung der Medienzusammensetzung und Kulturführung erreicht werden. Anleitungen hierzu findet der Fachmann unter anderem bei Martin et al. (Bio/Technology 5, 137-146 (1987)), bei Guerrero et al. (Gene 138, 35-41 (1994)), Tsuchiya und Morinaga (Bio/Technology 6, 428-430 (1988)), bei Eikmanns et al. (Gene 102, 93-98 (1991)), in EP-A-0 472 869, im US 4,601,893, bei Schwarzer und Pühler (Bio/Technology 9, 84-87 (1991), bei Reinscheid et al. (Applied and Environmental Microbiology 60, 126-132 (1994)), bei LaBarre et al. (Journal of Bacteriology 175, 1001-1007 (1993)), in WO-A-96/15246, bei Malumbres et al. (Gene 134, 15-24 (1993), in JP-A-10-229891, bei Jensen und Hammer (Biotechnology and Bioengineering 58, 191-195 (1998)) und in bekannten Lehrbüchern der Genetik und Molekularbiologie. Die vorstehend beschriebenen Maßnahmen führen ebenso wie die Mutationen zu gentechnisch veränderten Zellen.

Zur Erhöhung der Expression der jeweiligen Gene werden zum Beispiel episomale Plasmide eingesetzt. Als Plasmide bzw. Vektoren kommen im Prinzip alle dem Fachmann für diesen Zweck zur Verfügung stehenden Ausführungsformen in Frage. Derartige Plasmide und Vektoren können z. B. den Broschüren der Firmen Novagen, Promega, New England Biolabs, Clontech oder Gibco BRL entnommen werden. Weitere bevorzugte Plasmide und Vektoren können gefunden werden in: Glover, D. M. (1985), DNA cloning: a practical approach, Vol. I-III, IRL Press Ltd., Oxford; Rodriguez, R.L. und Denhardt, D. T (eds) (1988), Vectors : a survey of molecular cloning vectors and their uses, 179-204, Butterworth, Stoneham; Goeddel, D. V. (1990), Systems for heterologous gene expression, Methods Enzymol. 185, 3-7; Sambrook, J.; Fritsch, E. F. und Maniatis, T. (1989), Molecular cloning: a laboratory manual, 2nd ed., Cold Spring Harbor Laboratory Press, New York.

Die Vektoren, wie Expressionsvektoren, Gendeletions-, Geninsertions- oder Genüberexpressionskassetten, welche das zu amplifizierende Gen oder Teile des zu inaktivierenden Gens enthalten, werden anschließend durch Transformation in den gewünschten Stamm überführt. Methoden zur Transformation, insbesondere Elektroporation, Lithiumacetat-vermittelte Transformation, Freeze-Thaw-Transformation sind beispielsweise bei Gietz, R.D., Schiestl, R.H. (2007). Frozen competent yeast cells that can be transformed with high efficiency using the LiAc/SS carrier DNA/PEG method. Nat Protoc. 2:1-4; Suga, M., Hatakeyama, T. (2003). High-efficiency electroporation by freezing intact yeast cells with addition of calcium. Curr Genet. 43:206-211; Hubberstey, A.V., Wildeman, A.G. (1991). Transformation of Saccharomyces cerevisiae by use of frozen spheroplasts. Trends Genet. 7:41; Bröker, M. (1993). Rapid transformation of cryopreserved competent Schizosaccharomyces pombe cells. Biotechniques. 15:598 - 600; Gietz, R.D., Schiestl, R.H. (1989). High efficiency transformation of intact yeast cells using single stranded nucleic acids as a carrier. Curr Genet. 16:339-346 und in "Nonconventional yeast in biotechnology" (Hrsg. Klaus Wolf, Springer-Verlag Berlin, 1996) beschrieben. Nach Transformation integrieren die Vektoren, insbesondere Gendeletions-, Geninsertions- oder Genüberexpressionskassetten durch homologe oder heterologe, bevorzugt durch homologe Rekombination, mittels eines "crossover"-Ereignisses in die Chromosomen des gewünschten Stammes. Alternativ replizieren die Vektoren, insbesondere Expressionsvektoren, auch episomal, also als unabhängige Replikationseinheit, in Zellen des gewünschten Stammes. In allen Fällen ist so sicher gestellt, das die Vektoren, wie Expressionsvektoren, Gendeletions-, Geninsertions- oder Genüberexpressionskassetten, bei Zellteilung auch an die Tochterzellen weitergegeben werden.
Unter der vorstehend und in den nachfolgenden Ausführungen verwendeten Formulierung "eine im Vergleich zu ihrem Wildtyp gesteigerte Aktivität eines Enzyms Eₓ" ist vorzugsweise stets eine um einen Faktor von mindestens 1,5, besonders bevorzugt von mindestens 10, darüber hinaus bevorzugt von mindestens 100, darüber hinaus noch mehr bevorzugt von mindestens 1.000 und am meisten bevorzugt von mindestens 10.000 gesteigerte Aktivität des jeweiligen Enzyms Eₓ zu verstehen. Weiterhin umfasst die erfindungsgemäße Zelle, welche "eine gegenüber ihrem Wildtyp gesteigerte Aktivität eines Enzyms Eₓ" aufweist, insbesondere auch eine Zelle, deren Wildtyp keine oder zumindest keine nachweisbare Aktivität dieses Enzyms Eₓ aufweist und die erst nach Erhöhung der Enzymaktivität, beispielsweise durch Überexpression, eine nachweisbare Aktivität dieses Enzyms Eₓ zeigt. In diesem Zusammenhang umfasst der Begriff "Überexpression" oder die in den nachfolgenden Ausführungen verwendete Formulierung "Erhöhung der Expression" auch den Fall, dass eine Ausgangszelle, beispielsweise eine Wildtyp-Zelle, keine oder zumindest keine nachweisbare Expression aufweist und erst durch rekombinante Verfahren eine nachweisbare Synthese des Enzyms Eₓ induziert wird.

Unter der verwendeten Formulierung "verminderte Aktivität eines Enzyms Eₓ" wird dementsprechend vorzugsweise eine um einen Faktor von mindestens 0,5, besonders bevorzugt von mindestens 0,1, darüber hinaus bevorzugt von mindestens 0,01, darüber hinaus noch mehr bevorzugt von mindestens 0,001 und am meisten bevorzugt von mindestens 0,0001 verminderte Aktivität verstanden. Die Formulierung "verminderte Aktivität" beinhaltet auch keine detektierbare Aktivität ("Aktivität von null"). Die Verminderung der Aktivität eines bestimmten Enzyms kann beispielsweise durch gezielte Mutation oder durch andere, dem Fachmann bekannte Maßnahmen zur Verminderung der Aktivität eines bestimmten Enzyms erfolgen.
Verfahren zur Verminderung von enzymatischen Aktivitäten in Mikroorganismen sind dem Fachmann bekannt.
Insbesondere molekularbiologische Techniken bieten sich hier an. Anleitung zur Modifikation und Verminderung von Proteinexpressionen und damit einhergehender Enzymaktivitätsverringerung speziell für *Candida,* insbesondere zum Unterbrechen spezieller Gene findet der Fachmann in der WO91/006660 und WO03/100013.
Erfindungsgemäß bevorzugte Zellen sind dadurch gekennzeichnet, dass die Verminderung der enzymatischen Aktivität erreicht wird durch Modifikation eines Genes umfassend eine der genannten Nukleinsäuresequenzen, wobei die Modifikation ausgewählt ist aus der Gruppe umfassend, bevorzugt bestehend aus, Insertion von Fremd-DNA in das Gen, Deletion mindestens von Teilen des Gens, Punktmutationen in der Gensequenz, RNA-Interferenz (siRNA), *antisense*-RNA oder Modifikation (Insertion, Deletion oder Punktmutationen) von regulatorischen Sequenzen, welche das Gen flankieren.
Unter Fremd-DNA ist in diesem Zusammenhang jegliche DNA-Sequenz zu verstehe, die dem Gen (und nicht dem Organismus) "fremd" ist, d.h. auch *Candida bombicola* endogene DNA-Sequenzen können in diesem Zusammenhang als "Fremd-DNA" fungieren.
In diesem Zusammenhang ist es insbesondere bevorzugt, dass das Gen durch Insertion eines Selektionsmarkergens unterbrochen wird, somit die Fremd-DNA ein Selektionsmarkergen ist, wobei bevorzugt die Insertion durch homologe Rekombination in den Genlocus erfolgte.

Erfindungsgemäß bevorzugte Zellen sind dadurch gekennzeichnet, dass sie mit mindestens einer der im Folgenden beschriebenen, erfindungsgemäßen Nukleinsäure bzw. einem im Folgenden beschriebenen, erfindungsgemäßen Vektor transformiert wurden.

Erfindungsgemäße Zellen lassen sich vorteilhaft zur Herstellung von Sophorolipiden verwenden.
Somit ist ein weiterer Gegenstand der Erfindung die Verwendung erfindungsgemäßer Zellen zur Herstellung von Verbindungen der allgemeinen Formel (Ia) und (Ib) bei denen
- R¹: = H oder CO-CH₃,
- R²: = H oder CO-CH₃,
- R³: = ein unsubstituierter oder mit Hydroxyfunktionen substituierter, unverzweigter, gegebenenfalls ein bis drei Doppel- oder Dreifachbindungen enthaltender, zweibindiger organischer Rest, umfassend 6 bis 32, bevorzugt 7 bis 19 Kohlenstoffatome,
- R⁴: = H, CH₃ oder ein unsubstituierter oder mit Hydroxyfunktionen substituierter, unverzweigter, gegebenenfalls ein bis drei Doppel- oder Dreifachbindungen enthaltender, einbindiger organischer Rest, umfassend 2 bis 10 Kohlenstoffatome und
- n: = 0 oder 1
insbesondere von solchen Verbindungen der allgemeinen Formel (Ia) und (Ib)
bei denen
- R¹: = H oder CO-CH₃,
- R²: = H oder CO-CH₃,
- R³: = ein unsubstituierter oder mit Hydroxyfunktionen substituierter, unverzweigter, gegebenenfalls ein bis drei Doppel- oder Dreifachbindungen enthaltender, zweibindiger organischer Rest, umfassend 6 bis 32, bevorzugt 7 bis 19 Kohlenstoffatome,
- R⁴: = H, CH₃ oder C₉H₁₉ und
- n: = 0 oder 1
und ganz besonders bevorzugt Verbindungen der allgemeinen Formel (Ia) und (Ib)
bei denen
- R¹: = H oder CO-CH₃,
- R²: = H oder CO-CH₃,
- R³: = ein unsubstituierter oder mit Hydroxyfunktionen substituierter, unverzweigter, gegebenenfalls ein bis drei Doppel- oder Dreifachbindungen enthaltender, zweibindiger organischer Rest, umfassend 6 bis 32, bevorzugt 7 bis 19 Kohlenstoffatome, insbesondere C₈H₁₅=C₇H₁₄
- R⁴: = H, CH₃ oder C₉H₁₉, insbesondere H oder CH₃ und
- n: = 1.

Ein weiterer Gegenstand der vorliegenden Erfindung ist ein Verfahren zur Herstellung von Sophorolipiden,
bevorzugt von Verbindungen der allgemeinen Formel (Ia) und (Ib) bei denen
- R¹: = H oder CO-CH₃,
- R²: = H oder CO-CH₃,
- R³: = ein unsubstituierter oder mit Hydroxyfunktionen substituierter, unverzweigter, gegebenenfalls ein bis drei Doppel- oder Dreifachbindungen enthaltender, zweibindiger organischer Rest, umfassend 6 bis 32, bevorzugt 7 bis 19 Kohlenstoffatome,
- R⁴: = H, CH₃ oder ein unsubstituierter oder mit Hydroxyfunktionen substituierter, unverzweigter, gegebenenfalls ein bis drei Doppel- oder Dreifachbindungen enthaltender, einbindiger organischer Rest, umfassend 2 bis 10 Kohlenstoffatome und
- n: = 0 oder 1
insbesondere von solchen Verbindungen der allgemeinen Formel (Ia) und (Ib)
bei denen
- R¹: = H oder CO-CH₃,
- R²: = H oder CO-CH₃,
- R³: = ein unsubstituierter oder mit Hydroxyfunktionen substituierter, unverzweigter, gegebenenfalls ein bis drei Doppel- oder Dreifachbindungen enthaltender, zweibindiger organischer Rest, umfassend 6 bis 32, bevorzugt 7 bis 19 Kohlenstoffatome,
- R⁴: = H, CH₃ oder C₉H₁₉ und
- n: = 0 oder 1
und ganz besonders bevorzugt Verbindungen der allgemeinen Formel (Ia) und (Ib)
bei denen
- R¹: = H oder CO-CH₃,
- R²: = H oder CO-CH₃,
- R³: = ein unsubstituierter oder mit Hydroxyfunktionen substituierter, unverzweigter, gegebenenfalls ein bis drei Doppel- oder Dreifachbindungen enthaltender, zweibindiger organischer Rest, umfassend 6 bis 32, bevorzugt 7 bis 19 Kohlenstoffatome, insbesondere C₈H₁₅=C₇H₁₄,
- R⁴: = H, CH₃ oder C₉H₁₉, insbesondere H oder CH₃ und
- n: = 1
umfassend die Verfahrensschritte
I) in Kontakt Bringen der erfindungsgemäßen Zelle mit einem Medium beinhaltend eine Kohlenstoffquelle
II) Kultivieren der Zelle unter Bedingungen, die es der Zelle ermöglichen aus der Kohlenstoffquelle Sophorolipid zu bilden und
III) gegebenenfalls Isolierung der gebildeten Sophorolipide.

Die erfindungsgemäßen, gentechnisch veränderten Zellen können kontinuierlich oder diskontinuierlich im batch-Verfahren (Satzkultivierung) oder im fed-batch-Verfahren (Zulaufverfahren) oder repeated-fed-batch-Verfahren (repetitives Zulaufverfahren) zum Zwecke der Produktion der vorstehend genannten Produkte mit dem Nährmedium in Kontakt gebracht und somit kultiviert werden. Denkbar ist auch ein semi-kontinuierliches Verfahren, wie es in der GB-A-1009370 beschrieben wird. Eine Zusammenfassung über bekannte Kultivierungsmethoden sind im Lehrbuch von Chmiel ("Bioprozesstechnik 1. Einführung in die Bioverfahrenstechnik" (Gustav Fischer Verlag, Stuttgart, 1991)) oder im Lehrbuch von Storhas ("Bioreaktoren und periphere Einrichtungen", Vieweg Verlag, Braunschweig/Wiesbaden, 1994) beschrieben. Das zu verwendende Kulturmedium muss in geeigneter Weise den Ansprüchen der jeweiligen Stämme genügen. Beschreibungen von Kulturmedien verschiedener Hefestämme sind beispielsweise in "Nonconventional yeast in biotechnology" (Hrsg. Klaus Wolf, Springer-Verlag Berlin, 1996) enthalten.
Als Kohlenstoffquelle können Kohlehydrate wie z. B. Glukose, Saccharose, Arabinose, Xylose, Laktose, Fructose, Maltose, Melasse, Stärke, Cellulose und Hemicellulose, pflanzliche und tierische Öle und Fette wie z. B. Sojaöl, Distelöl, Erdnussöl, Hanföl, Jatrophaöl, Kokosnussfett, Kürbiskernöl, Leinöl, Maisöl, Mohnöl, Nachtkerzenöl, Olivenöl, Palmkernöl, Palmöl, Rapsöl, Sesamöl, Sonnenblumenöl, Traubenkernöl, Walnussöl, Weizenkeimöl und Kokosfett, Fettsäuren, wie z. B. Caprylsäure, Caprinsäure, Laurinsäure, Myristinsäure, Palmitinsäure, Palmitolensäure, Stearinsäure, Arachidonsäure, Behensäure, Ölsäure, Linolsäure, Linolensäure, Gamma-Linolensäure und deren Methyl- oder Ethylester sowie Fettsäuregemische, Mono-, Di- und Triglyceride mit den gerade erwähnten Fettsäuren, Alkohole wie z. B. Glycerin, Ethanol und Methanol, Kohlenwasserstoffe wie Methan, kohlenstoffhaltige Gase und Gasgemische, wie CO, CO₂, Syntheseoder Rauchgas, Aminosäuren wie L-Glutamat oder L-Valin oder organische Säuren wie z. B. Essigsäure verwendet werden. Diese Stoffe können einzeln oder als Mischung verwendet werden. Besonders bevorzugt ist der Einsatz von Kohlehydraten, insbesondere von Monosacchariden, Oligosacchariden oder Polysacchariden, als Kohlenstoffquelle wie dies in US 6,01,494 und US 6,136,576 beschrieben ist, sowie von Kohlenwasserstoffen, insbesondere von Alkanen, Alkenen und Alkinen sowie den daraus abgeleiteten Monokarbonsäuren und den aus diesen Monocarbonsäuren abgeleiteten Mono-, Di- und Triglyceriden, sowie von Glycerin und Acetat. Ganz besonders bevorzugt sind Mono-, Di- und Triglyceride, enthaltend die Veresterungsprodukte von Glycerin mit Caprylsäure, Caprinsäure, Laurinsäure, Myristinsäure, Palmitinsäure, Palmitolensäure, Stearinsäure, Arachidonsäure, Behensäure, Ölsäure, Linolsäure, Linolensäure und/oder Gamma-Linolensäure.
Als Stickstoffquelle können organische stickstoffhaltige Verbindungen wie Peptone, Hefeextrakt, Fleischextrakt, Malzextrakt, Maisquellwasser, Sojabohnenmehl und Harnstoff oder anorganische Verbindungen wie Ammoniumsulfat, Ammoniumchlorid, Ammoniumphosphat, Ammoniumcarbonat und Ammoniumnitrat, Ammoniak, Ammoniumhydroxid oder Ammoniakwasser verwendet werden. Die Stickstoffquellen können einzeln oder als Mischung verwendet werden.

Als Phosphorquelle können Phosphorsäure, Kaliumdihydrogenphosphat oder Dikaliumhydrogenphosphat oder die entsprechenden Natrium-haltigen Salze verwendet werden. Das Kulturmedium muss weiterhin Salze von Metallen enthalten wie z. B. Magnesiumsulfat oder Eisensulfat, die für das Wachstum notwendig sind. Schließlich können essentielle Wuchsstoffe wie Aminosäuren und Vitamine zusätzlich zu den oben genannten Stoffen eingesetzt werden. Dem Kulturmedium können überdies geeignete Vorstufen zugesetzt werden. Die genannten Einsatzstoffe können zur Kultur in Form eines einmaligen Ansatzes hinzugegeben oder in geeigneter Weise während der Kultivierung zugefüttert werden. Zur pH-Kontrolle der Kultur werden basische Verbindungen wie Natriumhydroxid, Kaliumhydroxid, Ammoniak bzw. Ammoniakwasser oder saure Verbindungen wie Phosphorsäure oder Schwefelsäure in geeigneter Weise eingesetzt. Zur Kontrolle der Schaumentwicklung können Antischaummittel wie z. B. Fettsäurepolyglykolester eingesetzt werden. Zur Aufrechterhaltung der Stabilität von Plasmiden können dem Medium geeignete selektiv wirkende Stoffe wie z. B. Antibiotika hinzugefügt werden. Um aerobe Bedingungen aufrechtzuerhalten, werden Sauerstoff oder Sauerstoffhaltige Gasmischungen wie z. B. Luft in die Kultur eingetragen.
Die Temperatur der Kultur liegt normalerweise bei mehr als 20°C, vorzugsweise bei mehr als 25°C, sie kann auch mehr als 40°C betragen, wobei vorteilhafterweise eine Kultivierungstemperatur von 95°C, besonders bevorzugt 90°C und am meisten bevorzugt 80°C nicht überschritten wird.

Im Schritt III) des erfindungsgemäßen Verfahrens können die durch die Zellen gebildeten Sophorolipide gegebenenfalls aus den Zellen und/oder dem Nährmedium isoliert werden, wobei zur Isolierung alle dem Fachmann bekannten Methoden zur Isolierung von niedermolekularen Substanzen aus komplexen Zusammensetzungen in Betracht kommen wie beispielsweise Filtration, Extraktion, Adsorption (Chromatographie) oder Kristallisation. Die Aufarbeitung der Sophorolipide geschieht in der Regel in Abhängigkeit von der Produktform. Im Falle eines Sophorolipids, welches in der wasser-unlöslichen Lactonform vorliegt, bietet sich folgende Vorgehensweise an: Die Abtrennung des Produktes in der Lactonform von der wässrigen Phase erfolgt durch Zentrifugation.
Darüber hinaus enthält die Produktphase Reste an Biomasse und verschiedene Verunreinigungen, wie Ölen, Fettsäuren und anderen Nährmedienbestandteilen. Ölreste können beispielsweise durch Extraktion mittels geeigneter Lösungsmittel vorteilhafterweise mittels organischer Lösungsmittel abgetrennt werden. Bevorzugt wird als Lösungsmittel ein Alkan wie z.B. n-Hexan. Die Abtrennung des Produkts von der wässrigen Phase kann beispielsweise mit einem geeigneten Ester, beispielsweise mittels Ethylacetat erfolgen. Die genannten Extraktionsschritte können in beliebiger Reihenfolge durchgeführt werden. Alternativ kann die Aufreinigung von Sophorolipiden aus dem Nährmedium durch Überführung der Lactonform in die wasserlösliche offene Säureform erfolgen. Beispielsweise erfolgt die Überführung in die offene Säureform mittels Hydrolyse, vorteilhafterweise durch alkalische Hydrolyse. Anschließend werden die offenkettigen Sophorolipide in einer wässrigen Säure, vorzugsweise wässriger Schwefelsäure gelöst, um ggf. gebildete Salze in der Lösung abzutrennen. Die weitere Aufreinigung des Produkts erfolgt mittels Extraktion. Hierbei werden vorzugsweise Lösungsmittel eingesetzt, insbesondere organische Lösungsmittel. Bevorzugt wird als Lösungsmittel n-Pentanol. Zur Entfernung des Lösungsmittels erfolgt beispielsweise eine Destillation. Anschließend kann das lyophilisierte Produkt, beispielsweise mittels chromatographischer Methoden, weiter aufgereinigt werden. Beispielhaft seien an dieser Stelle die Fällung mittels geeigneter Lösungsmittel, die Extraktion mittels geeigneter Lösungsmittel, die Komplexierung, beispielsweise mittels Cyclodextrinen oder Cyclodextrin-Derivaten, die Kristallisation, die Aufreinigung bzw. Isolierung mittels chromatographischer Methoden oder die Überführung der Sophorolipide in leicht abtrennbare Derivate genannt.

Die mit dem erfindungsgemäßen Verfahren hergestellten Sophorolipide lassen sich vorteilhaft in Reinigungsmitteln, in kosmetischen oder pharmazeutischen Formulierungen sowie in Pflanzenschutz-Formulierungen einsetzen.
Somit wird vorliegend die Verwendung der mit dem erfindungsgemäßen Verfahren erhaltenen Sophorolipide zur Herstellung von kosmetischen, dermatologischen oder pharmazeutischen Formulierungen, von Pflanzenschutz-Formulierungen sowie von Pflege- und Reinigungsmitteln und Tensidkonzentraten offenbart.

Unter dem Begriff "Pflegemittel" wird hier eine Formulierung verstanden, die den Zweck erfüllt, einen Gegenstand in seiner ursprünglichen Form zu erhalten, die Auswirkungen äußerer Einflüsse (z.B. Zeit, Licht, Temperatur, Druck, Verschmutzung, chemische Reaktion mit anderen, mit dem Gegenstand in Kontakt tretenden reaktiven Verbindungen) wie beispielsweise Altern, Verschmutzen, Materialermüdung, Ausbleichen, zu mindern oder zu vermeiden oder sogar gewünschte positive Eigenschaften des Gegenstandes zu verbessern. Für letzten Punkt sei etwa ein verbesserter Haarglanz oder eine größere Elastizität des betrachteten Gegenstandes genannt.
Unter "Pflanzenschutz-Formulierungen" sind solche Formulierungen zu verstehen, die von der Art ihrer Herrichtung nach augenfällig zum Pflanzenschutz verwendet werden; dies ist insbesondere dann der Fall, wenn in der Formulierung mindestens eine Verbindung aus den Klassen der Herbizide, Fungizide, Insektizide, Akarizide, Nematizide, Schutzstoffe gegen Vogelfraß, Pflanzennährstoffe und Bodenstrukturverbesserungsmittel enthalten ist.
Insbesondere werden mit dem erfindungsgemäßen Verfahren hergestellten Sophorolipide in Pflege- und Reinigungsmitteln für Haushalt, Industrie, insbesondere für harte Oberflächen, Leder oder Textilien verwendet.

Einen weiteren Beitrag zur Lösung der Aufgabe leistet eine isolierte DNA, welche ausgewählt ist aus den folgenden Sequenzen:
A1b) eine Sequenz gemäß Seq ID Nr. 56, Seq ID Nr. 58 oder Seq ID Nr. 60, wobei diese Sequenz für ein Protein kodiert, welches in der Lage ist,
   Z-9-Octadecensäure zu 17-Hydroxy-Z-9-Octadecensäure umzusetzen,
B1b) eine intronfreie Sequenz, die von einer Sequenz nach A1b) abgeleitet ist und die das gleiche Protein oder Peptid kodiert wie die Sequenz nach Seq ID Nr. 56, Seq ID Nr. 58 oder Seq ID Nr. 60,
C1b) eine Sequenz, die ein Protein oder Peptid kodiert, das die Aminosäuresequenz gemäß Seq ID Nr. 57, Seq ID Nr. 59 oder Seq ID Nr. 61 umfasst, und das bevorzugt in der Lage ist,
   Z-9-Octadecensäure zu 17-Hydroxy-Z-9-Octadecensäure umzusetzen
D1b) eine Sequenz, die mit einer Sequenz nach einer der Gruppen A1b) bis C1b), besonders bevorzugt nach Gruppe A1b), zu mindestens 95 % und am meisten bevorzugt zu mindestens 99 % identisch ist, wobei diese Sequenz vorzugsweise für ein Protein oder Peptid kodiert, welches in der Lage ist, Z-9-Octadecensäure zu 17-Hydroxy-Z-9-Octadecensäure umzusetzen,
E1b) eine Sequenz, die mit dem Gegenstrang einer Sequenz nach einer der Gruppen A1b) bis D1b), besonders bevorzugt nach Gruppe A1b), hybridisiert oder unter Berücksichtigung der Degeneration des genetischen Codes hybridisieren würde, wobei diese Sequenz vorzugsweise für ein Protein oder Peptid kodiert, welches in der Lage ist,
   Z-9-Octadecensäure zu 17-Hydroxy-Z-9-Octadecensäure umzusetzen,
F1b) ein durch Substitution, Addition, Inversion und/oder Deletion mindestens einer Base, vorzugsweise von mindestens 2 Basen, darüber hinaus bevorzugt von mindestens 5 Basen und am meisten bevorzugt mindestens 10 Basen, jedoch vorzugsweise von nicht mehr als 100 Basen, besonders bevorzugt von nicht mehr als 50 Basen und am meisten bevorzugt von nicht mehr als 25 Basen erhaltenes Derivat einer Sequenz nach einer der Gruppen A1b) bis
E1b), besonders bevorzugt nach Gruppe A1b), wobei dieses Derivat vorzugsweise für ein Protein oder Peptid kodiert, welches in der Lage ist,
   Z-9-Octadecensäure zu 17-Hydroxy-Z-9-Octadecensäure umzusetzen, und
G1b) eine komplementäre Sequenz zu einer Sequenz nach einer der Gruppen A1b) bis F1b), besonders bevorzugt nach Gruppe A1b).

Einen weiteren Beitrag zur Lösung der Aufgabe leistet eine isolierte DNA, welche ausgewählt ist aus den folgenden Sequenzen:
A1c) eine Sequenz gemäß Seq ID Nr. 62, wobei diese Sequenz für ein Protein kodiert, welches in der Lage ist,
   Z-9-0ctadecensäure zu 17-Hydroxy-Z-9-Octadecensäure umzusetzen,
B1c) eine intronfreie Sequenz, die von einer Sequenz nach A1c) abgeleitet ist und die das gleiche Protein oder Peptid kodiert wie die Sequenz nach Seq ID Nr. 62,
C1c) eine Sequenz, die ein Protein oder Peptid kodiert, das die Aminosäuresequenz gemäß Seq ID Nr. 63 umfasst, und das bevorzugt in der Lage ist,
   Z-9-Octadecensäure zu 17-Hydroxy-Z-9-Octadecensäure umzusetzen
D1c) eine Sequenz, die mit einer Sequenz nach einer der Gruppen A1c) bis C1c), besonders bevorzugt nach Gruppe A1c), zu mindestens 90 % und am meisten bevorzugt zu mindestens 99 % identisch ist, wobei diese Sequenz vorzugsweise für ein Protein oder Peptid kodiert, welches in der Lage ist, Z-9-Octadecensäure zu 17-Hydroxy-Z-9-Octadecensäure umzusetzen,
E1c) eine Sequenz, die mit dem Gegenstrang einer Sequenz nach einer der Gruppen A1c) bis D1c), besonders bevorzugt nach Gruppe A1c), hybridisiert oder unter Berücksichtigung der Degeneration des genetischen Codes hybridisieren würde, wobei diese Sequenz vorzugsweise für ein Protein oder Peptid kodiert, welches in der Lage ist,
   Z-9-Octadecensäure zu 17-Hydroxy-Z-9-Octadecensäure umzusetzen,
F1c) ein durch Substitution, Addition, Inversion und/oder Deletion mindestens einer Base, vorzugsweise von mindestens 2 Basen, darüber hinaus bevorzugt von mindestens 5 Basen und am meisten bevorzugt mindestens 10 Basen, jedoch vorzugsweise von nicht mehr als 100 Basen, besonders bevorzugt von nicht mehr als 50 Basen und am meisten bevorzugt von nicht mehr als 25 Basen erhaltenes Derivat einer Sequenz nach einer der Gruppen A1c) bis
E1c), besonders bevorzugt nach Gruppe A1c), wobei dieses Derivat vorzugsweise für ein Protein oder Peptid kodiert, welches in der Lage ist,
   Z-9-Octadecensäure zu 17-Hydroxy-Z-9-Octadecensäure umzusetzen, und
G1c) eine komplementäre Sequenz zu einer Sequenz nach einer der Gruppen A1c) bis F1c), besonders bevorzugt nach Gruppe A1c).

Ein weiterer Gegenstand der Erfindung ist eine isolierte DNA, welche ausgewählt ist aus den folgenden Sequenzen:
A2) eine Sequenz gemäß Seq ID Nr. 3, wobei diese Sequenz für ein Protein kodiert, welches in der Lage ist,
   UDP-Glukose und 17-Hydroxy-Z-9-Octadecensäure zu 17-(β-D-Glucopyranosyl-Oxy)-Z-9-Octadecensäure umzusetzen,
B2) eine intronfreie Sequenz, die von einer Sequenz nach A2) abgeleitet ist und die das gleiche Protein oder Peptid kodiert wie die Sequenz nach Seq ID Nr. 3,
C2) eine Sequenz, die ein Protein oder Peptid kodiert, das die Aminosäuresequenz gemäß Seq ID Nr. 8 umfasst, und welches bevorzugt in der Lage ist,
   UDP-Glukose und 17-Hydroxy-Z-9-Octadecensäure zu 17-(β-D-Glucopyranosyl-Oxy)-Z-9-Octadecensäure umzusetzen,
D2) eine Sequenz, die mit einer Sequenz nach einer der Gruppen A2) bis C2), besonders bevorzugt nach Gruppe A2), zu mindestens 90 %, darüber hinaus bevorzugt zu mindestens 95 % und am meisten bevorzugt zu mindestens 99 % identisch ist, wobei diese Sequenz vorzugsweise für ein Protein oder Peptid kodiert, welches in der Lage ist, UDP-Glukose und 17-Hydroxy-Z-9-Octadecensäure zu 17-(β-D-Glucopyranosyl-Oxy)-Z-9-Octadecensäure umzusetzen,
E2) eine Sequenz, die mit dem Gegenstrang einer Sequenz nach einer der Gruppen A2) bis D2), besonders bevorzugt nach Gruppe A2), hybridisiert oder unter Berücksichtigung der Degeneration des genetischen Codes hybridisieren würde, wobei diese Sequenz vorzugsweise für ein Protein oder Peptid kodiert, welches in der Lage ist,
   UDP-Glukose und 17-Hydroxy-Z-9-Octadecensäure zu 17-(β-D-Glucopyranosyl-Oxy)-Z-9-Octadecensäure umzusetzen,
F2) ein durch Substitution, Addition, Inversion und/oder Deletion mindestens einer Base, vorzugsweise von mindestens 2 Basen, darüber hinaus bevorzugt von mindestens 5 Basen und am meisten bevorzugt mindestens 10 Basen, jedoch vorzugsweise von nicht mehr als 100 Basen, besonders bevorzugt von nicht mehr als 50 Basen und am meisten bevorzugt von nicht mehr als 25 Basen erhaltenes Derivat einer Sequenz nach einer der Gruppen A2) bis E2), besonders bevorzugt nach Gruppe A2), wobei dieses Derivat vorzugsweise für ein Protein oder Peptid kodiert, welches in der Lage ist,
   UDP-Glukose und 17-Hydroxy-Z-9-Octadecensäure zu 17-(β-D-Glucopyranosyl-Oxy)-Z-9-Octadecensäure umzusetzen, und
G2) eine komplementäre Sequenz zu einer Sequenz nach einer der Gruppen A2) bis F2), besonders bevorzugt nach Gruppe A2).

Ein weiterer Gegenstand der Erfindung ist eine isolierte DNA, welche ausgewählt ist aus den folgenden Sequenzen:
A3) eine Sequenz gemäß Seq ID Nr. 4, wobei diese Sequenz für ein Protein kodiert, welches in der Lage ist, 17-L-[(2'-*O*-β-D-Glucopyranosyl-β-D-Glucopyranosyl)-Oxy]-Z-9-Octadecensäure-1',4''-Lacton und Acetyl-Coenzym A zu 17-L-[(2'-*O*-β-D-Glucopyranosyl-β-D-Glucopyranosyl)-Oxy]-Z-9-Octadecensäure-1',4"-Lacton-Monoacetat
   oder 17-L-[(2'-*O*-β-D-Glucopyranosyl-β-D-Glucopyranosyl)-Oxy]-Z-9-Octadecensäure-1',4''-Lacton-Monoacetat und Acetyl-Coenzym A zu 17-L-[(2'-*O*-β-D-Glucopyranosyl-β-D-Glucopyranosyl)-Oxy]-Z-9-Octadecensäure-1',4"-Lacton-Diacetat
   oder 17-L-[(2'-*O*-β-D-Glucopyranosyl-β-D-Glucopyranosyl)-Oxy]-Z-9-Octadecensäure-1',4''-Lacton und Acetyl-Coenzym A zu 17-L-[(2'-*O*-β-D-Glucopyranosyl-β-D-Glucopyranosyl)-Oxy]-Z-9-Octadecensäure-1',4''-Lacton-Diacetat, wobei ersteres bevorzugt ist, umzusetzen,
B3) eine intronfreie Sequenz, die von einer Sequenz nach A3) abgeleitet ist und die das gleiche Protein oder Peptid kodiert wie die Sequenz nach Seq ID Nr. 4,
C3) eine Sequenz, die ein Protein oder Peptid kodiert, das die Aminosäuresequenz gemäß Seq ID Nr. 9 umfasst, und welches bevorzugt in der Lage ist, 17-L-[(2'-*O*-β-D-Glucopyranosyl-β-D-Glucopyranosyl)-Oxy]-Z-9-Octadecensäure-1',4''-Lacton und Acetyl-Coenzym A zu 17-L-[(2'-*O*-β-D-Glucopyranosyl-β-D-Glucopyranosyl)-Oxy]-Z-9-Octadecensäure-1',4"-Lacton-Monoacetat
   oder 17-L-[(2'-*O*-β-D-Glucopyranosyl-β-D-Glucopyranosyl)-Oxy]-Z-9-Octadecensäure-1',4''-Lacton-Monoacetat und Acetyl-Coenzym A zu 17-L-[(2'-*O*-β-D-Glucopyranosyl-β-D-Glucopyranosyl)-Oxy]-Z-9-Octadecensäure-1',4"-Lacton-Diacetat
   oder 17-L-[(2'-*O*-β-D-Glucopyranosyl-β-D-Glucopyranosyl)-Oxy]-Z-9-Octadecensäure-1',4"-Lacton und Acetyl-Coenzym A zu 17-L-[(2'-*O*-β-D-Glucopyranosyl-β-D-Glucopyranosyl)-Oxy]-Z-9-Octadecensäure-1',4''-Lacton-Diacetat, wobei ersteres bevorzugt ist, umzusetzen,
D3) eine Sequenz, die mit einer Sequenz nach einer der Gruppen A3) bis C3), besonders bevorzugt nach Gruppe A3), zu mindestens 90 %, darüber hinaus bevorzugt zu mindestens 95 % und am meisten bevorzugt zu mindestens 99 % identisch ist, wobei diese Sequenz vorzugsweise für ein Protein oder Peptid kodiert, welches in der Lage ist, 17-L-[(2'-*O*-β-D-Glucopyranosyl-β-D-Glucopyranosyl)-Oxy]-Z-9-Octadecensäure-1',4''-Lacton und Acetyl-Coenzym A zu 17-L-[(2'-*O*-β-D-Glucopyranosyl-β-D-Glucopyranosyl)-Oxy]-Z-9-Octadecensäure-1',4"-Lacton-Monoacetat
   oder 17-L-[(2'-*O*-β-D-Glucopyranosyl-β-D-Glucopyranosyl)-Oxy]-Z-9-Octadecensäure-1',4''-Lacton-Monoacetat und Acetyl-Coenzym A zu 17-L-[(2'-*O*-β-D-Glucopyranosyl-β-D-Glucopyranosyl)-Oxy]-Z-9-Octadecensäure-1',4"-Lacton-Diacetat
   oder 17-L-[(2'-*O*-β-D-Glucopyranosyl-β-D-Glucopyranosyl)-Oxy]-Z-9-Octadecensäure-1',4"-Lacton und Acetyl-Coenzym A zu 17-L-[(2'-*O*-β-D-Glucopyranosyl-β-D-Glucopyranosyl)-Oxy]-Z-9-Octadecensäure-1',4''-Lacton-Diacetat, wobei ersteres bevorzugt ist, umzusetzen,
E3) eine Sequenz, die mit dem Gegenstrang einer Sequenz nach einer der Gruppen A3) bis D3), besonders bevorzugt nach Gruppe A3), hybridisiert oder unter Berücksichtigung der Degeneration des genetischen Codes hybridisieren würde, wobei diese Sequenz vorzugsweise für ein Protein oder Peptid kodiert, welches in der Lage ist, 17-L-[(2'-*O*-β-D-Glucopyranosyl-β-D-Glucopyranosyl)-Oxy]-Z-9-Octadecensäure-1',4''-Lacton und Acetyl-Coenzym A zu 17-L-[(2'-*O*-β-D-Glucopyranosyl-β-D-Glucopyranosyl)-Oxy]-Z-9-Octadecensäure-1',4"-Lacton-Monoacetat
   oder 17-L-[(2'-*O*-β-D-Glucopyranosyl-β-D-Glucopyranosyl)-Oxy]-Z-9-Octadecensäure-1',4''-Lacton-Monoacetat und Acetyl-Coenzym A zu 17-L-[(2'-*O*-β-D-Glucopyranosyl-β-D-Glucopyranosyl)-Oxy]-Z-9-Octadecensäure-1',4"-Lacton-Diacetat
   oder 17-L-[(2'-*O*-β-D-Glucopyranosyl-β-D-Glucopyranosyl)-Oxy]-Z-9-Octadecensäure-1',4''-Lacton und Acetyl-Coenzym A zu 17-L-[(2'-*O*-β-D-Glucopyranosyl-β-D-Glucopyranosyl)-Oxy]-Z-9-Octadecensäure-1',4''-Lacton-Diacetat, wobei ersteres bevorzugt ist, umzusetzen,
F3) ein durch Substitution, Addition, Inversion und/oder Deletion mindestens einer Base, vorzugsweise von mindestens 2 Basen, darüber hinaus bevorzugt von mindestens 5 Basen und am meisten bevorzugt mindestens 10 Basen, jedoch vorzugsweise von nicht mehr als 100 Basen, besonders bevorzugt von nicht mehr als 50 Basen und am meisten bevorzugt von nicht mehr als 25 Basen erhaltenes Derivat einer Sequenz nach einer der Gruppen A3) bis E3), besonders bevorzugt nach Gruppe A3), wobei dieses Derivat vorzugsweise für ein Protein oder Peptid kodiert,
   welches in der Lage ist, 17-L-[(2'-*O*-β-D-Glucopyranosyl-β-D-Glucopyranosyl)-Oxy]-Z-9-Octadecensäure-1',4''-Lacton und Acetyl-Coenzym A zu 17-L-[(2'-*O*-β-D-Glucopyranosyl-β-D-Glucopyranosyl)-Oxy]-Z-9-Octadecensäure-1',4"-Lacton-Monoacetat
   oder 17-L-[(2'-*O*-β-D-Glucopyranosyl-β-D-Glucopyranosyl)-Oxy]-Z-9-Octadecensäure-1',4''-Lacton-Monoacetat und Acetyl-Coenzym A zu 17-L-[(2'-*O*-β-D-Glucopyranosyl-β-D-Glucopyranosyl)-Oxy]-Z-9-Octadecensäure-1',4"-Lacton-Diacetat
   oder 17-L-[(2'-*O*-β-D-Glucopyranosyl-β-D-Glucopyranosyl)-Oxy]-Z-9-Octadecensäure-1',4''-Lacton und Acetyl-Coenzym A zu 17-L-[(2'-*O*-β-D-Glucopyranosyl-β-D-Glucopyranosyl)-Oxy]-Z-9-Octadecensäure-1',4''-Lacton-Diacetat, wobei ersteres bevorzugt ist, umzusetzen,
   und
G3) eine komplementäre Sequenz zu einer Sequenz nach einer der Gruppen A3) bis F3), besonders bevorzugt nach Gruppe A3).

Ein weiterer Gegenstand der Erfindung ist eine isolierte DNA, welche ausgewählt ist aus den folgenden Sequenzen:
A4) eine Sequenz gemäß Seq ID Nr. 5, wobei diese Sequenz für ein Protein kodiert, welches in der Lage ist, ein Sophorolipid aus einer Zelle heraus ins umgebende Medium zu transferieren,
B4) eine intronfreie Sequenz, die von einer Sequenz nach A4) abgeleitet ist und die das gleiche Protein oder Peptid kodiert wie die Sequenz nach Seq ID Nr. 5,
C4) eine Sequenz, die ein Protein oder Peptid kodiert, das die Aminosäuresequenz gemäß Seq ID Nr. 10 umfasst, und welches bevorzugt in der Lage ist, ein Sophorolipid aus einer Zelle heraus ins umgebende Medium zu transferieren,
D4) eine Sequenz, die mit einer Sequenz nach einer der Gruppen A4) bis C4), besonders bevorzugt nach Gruppe A4), zu mindestens 90 %, darüber hinaus bevorzugt zu mindestens 95 % und am meisten bevorzugt zu mindestens 99 % identisch ist, wobei diese Sequenz vorzugsweise für ein Protein oder Peptid kodiert, welches in der Lage ist, ein Sophorolipid aus einer Zelle heraus ins umgebende Medium zu transferieren,
E4) eine Sequenz, die mit dem Gegenstrang einer Sequenz nach einer der Gruppen A4) bis D4), besonders bevorzugt nach Gruppe A4), hybridisiert oder unter Berücksichtigung der Degeneration des genetischen Codes hybridisieren würde, wobei diese Sequenz vorzugsweise für ein Protein oder Peptid kodiert, welches in der Lage ist, ein Sophorolipid aus einer Zelle heraus ins umgebende Medium zu transferieren,
F4) ein durch Substitution, Addition, Inversion und/oder Deletion mindestens einer Base, vorzugsweise von mindestens 2 Basen, darüber hinaus bevorzugt von mindestens 5 Basen und am meisten bevorzugt mindestens 10 Basen, jedoch vorzugsweise von nicht mehr als 100 Basen, besonders bevorzugt von nicht mehr als 50 Basen und am meisten bevorzugt von nicht mehr als 25 Basen erhaltenes Derivat einer Sequenz nach einer der Gruppen A4) bis E4), besonders bevorzugt nach Gruppe A4), wobei dieses Derivat vorzugsweise für ein Protein oder Peptid kodiert, welches in der Lage ist, ein Sophorolipid aus einer Zelle heraus ins umgebende Medium zu transferieren, und
G4) eine komplementäre Sequenz zu einer Sequenz nach einer der Gruppen A4) bis F4), besonders bevorzugt nach Gruppe A4).

Ein weiterer Gegenstand der Erfindung ist eine isolierte DNA, welche ausgewählt ist aus den folgenden Sequenzen:
A5) eine Sequenz gemäß Seq ID Nr. 6, wobei diese Sequenz für ein Protein kodiert, welches in der Lage ist, UDP-Glukose und 17-Hydroxy-Z-9-Octadecensäure zu 17-(β-D-Glucopyranosyl-Oxy)-Z-9-Octadecensäure oder 17-(β-D-Glucopyranosyl-Oxy)-Z-9-Octadecensäure und UDP-Glukose zu 17-L-[(2'-*O*-β-D-Glucopyranosyl-β-D-Glucopyranosyl)-Oxy]-Z-9-Octadecensäure, wobei letzteres bevorzugt ist, umzusetzen,
B5) eine intronfreie Sequenz, die von einer Sequenz nach A5) abgeleitet ist und die das gleiche Protein oder Peptid kodiert wie die Sequenz nach Seq ID Nr. 6,
C5) eine Sequenz, die ein Protein oder Peptid kodiert, das die Aminosäuresequenz gemäß Seq ID Nr. 11 umfasst, und welches bevorzugt in der Lage ist,
   UDP-Glukose und 17-Hydroxy-Z-9-Octadecensäure zu 17-(β-D-Glucopyranosyl-Oxy)-Z-9-Octadecensäure oder 17-(β-D-Glucopyranosyl-Oxy)-Z-9-Octadecensäure und UDP-Glukose zu 17-L-[(2'-*O*-β-D-Glucopyranosyl-β-D-Glucopyranosyl)-Oxy]-Z-9-Octadecensäure, wobei letzteres bevorzugt ist, umzusetzen,
D5) eine Sequenz, die mit einer Sequenz nach einer der Gruppen A5) bis C5), besonders bevorzugt nach Gruppe A5), zu mindestens 90 %, darüber hinaus bevorzugt zu mindestens 95 % und am meisten bevorzugt zu mindestens 99 % identisch ist, wobei diese Sequenz vorzugsweise für ein Protein oder Peptid kodiert, welches in der Lage ist, UDP-Glukose und 17-Hydroxy-Z-9-Octadecensäure zu 17-(β-D-Glucopyranosyl-Oxy)-Z-9-Octadecensäure oder 17-(β-D-Glucopyranosyl-Oxy)-Z-9-Octadecensäure und UDP-Glukose zu 17-L-[(2'-*O*-β-D-Glucopyranosyl-β-D-Glucopyranosyl)-Oxy]-Z-9-Octadecensäure, wobei letzteres bevorzugt ist, umzusetzen,
E5) eine Sequenz, die mit dem Gegenstrang einer Sequenz nach einer der Gruppen A5) bis D5), besonders bevorzugt nach Gruppe A5), hybridisiert oder unter Berücksichtigung der Degeneration des genetischen Codes hybridisieren würde, wobei diese Sequenz vorzugsweise für ein Protein oder Peptid kodiert, welches in der Lage ist,
   UDP-Glukose und 17-Hydroxy-Z-9-Octadecensäure zu 17-(β-D-Glucopyranosyl-Oxy)-Z-9-Octadecensäure oder 17-(β-D-Glucopyranosyl-Oxy)-Z-9-Octadecensäure und UDP-Glukose zu 17-L-[(2'-*O*-β-D-Glucopyranosyl-β-D-Glucopyranosyl)-Oxy]-Z-9-Octadecensäure, wobei letzteres bevorzugt ist, umzusetzen,
F5) ein durch Substitution, Addition, Inversion und/oder Deletion mindestens einer Base, vorzugsweise von mindestens 2 Basen, darüber hinaus bevorzugt von mindestens 5 Basen und am meisten bevorzugt mindestens 10 Basen, jedoch vorzugsweise von nicht mehr als 100 Basen, besonders bevorzugt von nicht mehr als 50 Basen und am meisten bevorzugt von nicht mehr als 25 Basen erhaltenes Derivat einer Sequenz nach einer der Gruppen A5) bis E5), besonders bevorzugt nach Gruppe A5), wobei dieses Derivat vorzugsweise für ein Protein oder Peptid kodiert, welches in der Lage ist,
   UDP-Glukose und 17-Hydroxy-Z-9-Octadecensäure zu 17-(β-D-Glucopyranosyl-Oxy)-Z-9-Octadecensäure oder 17-(β-D-Glucopyranosyl-Oxy)-Z-9-Octadecensäure und UDP-Glukose zu 17-L-[(2'-*O*-β-D-Glucopyranosyl-β-D-Glucopyranosyl)-Oxy]-Z-9-Octadecensäure, wobei letzteres bevorzugt ist, umzusetzen, und
G5) eine komplementäre Sequenz zu einer Sequenz nach einer der Gruppen A5) bis F5), besonders bevorzugt nach Gruppe A5).

Die "Nukleotid-Identität" oder "Aminosäure-Identität" wird hier mit Hilfe bekannter Verfahren bestimmt. Generell werden besondere Computerprogramme mit Algorithmen unter Berücksichtigung spezieller Erfordernisse verwendet.
Bevorzugte Verfahren zur Bestimmung der Identität erzeugen zunächst die größte Übereinstimmung zwischen den zu vergleichenden Sequenzen. Computer-Programme zur Bestimmung der Identität umfassen, sind jedoch nicht beschränkt auf, das GCG-Programmpaket, einschließlich
GAP (Deveroy, J. et al., Nucleic Acid Research 12 (1984), Seite 387, Genetics Computer Group University of Wisconsin, Medicine (Wi), und BLASTP, BLASTN und FASTA (Altschul, S. et al., Journal of Molecular Biology 215 (1990), Seiten 403-410. Das BLAST-Programm kann erhalten werden vom National Center For Biotechnology Information (NCBI) und aus weiteren Quellen (BLAST Handbuch, Altschul S. et al., NCBI NLM NIH Bethesda ND 22894; Altschul S. et al., vorstehend).
Der bekannte Smith-Waterman Algorithmus kann ebenso zur Bestimmung der Nukleotid-Identität verwendet werden. Bevorzugte Parameter für die Bestimmung der "Nukleotid-Identität" sind bei Verwendung des BLASTN-Programms (Altschul, S. et al., Journal of Molecular Biology 215 (1990), Seiten 403-410 :

| | |
|---|---|
| Expect Threshold: | 10 |
| Word size: | 28 |
| Match Score: | 1 |
| Mismatch Score: | -2 |
| Gap costs: | Linear |

Die vorstehenden Parameter sind die default-Parameter im Nukleotidsequenzvergleich.
Das GAP-Programm ist ebenso zur Verwendung mit den vorstehenden Parametern geeignet.

Bevorzugte Parameter für die Bestimmung der "Aminosäure-Identität" sind bei Verwendung des BLASTP-Programms (Altschul, S. et al., Journal of Molecular Biology 215 (1990), Seiten 403-410 :

| | |
|---|---|
| Expect Threshold: | 10 |
| Word size: | 3 |
| Matrix: | BLOSUM62 |
| Gap costs: | Existence: 11; Extension: 1 |
| Compositional adjustments: | Conditional compositional score matrix adjustment |

Die vorstehenden Parameter sind die default-Parameter im Aminosäuresequenzvergleich.
Das GAP-Programm ist ebenso zur Verwendung mit den vorstehenden Parametern geeignet.

Eine Identität von 80 % gemäß dem vorstehenden Algorithmus bedeutet im Zusammenhang mit der vorliegenden Erfindung 80 % Identität. Das gleiche gilt für höhere Identitäten.

Das Merkmal "Sequenz, die mit dem Gegenstrang einer Sequenz hybridisiert oder unter Berücksichtigung der Degeneration des genetischen Codes hybridisieren würde" weist auf eine Sequenz hin, die unter vorzugsweise stringenten Bedingungen mit dem Gegenstrang einer Bezugssequenz hybridisiert oder unter Berücksichtigung der Degeneration des genetischen Codes hybridisieren würde. Beispielsweise können die Hybridisierungen bei 68°C in 2 x SSC oder nach dem Protokoll des Digoxigenin-Labelling-Kits der Firma Boehringer (Mannheim) durchgeführt werden. Bevorzugte Hybridisierungsbedingungen sind z. B. Inkubation bei 65°C über Nacht in 7 % SDS, 1 % BSA, 1 mM EDTA, 250 mM Natriumphosphatpuffer (pH 7,2) und nachfolgendes Waschen bei 65°C mit 2 x SSC; 0,1 % SDS.
Zu den Derivaten der erfindungsgemäßen isolierten DNA, welche gemäß Alternative F1a), F1b), F1b), F1c), F2), F3), F4) oder F5) durch Substitution, Addition, Inversion und/oder Deletion einer oder mehrerer Basen einer Sequenz nach einer der Gruppen A1a) bis E1a), A1b) bis E1b), A1c) bis E1c), A2) bis E2), A3) bis E3), A4) bis E4) und A5) bis E5) erhalten werden können, gehören insbesondere solche Sequenzen, welche in dem Protein, welches sie kodieren, zu konservativen Aminosäureaustauschen, wie z. B. dem Austausch von Glycin gegen Alanin oder von Asparaginsäure gegen Glutaminsäure führen. Solche funktionsneutralen Mutationen werden als Sinnmutationen (sense mutations) bezeichnet und führen zu keiner grundsätzlichen Veränderung der Aktivität des Polypeptids. Weiterhin ist bekannt, dass Änderungen am N- und/oder C-Terminus eines Polypeptids dessen Funktion nicht wesentlich beeinträchtigen oder dieses sogar stabilisieren können, so dass dementsprechend auch DNA-Sequenzen, bei denen am 3'-Ende oder am 5'-Ende der Sequenz mit den erfindungsgemäßen Nukleinsäuren Basen angefügt sind, von der vorliegenden Erfindung umfasst sind. Angaben hierzu findet der Fachmann unter anderem bei Ben-Bassat et al. (Journal of Bacteriology 169:751-757 (1987)), bei O'Regan et al. (Gene 77:237-251 (1989)), bei Sahin-Toth et al. (Protein Sciences 3:240-247 (1994)), bei Hochuli et al. (Bio/Technology 6:1321-1325 (1988)) und in bekannten Lehrbüchern der Genetik und Molekularbiologie.

Einen Beitrag zur Lösung der eingangs genannten Aufgaben leistet weiterhin ein Vektor, vorzugsweise ein Expressionsvektor, eine Gendeletions- Geninsertions- oder Genüberexpressionskassette, umfassend eine DNA mit einer Sequenz nach einer der Gruppen A1a) bis G1a), A1b) bis G1b), A1c) bis G1c), A2) bis G2), A3) bis G3), A4) bis G4) und A5) bis G5), wie vorstehend definiert. Als Vektoren kommen alle dem Fachmann bekannten Vektoren in Betracht, die üblicherweise zum Einschleusen von DNA in eine Wirtzelle eingesetzt werden. Diese Vektoren können sowohl autonom replizieren, da sie Replikationsursprünge, wie etwa jenen des 2µ-Plasmids oder ARS (autonom replizierende Sequenzen) besitzen oder in die Chromosomen integrieren (nicht-replikative Plasmide). Unter Vektoren werden auch lineare DNA-Fragmente verstanden, die keinerlei Replikationsursprünge besitzen, wie etwa Gendeletions- Geninsertions- oder Genüberexpressionskassetten. Gendeletionskassetten bestehen üblicherweise aus einem Selektionsmarker und DNA-Fragmenten, welche den zu deletierenden Bereich flankieren. Geninsertionskassetten bestehen üblicherweise aus einem Marker und Fragmenten des zu inaktivierenden Gens, Genüberexpressionskassetten bestehen üblicherweise aus einem Marker, dem überzuexprimierenden Gen sowie für die Expression des Gens relevanten regulatorischen Bereichen, wie etwa Promotor und Terminator. Bevorzugte Vektoren sind ausgewählt aus der Gruppe umfassend Plasmide und Kassetten, wie etwa *E*. *coli-*Hefe-Shuttle-Plasmide, besonders bevorzugt sind Expressionsvektoren, Gendeletions- Geninsertions- oder Genüberexpressionskassetten, insbesondere die unten beschriebenen Gendeletionskassetten mit Seq ID Nr. 12, Seq ID Nr. 13, Seq ID Nr. 14, Seq ID Nr. 15 und Seq ID Nr. 16 sowie die Expressionskassetten mit Seq ID Nr. 70, Seq ID Nr. 71, Seq ID Nr. 72, Seq ID Nr. 73 und Seq ID Nr. 74 . Gemäß einer bevorzugten Ausführungsform des erfindungsgemäßen Vektors liegt die DNA mit einer Sequenz nach einer der Gruppen A1) bis F5) unter der Kontrolle eines konstitutiven oder regulierbaren Promotors, welcher zur Expression des von diesen DNA-Sequenzen kodierten Polypeptids in der Zelle eines Mikroorganismus, vorzugsweise einer Bakterien-, Hefe- oder Pilzzelle, besonders bevorzugt einer Hefezelle, am meisten bevorzugt einer *Candida bombicola-, Candida bogoriensis-, Candida batistae-, Candida apicola-* oder *Wickerhamiella domericqiae-*Zelle geeignet ist. Beispiele für solche konstitutiven Promotoren sind etwa der TSC3-Promotor, der *ENO1* Promotor, der *FBA1* Promotor, der *GPD* Promotor, der *GPM* Promotor, der *FBA1* Promotor, der *ICL1* Promotor oder der *ACT1*-Promotor. Beispiele für solche regulierbaren Promotoren sind etwa der ***GAL1-***Promotor, der *GAL2* Promotor, der *GAL7* Promotor, der *MEL1* Promotor, der *GAL10* Promotor, der *SBG1* Promotor, der *SBG2* Promotor, der *SBG3* Promotor, der *SBG4* Promotor, der *SBG5* Promotor oder der *MAL2*-Promotor.
Der erfindungsgemäße Vektor sollte neben einem Promotor vorzugsweise eine Ribosomenbindungsstelle sowie einen Terminator umfassen. Dabei ist es besonders bevorzugt, dass die erfindungsgemäße DNA in eine Expressionskassette des Vektors umfassend den Promotor, die Ribosomenbindungsstelle und den Terminator eingebaut ist. Neben den vorstehend genannten strukturellen Elementen kann der Vektor des Weiteren dem Fachmann bekannte Selektionsmarkergene umfassen. Erfindungsgemäß bevorzugte Vektoren stellen die in den Beispielen beschriebenen Nukleinsäuren Seq ID Nr. 12, Seq ID Nr. 13, Seq ID Nr. 14, Seq ID Nr. 15, Seq ID Nr. 16, IntEx-CbSBG1 (Seq ID Nr. 70), IntEx-CbSBG2 (Seq ID Nr. 71), IntEx-CbSBG3 (Seq ID Nr. 72), IntEx-CbSBG4 (Seq ID Nr. 73) und IntEx-CbSBG5 (Seq ID Nr. 74) dar.

Einen weiteren Beitrag zur Lösung der Aufgabe leisten die neuen Enzyme E₁ bis E₅.
Somit ist ein weiterer Gegenstand der Erfindung ein isoliertes Polypeptid ausgewählt aus der Gruppe bestehend aus ein Enzym E₁ mit Polypeptidsequenz Seq ID Nr. 57, Seq ID Nr. 59, Seq ID Nr. 61 oder Seq ID Nr. 63 oder mit einer Polypeptidsequenz bei der bis zu 10, 9, 8, 7, 6, 5, 4, 3, 2, 1 % der Aminosäurereste gegenüber der jeweiligen Bezugssequenz Seq ID Nr. 57, Seq ID Nr. 59, Seq ID Nr. 61 oder Seq ID Nr. 63 durch Deletion, Insertion, Substitution oder eine Kombination davon verändert sind und die noch mindestens 50%, bevorzugt 65%, besonders bevorzugt 80%, insbesondere mehr als 90 % der enzymatischen Aktivität des Enzyms mit der jeweiligen Bezugssequenz besitzt, wobei unter enzymatischer Aktivität für ein Enzym E₁ die Fähigkeit verstanden wird, Z-9-Octadecensäure zu 17-Hydroxy-Z-9-Octadecensäure umzusetzen,
ein Enzym E₂ mit Polypeptidsequenz Seq ID Nr. 8 oder Seq ID Nr. 11 oder mit einer Polypeptidsequenz bei der bis zu 60%, bevorzugt bis zu 25%, besonders bevorzugt bis zu 15% insbesondere bis zu 10, 9, 8, 7, 6, 5, 4, 3, 2, 1 % der Aminosäurereste gegenüber Seq ID Nr. 8 oder Seq ID Nr. 11 durch Deletion, Insertion, Substitution oder eine Kombination davon verändert sind und die noch mindestens 50%, bevorzugt 65%, besonders bevorzugt 80%, insbesondere mehr als 90 % der enzymatischen Aktivität des Enzyms mit der jeweiligen Bezugssequenz Nr. 8 oder 11 besitzt, wobei unter enzymatischer Aktivität für ein Enzym E₂ die Fähigkeit verstanden wird, UDP-Glukose und 17-Hydroxy-Z-9-Octadecensäure zu 17-(β-D-Glucopyranosyl-Oxy)-Z-9-Octadecensäure umzusetzen,
ein Enzym E₃ mit Polypeptidsequenz Seq ID Nr. 11 oder mit einer Polypeptidsequenz bei der bis zu 60%, bevorzugt bis zu 25%, besonders bevorzugt bis zu 15% insbesondere bis zu 10, 9, 8, 7, 6, 5, 4, 3, 2, 1 % der Aminosäurereste gegenüber Seq ID Nr. 11 durch Deletion, Insertion, Substitution oder eine Kombination davon verändert sind und die noch mindestens 50%, bevorzugt 65%, besonders bevorzugt 80%, insbesondere mehr als 90 % der enzymatischen Aktivität des Enzyms mit der jeweiligen Bezugssequenz Nr. 11 besitzt, wobei unter enzymatischer Aktivität für ein Enzym E₃ die Fähigkeit verstanden wird, 17-(β-D-Glucopyranosyl-Oxy)-Z-9-Octadecensäure und UDP-Glukose zu 17-L-[(2'-*O*-β-D-Glucopyranosyl-β-D-Glucopyranosyl)-Oxy]-Z-9-Octadecensäure umzusetzen,
ein Enzym E₄ mit Polypeptidsequenz Seq ID Nr. 9 oder mit einer Polypeptidsequenz bei der bis zu 50%, bevorzugt bis zu 25%, besonders bevorzugt bis zu 15% insbesondere bis zu 10, 9, 8, 7, 6, 5, 4, 3, 2, 1 % der Aminosäurereste gegenüber Seq ID Nr. 9 durch Deletion, Insertion, Substitution oder eine Kombination davon verändert sind und die noch mindestens 50%, bevorzugt 65%, besonders bevorzugt 80%, insbesondere mehr als 90 % der enzymatischen Aktivität des Enzyms mit Seq ID Nr. 9 besitzt, wobei unter enzymatischer Aktivität für ein Enzym E₄ die Fähigkeit verstanden wird,
17-L-[(2'-*O*-β-D-Glucopyranosyl-β-D-Glucopyranosyl)-Oxy]-Z-9-Octadecensäure-1',4''-Lacton und Acetyl-Coenzym A zu 17-L-[(2'-*O*-β-D-Glucopyranosyl-β-D-Glucopyranosyl)-Oxy]-Z-9-Octadecensäure-1',4"-Lacton-Monoacetat
oder 17-L-[(2'-*O*-β-D-Glucopyranosyl-β-D-Glucopyranosyl)-Oxy]-Z-9-Octadecensäure-1',4"-Lacton-Monoacetat und Acetyl-Coenzym A zu 17-L-[(2'-*O*-β-D-Glucopyranosyl-β-D-Glucopyranosyl)-Oxy]-Z-9-Octadecensäure-1',4"-Lacton-Diacetat
oder 17-L-[(2'-*O*-β-D-Glucopyranosyl-β-D-Glucopyranosyl)-Oxy]-Z-9-Octadecensäure-1',4"-Lacton und Acetyl-Coenzym A zu 17-L-[(2'-*O*-β-D-Glucopyranosyl-β-D-Glucopyranosyl)-Oxy]-Z-9-Octadecensäure-1',4"-Lacton-Diacetat, wobei ersteres bevorzugt ist, umzusetzen,
und
ein Enzym E₅ mit Polypeptidsequenz Seq ID Nr. 10 oder mit einer Polypeptidsequenz bei der bis zu 45%, bevorzugt bis zu 25%, besonders bevorzugt bis zu 15% insbesondere bis zu 10, 9, 8, 7, 6, 5, 4, 3, 2, 1 % der Aminosäurereste gegenüber Seq ID Nr. 10 durch Deletion, Insertion, Substitution oder eine Kombination davon verändert sind und die noch mindestens 50%, bevorzugt 65%, besonders bevorzugt 80%, insbesondere mehr als 90 % der enzymatischen Aktivität des Enzyms mit Seq ID Nr. 10 besitzt, wobei unter enzymatischer Aktivität für ein Enzym E₅ die Fähigkeit verstanden wird, ein Sophorolipid aus einer Zelle heraus ins umgebende Medium zu transferieren.

In den nachfolgend aufgeführten Beispielen wird die vorliegende Erfindung beispielhaft beschrieben, ohne dass die Erfindung, deren Anwendungsbreite sich aus der gesamten Beschreibung und den Ansprüchen ergibt, auf die in den Beispielen genannten Ausführungsformen beschränkt sein soll.
Folgende Abbildungen sind Bestandteil der Beispiele:
Abbildung 1: Akkurate Massenspur für 17-L-[(6'-*O*-Acetyl-2'-*O*-β-D-Glucopyranosyl-6"-*O*-Acetyl-β-D-Glucopyranosyl)-Oxy]-Z-9-Octadecen-4"-*O*-Lacton
Abbildung 2: Akkurate Massenspur für 17-L-[(2'-*O*-β-D-Glucopyranosyl-β-D-Glucopyranosyl)-Oxy]-Z-9-Octadecen-4"-*O-*Lacton

### Beispiele:

### Beispiel 1: Generierung von Uracil-auxotrophen Mutanten von Candida bombicola ATCC 22214

Eine Uracil-auxotrophe Mutante von *Candida bombicola* ATCC 22214 wurde generiert wie vorbeschrieben (van Bogaert et al. Yeast. 2007. 24(3):201-8). Dieser Stamm wurde als *C. bombicola* ATCC 22214 *ura*⁻ bezeichnet.

### Beispiel 2: Inaktivierung der Strukturgene der an der Sophorolipid-Biosynthese beteiligten Enzyme in Candida bombicola ATCC 22214

Um die an der Sophorolipid-Biosynthese beteiligten Enzyme identifizieren zu können, wurde zunächst das Genom von *Candida bombicola* ATCC 22214 mittels GLS Flex Titanium-Technologie sequenziert. Bei Inspektion der genetischen Information von *Candida bombicola* ATCC 22214 wurde ein Cluster von fünf Genen (Seq ID Nr. 01) identifiziert, deren kodierenden Bereiche (Seq ID Nr. 02, Seq ID Nr. 03, Seq ID Nr. 04, Seq ID Nr. 05, Seq ID Nr. 06) für Genprodukte (Seq ID Nr. 07, Seq ID Nr. 08, Seq ID Nr. 09, Seq ID Nr. 10, Seq ID Nr. 11) kodieren.

Die fünf Gene wurden mit *SBG1* (Seq ID Nr. 02), *SBG2* (Seq ID Nr. 03), *SBG3* (Seq ID Nr. 04), *SBG4* (Seq ID Nr. 05) und *SBG5* (Seq ID Nr. 06) (SBG für Sophorolipid Biosynthesis Gene) bezeichnet.

Sie kodieren für die folgenden Proteine: Sbg1p (Seq ID Nr. 07), Sbg2p (Seq ID Nr. 08), Sbg3p (Seq ID Nr. 09), Sbg4p (Seq ID Nr. 10) und Sbg5p (Seq ID Nr. 11).

**Tabelle 1: Sbg1p, Sbg2p, Sbg3p, Sbg4p und Sbg5p sowie deren Funktionen in Sophorolipid-Biosynthese und -Export.**

| Seq ID Nr. | Protein | PFAM-Domäne | NCBI conserved domain | Funktion |
|---|---|---|---|---|
| 07 | Sbg1p | P450 (PFAM PF00067) | Cytochrom P450 | Fettsäure-[ω,ω-1, ω-2, ω-3]-hydroxylierende Monoxygenase |
| 08 | Sbg2p | UDP-Glycosyltransferase (PFAM PF00201) | Glycosyltransferase | UDP-Glukose: [ω,ω-1, ω-2, ω-3]-Hydroxyfettsäure-Glucosyltransferase |
| 09 | Sbg3p | keine | Maltose-O-Acetyltransferase (PRK10092) | Acetyl-CoA:Sophorolipid-Acetyltransferase |
| 10 | Sbg4p | ABC-Transporter (PFAM 00667) | ABC-Transporter | Sophorolipid-Exportprotein |
| 11 | Sbg5p | UDP-Glycosyltransferase (PFAM PF00201) | Glycosyltransferase | UDP-Glukose:[ω,ω-1, ω-2, ω-3]-Hydroxyfettsäure-Glucosyltransferase; UDP-Glukose: [ω,ω-1, ω-2, ω-3]-(β-D-Glucopyranosyl)-Oxy-Fettsäure-Glucosyltransferase |

Die Gene *SBG1, SBG2, SBG3, SBG4* und *SBG5* werden einzeln inaktiviert und der Phänotyp der entsprechenden Mutanten bezüglich Sophorolipid-Biosynthese charakterisiert. Zur Konstruktion der entsprechenden Mutanten in C. *bombicola* ATCC 22214 werden zunächst Deletionskassetten durch die GeneArt AG (Regensburg) synthetisiert. Diese Deletionskassetten (Seq ID Nr. 12, Seq ID Nr. 13, Seq ID Nr. 14, Seq ID Nr. 15, Seq ID Nr. 16) bestehen aus dem vorbeschriebenen, für die Orotidin-5-Phosphat-Decarboxylase von C. *bombicola* ATCC 22214 kodierenden, Gen *CbURA3* (van Bogaert et al. Yeast. 2007. 24(3):201-8), welches stromauf- und stromabwärts flankiert wird von jeweils etwa 1000 bp der die zu inaktivierenden Gene flankierenden Bereiche. Zwischen den flankierenden Bereichen und dem *CbURA3-*Gen werden jeweils *loxP-*Loci eingefügt, die ggf. die Deletion des *CbURA3-*Gen durch vorübergehende Einführung des für die Rekombinase Cre kodierenden Gens und dessen funktionelle Expression erlauben (für einen Überblick siehe Kühn & Torres. Methods Mol Biol. 2002. 180:175-204). Dabei sind die einzelnen Deletionskassetten entsprechend den Angaben in Tabelle 2 aufgebaut:

**Tabelle 2: Struktur der Deletionskassetten für die Sbg1p, Sbg2p, Sbg3p, Sbg4p und Sbg5p kodierenden Strukturgene von C. bombicola ATCC 22214.**

| Seq ID Nr. | Gen | 5'-flankieren der Bereich | loxP-Locus 1 | *CbURA3* | loxP-Locus 2 | 3'-flankierender Bereich |
|---|---|---|---|---|---|---|
| 12 | *SBG1* | 1 - 1003 | 1004 - 1037 | 1038 - 3106 | 3107 - 3140 | 3141 - 4143 |
| 13 | *SBG2* | 1 - 0999 | 1000 - 1033 | 1034 - 3102 | 3103 - 3136 | 3137 - 4143 |
| 14 | *SBG3* | 1 - 1002 | 1003 - 1036 | 1037 - 3105 | 3106 - 3139 | 3140 - 4140 |
| 15 | *SBG4* | 1 - 0997 | 0998 - 1031 | 1032 - 3100 | 3101 - 3134 | 3135 - 4130 |
| 16 | *SBG5* | 1 - 1002 | 1003 - 1036 | 1037 - 3105 | 3106 - 3139 | 3140 - 4141 |

Um die Deletionskassetten für die anschließende Transformation von *C*. *bombicola* ATCC 22214 *ura*⁻ in ausreichender Menge zur Verfügung zu stellen, werden diese per PCR amplifiziert. Dabei kommen folgende Oligonukleotide zum Einsatz:

**Amplifikation der Deletionskassette für Inaktivierung von CbSBG1:**

| | |
|---|---|
| SBG1-fw: | 5'-AAT TGT TCG ATG GAT AGC TTT GGA GTC -3' (Seq ID Nr. 17) |
| SBG1-rv: | 5'-TTC GGG GCT CCT GTC GTT GTC -3' (Seq ID Nr. 18) |

**Amplifikation der Deletionskassette für Inaktivierung von CbSBG2:**

| | |
|---|---|
| SBG2-fw: | 5'- GAA ATC TGA TCA ATT CTG CAA ACC TG -3' (Seq ID Nr. 19) |
| SBG2-rv: | 5'-ATG ACT CCT AGA AAA GAA ATT GAC CAG -3' (Seq ID Nr. 20) |

**Amplifikation der Deletionskassette für Inaktivierung von CbSBG3:**

| | |
|---|---|
| SBG3-fw: | 5'-TGC AGA CAA GTT CCT GCA GCT G -3' (Seq ID Nr. 21) |
| SBG3-rv: | 5'-ATG CTT TAT TCA GGC ACG CTA CG -3' (Seq ID Nr. 22) |

**Amplifikation der Deletionskassette für Inaktivierung von CbSBG4:**

| | |
|---|---|
| SBG4-fw: | 5'- GGA TGA GTC GCA GTC ACG AAC -3' (Seq ID Nr. 23) |
| SBG4-rv: | 5'-TCA ATC ATT GGC TCA AGA CTA GGA AC -3' (Seq ID Nr. 24) |

**Amplifikation der Deletionskassette für Inaktivierung von CbSBG5:**

| | |
|---|---|
| SBG5-fw: | 5'-ATT CTG GTG CTG ACC TCG CCA C -3' (Seq ID Nr. 25) |
| SBG5-rv: | 5'-ACT CAT GTC GTA CTT GCA AGA ACT G -3' (Seq ID Nr. 26) |

Folgende Parameter werden für die PCR eingesetzt: 1 x: initiale Denaturierung, 98 °C, 3 min; 25 x: Denaturierung, 98 °C, 0:10 min, Annealing, 60 °C, 0:30 min; Elongation, 72 °C, 2:00 min; 1 x: terminale Elongation, 72 °C, 10 min. Für die Amplifikation wird der Phusion™ High-Fidelity Master Mix von New England Biolabs (Frankfurt) entsprechend den Empfehlungen des Herstellers verwendet. Die PCR-Produkte werden mittels des QIAquick PCR-Purification Kit (Qiagen, Hilden) gemäß den Angaben des Herstellers aufgereinigt. Die Durchführung der PCR, die Überprüfung der erfolgreichen Amplifikation der PCR mittels Agarosegel-Elektrophorese, Ethidiumbromidfärbung der DNA, Bestimmung der PCR-Fragmentgrößen, Reinigung der PCR-Produkte und DNA-Konzentrationsbestimmung erfolgt in dem Fachmann bekannter Art und Weise.

Die Transformation von *C*. *bombicola* ATCC 22214 *ura*⁻ erfolgt wie vorbeschrieben (van Bogaert et al. Yeast. 2008. 25:273-278); van Bogaert et al. FEMS Yeast Res. 2009. 9:610-617).

Um die Deletion der Gene *SBG1, SBG2, SBG3, SBG4* und *SBG5* in *C*. *bombicola* ATCC 22214 *ura*⁻ -Transformanten nach Transformation mit den Deletionskassetten für *CbSBG1* (Seq ID Nr. 12), *CbSBG2* (Seq ID Nr. 13), *CbSBG3* (Seq ID Nr. 14), *CbSBG4* (Seq ID Nr. 15) und *CbSBG5* (Seq ID Nr. 16) zu überprüfen, werden von jeweils 5 Transformanten und *C*. *bombicola* ATCC 22214 *ura*⁻ die jeweiligen Loci per Kolonie-PCR amplifiziert. Dabei kommen folgende Oligonukleotide zum Einsatz:

**Kontrolle der genomischen Deletion von CbSBG1:**

| | |
|---|---|
| SBG1-KO-fw: | 5'- GTG TCG ACT CGC CAA ATT CCA TCG GAG -3' (Seq ID Nr. 27) |
| SBG1-KO-rv: | 5'- GGT TCA TAG CGA GTT TCT TTG CAT GTG C -3' (Seq ID Nr. 28) |

**Kontrolle der genomischen Deletion von CbSBG2:**

| | |
|---|---|
| SBG2-KO-fw: | 5'- CTC CTT TAT TAA CTC CGC AGC ATG ACT G -3' (Seq ID Nr. 29) |
| SBG2-KO-rv: | 5'- CTC CTC GAA GGA CCC TCA AAA CAA AGG -3' (Seq ID Nr. 30) |

**Kontrolle der genomischen Deletion von CbSBG3:**

| | |
|---|---|
| SBG3-KO-fw: | 5'- CAA ATT TAT CTG GGA GCA CAG TTA CAT TGC -3' (Seq ID Nr. 31) |
| SBG3-KO-rv: | 5'- CAC ACA TTG CTT TAG TCC AGC AAG AAC C -3' (Seq ID Nr. 32) |

**Kontrolle der genomischen Deletion von CbSBG4:**

| | |
|---|---|
| SBG4-KO-fw: | 5'-ATT CTC CTC GCA CGT TTC TCG GGG C -3' (Seq ID Nr. 33) |
| SBG4-KO-rv: | 5'- GGT TGA AAT ACT TGT TGC CGC ACT AAA G -3' (Seq ID Nr. 34) |

**Kontrolle der genomischen Deletion von CbSBG5:**

| | |
|---|---|
| SBG5-KO-fw: | 5'- CGC TTC CTG AAT TGA GTT GGT ATC GTT AAT G -3' (Seq ID Nr. 35) |
| SBG5-KO-rv: | 5'- GAC ATT GTT GGA ATT GGC TGC TTA GTG G -3' (Seq ID Nr. 36) |

Folgende Parameter werden für die PCR eingesetzt: 1 x: initiale Denaturierung, 94 °C, 3 min; 25 x: Denaturierung, 94 °C, 1:00 min, Annealing, 60 °C, 1:00 min; Elongation, 72 °C, 5:00 min; 1 x: terminale Elongation, 72 °C, 10 min. Für die Amplifikation wird der Taq PCR Master Mix Kit von QIAGEN (Hilden) entsprechend den Empfehlungen des Herstellers verwendet. Jeweils 10 µl der PCR-Reaktionen werden anschließend auf einem 0,8 %igen Agarosegel aufgetrennt. Die Durchführung der PCR, der Agarosegel-Elektrophorese, Ethidiumbromidfärbung der DNA und Bestimmung der PCR-Fragmentgrößen erfolgt in dem Fachmann bekannter Art und Weise.

Bei der Amplifikation der entsprechenden Loci sollten die in Tabelle 3 angegebenen PCR-Fragmentgrößen entstehen:

**Tabelle 3: Zu erwartende PCR-Fragmentgrößen bei Amplifikation der chromosomalen SBG1-, SBG2-, SBG3-, SBG4- und SBG5-Loci bei erfolgreicher Deletion bzw. Wildtypsituation.**

| Gen | Größe des PCR-Produktes bei chromosomaler Deletion | Größe des PCR-Produktes bei Wildtyp-Situation |
|---|---|---|
| *SBG1* | 4201 bp | 3678 bp |
| *SBG2* | 4199 bp | 3451 bp |
| *SBG3* | 4199 bp | 2839 bp |
| *SBG4* | 4190 bp | 5950 bp |
| *SBG5* | 4201 bp | 3360 bp |

Bei der Amplifikation der *CbSBG1-, CbSBG2-, CbSBG3-, CbSBG4-*bzw. *CbSBG5*-Loci aus *C. bombicola* ATCC 22214 *ura*⁻ werden ausschließlich die bei Vorliegen einer Wildtypsituation zu erwartenden Fragmentgrößen von etwa 3,7 kbp, 3,5 kbp, 2,8 kbp, 5,9 kbp bzw. 3,4 kbp erzielt.

Bei der Amplifikation des *SBG1*-Locus aus Transformanten nach Transformation der Deletionskassette für *CbSBG1* wird ausschließlich die nach erfolgreicher chromosomaler Deletion von *CbSBG1* zu erwartende Fragmentgröße von etwa 4,2 kbp erzielt.

Bei der Amplifikation des *SBG2*-Locus aus Transformanten nach Transformation der Deletionskassette für *CbSBG2* wird ausschließlich die nach erfolgreicher chromosomaler Deletion von *CbSBG2* zu erwartende Fragmentgröße von etwa 4,2 kbp erzielt.

Bei der Amplifikation des *SBG3*-Locus aus Transformanten nach Transformation der Deletionskassette für *CbSBG3* wird ausschließlich die nach erfolgreicher chromosomaler Deletion von *CbSBG3* zu erwartende Fragmentgröße von etwa 4,2 kbp erzielt.

Bei der Amplifikation des *SBG4*-Locus aus Transformanten nach Transformation der Deletionskassette für *CbSBG4* wird ausschließlich die nach erfolgreicher chromosomaler Deletion von *CbSBG4* zu erwartende Fragmentgröße von etwa 4,2 kbp erzielt.

Bei der Amplifikation des *SBG5*-Locus aus Transformanten nach Transformation der Deletionskassette für *CbSBG5* wird ausschließlich die nach erfolgreicher chromosomaler Deletion von *CbSBG5* zu erwartende Fragmentgröße von etwa 4,2 kbp erzielt. Somit können in allen fünf Fällen Klone identifiziert werden, in denen die Gene *CbSBG1, CbSBG2, CbSBG3, CbSBG4* oder *CbSBG5* chromosomal deletiert wurden. Die entsprechenden Stämme werden im weiteren als *C. bombicola* ATCC 22214 *sbg1, C. bombicola* ATCC 22214 *sbg2, C. bombicola* ATCC 22214 *sbg3, C. bombicola* ATCC 22214 *sbg4* bzw. *C. bombicola* ATCC 22214 *sbg5* bezeichnet.

### Beispiel 3: Charakterisierung der Sophorolipidbildung durch C. bombicola ATCC 22214, C. bombicola ATCC 22214 sbg1, C. bombicola ATCC 22214 sbg2, C. bombicola ATCC 22214 sbg3, C. bombicola ATCC 22214 sbg4 und C. bombicola ATCC 22214 sbg5.

Die Propagierung der Stämme *C*. *bombicola* ATCC 22214, *C*. *bombicola* ATCC 22214 *sbg1, C. bombicola* ATCC 22214 *sbg2, C. bombicola* ATCC 22214 *sbg3, C. bombicola* ATCC 22214 *sbg4* und *C*. *bombicola* ATCC 22214 *sbg5* erfolgt auf YPD-Agar-Platten. Zur Produktion der Sophorolipide wird das im Folgenden als SL-Produktionsmedium bezeichnete Medium verwendet. Dieses besteht aus 0,1 % KH₂PO₄, 0,5 % MgSO₄ x 7 H₂O, 0,01 % FeCl₃, 0,01 % NaCl, 0,01 % Uracil, 0,4 % Hefeextrakt, 0,1 % Urea, 10,5 % Rapsöl und 10 % Glucose. Der pH-Wert wird auf 4,5 eingestellt und das Medium folgend mittels Autoklav (121 °C, 20 min) sterilisiert. Ein Einstellen des pH-Wertes während der Kultivierung ist nicht nötig.

Zur Untersuchung der Sophorolipidproduktion im Schüttelkolben wird zunächst eine Vorkultur angesetzt. Hierzu wird eine Impföse eines frisch auf YPD-Agarplatte ausgestrichenen Stammes verwendet und 10 ml YPD-Medium in einem 100 ml Erlenmeyerkolben beimpft. Die Kultivierung erfolgt bei 30 °C und 200 rpm über Nacht. Diese Vorkultur wird folgend verwendet, um 100 ml SL-Medium im 1000 ml Erlenmeyerkolben zu beimpfen (Start-OD₆₀₀ 0,2). Die Kulturen werden bei 200 rpm und 30 °C für 7 Tage kultiviert und jeden Tag eine Probe von 2 ml Brühe genommen, wobei strikt auf gute Durchmischung des Kulturmediums vor Probenahme geachtet wird.

Die Probenvorbereitung für die nachfolgenden chromatographischen Analysen erfolgt folgendermaßen: mit einer Verdrängerpipette (Combitip) werden in einem 2 ml-Reaktionsgefäß 800 µl Aceton vorgelegt und das Reaktionsgefäß zur Minimierung von Verdunstung unmittelbar verschlossen. Es folgt die Zugabe von 200 µl Brühe. Nach Vortexen des Brühe/Acetongemisches wird dieses für 1 min bei 13.000 rpm abzentrifugiert, und 800 µl des Überstands in ein HPLC-Gefäß überführt.

Zum Nachweis und zur Quantifizierung von Sophorolipiden bzw. Ölsäure wird ein Evaporative Light Scattering Detektor (ELSD) benutzt. Die eigentliche Messung wird mittels Agilent Technologies 1200 Series (Santa Clara, Kalifornien) und der Zorbax SB-C8 Rapid Resolution Säule (4,6 x 150 mm, 3,5 µm, Agilent) durchgeführt. Das Injektionsvolumen beträgt 5 µl und die Laufzeit der Methode liegt bei 20 min. Als mobile Phase wird H₂O und 0,1 % TFA (Trifluor-Essigsäure, Lösung A) und Methanol (Lösung B) verwendet. Die Säulentemperatur beträgt 40 °C. Als Detektoren dienten der ELSD (Detektortemperatur 60 °C) und der DAD (Diodenarray, 210 nm). Der in der Methode verwendete Gradient ist in Tabelle 4 dargestellt.

**Tabelle 4: Beschreibung des für die HPLC-basierte Quantifizierung von Sophorolipiden zu verwendenden Gradientenprofils der mobilen Phase.**

| **t [min]** | **Lösung B %** | **Fluss [ml/min]** |
|---|---|---|
| 0,00 | 70% | 1,00 |
| 15,00 | 100% | 1,00 |
| 15,01 | 70% | 1,00 |
| 20,00 | 70% | 1,00 |

Während *C*. *bombicola* ATCC 22214 Sophorolipide produziert, ist bei den Stämmen *C*. *bombicola* ATCC 22214 *sbg1, C. bombicola* ATCC 22214 *sbg2* und *C*. *bombicola* ATCC 22214 *sbg4* keine Sophorolipidbildung nachweisbar. Dies zeigt eindeutig, dass diese Gene an der Sophorolipidbildung beteiligt sind und dabei die oben angegebenen Funktionen erfüllen. Die Stämme *C*. *bombicola* ATCC 22214 *sbg3* und *C*. *bombicola* ATCC 22214 *sbg5* sind in der Lage Sophorolipide zu bilden, jedoch zeigen diese eine veränderte Retentionszeit in der HPLC-Analyse.

Per LC-MS² kann nachgewiesen werden, dass die durch *C*. *bombicola* ATCC 22214 *sbg3* gebildeten Sophorolipide im Gegensatz zu den durch *C*. *bombicola* ATCC 22214 gebildeten Sophorolipiden ausschließlich Verbindungen der allgemeinen Formel (Ia) und (Ib) entsprechen, bei denen R¹ = H und R² = H.
Dies beweist die Funktion von Sbg3p als Acetyltransferase (E₄) in der Sophorolipidbiosynthese.

Per LC-MS kann ebenfalls nachgewiesen werden, dass die durch *C*. *bombicola* ATCC 22214 *sbg5* gebildeten Sophorolipide im Gegensatz zu den durch *C*. *bombicola* ATCC 22214 gebildeten Sophorolipiden ausschließlich Verbindungen der allgemeinen Formel (Ia) entsprechen, bei denen n = 0 ist.

Dies beweist die Funktion von Sbg5p als Glycosyltransferase II (E₃) in der Sophorolipidbiosynthese.

### Beispiel 4: Konstruktion von Candida bombicola ATCC 22214-Stämmen, die die an der Sophorolipid-Biosynthese beteiligten Enzyme überproduzieren

Um die Konstruktion von *Candida bombicola* ATCC 22214-Stämmen, die die an der Sophorolipid-Biosynthese beteiligten Enzyme überproduzieren, zu ermöglichen, wird zunächst eine Integrations/Überexpressionskassette durch die GeneArt AG synthetisiert (Seq ID Nr. 75).

Diese Integrations/Überexpressionskassette enthält die in Tabelle 5 angegebenen Komponenten:

**Tabelle 5: Übersicht über die in der für Candida bombicola ATCC 22214 zu entwickelnden Integrations/Überexpressionskassette vorhandenen Module und wichtigen Restriktionsschnittstellen.**

| Position (bp) | Komponente |
|---|---|
| 1 - 8 | *Not*I-Erkennungsstelle |
| 9 - 507 | DNA-Abschnitt stromaufwärts des *C. bombicola* ATCC 22214 *LEU2*-Gens |
| 508 - 513 | *Pci*I-Erkennungsstelle |
| 514 - 1217 | Promotorbereich des *C. bombicola* ATCC 22214 *URA3*-Gens |
| 1217 - 2005 | Kodierender Bereich des *C. bombicola* ATCC 22214 *URA3*-Gens |
| 2006 - 2586 | Terminatorbereich des *C. bombicola* ATCC 22214 *URA3*-Gens |
| 2587 - 2592 | *Pci*I-Erkennungsstelle |
| 2593 - 2600 | *Asi*SI-Erkennungsstelle |
| 2601 - 3012 | Promotorbereich des *C. bombicola* ATCC 22214 *TSC3*-Gens |
| 3011 - 3016 | *Nde*I-Erkennungsstelle |
| 3025 - 3032 | *Fse*I-Erkennungsstelle |
| 3033 - 3210 | Terminatorbereich des *C. bombicola* ATCC 22214 *TSC3*-Gens |
| 3211 - 3218 | *Asi*SI-Erkennungsstelle |
| 3219 - 3224 | *Mlu*I-Erkennungsstelle |
| 3225 - 3724 | DNA-Abschnitt stromabwärts des *C. bombicola* ATCC 22214 *LEU2*-Gens |
| 3725 - 3732 | *Sbf*I-Erkennungsstelle |

Diese Integrations/Überexpressionskassette ermöglicht die Insertion beliebiger Strukturgene von Startcodon bis Stopcodon via *Nde*I und *Fse*I zwischen Promotor- und Terminatorbereich des *C. bombicola* ATCC 22214 *TSC3*-Gens, welches für die Glyceraldehyd-3-Phosphat-Dehydrogenase kodiert (van Bogaert *et al*.; 2008). Glyceraldehyd-3-Phosphat-Dehydrogenase ist in vielen Hefen ein hochabundantes Protein, so dass davon ausgegangen werden kann, dass auf diese Art und Weise eine starke Expression des inserierten Gens erzielt werden kann. Als Selektionsmarker wird das *C. bombicola* ATCC 22214 *URA3-*Gen gewählt, so dass diese Integrations/Überexpressionskassette nur für die Transformation von Uracil-auxotrophen Stämmen von *C*. *bombicola* ATCC 22214 genutzt werden kann. Deren Generierung sowie das *C. bombicola* ATCC 22214 *URA3-*Gen wurden bereits vorbeschrieben (van Bogaert et *al.,* 2007; van Bogaert et *al.,* 2008). Die 5'- und 3'-terminalen DNA-Abschnitte erlauben die Insertion der Kassette am *C. bombicola* ATCC 22214 *LEU2*-Locus (Seq ID Nr. 37), wobei das *LEU2-Gen* inaktiviert wird. *LEU2*-kodiert für die einzige Isopropylmalat-Dehydrogenase in *C*. *bombicola* ATCC 22214. Da die Isopropylmalat-Dehydrogenase essentieller Bestandteil der Leucin-Biosynthese ist, können Transformanten mit korrekter Integration der Integrations/Überexpressionskassette über ihre Leucin-Auxotrophie identifiziert werden. Diverse unikale und redundante Erkennungssequenzen (*NotI, Pci*I, *Ase*SI*, Mlu*I*, Sbf*I) erlauben den Austausch einzelner Module der Integrations/Überexpressionskassette. Die Kassette wird von der GeneArt AG in den proprietären Vektor pMA kloniert, der keine der oben beschriebenen Schnittstellen enthält, so dass diese Schnittstellen in vollem Umfang genutzt werden können.

Zur Insertion der Gene *CbSBG1, CbSBG3* und *CbSBG5* in die beschriebene Integrations/Überexpressionskassette werden diese aus chromosomaler DNA von *C*. *bombicola* ATCC 22214 per PCR amplifiziert und gleichzeitig über die verwendeten Oligonukleotide eine *Nde*I-Schnittstelle stromaufwärts des Startcodons und eine *Fse*I-Schnittstelle stromabwärts des Stopcodons eingeführt. Zur Insertion der Gene *CbSBG2* und *CbSBG4* in die beschriebene Integrations/ Überexpressionskassette werden diese zunächst von der GeneArt AG (Regensburg) *de novo* synthetisiert, um deren Sequenz so zu modifizieren, dass die internen *Fse*I und *Not*I-Schnittstellen (*CbSBG2*) bzw. *Nde*I-Schnittstellen (*CbSBG4*) entfernt werden, ohne die Aminosäuresequenz des kodierten Proteins zu verändern. Im Anschluss werden die durch die GeneArt AG (Regensburg) bereit gestellten modifizierten Gene *CbSBG2mod* und *CbSBG4mod* per PCR amplifiziert und gleichzeitig über die verwendeten Oligonukleotide eine *Nde*I-Schnittstelle stromaufwärts des Startcodons und eine *Fse*I-Schnittstelle stromabwärts des Stopcodons eingeführt. Dabei kommen folgende Oligonukleotide zum Einsatz:

**CbSBG1:**

| | |
|---|---|
| SBG1-OE-fw: | 5'-ATA TAT ATA CAT ATG TTA ATC AAA GAC ATT ATT CTA ACT CCA ATG-3' (Seq ID Nr. 38) |
| SBG1-OE-rv: | 5'-ATA TAT GGC CGG CCA ACT TAA GAA AAC CGC ACA ACC ACA CCG-3' (Seq ID Nr. 39) |

**CbSBG2mod:**

| | |
|---|---|
| SBG2-OE-fw: | 5'-ATA TAT ATA CAT ATG AGC CCT TCA TCA CAC AAA CCC CTG -3' (Seq ID Nr. 40) |
| SBG2-OE-rv: | 5'-ATA TAT GGC CGG CCA TTC TAA GAA CTC ACC GCT AAG GCC -3' (Seq ID Nr. 41) |

**CbSBG3:**

| | |
|---|---|
| SBG3-OE-fw: | 5'-ATA TAT ATA CAT ATG GTT GTA AAC TCC TCG AAG GAC CC-3' (Seq ID Nr. 42) |
| SBG3-OE-rv: | 5'-ATA TAT GGC CGG CCT ACC TAG ACC TTC TGG TTA GCG GTA TTG -3' (Seq ID Nr. 43) |

**CbSBG4mod:**

| | |
|---|---|
| SBG4-OE-fw: | 5'-ATA TAT ATA CAT ATG GTG GAT GAT ATA CAG GTA GAG AAG C-3' (Seq ID Nr. 44) |
| SBG4-OE-rv: | 5'-ATA TAT GGC CGG CCA CGT CAA ATC TCT CCG AGA CCT TGC AAG -3' (Seq ID Nr. 45) |

**CbSBG5 :**

| | |
|---|---|
| SBG5-OE-fw: | 5'-ATA TAT ATA CAT ATG GCC ATC GAG AAA CCA GTG ATA GTT G -3' (Seq ID Nr. 46) |
| SBG5-OE-rv: | 5'-ATA TAT GGC CGG CCA GGT TAA GAA GCT AAT TCA CTA ATT GCC GAC -3' (Seq ID Nr. 47) |

Folgende Parameter werden für die PCR eingesetzt: 1 x: initiale Denaturierung, 98 °C, 3 min; 25 x: Denaturierung, 98 °C, 0:10 min, Annealing, 60 °C, 0:30 min; Elongation, 72 °C, 2:00 min; 1 x: terminale Elongation, 72 °C, 10 min. Für die Amplifikation wird der Phusion™ High-Fidelity Master Mix von New England Biolabs (Frankfurt) entsprechend den Empfehlungen des Herstellers verwendet. Jeweils 10 µl der PCR-Reaktionen werden anschließend auf einem 0,8 %igen Agarosegel aufgetrennt. Die Durchführung der PCR, der Agarosegel-Elektrophorese, Ethidiumbromidfärbung der DNA und Bestimmung der PCR-Fragmentgrößen erfolgt in dem Fachmann bekannter Art und Weise. In allen Fällen können PCR-Fragmente der erwarteten Größe amplifiziert werden. Diese betragen für *CbSBG1* 1646 bp, für *CbSBG2* 1421 bp, für *CbSBG3* 809 bp, für *CbSBG4* 3929 bp und für *CbSBG5* 1328 bp. Die PCR-Produkte werden mit NdeI und FseI entsprechend den Empfehlungen des Herstellers der Restriktionsendonukleasen (New England Biolabs; Frankfurt/Main) verdaut und in den mit NdeI und FseI geschnittenen Vektor pMA-ExCat (Seq ID Nr. 64) ligiert. Ligation sowie Transformation chemisch kompetenter *E. coli* DH5α-Zellen (New England Biolabs; Frankfurt/Main) erfolgen in dem Fachmann bekannter Art und Weise. Die korrekte Insertion der *CbSBG1-, CbSBG2-, CbSBG3-, CbSBG4-* bzw. *CbSBG5*-Fragmente in pMA-ExCat wird durch eine Restriktion mit *Nde*I und *Fse*I überprüft und bestätigt. Die erhaltenen Vektoren werden als pMA_ExCat-*CbSBG1* (Seq ID Nr. 65), *pMA_ExCat-CbSBG2* (Seq ID Nr. 66), *pMA_ExCat-CbSBG3* (Seq ID Nr. 67), *pMA_ExCat-CbSBG4* (Seq ID Nr. 68) und *pMA_ExCat-CbSBG5* (Seq ID Nr. 69) bezeichnet.

Um die einzelnen Integrations/Überexpressionskassetten sowie die Kontrollkassette ExCat für die anschließende Transformation von *C. bombicola* ATCC 22214 *ura*⁻ ausreichender Menge zur Verfügung zu stellen, werden diese per PCR amplifiziert. Dabei kommen folgende Oligonukleotide zum Einsatz:

| | |
|---|---|
| OEx-LEU2-fw: | 5'- GGA CCT GCG CCC TAA AAT GGG AC -3' (Seq ID Nr. 48) |
| OEx-LEU2-rv: | 5'-ATC CTA GAA AAC AGC TGG ATA TGG ATA AAC -3' (Seq ID Nr. 49) |

Die PCR-Produkte werden mittels des QIAquick PCR-Purification Kit (Qiagen, Hilden) gemäß den Angaben des Herstellers aufgereinigt. Die Durchführung der PCR, die Überprüfung der erfolgreichen Amplifikation der PCR mittels Agarosegel-Elektrophorese, Ethidiumbromidfärbung der DNA, Bestimmung der PCR-Fragmentgrößen, Reinigung der PCR-Produkte und DNA-Konzentrationsbestimmung erfolgt in dem Fachmann bekannter Art und Weise.

Die erhaltenen Integrations/Überexpressionskassetten erhalten die Namen IntEx-*CbSBG1* (Seq ID Nr. 70), IntEx-CbSBG2 (Seq ID Nr. 71), IntEx*-CbSBG3* (Seq ID Nr. 72), IntEx*-CbSBG4* (Seq ID Nr. 73) und IntEx-*CbSBG5* (Seq ID Nr. 74). Ebenfalls erhalten wird die Kontrollkassette ExCat (Seq ID Nr. 75).

Die Transformation von *C*. *bombicola* ATCC 22214 *ura*⁻ erfolgt wie vorbeschrieben (van Bogaert et al. Yeast. 2008. 25:273-278); van Bogaert et al. FEMS Yeast Res. 2009. 9:610-617).

Um die Insertion der Integrations/Überexpressionskassetten für die Überexpression *CbSBG1, CbSBG2, CbSBG3, CbSBG4* und *CbSBG5* sowie der Kontrollkassette ExCat in den *LEU2*-Locus von *C*. *bombicola* ATCC 22214 *ura*⁻ zu überprüfen, wird von jeweils 5 Transformanten (nach Transformation der Integrations/Überexpressionskassetten für *CbSBG1, CbSBG2, CbSBG3, CbSBG4* und *CbSBG5* sowie der Kontrollkassette ExCat) und *C. bombicola* ATCC 22214 *ura*⁻ der *LEU2*-Locus per Kolonie-PCR amplifiziert. Dabei kommen folgende Oligonukleotide zum Einsatz:

| | |
|---|---|
| LEU2-KI-fw: | 5'- GTG CCC GAC CAC CAT GAG CTG TC -3' (Seq ID Nr. 50) |
| LEU2-KI-rv: | 5'- CCC AAG CAT GAG GGT CGT GCC GG -3' (Seq ID Nr. 51) |

Folgende Parameter werden für die PCR eingesetzt: 1 x: initiale Denaturierung, 94 °C, 3 min; 25 x: Denaturierung, 94 °C, 1:00 min, Annealing, 60 °C, 1:00 min; Elongation, 72 °C, 5:00 min; 1 x: terminale Elongation, 72 °C, 10 min. Für die Amplifikation wird der Taq PCR Master Mix Kit von QIAGEN (Hilden) entsprechend den Empfehlungen des Herstellers verwendet. Jeweils 10 µl der PCR-Reaktionen werden anschließend auf einem 0,8 %igen Agarosegel aufgetrennt. Die Durchführung der PCR, der Agarosegel-Elektrophorese, Ethidiumbromidfärbung der DNA und Bestimmung der PCR-Fragmentgrößen erfolgt in dem Fachmann bekannter Art und Weise.

Bei der Amplifikation der entsprechenden Loci sollen die in Tabelle 6 angegebenen PCR-Fragmentgrößen entstehen:

**Tabelle 6: Zu erwartende PCR-Fragmentgrößen bei Amplifikation des chromosomalem LEU2-Locus nach homologer Rekombination der SBG1-, SBG2-, SBG3-, SBG4- und SBG5-Expressionskassetten sowie der Kontrollkassette ExCat in den chromosomalen C. bombicola LEU2-Locus bzw. bei nicht-homologer Integration.**

| Gen | Größe des PCR-Produktes bei homologer Integration in den *CbLEU2*-Locus | Größe des PCR-Produktes bei nicht-homologer Integration an anderer Stelle des Genoms |
|---|---|---|
| *SBG1* | 5452 bp | 2235 bp |
| *SBG2* | 5227 bp | 2235 bp |
| *SBG3* | 4615 bp | 2235 bp |
| *SBG4* | 7735 bp | 2235 bp |
| *SBG5* | 5125 bp | 2235 bp |
| ExCat | 3844 bp | 2235 bp |

Bei der Amplifikation des *LEU2*-Locus aus *C. bombicola* ATCC 22214 *ura*⁻ wird ausschließlich das bei Vorliegen der Wildtypsituation zu erwartende Fragment mit einer Größe von 2,2 kbp erzielt.

Bei der Amplifikation des *LEU2*-Locus aus *C. bombicola* ATCC 22214-Transformanten nach Transformation mit den Integrations/Überexpressionskassetten für die Überexpression von *CbSBG1, CbSBG2 mod, CbSBG3, CbSBG4 mod* und *CbSBG5* werden ausschließlich die bei erfolgreicher chromosomaler Integration der Integrations/ Überexpressionskassetten IntEx-*CbSBG1* (Seq ID Nr. 70), IntEx-*CbSBG2* (Seq ID Nr. 71), IntEx*-CbSBG3* (Seq ID Nr. 72), IntEx-*CbSBG4* (Seq ID Nr. 73) und IntEx*-CbSBG5* (Seq ID Nr. 74) zu erwartenden Fragmentgrößen von etwa 5,5 kbp, 5,2 kbp, 4,6 kbp, 7,7 kbp bzw. 5,1 kbp erzielt.

Somit können in allen fünf Fällen Klone identifiziert werden, in denen die Gene *CbSBG1, CbSBG2, CbSBG3, CbSBG4* oder *CbSBG5* unter Kontrolle des *C*. *bombicola* ATCC 22214 TSC3-Promotors gebracht werden konnten, so dass deren Überexpression postuliert werden kann.

Die entsprechenden Stämme werden im weiteren als C. *bombicola* ATCC 22214 *P_{TSC3}-SBG1-T_{TSC3}, C. bombicola* ATCC 22214 *P_{TSC3}-SBG2-T_{TSC3}, C. bombicola* ATCC 22214 *P_{TSC3}-SBG3-T_{TSC3}, C. bombicola* ATCC 22214 *P_{TSC3}-SBG4-T_{TSC3}* und *C*. *bombicola* ATCC 22214 *P_{TSC3}-SBG5-T_{TSC3}* bezeichnet.

### Beispiel 5: Charakterisierung der Sophorolipidbildung durch C. bombicola ATCC 22214 ExCat, C. bombicola ATCC 22214 P_{TSC3}-SBG1-T_{TSC3}, C. bombicola ATCC 22214 P_{TSC3}-SBG2-T_{TSC3}, C. bombicola ATCC 22214 P_{TSC3}-SBG3-T_{TSC3}, C. bombicola ATCC 22214 P_{TSC3}-SBG4-T_{TSC3} und C. bombicola ATCC 22214 P_{TSC3}-SBG5-T_{TSC3}

Die Propagierung der Stämme *C*. *bombicola* ATCC 22214 ExCat, *C*. *bombicola* ATCC 22214 *P_{TSC3}-SBG1-T_{TSC3}, C. bombicola* ATCC 22214 *P_{TSC3}-SBG2-T_{TSC3}, C*. *bombicola* ATCC 22214 *P_{TSC3}-SBG3-T_{TSC3}, C. bombicola* ATCC 22214 *P_{TSC3}-SBG4-T_{TSC3} und C. bombicola* ATCC 22214 *P_{TSC3}-SBG5-T_{TSC3}* erfolgt auf YPD-Agar-Platten. Zur Produktion der Sophorolipide wird das im Folgenden als SL-Produktionsmedium bezeichnete Medium verwendet. Dieses besteht aus 0,1 % KH₂PO₄, 0,5 % MgSO₄ x 7 H₂O, 0,01 % FeCl₃, 0,01 % NaCl, 0,01 % Uracil, 0,4 % Hefeextrakt, 0,1 % Urea, 10,5 % Rapsöl und 10 % Glucose. Der pH-Wert wird auf 4,5 eingestellt und das Medium folgend mittels Autoklav (121 °C, 20 min) sterilisiert. Ein Einstellen des pH-Wertes während der Kultivierung ist nicht nötig.

Zur Untersuchung der Sophorolipidproduktion im Schüttelkolben wird zunächst eine Vorkultur angesetzt. Hierzu wird eine Impföse eines frisch auf YPD-Agarplatte ausgestrichenen Stammes verwendet und 10 ml YPD-Medium in einem 100 ml Erlenmeyerkolben beimpft. Die Kultivierung erfolgt bei 30 °C und 200 rpm über Nacht. Diese Vorkultur wird folgend verwendet um 100 ml SL-Medium im 1000 ml Erlenmeyerkolben zu beimpfen (Start-OD₆₀₀ 0,2). Die Kulturen werden bei 200 rpm und 30 °C für 7 Tage kultiviert und jeden Tag eine Probe von 2 ml Brühe genommen, wobei strikt auf gute Durchmischung des Kulturmediums vor Probenahme geachtet wird.

Die Probenvorbereitung für die nachfolgenden chromatographischen Analytiken erfolgt folgendermaßen: mit einer Verdrängerpipette (Combitip) werden in einem 2 ml-Reaktionsgefäß 800 µl Aceton vorgelegt und das Reaktionsgefäß zur Minimierung von Verdunstung unmittelbar verschlossen. Es folgt die Zugabe von 200 µl Brühe. Nach Vortexen des Brühe/Acetongemisches wird dieses für 1 min bei 13.000 rpm abzentrifugiert, und 800 µl des Überstands in ein HPLC-Gefäß überführt.

Zum Nachweis und zur Quantifizierung von Sophorolipiden bzw. Ölsäure wird ein Evaporative Light Scattering Detektor (ELSD) benutzt. Die eigentliche Messung wird mittels Agilent Technologies 1200 Series (Santa Clara, Kalifornien) und der Zorbax SB-C8 Rapid Resolution Säule (4,6 x 150 mm, 3,5 µm, Agilent) durchgeführt. Das Injektionsvolumen beträgt 5 µl und die Laufzeit der Methode liegt bei 20 min. Als mobile Phase wird H₂O und 0,1 % TFA (Trifluor-Essigsäure, Lösung A) und Methanol (Lösung B) verwendet. Die Säulentemperatur beträgt 40 °C. Als Detektoren dient der ELSD (Detektortemperatur 60 °C) und der DAD (Diodenarray, 210 nm). Der in der Methode verwendete Gradient ist in Tabelle 3 dargestellt.

Wie der Kontrollstamm *C*. *bombicola* ATCC 22214 ExCat produzieren alle Stämme Sophorolipide. Jedoch weisen die Stämme *C*. *bombicola* ATCC 22214 *P_{TSC3}-SBG1-T_{TSC3}, C. bombicola* ATCC 22214 *P_{TSC3}-SBG2-T_{TSC3}, C. bombicola* ATCC 22214 *P_{TSC3}-SBG3-T_{TSC3}, C. bombicola* ATCC 22214 *P_{TSC3}-SBG4-T_{TSC3}* und *C*. *bombicola* ATCC 22214 *P_{TSC3}-SBG5-T_{TSC3}* gegenüber *C*. *bombicola* ATCC 22214 ExCat eine erhöhte Raum-ZeitAusbeute der Sophorolipidbildung auf. Während *C*. *bombicola* ATCC 22214 ExCat unter den gewählten Bedingungen etwa 2 mg Sophorolipide pro Liter, Stunde und OD₆₀₀ produziert, liegt dieser Parameter bei den Stämmen *C*. *bombicola* ATCC 22214 *P_{TSC3}*-*SBG1-T_{TSC3}, C. bombicola* ATCC 22214 *P_{TSC3}-SBG2-T_{TSC3}, C. bombicola* ATCC 22214 *P_{TSC3}-SBG3-T_{TSC3}, C. bombicola* ATCC 22214 *P_{TSC3}-SBG4-T_{TSC3}* und *C*. *bombicola* ATCC 22214 *P_{TSC3}-SBG5-T_{TSC3}* zwischen 2,5 mg und 6 mg. Somit kann gezeigt werden, dass die Verstärkung der Enzyme *CbSBG1, CbSBG2, CbSBG3, CbSBG4* und *CbSBG5* in *C*. *bombicola* ATCC 22214 zu einer Steigerung der Sophorolipid-Bildung führt.

### Beispiel 6: Vektor pTZ_E02_His-GlcTrI zur Überexpression des Candida bombicola Gens SBG2 mit N-terminalem His-tag

Zur Überexpression des *Candida bombicola* ATCC22214 Gens *SBG2* (Seq ID Nr. 03) in *Escherichia coli* wurde das Plasmid pTZ_E02_His-GlcTrI konstruiert. Chromosomale DNA aus *Candida bombicola* ATCC22214 diente als Matrize für eine PCR mit dem "Expand™ High Fidelity"-PCR-Kit von Roche Diagnostics (Mannheim), gemäß Angaben des Herstellers. Das *SBG2-Gen* wurde mit Hilfe der Oligonukleotide 1373_GlcTrI_BsmBI_His_fp (Seq ID Nr. 76) und 1373_GlcTrI_AscI_rp (Seq ID Nr. 77) aus der chromosomalen DNA amplifiziert ("PCR protocols. A guide to methods and applications", 1990, Academic Press) und auf diesem Wege am 5'-Ende mit einem 6-fachen N-terminalen Histidin-Tag versehen. Außerdem wurden die Schnittstellen *Bsm*BI und *Asc*I eingefügt. Dafür wurden die folgenden Oligonukleotide eingesetzt:
1373_GlcTrI_BsmBI_His_fp (Seq ID Nr. 76):
   5' -AAACGTCTCAGATG*CACCACCACCACCACCAC*ATGGTTGTAAACTCCTCG-3'
1373_GlcTrI_AscI_rp (Seq ID Nr. 77):
   5'-AAAGGCGCGCCCTAGACCTTCTGGTTAGCG-3'

Das PCR-Produkt (1435 bp) wurde mittels des QIAquick PCR-Purification Kits (Qiagen, Hilden) gemäß Angaben des Herstellers aufgereinigt, mit *BsmBI* und AscI geschnitten und anschließend in den auf die gleiche Weise geschnittenen Expressionsvektor pTZ_E02 (pET24d-basierter Vektor; Merck Chemicals, Darmstadt) der Firma Trenzyme GmbH, Konstanz, ligiert. Das resultierende Plasmid pTZ_E02_His-GlcTrI (Seq ID Nr. 78) ist 6700 Basenpaare groß. Die Ligation sowie die Transformation chemisch kompetenter *E. coli* DH5α-Zellen (Gibco-BRL, Karlsruhe) erfolgten in dem Fachmann bekannter Art und Weise. Die Authentizität des Inserts wurde durch eine DNA-Sequenzanalyse überprüft.

Das Plasmid pTZ_E02_His-GlcTrI wurde mittels Transformation in die Stämme *Escherichia coli* BL21(DE3) und *Escherichia coli* Rosetta (DE3) (beide Merck Chemicals, Darmstadt) eingebracht. Die resultierenden Stämme wurden *E. coli* BL21(DE3)/pTZ_E02_His-GlcTrI und *E. coli* Rosetta (DE3)/pTZ_E02_His-GlcTrI genannt.

### Beispiel 7: Vektor pTZ_E02_His-GlcTrII zur Überexpression des Candida bombicola Gens SBG5 mit N-terminalem His-tag

Zur Überexpression des *Candida bombicola* ATCC22214 Gens *SBG5* (Seq ID Nr. 06) in *Escherichia coli* wurde das Plasmid pTZ_E02_His-GlcTrII konstruiert. Chromosomale DNA aus *Candida bombicola* ATCC22214 diente als Matrize für eine PCR mit dem "Expand™ High Fidelity"-PCR-Kit von Roche Diagnostics (Mannheim), gemäß Angaben des Herstellers. Das *SBG5-*Gen wurde mit Hilfe der Oligonukleotide 1373_GlcTrII_BsmBI_His_fp (Seq ID Nr. 79) und 1373_GlcTrII_AscI_rp (Seq ID Nr. 80) aus der chromosomalen DNA amplifiziert ("PCR protocols. A guide to methods and applications", 1990, Academic Press) und auf diesem Wege am 5'-Ende mit einem 6-fachen N-terminalen Histidin-Tag versehen. Außerdem wurden die Schnittstellen *Bsm*BI und *Asc*I eingefügt. Dafür wurden die folgenden Oligonukleotide eingesetzt:
1373_GlcTrII_BsmBI_His_fp (Seq ID Nr. 79):
   5'-AAACGTCTCAGATG*CACCACCACCACCACCAC*ATGGCCATCGAGAAACCAG-3'
1373_GlcTrII_AscI_rp (Seq ID Nr. 80):
   5'-AAAGGCGCGCCTTAAGAAGCTAATTCACTAATTGCC-3'

Das PCR-Produkt (1342 bp) wurde mittels des QIAquick PCR-Purification Kits (Qiagen, Hilden) gemäß Angaben des Herstellers aufgereinigt, mit *Bsm*BI und AscI geschnitten und anschließend in den auf die gleiche Weise geschnittenen Expressionsvektor pTZ_E02 (pET24d-basierter Vektor; Merck Chemicals, Darmstadt) der Firma Trenzyme GmbH, Konstanz, ligiert. Das resultierende Plasmid pTZ_E02_His-GlcTrII (Seq ID Nr. 81) ist 6607 Basenpaare groß. Die Ligation sowie die Transformation chemisch kompetenter *E. coli* DH5α-Zellen (Gibco-BRL, Karlsruhe) erfolgten in dem Fachmann bekannter Art und Weise. Die Authentizität des Inserts wurde durch eine DNA-Sequenzanalyse überprüft.

Das Plasmid pTZ_E02_His-GlcTrII wurde mittels Transformation in die Stämme *Escherichia coli* BL21(DE3) und *Escherichia coli* Rosetta (DE3) (beide Merck Chemicals, Darmstadt) eingebracht. Die resultierenden Stämme wurden *E. coli* BL21(DE3)/pTZ_E02_His-GlcTrII und *E. coli* Rosetta (DE3)/pTZ_E02_His-GlcTrII genannt.

### Beispiel 8: Vektor pTZ_E02_His-AcTr zur Überexpression des Candida bombicola Gens SBG3 mit N-terminalem His-tag

Zur Überexpression des *Candida bombicola* ATCC22214 Gens *SBG3* (Seq ID Nr. 04) in *Escherichia coli* wurde das Plasmid pTZ_E02_His-AcTr konstruiert. Chromosomale DNA aus *Candida bombicola* ATCC22214 diente als Matrize für eine PCR mit dem "Expand™ High Fidelity"-PCR-Kit von Roche Diagnostics (Mannheim), gemäß Angaben des Herstellers. Das *SBG3-Gen* wurde mit Hilfe der Oligonukleotide 1373_AcTr_BsmBI_His_fp (Seq ID Nr. 82) und 1373_AcTr_AscI_rp (Seq ID Nr. 83) aus der chromosomalen DNA amplifiziert ("PCR protocols. A guide to methods and applications", 1990, Academic Press) und auf diesem Wege am 5'-Ende mit einem 6-fachen N-terminalen Histidin-Tag versehen. Außerdem wurden die Schnittstellen *Bsm*BI und *Asc*I eingefügt. Dafür wurden die folgenden Oligonukleotide eingesetzt:
1373_AcTr_BsmBI_His_fp (Seq ID Nr. 82):
   5'-AAACGTCTCAGATG*CACCACCACCACCACCAC*ATGGTTGTAAACTCCTCG-3'
1373_AcTr_AscI_rp (Seq ID Nr. 83):
   5'-AAAGGCGCGCCCTAGACCTTCTGGTTAGCG-3'

Das PCR-Produkt (823 bp) wurde mittels des QIAquick PCR-Purification Kits (Qiagen, Hilden) gemäß Angaben des Herstellers aufgereinigt, mit *Bsm*BI und AscI geschnitten und anschließend in den auf die gleiche Weise geschnittenen Expressionsvektor pTZ_E02 (pET24d-basierter Vektor; Merck Chemicals, Darmstadt) der Firma Trenzyme GmbH, Konstanz, ligiert. Das resultierende Plasmid pTZ_E02_His-AcTr (Seq ID Nr. 84) ist 6088 Basenpaare groß. Die Ligation sowie die Transformation chemisch kompetenter *E. coli* DH5α-Zellen (Gibco-BRL, Karlsruhe) erfolgten in dem Fachmann bekannter Art und Weise. Die Authentizität des Inserts wurde durch eine DNA-Sequenzanalyse überprüft.

Das Plasmid pTZ_E02_His-AcTr wurde mittels Transformation in die Stämme *Escherichia coli* BL21(DE3) und *Escherichia coli* Rosetta (DE3) (beide Merck Chemicals, Darmstadt) eingebracht. Die resultierenden Stämme wurden *E. coli* BL21(DE3)/pTZ_E02_His-AcTr und *E. coli* Rosetta (DE3)/pTZ_E02_His-AcTr genannt.

### Beispiel 9: Heterologe Expression der an der Sophorolipid-Biosynthese beteiligten Enzyme SBG2, SBG3 und SBG5

Je eine Einzelkolonie der unter Punkt 1-3 konstruierten *E. coli-*Stämme wurde zunächst für 8 h in 5 ml LB-Medium (10 g/l Trypton, 5 g/l Hefeextrakt, 10 g/l NaCl) mit 50 µg/ml Kanamycin bei 37°C und 175 rpm kultiviert. Anschließend wurden 100 ml LB-Medium in 500 ml Schüttelkolben mit der ersten Vorkultur inokuliert und über Nacht bei 37°C und 175 rpm kultiviert. Am nächsten Morgen wurde die zweite Vorkultur dazu genutzt um 1 l LB-Medium mit einer Start OD₆₀₀ von 0,1 zu inokulieren (5 l Schüttelkolben). Alle Kulturen wurden bei 37°C und 175 rpm inkubiert. Das Wachstum der Kulturen wurde anhand der scheinbaren optischen Dichte (OD₆₀₀) verfolgt. Beim Erreichen einer OD₆₀₀ von ∼0,3 wurde die Kultivierungstemperatur von 37°C auf 20°C reduziert. Bei einer OD₆₀₀ von 0,6 wurde die Expression der jeweiligen Zielgene durch Zugabe von 0,5 mM IPTG (End-Konzentration) induziert. Während der gesamten Kultivierungsschritte wurden die entsprechenden Antibiotika zugegeben (Kanamycin 50 µg/ml). Sowohl vor der IPTG-Zugabe als auch 24 h nach der Induktion wurden Proben für die Analytik genommen. Die Zellen wurden mittels Bugbuster (Merck Chemicals, Darmstadt) gemäß Angaben des Herstellers aufgeschlossen, um lösliche und unlösliche Proteine voneinander zu trennen. Vergleichbare Mengen der Zellextrakte wurden mittels SDS-PAGE aufgetrennt und die Gele anschließend mit kolloidalem Coomassie gefärbt. Für alle drei his-getagten, rekombinant hergestellten Proteine Sbg2p, Sbg3p und Sbg5p konnte eine Überproduktion in der löslichen Fraktion des Zellextraktes nachgewiesen werden.

### Beispiel 10: Aufreinigung der an der Sophorolipid-Biosynthese beteiligten Enzyme Sbg2p, Sbg3p und Sbg5p

24 h nach Induktion der Genexpression wurden die Zellen mittels Zentrifugation (8000 g, 20 min, 4°C) geerntet. 1 l Kultur resultierte in ∼5 g Biofeuchtmasse. Die Zellpellets wurden in 100 ml Puffer A (100 mM Tris, pH 7,8, 50 mM NaCl, 20 mM Imidazol) resuspendiert, der zusätzlich einen Protease-Inhibitor (Roche, Bestell-Nr. 11 873 580 001) enthielt. Die resuspendierten Zellen wurden durch 6malige Passage durch einen Microfluidizer aufgeschlossen. Nach einem weiteren Zentrifugationsschritt (10.000 g, 20 min, 4°C) wurde der Überstand filtriert (Porendurchmesser: 0,45 µm), um die lösliche Proteinfraktion zu erhalten. Die Zielproteine wurden über eine His-Tag Affinitätschromatographiesäule (GE, HisTrap FF 1 ml Säulen, Bestell-Nr. 17-5319-01) gereinigt. Die Flussrate betrug 1 ml/min. Es erfolgte eine lineare Elution von 0 - 100 % mit Puffer B (100 mM Tris, pH 7,8, 50 mM NaCl, 500 mM Imidazol). Dabei wurde das 20fache Säulenvolumen an Puffer B eingesetzt und 2 ml Fraktionen gesammelt. Die Eluatfraktionen mit Protein wurden gepoolt und mittels einer Filtrationseinheit aufkonzentriert (Amicon Ultra-15, NMWL 10 kDa Centricons, Millipore, Bestell-Nr. UFC901024). Anschließend wurden die jeweiligen Proteinfraktionen mittels Gelfiltration mit Sephadex 25 (PD-10-Säulen, GE, Bestell-Nr. 17-0851-01) in den finalen Puffer (100 mM Tris, pH 7.8, 50 mM NaCl) umgepuffert. Die Proteinaufreinigung wurde mittels SDS-PAGE überprüft. Aus 1 l Kultur wurden 3,3 mg Sbg2p (Proteinkonzentration 1,0 µg/µl), 7,3 mg Sbg5p (Proteinkonzentration 2,2 µg/µl) und 6,9 mg Sbg3p (Proteinkonzentration (2,1 µg/µl) aufgereinigt.

### Beispiel 11: Charakterisierung der an der Sophorolipid-Biosynthese beteiligten Enzyme Sbg2p, Sbg3p und Sbg5p

Um die Funktion der an der Sophorolipid-Biosynthese beteiligten Enzyme Sbg2p, Sbg3p und Sbg5p nachzuweisen, wurde mit den drei aufgereinigten Enzymen Sbg2p, Sbg3p und Sbg5p jeweils einzeln sowie in allen möglichen Kombinationen Enzymassays durchgeführt. Die Durchführung erfolgte in einem Gesamtvolumen von 350 µl nach folgendem Schema:

**Tabelle 7: Zusammensetzung der Enzymassay-Ansätze in µl**

| | **1** | **2** | **3** | **4** | **5** | **6** | **7** | **8** |
|---|---|---|---|---|---|---|---|---|
| 10 mM Tris-HCl (pH 7.5) | 327,5 | 277,5 | 227,5 | 277,5 | 177,5 | 227,5 | 177,5 | 227,5 |
| 125 mM UDP-Glucose | 5 | 5 | 5 | 5 | 5 | 5 | 5 | 5 |
| 100 mM Acetyl-CoA | 3,5 | 3,5 | 3,5 | 3,5 | 3,5 | 3,5 | 3,5 | 3,5 |
| Sbg3p (2,1 µg/µl) | - | 50 | - | - | 50 | 50 | - | 50 |
| Sbg2p (1 µg/µl) | - | - | 100 | - | 100 | - | 100 | 100 |
| Sbg5p (2,2 µg/µl) | - | - | - | 50 | | 50 | 50 | 50 |
| 13,4 mM 18-Hydroxy-Z-9-Octadecensäure | 14 | 14 | 14 | 14 | 14 | 14 | 14 | 14 |
| ∑ | 350 | 350 | 350 | 350 | 350 | 350 | 350 | 350 |

Die Reaktion wurde durch die Zugabe von 14 µl 13,4 mM ethanolischer Substratlösung (18-Hydroxy-Z-9-Octadecensäure), gestartet und 6 h bei 30°C unter Schütteln (600 rpm) inkubiert. Anschließend wurde die Reaktion durch die Zugabe von 1,4 ml Aceton gestoppt. Ungelöste Bestandteile wurden durch Zentrifugation (16.100 g, 5 min, RT) sedimentiert. Der Überstand wurde anschließend in ein neues Gefäß überführt und auf das ursprüngliche Reaktionsvolumen (350 µl) mittels Vakuumverdampfer (25 °C) eingeengt. Die Proben wurden mittels LC-ESI-MS analysiert und die Identifizierung der Produkte erfolgte durch Analyse der entsprechenden Massenspuren und der MS-Spektren.

Für die Identifizierung der entstandenen Produkte wurden 5 µl in eine UPLC-Anlage Accela (Thermo Scientific, Dreieich) injiziert. Die zu untersuchenden Substanzen wurden mit einer semi UPLC-Säule "Pursuit XRs ULTRA (C8, 2,8 µm, 2,1 x 100 mm) (Varian, Darmstadt) analysiert. Die Trennung erfolgte innerhalb von 25 min mittels eines Gradienten bestehend aus der mobilen Phase A1 (H₂O, 0,1 % (v/v) TFA) und der mobilen Phase B1 (Methanol, 0,1 % (v/v) TFA) mit einer Flussrate von 0,3 ml/min bei 40 °C. Der zeitliche Verlauf des Gradienten ist in Tabelle 8 dargestellt.

**Tab. 8: HPLC Gradientenverlauf**

| Zeit [min] | Mobile Phase A1 [%] | Mobile Phase B1 [%] |
|---|---|---|
| 0 | 30 | 70 |
| 15 | 0 | 100 |
| 25 | 0 | 100 |
| 25,01 | 30 | 70 |
| 32 | 30 | 70 |

Die Detektion erfolgte mittels DAD-Detektor im Wellenlängenbereich von 200 - 600 nm sowie massenselektiv mit einem hochauflösenden FT-ICR Massenspektrometer LTQ-FT (Thermo Scientific, Dreieich) im Scanbereich m/z 100 - 1000. Die Ionisierung erfolgte mittels ESI (electrospray ionization). Exakte Massen und chemische Summenformeln wurden mit Hilfe des FT-ICR Massenanalysators, mit einer Auflösung von R = 100000 und einer Massengenauigkeit von ≤ 2 ppm ermittelt.

Als Kontrollreaktion diente ein Ansatz, der nur die Substrate UDP-Glucose, Acetyl-CoA und 18-Hydroxy-Z-9-Octadecensäure, aber keine Enzyme enthielt. (siehe Tab. 7). In dieser Probe konnte mittels MS lediglich das Substrat 18-Hydroxy-Z-9-Octadecensäure (C₁₈H₃₄O₃; 298,2502 g/mol) nachgewiesen werden.
Ansatz 2 (siehe Tab. 7) enthielt neben den Substraten 105 µg Sbg3p. In dieser Probe konnte wie in Ansatz 1 nur 18-Hydroxy-Z-9-Octadecensäure nachgewiesen werden.
Ansatz 3 (siehe Tab. 7) enthielt neben den Substraten 100 µg Sbg2p. Neben dem Substrat 18-Hydroxy-Z-9-Octadecensäure wurde 18-(β-D-Glucopyranosyl-Oxy)-Z-9-Octadecensäure (Summenformel, C₂₄H₄₄O₈; Molekulargewicht, 460,3031 g/mol) detektiert. Dies beweist, dass Sbg2p UDP-Glukose und 18-Hydroxy-Z-9-Octadecensäure zu 18-(β-D-Glucopyranosyl-Oxy)-Z-9-Octadecensäure umzusetzen vermag.
Ansatz 4 (siehe Tab. 7) enthielt neben den Substraten zusätzlich 110 µg Sbg5p. Neben dem Substrat 18-Hydroxy-Z-9-Octadecensäure wurden 18-(β-D-Glucopyranosyl-Oxy)-Z-9-Octadecensäure und 18-L-[(2'-*O*-β-D-Glucopyranosyl-β-D-Glucopyranosyl)-Oxy]-Z-9-Octadecensäure (Summenformel, C₃₀H₅₄O₁₃; Molekulargewicht, 622,3559 g/mol) detektiert. Dies beweist, dass Sbg5p UDP-Glukose und 18-Hydroxy-Z-9-Octadecensäure zu 18-(β-D-Glucopyranosyl-Oxy)-Z-9-Octadecensäure und weiter zu 18-L-[(2'-*O*-β-D-Glucopyranosyl-β-D-Glucopyranosyl)-Oxy]-Z-9-Octadecensäure umzusetzen vermag.
Ansatz 5 (siehe Tab. 7) enthielt neben den Substraten zusätzlich 100 µg Sbg2p und 105 µg Sbg3p. Neben dem Substrat 18-Hydroxy-Z-9-Octadecensäure wurden 18-(β-D-Glucopyranosyl-Oxy)-Z-9-Octadecensäure und 18-(6-*O*-Acetyl-β-D-Glucopyranosyl-Oxy)-Z-9-Octadecensäure (Summenformel, C₂₆H₄₆O₉; Molekulargewicht, 502,3136 g/mol) detektiert. Dies bestätigt wie schon in Ansatz 3 gezeigt, dass Sbg2p UDP-Glukose und 18-Hydroxy-Z-9-Octadecensäure zu 18-(β-D-Glucopyranosyl-Oxy)-Z-9-Octadecensäure umzusetzen vermag und beweist weiterhin, dass Sbg3p 18-(β-D-Glucopyranosyl-Oxy)-Z-9-Octadecensäure in Gegenwart von Acetyl-CoA zu 18-(6-*O*-Acetyl-β-D-Glucopyranosyl-Oxy)-Z-9-Octadecensäure acetylieren kann. Ansatz 6 (siehe Tab. 7) enthielt neben den Substraten zusätzlich 110 µg Sbg5p und 105 µg Sbg3p. Neben dem Substrat 18-Hydroxy-Z-9-Octadecensäure wurden 18-(β-D-Glucopyranosyl-Oxy)-Z-9-Octadecensäure, 18-(6-*O*-Acetyl-β-D-Glucopyranosyl-Oxy)-Z-9-Octadecensäure, 18-L-[(2'-*O*-β-D-Glucopyranosyl-β-D-Glucopyranosyl)-Oxy]-Z-9-Octadecensäure, 18-L-[(6'-*O*-Acetyl-2'-*O*-β-D-Glucopyranosyl-β-D-Glucopyranosyl)-Oxy]-2-9-Octadecensäure (Summenformel, C₃₂H₅₆O₁₄; Molekulargewicht, 664,3665 g/mol) und 18-L-[(6'-*O*-Acetyl-2'-O-β-D-Glucopyranosyl-6"-*O*-Acetyl-β-D-Glucopyranosyl)-Oxy]-Z-9-Octadecensäure (Summenformel, C₃₄H₅₈O₁₅; Molekulargewicht, 706,3770 g/mol) detektiert. Dies bestätigt wie schon in Ansatz 4 gezeigt, dass Sbg5p UDP-Glukose und 18-Hydroxy-Z-9-Octadecensäure zu 18-(β-D-Glucopyranosyl-Oxy)-Z-9-Octadecensäure und weiter zu 18-L-[(2'-*O*-β-D-Glucopyranosyl-β-D-Glucopyranosyl)-Oxy]-Z-9-Octadecensäure umzusetzen vermag und beweist weiterhin, dass die gebildeten Produkte in Gegenwart von Acetyl-CoA durch Sbg3p zu 18-L-[(6'-*O*-Acetyl-2'-O-β-D-Glucopyranosyl-β-D-Glucopyranosyl)-Oxy]-Z-9-Octadecensäure und/oder 18-L-[(2'-*O*-β-D-Glucopyranosyl-6"-*O*-Acetyl-β-D-Glucopyranosyl)-Oxy]-Z-9-Octadecensäure sowie 18-L-[(6'-*O-*Acetyl-2'-*O*-β-D-Glucopyranosyl-6"-*O*-Acetyl-β-D-Glucopyranosyl)-Oxy]-Z-9-Octadecensäure acetyliert werden können.
Ansatz 7 (siehe Tab. 7) enthielt neben den Substraten zusätzlich 100 µg Sbg2p und 110 µg Sbg5p. Neben dem Substrat 18-Hydroxy-Z-9-Octadecensäure wurden 18-(β-D-Glucopyranosyl-Oxy)-Z-9-Octadecensäure und 18-L-[(2'-*O*-β-D-Glucopyranosyl-β-D-Glucopyranosyl)-Oxy]-Z-9-Octadecensäure detektiert. Dies beweist, dass Sbg2p und Sbg5p in einem Ansatz UDP-Glukose und 18-Hydroxy-Z-9-Octadecensäure zu 18-(β-D-Glucopyranosyl-Oxy)-Z-9-Octadecensäure und weiter zu 18-L-[(2'-*O*-β-D-Glucopyranosyl-β-D-Glucopyranosyl)-Oxyl-Z-9-Octadecensäure umzusetzen vermögen. Ansatz 8 (siehe Tab. 7) enthielt neben den Substraten zusätzlich 100 µg Sbg2p, 105 µg Sbg3p und 110 µg Sbg5p. Neben dem Substrat 18-Hydroxy-Z-9-Octadecensäure wurden 18-(ß-D-Glucopyranosyl-Oxy)-Z-9-Octadecensäure, 18-(6-*O*-Acetyl-β-D-Glucopyranosyl-Oxy)-Z-9-Octadecensäure, 18-L-[(2'-*O*-β-D-Glucopyranosyl-β-D-Glucopyranosyl)-Oxy]-Z-9-Octadecensäure, 18-L-[(6'-*O*-Acetyl-2'-*O*-β-D-Glucopyranosyl-β-D-Glucopyranosyl)-Oxy]-Z-9-Octadecensäure und 18-L-[(6'-*O*-Acetyl-2'-*O*-β-D-Glucopyranosyl-6"-*O*-Acetyl-β-D-Glucopyranosyl)-Oxy]-Z-9-Octadecensäure detektiert. Dies bestätigt, wie schon in Ansatz 7 gezeigt, dass Sbg2p und Sbg5p gemeinsam UDP-Glukose und 18-Hydroxy-Z-9-Octadecensäure zu 18-(β-D-Glucopyranosyl-Oxy)-Z-9-Octadecensäure und weiter zu 18-L-[(2'-*O*-β-D-Glucopyranosyl-β-D-Glucopyranosyl)-Oxy]-Z-9-Octadecensäure umzusetzen vermögen und beweist wie ebenfalls schon in den Ansätzen 5 und 6 gezeigt, dass die gebildeten Produkte in Gegenwart von Acetyl-CoA durch Sbg3p zu 18-L-[(6'-*O-*Acetyl-2'-*O*-β-D-Glucopyranosyl-β-D-Glucopyranosyl)-Oxy]-Z-9-Octadecensäure und/oder 18-L-[(2'-*O*-β-D-Glucopyranosyl-6"-*O-*Acetyl-β-D-Glucopyranosyl)-Oxy]-Z-9-Octadecensäure sowie 18-L-[(6'-*O*-Acetyl-2'-*O*-β-D-Glucopyranosyl-6"-*O*-Acetyl-β-D-Glucopyranosyl)-Oxy]-Z-9-Octadecensäure acetyliert werden können.

### Beispiel 12: Alternativer Weg zur Inaktivierung der Acetyltransferase (SBG3) in Candida bombicola ATCC 22214

Das Gen *SBG3* wurde auf einem alternativen Weg einzeln inaktiviert und der Phänotyp der entsprechenden Mutante bezüglich Sophorolipid-Biosynthese charakterisiert. Zur Konstruktion der entsprechenden Mutante in *C*. *bombicola* ATCC 22214 wurde zunächst eine Deletionskassette für *CbSBG3* durch die GeneArt AG (Regensburg) synthetisiert (Seq ID Nr. 14; vgl. Beispiel 2). Anschließend wurde bei der Trenzyme GmbH (Konstanz) das die Orotidin-5-Phosphat-Decarboxylase von *C*. *bombicola* ATCC 22214 kodierende Gen *CbURA3* (van Bogaert et al. Yeast. 2007. 24(3):201-8) durch eine Hygromyzinresistenz-Kassette ausgetauscht. Dazu wurde die Hygromyzinkassette von der DNA des Vektors p-Col-5 (Seq ID Nr. 85) mit folgenden Oliginukleotiden amplifiziert:
1390_hygR_fp_EcoRV: 5'- AAA GAT ATC TCT ATG CGC ACC CGT TCT C -3' (Seq ID Nr. 86)
1390_hygR_rp_Hind/Bgl: 5'- TTT AGA TCT AAG CTT GAG ACA CCT CAG CAT GCA CCA TTC -3' (Seq ID Nr. 87)

Folgende Parameter wurden für die PCR eingesetzt: 1 x: initiale Denaturierung, 98 °C, 3 min; 25 x: Denaturierung, 98 °C, 0:10 min, Annealing, 60 °C, 0:30 min; Elongation, 72 °C, 2:00 min; 1 x: terminale Elongation, 72 °C, 10 min. Für die Amplifikation wurde der Phusion™ High-Fidelity Master Mix von New England Biolabs (Frankfurt) entsprechend den Empfehlungen des Herstellers verwendet. Das PCR-Produkt wurde mittels des QIAquick PCR-Purification Kit (Qiagen, Hilden) gemäß den Angaben des Herstellers aufgereinigt. Das erhaltende PCR-Produkt hatte eine Größe von 1831 bp. Die Durchführung der PCR, die Überprüfung der erfolgreichen Amplifikation der PCR mittels Agarosegel-Elektrophorese, Ethidiumbromidfärbung der DNA, Bestimmung der PCR-Fragmentgrößen, Reinigung der PCR-Produkte und DNA-Konzentrationsbestimmung erfolgte in dem Fachmann bekannter Art und Weise. Die Hygromyzinkassette wurde in den Vektor pCR4_AcTr_URA (Seq ID Nr. 88) kloniert, in dem der Vektor mit den Restriktionsendonukleasen *Bgl*II und *Pml*I linearisiert wurde. Das Insert wurde mit den Restriktionsendonukleasen *Eco*RV und *Bgl*II für die anschließende Ligation vorbereitet. Die Ligation und anschließende Transformation in *E. coli* DH5α Zellen erfolgte in dem Fachmann bekannter Art und Weise. Die Authentizität des *Inserts* wurde durch DNA-Sequenzanalyse überprüft.
Das erzeugte Plasmid wurde pCR4_AcTr_HygR (Seq ID Nr. 89) genannt und ist 8578 bp groß.

Die Deletionskassette *CbSbg3-hyg* (Seq ID Nr. 90) besteht aus dem *Klebsiella pneumoniae* Hygromyzin-Resistenz-Gen (*hph*), welches die Hygromyzin B Phosphatase kodiert (Gritz L und Davies J 1983 Plasmid-encoded hygromycin B resistance: the sequence of hygromycin B phosphotransferase gene and its expression in Escherichia coli and Saccharomyces cerevisiae. Gene 25 (2-3): 179-188). Bei dem Promotor für das Resistenz-Gen handelt es sich um den konstitutiven Hybridpromotor hp4d (Madzak et al. 2000, Strong hybrid promotors and integrative expression/secretion vectors for quasi-constitutive expression of heterologous proteins in the yeast Yarrowia lipolytica. J. Mol. Microbiol. Biotechnol. 2, 207 - 216). Das Resistenz-Gen wird von dem Terminator des *XPR2*-Gens flankiert, welches für eine extrazelluläre Protease aus *Y. lipolytica* kodiert (Nicaud et al. 1989a. Cloning, sequencing and amplification of the alkaline extracellular protease (XPR2) gene of the yeast Yarrowia lipolytica. J. Biotechnol. 12, 285 - 298). Stromauf- und stromabwärts wird das Resistenzgen von etwa 1000 bp des angrenzenden Bereiches des zu inaktivierenden Genes flankiert.

Zwischen den flankierenden Bereichen und dem *hph*-Gen wurden jeweils *loxP-*Loc*i* eingefügt, die ggf. die Deletion des *hph*-Gens durch vorübergehende Einführung des für die Rekombinase Cre kodierenden Gens und dessen funktionelle Expression erlauben (für einen Überblick siehe Kühn & Torres. Methods Mol Biol. 2002. 180:175-204). Die Deletionskassette ist entsprechend der Angaben in der folgenden Tabelle 9 aufgebaut:

**Tabelle 9: Struktur der Deletionskassette für das Sbg3p kodierende Strukturgen von C. bombicola ATCC 22214.**

| Seq ID Nr. | Gen | 5' - flankierender Bereich | loxP-Locus 1 | *hph* | loxP-Locus 2 | 3'-flankierender Bereich |
|---|---|---|---|---|---|---|
| 90 | *SBG3* | 1 - 1033 | 1034 - 1066 | 1067 - 3599 | 3600 - 3633 | 3634 - 4635 |

Um die Deletionskassette für die anschließende Transformation von *C*. *bombicola* ATCC 22214 in ausreichender Menge zur Verfügung zu stellen, wurde diese per PCR amplifiziert. Dabei kamen folgende Oligonukleotide zum Einsatz:
Amplifikation der Deletionskassette für Inaktivierung von *CbSBG3:*
SBG3-fw: 5'- TGC AGA CAA GTT CCT GCA GCT G -3' (Seq ID Nr. 21)
SBG3-rv: 5'- ATG CTT TAT TCA GGC ACG CTA CG -3' (Seq ID Nr. 22)

Folgende Parameter wurden für die PCR eingesetzt: 1 x: initiale Denaturierung, 98 °C, 3 min; 25 x: Denaturierung, 98 °C, 0:10 min, Annealing, 60 °C, 0:30 min; Elongation, 72 °C, 2:00 min; 1 x: terminale Elongation, 72 °C, 10 min. Für die Amplifikation wurde der Phusion™ High-Fidelity Master Mix von New England Biolabs (Frankfurt) entsprechend den Empfehlungen des Herstellers verwendet. Das PCR-Produkt wurde mittels des QIAquick PCR-Purification Kit (Qiagen, Hilden) gemäß den Angaben des Herstellers aufgereinigt. Die Durchführung der PCR, die Überprüfung der erfolgreichen Amplifikation der PCR mittels Agarosegel-Elektrophorese, Ethidiumbromidfärbung der DNA, Bestimmung der PCR-Fragmentgrößen, Reinigung der PCR-Produkte und DNA-Konzentrationsbestimmung erfolgte in dem Fachmann bekannter Art und Weise.

Die Transformation von *C*. *bombicola* ATCC 22214 erfolgte wie vorbeschrieben (van Bogaert et al. Yeast. 2008. 25:273-278); van Bogaert et al. FEMS Yeast Res. 2009. 9:610-617).

Um die Deletion des Gens *SBG3* in *C*. *bombicola* ATCC 22214 - Transformanten nach Transformation mit der Deletionskassette für *CbSBG3* (Seq ID Nr. 90) zu überprüfen, wurde von jeweils 5 Transformanten und C. *bombicola* ATCC 22214 der jeweilige Locus per Kolonie-PCR amplifiziert. Dabei kamen folgende Oligonukleotide zum Einsatz:

**Kontrolle der genomischen Deletion von CbSBG3:**

| | |
|---|---|
| SBG3-KO-fw: | 5'- CAA ATT TAT CTG GGA GCA CAG TTA CAT TGC -3' (Seq ID Nr. 31) |
| SBG3-KO-rv: | 5'- CAC ACA TTG CTT TAG TCC AGC AAG AAC C -3' (Seq ID Nr. 32) |

Folgende Parameter wurden für die PCR eingesetzt: 1 x: initiale Denaturierung, 94 °C, 3 min; 25 x: Denaturierung, 94 °C, 1:00 min, Annealing, 60 °C, 1:00 min; Elongation, 72 °C, 5:00 min; 1 x: terminale Elongation, 72 °C, 10 min. Für die Amplifikation wurde der Taq PCR Master Mix Kit von QIAGEN (Hilden) entsprechend den Empfehlungen des Herstellers verwendet. Jeweils 10 µl der PCR-Reaktionen wurden anschließend auf einem 0,8 %igen Agarosegel aufgetrennt. Die Durchführung der PCR, der Agarosegel-Elektrophorese, Ethidiumbromidfärbung der DNA und Bestimmung der PCR-Fragmentgrößen erfolgte in dem Fachmann bekannter Art und Weise.
Bei der Amplifikation des *CbSBG3-*Locus aus *C*. *bombicola* ATCC 22214 wurden ausschließlich die bei vorliegen der Wildtypsituation zu erwartenden Fragmentgrößen von 2839 bp ermittelt.
Bei der Amplifikation des *SBG3-*Locus aus Transformanten nach Transformation der Deletionskassette *CbSBG3-hyg* wurde ausschließlich die nach erfolgreicher chromosomaler Deletion von *CbSBG3* zu erwartenden Fragmentgröße von 4693 bp erzielt.

Somit konnten Klone identifiziert werden, in denen das Gen *CbSBG3* chromosomal deletiert wurde. Der entsprechende Stamm wurde im Weiteren als *C*. *bombicola* ATCC 22214 *sbg3-hyg* bezeichnet.

### Beispiel 13: Charakterisierung der Sophorolipidbildung durch C. bombicola ATCC 22214 sbg3-hyg.

Die Propagierung der Stämme *C*. *bombicola* ATCC 22214 und *C*. *bombicola* ATCC 22214 *sbg3-hyg* erfolgte auf YPD-Agar-Platten. Zur Produktion der Sophorolipide wurde das im Folgenden als SL-Produktionsmedium bezeichnete Medium verwendet. Dieses besteht aus 0,1 % KH₂PO₄, 0,5 % MgSO₄ x 7 H₂O, 0,01 % FeCl₃, 0,01 % NaCl, 0,4 % Hefeextrakt, 0,1 % Urea, 10,5 % Rapsöl und 10 % Glucose. Der pH-Wert wurde auf 4,5 eingestellt und das Medium folgend mittels Autoklav (121 °C, 20 min) sterilisiert. Ein Einstellen des pH-Wertes während der Kultivierung war nicht nötig.

Zur Untersuchung der Sophorolipidproduktion im Schüttelkolben wurde zunächst eine Vorkultur angesetzt. Hierzu wurde eine Impföse eines frisch auf YPD-Agarplatte ausgestrichenen Stammes verwendet und 10 ml YPD-Medium in einem 100 ml Erlenmeyerkolben beimpft. Die Kultivierung erfolgte bei 30 °C und 200 rpm über Nacht. Diese Vorkultur wurde folgend verwendet, um 100 ml SL-Medium im 1000 ml Erlenmeyerkolben zu beimpfen (Start-OD₆₀₀ 0,2). Die Kulturen wurden bei 200 rpm und 30 °C für 7 Tage kultiviert und jeden Tag eine Probe von 2 ml Brühe genommen, wobei strikt auf gute Durchmischung des Kulturmediums vor Probenahme geachtet wurde.

Die Probenvorbereitung für die nachfolgenden chromatographischen Analysen erfolgte folgendermaßen: mit einer Verdrängerpipette (Combitip) wurden in einem 2 ml-Reaktionsgefäß 800 µl Aceton vorgelegt und das Reaktionsgefäß zur Minimierung von Verdunstung unmittelbar verschlossen. Es folgte die Zugabe von 200 µl Brühe. Nach Vortexen des Brühe/Acetongemisches wurde dieses für 1 min bei 13.000 rpm abzentrifugiert, und 800 µl des Überstands in ein HPLC-Gefäß überführt.

Zum Nachweis und zur Quantifizierung von Sophorolipiden bzw. Ölsäure wurde ein Evaporative Light Scattering Detektor (ELSD) benutzt. Die eigentliche Messung wurde mittels Agilent Technologies 1200 Series (Santa Clara, Kalifornien) und der Zorbax SB-C8 Rapid Resolution Säule (4,6 x 150 mm, 3,5 µm, Agilent) durchgeführt. Das Injektionsvolumen betrug 5 µl und die Laufzeit der Methode lag bei 20 min. Als mobile Phase wurde H₂O und 0,1 % TFA (Trifluor-Essigsäure, Lösung A) und Methanol (Lösung B) verwendet. Die Säulentemperatur betrug 40 °C. Als Detektoren dienten der ELSD (Detektortemperatur 60 °C) und der DAD (Diodenarray, 210 nm). Der in der Methode verwendete Gradient ist in der folgenden Tabelle 10 dargestellt.

**Tabelle 10: Beschreibung des für die HPLC-basierte Quantifizierung von Sophorolipiden zu verwendenden Gradientenprofils der mobilen Phase.**

| **t [min]** | **Lösung B %** | **Fluss [ml/min]** |
|---|---|---|
| 0,00 | 70% | 1,00 |
| 15,00 | 100% | 1,00 |
| 15,01 | 70% | 1,00 |
| 20,00 | 70% | 1,00 |

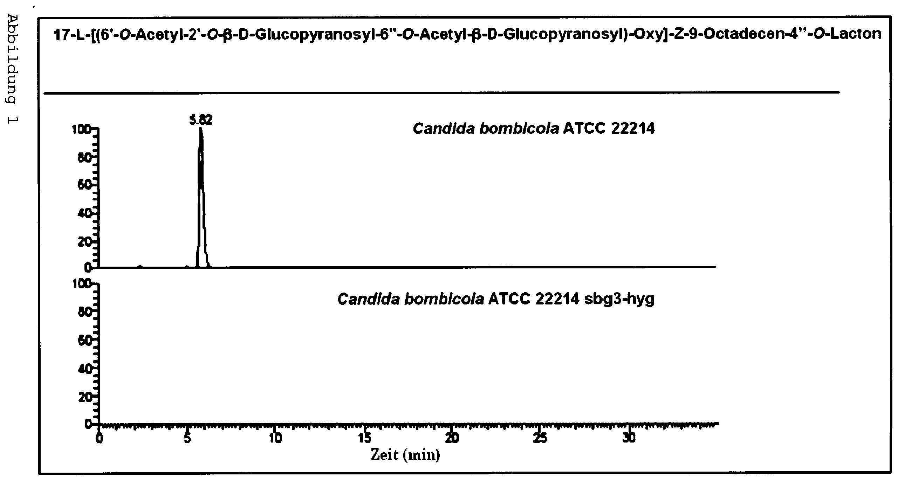

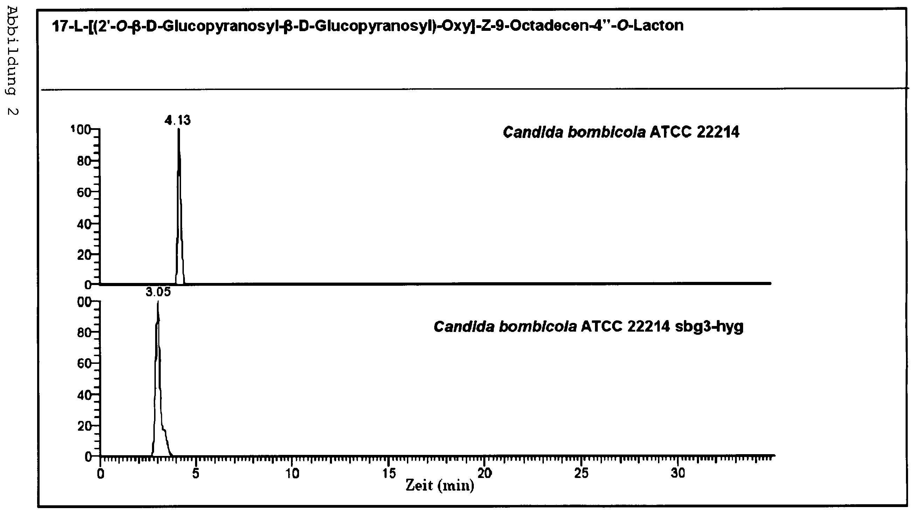

Die Analyse zeigte, dass sowohl *C. bombicola* ATCC 22214 und *C*. *bombicola* ATCC 22214 *sbg3-hyg* Sophorolipide produzieren. Mittels LC-MS² wurde nachgewiesen, dass die durch *C*. *bombicola* ATCC 22214 *sbg3-hyg* gebildeten Sophorolipide im Gegensatz zu den durch *C*. *bombicola* ATCC 22214 gebildeten Sophorolipiden ausschließlich Verbindungen der allgemeinen Formel (Ia) und (Ib) entsprechen, bei denen R¹ = H und R² = H (siehe Abbildung 1 und 2) und die Konzentration dieser Verbindungen im Vergleich zu *C*. *bombicola* ATCC 22214 um den Faktor 10 erhöht vorliegt. Dies beweist die Funktion von Sbg3p als Acetyltransferase in der Sophorolipidbiosynthese.

## Patentansprüche

1. Eine Sophorolipid bildende Zelle, welche derart gentechnisch verändert wird, dass sie verglichen zu ihrem Wildtyp eine geänderte, wie jeweils im Folgenden spezifizierte Aktivität mindestens eines der Enzyme ausgewählt aus der Gruppe bestehend aus,
mindestens ein Enzym E₁ mit Polypeptidsequenz Seq ID Nr. 7, Seq ID Nr. 53, Seq ID Nr. 55, Seq ID Nr. 57, Seq ID Nr. 59, Seq ID Nr. 61 oder Seq ID Nr. 63 oder mit einer Polypeptidsequenz bei der bis zu 25% der Aminosäurereste gegenüber Seq ID Nr. 7, Seq ID Nr. 53, Seq ID Nr. 55, Seq ID Nr. 57, Seq ID Nr. 59, Seq ID Nr. 61 oder Seq ID Nr. 63 durch Deletion, Insertion, Substitution oder eine Kombination davon verändert sind und die noch mindestens 50% der enzymatischen Aktivität des Enzyms mit der jeweiligen Bezugssequenz Seq ID Nr. 7, Seq ID Nr. 53, Seq ID Nr. 55, Seq ID Nr. 57, Seq ID Nr. 59, Seq ID Nr. 61 oder Seq ID Nr. 63 besitzt, wobei unter enzymatische Aktivität für ein Enzym E₁ die Fähigkeit verstanden wird, Z-9-Octadecensäure zu 17-Hydroxy-Z-9-Octadecensäure umzusetzen,
mindestens ein Enzym E₂ mit Polypeptidsequenz Seq ID Nr. 8 oder Seq ID Nr. 11 oder mit einer Polypeptidsequenz bei der bis zu 60% der Aminosäurereste gegenüber Seq ID Nr. 8 oder Seq ID Nr. 11 durch Deletion, Insertion, Substitution oder eine Kombination davon verändert sind und die noch mindestens 50% der enzymatischen Aktivität des Enzyms mit der jeweiligen Bezugssequenz Seq ID Nr. 8 oder Seq ID Nr. 11 besitzt, wobei unter enzymatische Aktivität für ein Enzym E₂ die Fähigkeit verstanden wird, UDP-Glukose und 17-Hydroxy-Z-9-Octadecensäure zu 17-(β-D-Glucopyranosyl-Oxy)-Z-9-Octadecensäure umzusetzen,
mindestens ein Enzym E₃ mit Polypeptidsequenz Seq ID Nr. 11 oder mit einer Polypeptidsequenz bei der bis zu 60% der Aminosäurereste gegenüber Seq ID Nr. 11 durch Deletion, Insertion, Substitution oder eine Kombination davon verändert sind und die noch mindestens 50% der enzymatischen Aktivität des Enzyms mit der Bezugssequenz Seq ID Nr. 11 besitzt, wobei unter enzymatische Aktivität für ein Enzym E₃ die Fähigkeit verstanden wird, 17-(β-D-Glucopyranosyl-Oxy)-Z-9-Octadecensäure und UDP-Glukose zu 17-L-[(2'-O-β-D-Glucopyranosyl-β-D-Glucopyranosyl)-Oxy]-Z-9-Octadecensäure umzusetzen,
mindestens ein Enzym E₄ mit Polypeptidsequenz Seq ID Nr. 9 oder mit einer Polypeptidsequenz bei der bis zu 50% der Aminosäurereste gegenüber Seq ID Nr. 9 durch Deletion, Insertion, Substitution oder eine Kombination davon verändert sind und die noch mindestens 50% der enzymatischen Aktivität des Enzyms mit Seq ID Nr. 9 besitzt, wobei unter enzymatische Aktivität für ein Enzym E₄ die Fähigkeit verstanden wird, 17-L-[(2'-*O*-β-D-Glucopyranosyl-β-D-Glucopyranosyl)-Oxy]-Z-9-Octadecensäure-1',4"-Lacton und Acetyl-Coenzym A zu 17-L-[(2'-*O*-β-D-Glucopyranosyl-β-D-Glucopyranosyl)-Oxy]-Z-9-Octadecensäure-1',4"-Lacton-Monoacetat oder 17-L-[(2'-O-β-D-Glucopyranosyl-β-D-Glucopyranosyl)-Oxy]-Z-9-Octadecensäure-1',4"-Lacton-Monoacetat und Acetyl-Coenzym A zu 17-L-[(2'-*O*-β-D-Glucopyranosyl-β-D-Glucopyranosyl)-Oxy]-Z-9-Octadecensäure-1',4"-Lacton-Diacetat oder 17-L-[(2'-*O-*β-D-Glucopyranosyl-β-D-Glucopyranosyl)-Oxy]-Z-9-Octadecensäure-1',4"-Lacton und Acetyl-Coenzym A zu 17-L-[(2'-*O*-β-D-Glucopyranosyl-β-D-Glucopyranosyl)-Oxy]-Z-9-Octadecensäure-1',4"-Lacton-Diacetat umzusetzen,
mindestens ein Enzym E₅ mit Polypeptidsequenz Seq ID Nr. 10 oder mit einer Polypeptidsequenz bei der bis zu 45% der Aminosäurereste gegenüber Seq ID Nr. 10 durch Deletion, Insertion, Substitution oder eine Kombination davon verändert sind und die noch mindestens 50% der enzymatischen Aktivität des Enzyms mit Seq ID Nr. 10 besitzt, wobei unter enzymatische Aktivität für ein Enzym E₅ die Fähigkeit verstanden wird, ein Sophorolipid aus einer Zelle heraus ins umgebende Medium zu transferieren,
**dadurch gekennzeichnet, dass**
sie gesteigerte Aktivitäten folgender Enzymkombinationen:
E₁E₂, E₁E₃, E₁E₄, E₁E₅, E₂E₃, E₂E₄, E₂E₅, E₃E₄, E₃E₅, E₄E₅, E₁E₂E₃, E₁E₂E₄, E₁E₂E₅, E₁E₃E₄, E₁E₃E₅, E₁E₄E₅, E₂E₃E₄, E₂E₄E₅, E₃E₄E₅, E₁E₂E₃E₄, E₂E₃E₄E₅, E₁E₃E₄E₅, E₁E₂E₄E₅, E₁E₂E₃E₅, E₁E₂E₃E₄ und E₁E₂E₃E₄E₅
oder dass sie eine verminderte Aktivität des Enzyms E₃ und gegebenenfalls eine gesteigerte Aktivität folgender Enzymkombinationen:
E₁E₂, E₁E₄, E₁E₅, E₂E₄, E₂E₅, E₄E₅, E₁E₂E₄, E₁E₂E₅, E₁E₄E₅ und E₁E₂E₄E₅
oder dass sie eine verminderte Aktivität des Enzyms E₄ und gegebenenfalls eine gesteigerte Aktivität folgender Enzymkombinationen:
E₁E₂, E₁E₃, E₁E₃, E₂E₃, E₂E₅, E₃E₅, E₁E₂E₃, E₁E₂E₅, E₁E₃E₅ und E₁E₂E₃E₅
oder dass sie eine verminderte Aktivität der Enzyme E₃ und E₄ und gegebenenfalls eine gesteigerte Aktivität folgender Enzymkombinationen:
E₁E₂, E₁E₅, E₂E₅, E₁E₂E₅,
aufweist.

2. Zelle gemäß Anspruch 1, **dadurch gekennzeichnet, dass** sie mindestens teilweise in ihrer β-Oxidation blockiert ist.

3. Zelle gemäß Anspruch 1 oder 2, **dadurch gekennzeichnet, dass** sie mit mindestens einer Nukleinsäure gemäß Anspruch 5 transformiert wird.

4. Verfahren zur Herstellung von Sophorolipiden umfassend die Verfahrensschritte
I) in Kontakt Bringen einer Zelle gemäß mindestens einem der Ansprüche 1 oder 2 mit einem Medium beinhaltend eine Kohlenstoffquelle,
II) Kultivieren der Zelle unter Bedingungen, die es der Zelle ermöglichen aus der Kohlenstoffquelle Sophorolipid zu bilden und
III) gegebenenfalls Isolierung der gebildeten Sophorolipide.

5. Isolierte DNA, welche ausgewählt ist aus den folgenden Sequenzen:
A) eine Sequenz gemäß Seq ID Nr. 3, Seq ID Nr. 4, Seq ID Nr. 5, Seq ID Nr. 6, Seq ID Nr. 56, Seq ID Nr. 58, Seq ID Nr. 60 oder Seq ID Nr. 62,
wobei die Sequenz gemäß Seq ID Nr. 56, Seq ID Nr. 58, Seq ID Nr. 60 oder Seq ID Nr. 62, für ein Protein kodiert, welches in der Lage ist, Z-9-Octadecensäure zu 17-Hydroxy-Z-9-Octadecensäure umzusetzen,
wobei die Sequenz Seq ID Nr. 3 für ein Protein kodiert, welches in der Lage ist,
UDP-Glukose und 17-Hydroxy-Z-9-Octadecensäure zu 17-(β-D-Glucopyranosyl-Oxy)-Z-9-Octadecensäure umzusetzen,
wobei die Sequenz Seq ID Nr. 4 für ein Protein kodiert, welches in der Lage ist,
17-L-[(2'-*O*-β-D-Glucopyranosyl-β-D-Glucopyranosyl)-Oxy]-Z-9-Octadecensäure-1',4"-Lacton und Acetyl-Coenzym A zu 17-L-[(2'-*O*-β-D-Glucopyranosyl-β-D-Glucopyranosyl)-Oxy]-Z-9-Octadecensäure-1',4"-Lacton-Monoacetat oder 17-L-[(2'-*O*-β-D-Glucopyranosyl-ß-D-Glucopyranosyl)-Oxy]-Z-9-Octadecensäure-1',4"-Lacton-Monoacetat und Acetyl-Coenzym A zu 17-L-[(2'-*O*-β-D-Glucopyranosyl-β-D-Glucopyranosyl)-Oxy]-Z-9-Octadecensäure-1',4"-Lacton-Diacetat
oder 17-L-[(2'-*O*-β-D-Glucopyranosyl-β-D-Glucopyranosyl)-Oxy]-Z-9-Octadecensäure-1',4"-Lacton und Acetyl-Coenzym A zu 17-L-[(2'-*O*-β-D-Glucopyranosyl-ß-D-Glucopyranosyl)-Oxy]-Z-9-Octadecensäure-1',4"-Lacton-Diacetat umzusetzen,
wobei die Sequenz Seq ID Nr. 5 für ein Protein kodiert, welches in der Lage ist, ein Sophorolipid aus einer Zelle heraus ins umgebende Medium zu transferieren
wobei die Sequenz Seq ID Nr. 6, für ein Protein kodiert, welches in der Lage ist,
UDP-Glukose und 17-Hydroxy-Z-9-Octadecensäure zu 17-(β-D-Glucopyranosyl-Oxy)-Z-9-Octadecensäure oder 17-(β-D-Glucopyranosyl-Oxy)-Z-9-Octadecensäure und UDP-Glukose zu 17-L-[(2'-*O*-β-D-Glucopyranosyl-β-D-Glucopyranosyl)-Oxy]-Z-9-Octadecensäure umzusetzen,
B) eine intronfreie Sequenz, die von einer Sequenz nach A) abgeleitet ist und die das gleiche Protein oder Peptid kodiert wie die Sequenz nach Seq ID Nr. 3, Seq ID Nr. 4, Seq ID Nr. 5, Seq ID Nr. 6, Seq ID Nr. 56, Seq ID Nr. 58, Seq ID Nr. 60 oder Seq ID Nr. 62,
C) eine Sequenz, die ein Protein oder Peptid kodiert, das die Aminosäuresequenz gemäß Seq ID Nr. 8, Seq ID Nr. 9, Seq ID Nr. 10, Seq ID Nr. 11, Seq ID Nr. 57, Seq ID Nr. 59, Seq ID Nr. 61 oder Seq ID Nr. 63 umfasst, wobei das Protein oder Peptid, das die Aminosäuresequenz gemäß Seq ID Nr. 57, Seq ID Nr. 59, Seq ID Nr. 61 oder Seq ID Nr. 63 umfasst, in der Lage ist,
Z-9-Octadecensäure zu 17-Hydroxy-Z-9-Octadecensäure umzusetzen,
D) eine Sequenz, die mit einer Sequenz nach einer der Gruppen A) bis C) mit Seq ID Nr. 3, Seq ID Nr. 4, Seq ID Nr. 5, Seq ID Nr. 6, Seq ID Nr. 62, Seq ID Nr. 8, Seq ID Nr. 9, Seq ID Nr. 10, Seq ID Nr. 11 oder Seq ID Nr. 63 als Bezugssequenz zu mindestens 90 %, mit Seq ID Nr. 56, Seq ID Nr. 58, Seq ID Nr. 60, Seq ID Nr. 57, Seq ID Nr. 59 oder Seq ID Nr. 61 als Bezugssequenz zu mindestens 95 %, identisch ist,
E) eine Sequenz, die mit dem Gegenstrang einer Sequenz nach einer der Gruppen A) bis D) hybridisiert oder unter Berücksichtigung der Degeneration des genetischen Codes hybridisieren würde,
F) ein durch Substitution, Addition, Inversion und/oder Deletion einer oder mehr Basen erhaltenes Derivat einer Sequenz nach einer der Gruppen A) bis E) und
G) eine komplementäre Sequenz zu einer Sequenz nach einer der Gruppen A) bis F).

6. Ein Vektor, umfassend eine DNA-Sequenz nach einer der Gruppen A) bis G), wie im Anspruch 5 definiert.

7. Die Verwendung des Vektors nach Anspruch 6 zur Transformation einer Zelle.

8. Isoliertes Polypeptid ausgewählt aus der Gruppe bestehend aus
ein Enzym E₁ mit Polypeptidsequenz Seq ID Nr. 57, Seq ID Nr. 59, Seq ID Nr. 61 oder Seq ID Nr. 63 oder mit einer Polypeptidsequenz bei der bis zu 10% der Aminosäurereste gegenüber der jeweiligen Bezugssequenz Seq ID Nr. 57, Seq ID Nr. 59, Seq ID Nr. 61 oder Seq ID Nr. 63 durch Deletion, Insertion, Substitution oder eine Kombination davon verändert sind und die noch mindestens 50% der enzymatischen Aktivität des Enzyms mit der jeweiligen Bezugssequenz besitzt, wobei unter enzymatische Aktivität für ein Enzym E₁ die Fähigkeit verstanden wird, Z-9-Octadecensäure zu 17-Hydroxy-Z-9-Octadecensäure umzusetzen,
ein Enzym E₂ mit Polypeptidsequenz Seq ID Nr. 8 oder Seq ID Nr. 11 oder mit einer Polypeptidsequenz bei der bis zu 60% der Aminosäurereste gegenüber Seq ID Nr. 8 oder Seq ID Nr. 11 durch Deletion, Insertion, Substitution oder eine Kombination davon verändert sind und die noch mindestens 50% der enzymatischen Aktivität des Enzyms mit der jeweiligen Bezugssequenz Nr. 8 oder 11 besitzt, wobei unter enzymatische Aktivität für ein Enzym E₂ die Fähigkeit verstanden wird, UDP-Glukose und 17-Hydroxy-Z-9-Octadecensäure zu 17-(β-D-Glucopyranosyl-Oxy)-Z-9-Octadecensäure umzusetzen,
ein Enzym E₃ mit Polypeptidsequenz Seq ID Nr. 11 oder mit einer Polypeptidsequenz bei der bis zu 60% der Aminosäurereste gegenüber Seq ID Nr. 11 durch Deletion, Insertion, Substitution oder eine Kombination davon verändert sind und die noch mindestens 50% der enzymatischen Aktivität des Enzyms mit der Bezugssequenz 11 besitzt, wobei unter enzymatische Aktivität für ein Enzym E₃ die Fähigkeit verstanden wird, 17-(β-D-Glucopyranosyl-Oxy)-Z-9-Octadecensäure und UDP-Glukose zu 17-L-[(2'-*O*-β-D-Glucopyranosyl-β-D-Glucopyranosyl)-Oxy]-Z-9-Octadecensäure umzusetzen,
ein Enzym E₄ mit Polypeptidsequenz Seq ID Nr. 9 oder mit einer Polypeptidsequenz bei der bis zu 50% der Aminosäurereste gegenüber Seq ID Nr. 9 durch Deletion, Insertion, Substitution oder eine Kombination davon verändert sind und die noch mindestens 50% der enzymatischen Aktivität des Enzyms mit Seq ID Nr. 9 besitzt, wobei unter enzymatische Aktivität für ein Enzym E₄ die Fähigkeit verstanden wird, 17-L-[(2'-*O*-β-D-Glucopyranosyl-β-D-Glucopyranosyl)-Oxy]-Z-9-Octadecensäure-1',4"-Lacton und Acetyl-Coenzym A zu 17-L-[(2'-*O*-β-D-Glucopyranosyl-β-D-Glucopyranosyl)-Oxy]-Z-9-Octadecensäure-1',4"-Lacton-Monoacetat oder (2'-*O*-β-D-Glucopyranosyl-β-D-Glucopyranosyl)-Oxy]-Z-9-Octadecensäure-1',4"-Lacton-Monoacetat und Acetyl-Coenzym A zu 17-L-[(2'-*O*-β-D-Glucopyranosyl-β-D-Glucopyranosyl)-Oxy]-Z-9-Octadecensäure-1',4"-Lacton-Diacetat oder 17-L-[(2'-*O*-β-D-Glucopyranosyl-β-D-Glucopyranosyl)-Oxy]-Z-9-Octadecensäure-1',4"-Lacton und Acetyl-Coenzym A zu 17-L-[(2'-*O*-β-D-Glucopyranosyl-β-D-Glucopyranosyl)-Oxy]-Z-9-Octadecensäure-1',4"-Lacton-Diacetat umzusetzen und
ein Enzym E₅ mit Polypeptidsequenz Seq ID Nr. 10 oder mit einer Polypeptidsequenz bei der bis zu 45% der Aminosäurereste gegenüber Seq ID Nr. 10 durch Deletion, Insertion, Substitution oder eine Kombination davon verändert sind und die noch mindestens 50% der enzymatischen Aktivität des Enzyms mit Seq ID Nr. 10 besitzt, wobei unter enzymatische Aktivität für ein Enzym E₅ die Fähigkeit verstanden wird, ein Sophorolipid aus einer Zelle heraus ins umgebende Medium zu transferieren.

## Claims

1. Sophorolipid-forming cell which is genetically modified in such a way that it has an activity, as specified in each case hereinbelow, of at least one of the enzymes selected from the group hereafter, which activity is modified in comparison with its wild type:
at least one enzyme E₁ with the polypeptide sequence Seq ID No. 7, Seq ID No. 53, Seq ID No. 55, Seq ID No. 57, Seq ID No. 59, Seq ID No. 61 or Seq ID No. 63 or with a polypeptide sequence where up to 25% of the amino acid residues are modified over Seq ID No. 7, Seq ID No. 53, Seq ID No. 55, Seq ID No. 57, Seq ID No. 59, Seq ID No. 61 or Seq ID No. 63 by deletion, insertion, substitution or a combination of these and which retains at least 50% of the enzymatic activity of the enzyme with the respective reference sequence Seq ID No. 7, Seq ID No. 53, Seq ID No. 55, Seq ID No. 57, Seq ID No. 59, Seq ID No. 61 or Seq ID No. 63, where enzymatic activity for an enzyme E₁ is understood as meaning the ability to convert Z-9-octadecenoic acid into 17-hydroxy-Z-9-octadecenoic acid,
at least one enzyme E₂ with the polypeptide sequence Seq ID No. 8 or Seq ID No. 11 or with a polypeptide sequence where up to 60% of the amino acid residues are modified over Seq ID No. 8 or Seq ID No. 11 by deletion, insertion, substitution or a combination of these and which retains at least 50% of the enzymatic activity of the enzyme with the respective reference sequence Seq ID No. 8 or Seq ID No. 11, where enzymatic activity for an enzyme E₂ is understood as meaning the ability to convert UDP-glucose and 17-hydroxy-Z-9-octadecenoic acid into 17-(β-D-glucopyranosyloxy)-Z-9-octadecenoic acid,
at least one enzyme E₃ with the polypeptide sequence Seq ID No. 11 or with a polypeptide sequence where up to 60% of the amino acid residues are modified over Seq ID No. 11 by deletion, insertion, substitution or a combination of these and which retains at least 50% of the enzymatic activity of the enzyme with the reference sequence Seq ID No. 11, where enzymatic activity for an enzyme E₃ is understood as meaning the ability to convert 17-(β-D-glucopyranosyloxy)-Z-9-octadecenoic acid and UDP-glucose into 17-L-[(2'-*O*-β-D-glucopyranosyl-β-D-glucopyranosyl)oxy]-Z-9-octadecenoic acid,
at least one enzyme E₄ with the polypeptide sequence Seq ID No. 9 or with a polypeptide sequence where up to 50% of the amino acid residues are modified over Seq ID No. 9 by deletion, insertion, substitution or a combination of these and which retains at least 50% of the enzymatic activity of the enzyme with Seq ID No. 9, where enzymatic activity for an enzyme E₄ is understood as meaning the ability to convert 17-L-[(2'-*O*-β-D-glucopyranosyl-β-D-glucopyranosyl)oxy]-Z-9-octadecenoic acid 1',4"-lactone and acetyl-coenzyme A into 17-L-[(2'-*O*-β-D-glucopyranosyl-β-D-glucopyranosyl)oxy]-Z-9-octadecenoic acid 1',4"-lactone monoacetate or 17-L-[(2'-*O*-β-D-glucopyranosyl-β-D-glucopyranosyl)oxy]-Z-9-octadecenoic acid 1',4"-lactone monoacetate and acetyl-coenzyme A into 17-L-[(2'-*O*-β-D-glucopyranosyl-β-D-glucopyranosyl)oxy]-2-9-octadecenoic acid 1',4"-lactone diacetate or 17-L-[(2'-*O*-β-D-glucopyranosyl-β-D-glucopyranosyl)oxy]-Z-9-octadecenoic acid 1',4"-lactone and acetyl-coenzyme A into 17-L-[(2'-*O*-β-D-glucopyranosyl-β-D-glucopyranosyl)oxy]-Z-9-octadecenoic acid 1',4"-lactone diacetate,
at least one enzyme E₅ with the polypeptide sequence Seq ID No. 10 or with a polypeptide sequence where up to 45% of the amino acid residues are modified over Seq ID No. 10 by deletion, insertion, substitution or a combination of these and which retains at least 50% of the enzymatic activity of the enzyme with Seq ID No. 10, where enzymatic activity for an enzyme E₅ is understood as meaning the ability to transfer a sophorolipid out of a cell into the surrounding medium,
**characterized in that**
it has increased activities of the following enzyme combinations:
E₁E₂, E₁E₃, E₁E₄, E₁E₅, E₂E₃, E₂E₄, E₂E₅, E₃E₄, E₃E₅, E₄E₅, E₁E₂E₃, E₁E₂E₄, E₁E₂E₅, E₁E₃E₄, E₁E₃E₅, E₁E₄E₅, E₂E₃E₄, E₂E₄E₅, E₃E₄E₅, E₁E₂E₃E₄, E₂E₃E₄E₅, E₁E₃E₄E₅, E₁E₂E₄E₅, E₁E₂E₃E₅, E₁E₂E₃E₄ and E₁E₂E₃E₄E₅
or **in that** it has a reduced activity of the enzyme E₃ and optionally an increased activity of the following enzyme combinations:
E₁E₂, E₁E₄, E₁E₅, E₂E₄, E₂E₅, E₄E₅, E₁E₂E₄, E₁E₂E₅, E₁E₄E₅ and E₁E₂E₄E₅
or **in that** it has a reduced activity of the enzyme E₄ and optionally an increased activity of the following enzyme combinations:
E₁E₂, E₁E₃, E₁E₅, E₂E₃, E₂E₅, E₃E₅, E₁E₂E₃, E₁E₂E₅, E₁E₃E₅ and E₁E₂E₃E₅
or **in that** it has a reduced activity of the enzymes E₃ and E₄ and optionally an increased activity of the following enzyme combinations:
E₁E₂, E₁E₅, E₂E₅, E₁E₂E₅.

2. Cell according to Claim 1, **characterized in that** it is at least partially blocked in its β-oxidation.

3. Cell according to Claim 1 or 2, **characterized in that** it is transformed with at least one nucleic acid according to Claim 5.

4. Process for the production of sophorolipids, comprising the process steps:
I) bringing a cell according to at least one of Claims 1 and 2 into contact with a medium comprising a carbon source,
II) culturing the cell under conditions which allow the cell to form a sophorolipid from the carbon source, and
III) optionally isolating the formed sophorolipids.

5. Isolated DNA which is selected from among the following sequences:
A) a sequence according to Seq ID No. 3, Seq ID No. 4, Seq ID No. 5, Seq ID No. 6, Seq ID No. 56, Seq ID No. 58, Seq ID No. 60 or Seq ID No. 62,
where the sequence according to Seq ID No. 56, Seq ID No. 58, Seq ID No. 60 or Seq ID No. 62 encodes a protein which is capable of converting
Z-9-octadecenoic acid into 17-hydroxy-Z-9-octadecenoic acid,
where the sequence Seq ID No. 3 encodes a protein which is capable of converting UDP-glucose and 17-hydroxy-Z-9-octadecenoic acid into 17-(β-D-glucopyranosyloxy)-Z-9-octadecenoic acid,
where the sequence Seq ID No. 4 encodes a protein which is capable of converting 17-L-[(2'-*O*-β-D-glucopyranosyl-β-D-glucopyranosyl)oxy]-Z-9-octadecenoic acid 1',4"-lactone and acetyl-coenzyme A into 17-L-[(2'-*O*-β-D-glucopyranosyl-β-D-glucopyranosyl)oxy]-Z-9-octadecenoic acid 1',4"-lactone monoacetate or 17-L-[(2'-*O*-β-D-glucopyranosyl-β-D-glucopyranosyl)oxy]-Z-9-octadecenoic acid 1',4"-lactone monoacetate and acetyl-coenzyme A into 17-L-[(2'-*O*-β-D-glucopyranosyl-β-D-glucopyranosyl)oxy]-Z-9-octadecenoic acid 1',4"-lactone diacetate or 17-L-[(2'-*O*-β-D-glucopyranosyl-β-D-glucopyranosyl)oxy]-Z-9-octadecenoic acid 1',4"-lactone and acetyl-coenzyme A into 17-L-[(2'-*O*-β-D-glucopyranosyl-β-D-glucopyranosyl)oxy]-Z-9-octadecenoic acid 1',4"-lactone diacetate,
where the sequence Seq ID No. 5 encodes a protein which is capable of transferring a sophorolipid out of a cell into the surrounding medium,
where the sequence Seq ID No. 6 encodes a protein which is capable of converting UDP-glucose and 17-hydroxy-Z-9-octadecenoic acid into 17-((β-D-glucopyranosyloxy)-Z-9-octadecenoic acid or 17-(β-D-glucopyranosyloxy)-Z-9-octadecenoic acid and UDP-glucose into 17-L-[(2'-*O*-β-D-glucopyranosyl-β-D-glucopyranosyl)oxy]-Z-9-octadecenoic acid,
B) an intron-free sequence which is derived from a sequence according to A) and which encodes the same protein or peptide as the sequence according to Seq ID No. 3, Seq ID No. 4, Seq ID No. 5, Seq ID No. 6, Seq ID No. 56, Seq ID No. 58, Seq ID No. 60 or Seq ID No. 62,
C) a sequence which encodes a protein or peptide which comprises the amino acid sequence according to Seq ID No. 8, Seq ID No. 9, Seq ID No. 10, Seq ID No. 11, Seq ID No. 57, Seq ID No. 59, Seq ID No. 61 or Seq ID No. 63, where the protein or peptide which comprises the amino acid sequence according to Seq ID No. 57, Seq ID No. 59, Seq ID No. 61 or Seq ID No. 63 is capable of converting Z-9-octadecenoic acid into 17-hydroxy-Z-9-octadecenoic acid,
D) a sequence which is to at least 90% identical to a sequence according to one of groups A) to C) with Seq ID No. 3, Seq ID No. 4, Seq ID No. 5, Seq ID No. 6, Seq ID No. 62, Seq ID No. 8, Seq ID No. 9, Seq ID No. 10, Seq ID No. 11 or Seq ID No. 63 as reference sequence, and to at least 95% identical to a sequence according to one of groups A) to C) with Seq ID No. 56, Seq ID No. 58, Seq ID No. 60, Seq ID No. 57, Seq ID No. 59 or Seq ID No. 61 as reference sequence,
E) a sequence which hybridizes or, taking into consideration the degeneracy of the genetic code, would hybridize to the counterstrand of a sequence according to one of groups A) to D),
F) a derivative of a sequence according to one of groups A) to E) which is obtained by substitution, addition, inversion and/or deletion of one or more bases, and
G) a complementary sequence to a sequence according to one of groups A) to F).

6. Vector comprising a DNA sequence according to one of groups A) to G) as defined in Claim 5.

7. Use of the vector according to Claim 6 for transforming a cell.

8. Isolated polypeptide selected from the group consisting of
an enzyme E₁ with the polypeptide sequence Seq ID No. 57, Seq ID No. 59, Seq ID No. 61 or Seq ID No. 63 or with a polypeptide sequence where up to 10% of the amino acid residues are modified over the respective reference sequence Seq ID No. 57, Seq ID No. 59, Seq ID No. 61 or Seq ID No. 63 by deletion, insertion, substitution or a combination of these and which retains at least 50% of the enzymatic activity of the enzyme with the respective reference sequence, where enzymatic activity for an enzyme E₁ is understood as meaning the ability to convert Z-9-octadecenoic acid into 17-hydroxy-Z-9-octadecenoic acid, an enzyme E₂ with the polypeptide sequence Seq ID No. 8 or Seq ID No. 11 or with a polypeptide sequence where up to 60% of the amino acid residues are modified over Seq ID No. 8 or Seq ID No. 11 by deletion, insertion, substitution or a combination of these and which retains at least 50% of the enzymatic activity of the enzyme with the respective reference sequence No. 8 or 11, where enzymatic activity for an enzyme E₂ is understood as meaning the ability to convert UDP-glucose and 17-hydroxy-Z-9-octadecenoic acid into 17-(β-D-glucopyranosyloxy)-Z-9-octadecenoic acid, an enzyme E₃ with the polypeptide sequence Seq ID No. 11 or with a polypeptide sequence where up to 60% of the amino acid residues are modified over Seq ID No. 11 by deletion, insertion, substitution or a combination of these and which retains at least 50% of the enzymatic activity of the enzyme with the respective reference sequence 11, where enzymatic activity for an enzyme E₃ is understood as meaning the ability to convert 17-(β-D-glucopyranosyloxy)-Z-9-octadecenoic acid and UDP-glucose into 17-L-[(2'-*O*-β-D-glucopyranosyl-β-D-glucopyranosyl)oxy]-Z-9-octadecenoic acid, an enzyme E₄ with the polypeptide sequence Seq ID No. 9 or with a polypeptide sequence where up to 50% of the amino acid residues are modified over Seq ID No. 9 by deletion, insertion, substitution or a combination of these and which retains at least 50% of the enzymatic activity of the enzyme with Seq ID No. 9, where enzymatic activity for an enzyme E₄ is understood as meaning the ability to convert 17-L-[(2'-*O*-β-D-glucopyranosyl-β-D-glucopyranosyl)oxy]-Z-9-octadecenoic acid 1',4"-lactone and acetyl-coenzyme A into 17-L-[(2'-*O*-β-D-glucopyranosyl-β-D-glucopyranosyl)oxy]-Z-9-octadecenoic acid 1',4"-lactone monoacetate or 17-L-[(2'-*O*-β-D-glucopyranosyl-β-D-glucopyranosyl)oxy]-Z-9-octadecenoic acid 1',4"-lactone monoacetate and acetyl-coenzyme A into 17-L-[(2'-*O*-β-D-glucopyranosyl-β-D-glucopyranosyl)oxy]-Z-9-octadecenoic acid 1',4"-lactone diacetate or 17-L-[(2'-*O*-β-D-glucopyranosyl-β-D-glucopyranosyl)oxy]-Z-9-octadecenoic acid 1',4"-lactone and acetyl-coenzyme A into 17-L-[(2'-*O*-β-D-glucopyranosyl-β-D-glucopyranosyl)oxy]-Z-9-octadecenoic acid 1',4"-lactone diacetate and an enzyme E₅ with the polypeptide sequence Seq ID No. 10 or with a polypeptide sequence where up to 45% of the amino acid residues are modified over Seq ID No. 10 by deletion, insertion, substitution or a combination of these and which retains at least 50% of the enzymatic activity of the enzyme with Seq ID No. 10, where enzymatic activity for an enzyme E₅ is understood as meaning the ability to transfer a sophorolipid out of a cell into the surrounding medium.

## Revendications

1. Cellule formant des sophorolipides, qui est modifiée par génie génétique de sorte qu'elle présente en comparaison de son type sauvage une activité modifiée, telle que spécifiée respectivement par la suite, d'au moins une des enzymes choisies dans le groupe constitué par :
au moins une enzyme E₁ ayant une séquence polypeptidique selon SEQ ID NO. 7, SEQ ID NO. 53, SEQ ID NO. 55, SEQ ID NO. 57, SEQ ID NO. 59, SEQ ID NO. 61 ou SEQ ID NO. 63, ou ayant une séquence polypeptidique dans laquelle jusqu'à 25 % des radicaux acides aminés sont modifiés par rapport à SEQ ID NO. 7, SEQ ID NO. 53, SEQ ID NO. 55, SEQ ID NO. 57, SEQ ID NO. 59, SEQ ID NO. 61 ou SEQ ID NO. 63 par délétion, insertion, substitution ou une combinaison d'entre elles, et qui présente encore au moins 50 % de l'activité enzymatique de l'enzyme ayant la séquence de référence respective SEQ ID NO. 7, SEQ ID NO. 53, SEQ ID NO. 55, SEQ ID NO. 57, SEQ ID NO. 59, SEQ ID NO. 61 ou SEQ ID NO. 63, l'activité enzymatique pour une enzyme E₁ se rapportant à la capacité à transformer l'acide Z-9-octadécénique en acide 17-hydroxy-Z-9-octadécénique,
au moins une enzyme E₂ ayant une séquence polypeptidique selon SEQ ID NO. 8 ou SEQ ID NO. 11 ou ayant une séquence polypeptidique dans laquelle jusqu'à 60 % des radicaux acides aminés sont modifiés par rapport à SEQ ID NO. 8 ou SEQ ID NO. 11 par délétion, insertion, substitution ou une combinaison d'entre elles, et qui présente encore au moins 50 % de l'activité enzymatique de l'enzyme ayant la séquence de référence respective SEQ ID NO. 8 ou SEQ ID NO. 11, l'activité enzymatique pour une enzyme E₂ se rapportant à la capacité à transformer l'UDP-glucose et l'acide 17-hydroxy-Z-9-octadécénique en acide 17-(β-D-glucopyranosyl-oxy)-Z-9-octadécénique,
au moins une enzyme E₃ ayant une séquence polypeptidique SEQ ID NO. 11 ou ayant une séquence polypeptidique dans laquelle jusqu'à 60 % des radicaux acides aminés sont modifiés par rapport à SEQ ID NO. 11 par délétion, insertion, substitution ou une combinaison d'entre elles, et qui présente encore au moins 50 % de l'activité enzymatique de l'enzyme ayant la séquence de référence SEQ ID NO. 11, l'activité enzymatique pour une enzyme E₃ se rapportant à la capacité à transformer l'acide 17-(β-D-glucopyranosyl-oxy)-Z-9-octadécénique et l'UDP-glucose en acide 17-L-[(2'-*O*-β-D-glucopyranosyl-β-D-glucopyranosyl)-oxy]-Z-9-octadécénique,
au moins une enzyme E₄ ayant une séquence polypeptidique SEQ ID NO. 9 ou ayant une séquence polypeptidique dans laquelle jusqu'à 50 % des radicaux acides aminés sont modifiés par rapport à SEQ ID NO. 9 par délétion, insertion, substitution ou une combinaison d'entre elles, et qui présente encore au moins 50 % de l'activité enzymatique de l'enzyme ayant la SEQ ID NO. 9, l'activité enzymatique pour une enzyme E₄ se rapportant à la capacité à transformer l'acide 17-L-[(2'-*O*-β-D-glucopyranosyl-β-D-glucopyranosyl)-oxy]-Z-9-octadécénique-1',4"-lactone et l'acétyl-coenzyme A en monoacétate de l'acide 17-L-[(2'-*O*-β-D-glucopyranosyl-β-D-glucopyranosyl)-oxy]-2-9-octadécénique-1',4"-lactone ou le monoacétate de l'acide 17-L-[(2'-*O*-β-D-glucopyranosyl-β-D-glucopyranosyl)-oxy]-Z-9-octadécénique-l',4"-lactone et l'acétyl-coenzyme A en diacétate de l'acide 17-L-[(2'-*O*-β-D-glucopyranosyl-β-D-glucopyranosyl)-oxy]-Z-9-octadécénique-1',4"-lactone ou l'acide 17-L-[(2'-*O*-β-D-glucopyranosyl-β-D-glucopyranosyl)-oxy]-Z-9-octadécénique-1',4"-lactone et l'acétyl-coenzyme A en diacétate de l'acide 17-L-[(2'-*O*-β-D-glucopyranosyl-β-D-glucopyranosyl)-oxy]-Z-9-octadécénique-l',4"-lactone, au moins une enzyme E₅ ayant une séquence polypeptidique selon SEQ ID NO. 10 ou ayant une séquence polypeptidique dans laquelle jusqu'à 45 % des radicaux acides aminés sont modifiés par rapport à SEQ ID NO. 10 par délétion, insertion, substitution ou une combinaison d'entre elles, et qui présente encore au moins 50 % de l'activité enzymatique de l'enzyme ayant la SEQ ID NO. 10, l'activité enzymatique pour une enzyme E₅ se rapportant à la capacité à transférer un sophorolipide issu d'une cellule dans le milieu environnant,
**caractérisée en ce qu'**elle présente des activités augmentées des combinaisons d'enzymes suivantes :
E₁E₂, E₁E₃, E₁E₄, E₁E₅, E₂E₃, E₂E₄, E₂E₅, E₃E₄, E₃E₅, E₄E₅, E₁E₂E₃, E₁E₂E₄, E₁E₂E₅, E₁E₃E₄, E₁E₃E₅, E₁E₄E₅, E₂E₃E₄, E₂E₄E₅, E₃E₄E₅, E₁E₂E₃E₄, E₂E₃E₄E₅, E₁E₃E₄E₅, E₁E₂E₄E₅, E₁E₂E₃E₅, E₁E₂E₃E₄ et E₁E₂E₃E₄E₅,
ou **en ce qu'**elle présente une activité réduite de l'enzyme E₃ et éventuellement une activité augmentée des combinaisons d'enzymes suivantes :
E₁E₂, E₁E₄, E₁E₅, E₂E₄, E₂E₅, E₄E₅, E₁E₂E₄, E₁E₂E₅, E₁E₄E₅ et E₁E₂E₄E₅,
ou **en ce qu'**elle présente une activité réduite de l'enzyme E₄ et éventuellement une activité augmentée des combinaisons d'enzymes suivantes :
E₁E₂, E₁E₃, E₁E₅, E₂E₃, E₂E₅, E₃E₅, E₁E₂E₃, E₁E₂E₅, E₁E₃E₅ et E₁E₂E₃E₅,
ou **en ce qu'**elle présente une activité réduite des enzymes E₃ et E₄ et éventuellement une activité augmentée des combinaisons d'enzymes suivantes :
E₁E₂, E₁E₅, E₂E₅, E₁E₂E₅.

2. Cellule selon la revendication 1, **caractérisée en ce qu'**elle est au moins partiellement bloquée dans son oxydation β.

3. Cellule selon la revendication 1 ou 2, **caractérisée en ce qu'**elle est transformée avec au moins un acide nucléique selon la revendication 5.

4. Procédé de fabrication de sophorolipides comprenant les étapes de procédé suivantes :
I) la mise en contact d'une cellule selon au moins l'une quelconque des revendications 1 ou 2 avec un milieu contenant une source de carbone,
II) la culture de la cellule dans des conditions qui permettent à la cellule de former des sophorolipides à partir de la source de carbone, et
III) éventuellement l'isolement des sophorolipides formés.

5. ADN isolé, qui est choisi parmi les séquences suivantes :
A) une séquence selon SEQ ID NO 3, SEQ ID NO 4, SEQ ID NO 5, SEQ ID NO 6, SEQ ID NO 56, SEQ ID NO 58, SEQ ID NO 60 ou SEQ ID NO 62,
la séquence selon SEQ ID NO 56, SEQ ID NO 58, SEQ ID NO 60 ou SEQ ID NO 62 codant pour une protéine qui est en mesure de transformer l'acide Z-9-octadécénique en acide 17-hydroxy-Z-9-octadécénique,
la séquence SEQ ID NO 3 codant pour une protéine qui est en mesure de transformer l'UDP-glucose et l'acide 17-hydroxy-Z-9-octadécénique en acide 17-(β-D-glucopyranosyl-oxy)-Z-9-octadécénique,
la séquence SEQ ID NO 4 codant pour une protéine qui est en mesure de transformer l'acide 17-L-[(2'-*O*-β-D-glucopyranosyl-β-D-glucopyranosyl)-oxy]-Z-9-octadécénique-1',4"-lactone et l'acétyl-coenzyme A en monoacétate de l'acide 17-L-[(2'-*O*-β-D-glucopyranosyl-β-D-glucopyranosyl)-oxy]-Z-9-octadécénique-1',4"-lactone ou le monoacétate de l'acide 17-L-[(2'-*O*-β-D-glucopyranosyl-β-D-glucopyranosyl)-oxy]-Z-9-octadécénique-1',4"-lactone et l'acétyl-coenzyme A en diacétate de l'acide 17-L-[(2'-*O*-β-D-glucopyranosyl-β-D-glucopyranosyl)-oxy]-Z-9-octadécénique-l',4"-lactone ou l'acide 17-L-[(2'-*O*-β-D-glucopyranosyl-β-D-glucopyranosyl)-oxy]-Z-9-octadécénique-l',4"-lactone et l'acétyl-coenzyme A en diacétate de l'acide 17-L-[(2'-*O*-β-D-glucopyranosyl-β-D-glucopyranosyl)-oxy]-Z-9-octadécénique-1',4"-lactone,
la séquence SEQ ID NO 5 codant pour une protéine qui est en mesure de transférer un sophorolipide issu d'une cellule dans le milieu environnant,
la séquence SEQ ID NO 6 codant pour une protéine qui est en mesure de transformer l'UDP-glucose et l'acide 17-hydroxy-Z-9-octadécénique en acide 17-(β-D-glucopyranosyl-oxy)-Z-9-octadécénique ou l'acide 17-(β-D-glucopyranosyl-oxy)-Z-9-octadécénique et l'UDP-glucose en acide 17-L-[(2'-*O*-β-D-glucopyranosyl-β-D-glucopyranosyl)-oxy]-Z-9-octadécénique,
B) une séquence exempte d'introns, qui est dérivée d'une séquence selon A) et qui code pour la même protéine ou le même peptide que la séquence selon SEQ ID NO 3, SEQ ID NO 4, SEQ ID NO 5, SEQ ID NO 6, SEQ ID NO 56, SEQ ID NO 58, SEQ ID NO 60 ou SEQ ID NO 62,
C) une séquence qui code pour une protéine ou un peptide qui comprend la séquence d'acides aminés selon SEQ ID NO 8, SEQ ID NO 9, SEQ ID NO 10, SEQ ID NO 11, SEQ ID NO 57, SEQ ID NO 59, SEQ ID NO 61 ou SEQ ID NO 63, la protéine ou le peptide qui comprend la séquence d'acides aminés selon SEQ ID NO 57, SEQ ID NO 59, SEQ ID NO 61 ou SEQ ID NO 63 étant en mesure de transformer l'acide Z-9-octadécénique en acide 17-hydroxy-Z-9-octadécénique,
D) une séquence qui est identique à une séquence selon l'un quelconque des groupes A) à C) avec SEQ ID NO 3, SEQ ID NO 4, SEQ ID NO 5, SEQ ID NO 6, SEQ ID NO 62, SEQ ID NO 8, SEQ ID NO 9, SEQ ID NO 10, SEQ ID NO 11 ou SEQ ID NO 63 en tant que séquence de référence à hauteur d'au moins 90 %, avec SEQ ID NO 56, SEQ ID NO 58, SEQ ID NO 60, SEQ ID NO 57, SEQ ID NO 59 ou SEQ ID NO 61 en tant que séquence de référence à hauteur d'au moins 95 %,
E) une séquence qui s'hybride avec le brin opposé d'une séquence selon l'un quelconque des groupes A) à D) ou s'hybriderait en prenant en compte la dégénération du code génétique,
F) un dérivé d'une séquence selon l'un quelconque des groupes A) à E) obtenu par substitution, addition, inversion et/ou délétion d'une ou de plusieurs bases, et
G) une séquence complémentaire d'une séquence selon l'un quelconque des groupes A) à F).

6. Vecteur, comprenant une séquence d'ADN selon l'un quelconque des groupes A) à G), tels que définis dans la revendication 5.

7. Utilisation du vecteur selon la revendication 6 pour la transformation d'une cellule.

8. Polypeptide isolé choisi dans le groupe constitué par :
une enzyme E₁ ayant une séquence polypeptidique SEQ ID SEQ ID NO. 57, SEQ ID NO. 59, SEQ ID NO. 61 ou SEQ ID NO. 63, ou ayant une séquence polypeptidique dans laquelle jusqu'à 10 % des radicaux acides aminés sont modifiés par rapport à la séquence de référence respective SEQ ID NO. 57, SEQ ID NO. 59, SEQ ID NO. 61 ou SEQ ID NO. 63 par délétion, insertion, substitution ou une combinaison d'entre elles, et qui présente encore au moins 50 % de l'activité enzymatique de l'enzyme ayant la séquence de référence respective, l'activité enzymatique pour une enzyme E₁ se rapportant à la capacité à transformer l'acide Z-9-octadécénique en acide 17-hydroxy-Z-9-octadécénique,
une enzyme E₂ ayant une séquence polypeptidique SEQ ID NO. 8 ou SEQ ID NO. 11 ou ayant une séquence polypeptidique dans laquelle jusqu'à 60 % des radicaux acides aminés sont modifiés par rapport à SEQ ID NO. 8 ou SEQ ID NO. 11 par délétion, insertion, substitution ou une combinaison d'entre elles, et qui présente encore au moins 50 % de l'activité enzymatique de l'enzyme ayant la séquence de référence respective SEQ ID NO. 8 ou 11, l'activité enzymatique pour une enzyme E₂ se rapportant à la capacité à transformer l'UDP-glucose et l'acide 17-hydroxy-Z-9-octadécénique en acide 17-(β-D-glucopyranosyl-oxy)-Z-9-octadécénique,
une enzyme E₃ ayant une séquence polypeptidique SEQ ID NO. 11 ou ayant une séquence polypeptidique dans laquelle jusqu'à 60 % des radicaux acides aminés sont modifiés par rapport à SEQ ID NO. 11 par délétion, insertion, substitution ou une combinaison d'entre elles, et qui présente encore au moins 50 % de l'activité enzymatique de l'enzyme ayant la séquence de référence SEQ ID NO. 11, l'activité enzymatique pour une enzyme E₃ se rapportant à la capacité à transformer l'acide 17-(β-D-glucopyranosyl-oxy)-Z-9-octadécénique et l'UDP-glucose en acide 17-L-[(2'-*O*-β-D-glucopyranosyl-β-D-glucopyranosyl)-oxy]-Z-9-octadécénique,
une enzyme E₄ ayant une séquence polypeptidique SEQ ID NO. 9 ou ayant une séquence polypeptidique dans laquelle jusqu'à 50 % des radicaux acides aminés sont modifiés par rapport à SEQ ID NO. 9 par délétion, insertion, substitution ou une combinaison d'entre elles, et qui présente encore au moins 50 % de l'activité enzymatique de l'enzyme ayant la SEQ ID NO. 9, l'activité enzymatique pour une enzyme E₄ se rapportant à la capacité à transformer l'acide 17-L-[(2'-*O*-β-D-glucopyranosyl-β-D-glucopyranosyl)-oxy]-Z-9-octadécénique-1',4"-lactone et l'acétyl-coenzyme A en monoacétate de l'acide 17-L-[(2'-*O*-β-D-glucopyranosyl-β-D-glucopyranosyl)-oxy]-2-9-octadécénique-1',4"-lactone ou le monoacétate de l'acide 17-L-[(2'-*O*-β-D-glucopyranosyl-β-D-glucopyranosyl)-oxy]-Z-9-octadécénique-1',4"-lactone et l'acétyl-coenzyme A en diacétate de l'acide 17-L-[(2'-*O*-β-D-glucopyranosyl-β-D-glucopyranosyl)-oxyl]-Z-9-octadécénique-1',4"-lactone ou l'acide 17-L-[(2'-*O*-β-D-glucopyranosyl-β-D-glucopyranosyl)-oxy]-Z-9-octadécénique-1',4"-lactone et l'acétyl-coenzyme A en diacétate de l'acide 17-L-[(2'-*O*-β-D-glucopyranosyl-β-D-glucopyranosyl)-oxy]-Z-9-octadécénique-1',4"-lactone, et
une enzyme E₅ ayant une séquence polypeptidique SEQ ID NO. 10 ou ayant une séquence polypeptidique dans laquelle jusqu'à 45 % des radicaux acides aminés sont modifiés par rapport à SEQ ID NO. 10 par délétion, insertion, substitution ou une combinaison d'entre elles, et qui présente encore au moins 50 % de l'activité enzymatique de l'enzyme ayant la SEQ ID NO. 10, l'activité enzymatique pour une enzyme E₅ se rapportant à la capacité à transférer un sophorolipide issu d'une cellule dans le milieu environnant.


### SEQUENCE LISTING

<110> Evonik Degussa GmbH
<120> Zellen, Nukleinsäuren, Enzyme und deren Verwendung sowie Verfahren zur Herstellung von Sophorolipiden
<130> 200900338
<160> 90
<170> PatentIn version 3.4
<210> 1
   <211> 18013
   <212> DNA
   <213> Candida bombicola
<400> 1
   caaactcgac gctaaacaga ccttaaatga caccaatcaa tgtgaaaaaa tcaagttttt     60
   ttgttcactc tatattgact gtttccgatg tgtgctatgc agccctcttt gaatcggtgg    120
   aagcatgtag ttgaagaaag atggacgtag gagaaacatc aaactgaaca atagtaactt    180
   aaacgtggtt tagaatgcaa gagcaggctc gctgctatgg cattcatagc caggaaagaa    240
   acacggatga tctcacactt tgttggatcg acagtcggat ttttttgaaa atttatactt    300
   ggcatacatc ttaatacagg ggtagaagga gaagtcgcga gagcgatttc tccgtcattt    360
   attcgccgac aaatgtggat ccgtatttag cagattcgaa gtaaattgca ctcgacacca    420
   cccacgtgat cgacactgtc gcgtcgatct ccatatatgt acgtgcctat ataaacaagc    480
   aacacgcaga ttttgaaatc acatagggag ttgcccgtat gaatccggtt caaataataa    540
   tactttgttt tcagatagga gaaacaaaac acccttggta ctcagaagac aaataacgat    600
   ccattgtttt caactggaag aaataataca cattgatatt cagaagacaa ataactatcc    660
   catttcttta gtatgtgcga aggtaaacag ttctatttca ccttaaaaac actactgaaa    720
   gtgcgacata ctgtcgtacg taaaatataa aagcaatcac tatcatttcg ccattatcct    780
   tgtcttgtaa taatccaaaa ctgagatcgg gaacggttcc cgttcttgac ataagcagga    840
   gctgagaaca ggaacggttc ctggtcttga aatcagcagt aatagagaac gggattggtt    900
   cccgttcttg acataagcag gaattggaaa caggaacggt tcccggtctt gacatcagca    960
   gggatcgaaa acaggagcgg tttccggtct tgacatgata caaagaatga ttctttgtat   1020
   cgggtctatg ggaggaaaaa cagctcattt tcacagaaaa tacagagaac aaaataattg   1080
   aaagcgcgac ataatgtcgt acgtagaatt tagaagcaat tactcttatt tttccattat   1140
   ccgcgctatt gtacacacac ccaaaccaga acgcgacttg agtgcaatgc ttactaacgc   1200
   gcacattaat aagcaaatat agatacgcgg agagcacgcg aaatttgttt accagtacac   1260
   tagtgcttag cacaatgaaa tagaccgtac tccggctgag gctcaaagtc cagaagttag   1320
   agatttgcca gtttcgttac tagacggttc gttgtgccag gtatgtcgta cagcgcattt   1380
   atcagggacg gaaatgggtc ttccatccct gttttggaat gcgctgtcga tccggacgca 1440
   gcctcagccg cgtctatttc aaccccccat tagacaggcg gtacattagc tgtttggcct 1500
   tcacgctaca gcataattct ccgtcatgtg tgtttccatg accaagaatt gttttggccc 1560
   acgaaccaag atcatcgccg tcatataaac ccacattgga gtgttgactc tccatagctt 1620
   gtcgttgaat gcaaacttga tgcccgcaaa agtgcttatt agcctacgca ctgattcgcc 1680
   ccactctgcg agccacattt ccgctagctt aacatcaggc accgcaatcg gtgcctggac 1740
   tgtctccggg ctcggccgag cccggttgag accatcttct tcaaattcat cttctgatag 1800
   ctcatctaac atcctagagc tgttcctctt tttccttctt tttgttaatt ggtatttaaa 1860
   ccaccaagtg tgtaaacttg tatttttgtc atccgagaga tatctaatag caagtttgcg 1920
   attagttaca aatttgttgc gctcttgttc ggtactctta ttgaaacaag ggtgtcgact 1980
   cgccaaattc catcggagaa aattgttcga tggatagctt tggagtctgt cccatcatga 2040
   tacgaaaagc gtgaagctcc tctgacaatc aaaactttgt ttcaatgggg tgtaggatgg 2100
   accccggatc caaacgaccg cgagtcaaaa aacctacggg tgcatttacc cgtagttgat 2160
   ctggaaagtc gagatcaact ttttgtagtt tagttacatt catttcacgg tcgaaaaact 2220
   cacacacaac gattgcagta tatttaccaa aatcgtctga agagaagcat ctgattgaga 2280
   gttcaccatg acgaatccca taaacgacta ctccactgga cacaccgaca gacgccctgg 2340
   ggatagtgaa actgaatttg tcggtataat ggcccgtctc acaggccggg cagaacactt 2400
   tcatgtcctt tcgcaggtct cgacattgga caagtatgtt gtcgtgggtg acgacaaatt 2460
   ggtcctcatc cttgaataag atgctccctt tgttctcagg aactggcacc attccattat 2520
   gggcgaataa tttctgctca tcttcgggac tgatgccata ttcttctaac agaagacggc 2580
   gctcacatgg gacctggtgc tctcgccggc ctctcaaatc gccggtgcat ctccacacgc 2640
   aaattcacgg gtgtataccc ctgatcaaac gtatcttgcg cgttctgtta ttcattggag 2700
   cgagggcccg atcctgtcct atcaaatgat ttcatgtggg aataatccat caattgttct 2760
   ggattgaggt atacttcgag ctgtaaagat gtcgcttcta tgtcaagaat agtcggttaa 2820
   acgcactcct tcaagattta catgatttac atgattcttc ataaagagca taaataaaga 2880
   actgcagcca ttcttgagta aagtgctcag aataataaaa aggttgccac aggttgagtt 2940
   aacatgggtt gattgaacca attaaggagg gaacgtttct tccatgggag gctaagaaac 3000
   ttaagaaaac cgcacaacca caccgggagg agcgtgttga gctgtaagcg ttgttgagaa 3060
   acgaggggac tctgggaagt cgggacccat ctcaatcttg gaatactcct gtaagagtct 3120
   caccagagtt agcgaaagct ctgtcagggc gaattgttgg ccgagacaaa ttcggggacc 3180
   gccattgaag ggcaagaatg cccacacatt atctagcttc aagttctccc atcgattggg 3240
   attgaattcg tgggcgtcag gaccccaata cttgatgtcc ctgtggacca tgtaaattga 3300
   atagtaaact gcggtgccct taggaacgaa gatcggatcc ttctgctcgg gaccaccacc 3360
   tatgggtaga gttgtatctc tcacagcagt acggaagttc aatggcaata ccggcgcaag 3420
   acgcaagact tcatttataa cttgcttcaa ataaggtgct tgcttcagaa gttcgaatga 3480
   taaaggcctt tgctcctcct tggttccaaa atgatcgagg acctcctcac gtagtttgtt 3540
   gaatacgtca ggatttctgg caaggaaatg aatagcgaag ctcaacgtag cagctgttgt 3600
   atctctacca gcaatgagaa tgttgaaaat ttgatcacgt atcgtcactg ggtctcgggt 3660
   aactttagcc atctcaagcg agaacacata gatgccacta gactctgcag cagcatcctt 3720
   ctctgcaata gagttctcag cagcgaaaga tgtggcgtaa agagccttat caacgtagta 3780
   gtcaatatag gactgagcac gtttcttgtg atctcggaat tccttagagt tgaacaacca 3840
   gtagactttg cttgataggg tccgtttgaa agcgtaattc agtagaaagt tgtaggactc 3900
   cacgaattgt tcggcagtaa tctccgaacc atcacgggct acaatacatg actgattctc 3960
   agggttcaag ctctcgcagg actccccaaa taggaattca gtcgctgtat ccagcgtaag 4020
   tttgtggaaa taatgttgaa catcaataaa ttggtccact ttcattgcac ggttcatctc 4080
   ctttattaac tccgcagcat gactggaaat ctgatcaatt ctgcaaacct gatctttagt 4140
   gaactgaggt ctcaacatcg atcgagactg tttccatcca tttccgctga gtgtaaatat 4200
   cccttggcca aacacttttc ccactgtgtg gaaacgtgct ccaagaccaa aatcattgaa 4260
   tttggttgcc aggattgtct taatgttttc tggctcgatt gtgaagattt ggtattgaag 4320
   gggagcttgt cgaagatacg tccgtgcttt gaacttattg aagactctgt cgtattgaac 4380
   ttccagtaag gtgtatgact tggccgtctt gatcatgtcc atggttcttt gtattcccag 4440
   tgggaacgat ttctcaatga agcgaggcat actacacttg tgcctacgtg ctgcatagcg 4500
   gtaccatagg agccagatag gctcgtgtag aactaagaaa gctacgaaga gcagtggcaa 4560
   caagccagca acagcggata aactcattgg agttagaata atgtctttga ttaacatata 4620
   tgtacttttc aatatgataa acggagaaat aacgcccggc tctatatgca agctgcatca 4680
   accctaatat atattagcga gtttctcatg caggctgtag tttgagtcgc tgtaacctca 4740
   gcctcaagac tcttacacca taggtagagt ttcgtcactg ggaaactcag ttactatcta 4800
   aaccaaactg tgctaatgct caaacctatc actcagaatt tagattgaat caatctaagt 4860
   ctgttgagaa acagatatgc atcaggggca cagactaaaa gctgctctca gcgagtaccc 4920
   ttacctcttg agaaccctca aaatttaccc agcctgcagc atatcatgca ccatggttaa 4980
   attcggaaat gaatttaccg gtggccttga accacgttcc tccaattatt taaggcaata 5040
   acctgccact ctcttgattt gattaagaaa gactttcaat ttagcttctc cctacgaata 5100
   ttcaatgagc ccttcatcac acaaacccct gattctcgct tgcggcttgc ctctttcagg 5160
   ccatataatg cccgttttga gtctggtaca cggccttacg gacgacggat acgaagctac 5220
   tgttgtgaca ggcagagcgt ttgaacaaaa agttcgagat gtgggtgcag actttgttcc 5280
   tttagaaggg aacgcagatt ttgatgacca caccttagac gatctggtcc cgggccgtaa 5340
   agacatggcc ccaagcttcg atcgtacagt tcaagatgtg gagcacatga tggtagctac 5400
   tcttcctgag cagtttgccg ctattcagag ggctttcaaa aagctcagcg caagcggccg 5460
   ccctgtcgtt cttgtcagtg aagtgctgtt tttcggtgca caccctatca gcctcggtgc 5520
   tcctggtttc aaacccgctg gctggatttg tttaggggtt ttgcctcttt tgatccgcag 5580
   tgatcatacc ttaggacttg acaacgacag gagccccgaa gcacatgcaa agaaactcgc 5640
   tatgaaccac gctcttgagc accaaatttt cgttaaagcc actgctaagc acaaggaaat 5700
   ctgccgagag ttaggttgca ctgaagatcc caaatttatc tgggagcaca gttacattgc 5760
   tgcagacaag ttcctgcagc tgtgcccgcc ttctcttgag ttcagcagag accatctgcc 5820
   tagcaacttc aaattcgccg gctcaacgcc caagcaccga actcaattca cccctccttc 5880
   ctggtggggg gatgttctga gtgccaagcg agtcatcatg gtcactcaag gaacttttgc 5940
   tgtcagttac aagcatctta ttgtgcctac tcttgaggcc ttgaaggacg agcctgacac 6000
   tttaacagta gccatattgg gccgccgcgg tgccaagcta ccggatgatg ttgtggttcc 6060
   tgagaatgct cgcgtgatcg actacttcaa ctacgatgct ctacttcctc acgttgatgc 6120
   tcttgtctac aatggtggat atggcggact tcagcacagc ttaagccact ctgttccagt 6180
   tgttattgct ggtgactctg aagacaagcc aatggtggca tcgagagctg aggccgctgg 6240
   cgtggcaatt gatttgaaaa ctggcttgcc tacagtggag caaatcaaag aagctgttga 6300
   ttcgataatt ggaaatccga aattccacga agcctcgaag aaggttcaaa tggagttgga 6360
   aagccacaac tccttgaaaa ttcttgagga aagcatcgag gaaatcgcca gccatgactt 6420
   tggtcttttg accaagagtg acgaggaaac tgaagatata cctgtcaaag ggccggcctt 6480
   agcggtgagt tcttagaatc gtacgatcaa atcagatcag ggaagagagg tagggttttt 6540
   tttatttatg tctttgtttt tattgattga aatttacaat acaacaacca tcaaattaat 6600
   ttgaacaaac aacaacacac acacacactg caactttcaa aaaaataagt aaaaggaaga 6660
   gaggagtttg ccaatatatt taccttcttc taattctgtt atttttttta attgttttgt 6720
   ggaaagaaag aagaaaaggc tgtcatgaat ttagtttacc tagaccttct ggttagcggt 6780
   attgacgttc atttcaactg gaagaaggaa ttccagttcc tctccttcag cctcgtcggg 6840
   atcctcctct ggaatatgct tgaggattcg cgcagggact cctcccacca cagtacgagg 6900
   aggaacatct tctcgaacga cagcaccagc cgcaattgtt gagccatctc caatcgtaac 6960
   acccggcagg acagtcacat tcgcaccaat ccatacatta ttccccacct tgataggaag 7020
   agcatacaca attctcctcg cacgtttctc ggggctaata ggatgagtcg cagtcacgaa 7080
   cgttgtattg ggccctacaa tcacctcatc accaaagatt attggagccg agtccaagaa 7140
   gcaaacgttg aagttggcgt aaaagtgctc gcctacgctg atgttgaatc caaaatcaac 7200
   tgagaatgga gcggtcagcc agacaatatc ctttgtttga ccaaaagtgt ctttgagaat 7260
   ctcgaccttc ttgatataag cagcgtgatt tgactcaaaa gtacgacttt cacttgcaat 7320
   ggtattgaac tccctaactt tctcactagt agccagggct ctaaacataa gatctggatc 7380
   gtatggattg taaggaactc ctgagaccat cttctcatag ttttcattgc caggggtgtt 7440
   tttgaggttt tttttggccc aagagaccat ttcctggtca atttcttttc taggagtcat 7500
   tcctttgttt tgagggtcct tcgaggagtt tacaaccatt gaattctaga atgtgaggtg 7560
   gaatgaggca aggaaggagg aacgtattga gttgtacctt aagatatctc aaagtgctta 7620
   tctccgacta ccggaatatg ctccgggtaa tgcaagtcag tgtgcatatg ggtaaggtga 7680
   tgcaagctaa ccctcagggc atatctaatt cgcgtgaggg ttattattgg tctacattac 7740
   ctcagtcata gcccgtcaaa gcaaaagccc aaaatcagca cgaaatccca gagatagatt 7800
   gttgctgtct cttcaagtac tacgacagtt ccctatatct acagattatc gtcacgagtg 7860
   aattatgcag gataggtgac tcaggggtca taatcagagg aatccaatgt gctatttcaa 7920
   ttaacgagtc cctttaatca gacaatgtat ggtgactcag gggccataac tagagaaatt 7980
   cgatatgcta tttcaattaa tgagtgcctt taatcaaata atgtatgcaa gcagtggcca 8040
   aaaataaatg aacgtcaaat ctctccgaga ccttgcaagt tcaccaattc agcgtaccat 8100
   ccattgagtt caaggaggct ctgatggtcg ccctgctcca cgatgcgccc tcctgagaac 8160
   acatatatga catctgcttt ctgaattgtt gataatctat gcgcaacggc gattgtagta 8220
   cggcccttcg ctgctgcgtc gagtgctgct tgaactactt tctcagattc ggaatccaga 8280
   gctgaggtgg cctcatcgag gaggagtacc tttggatttc tgatcagggc ccttgcaatt 8340
   gcaattcgct gcttttgccc cccagatagc aacgatcccc tagatccgct gagcgtttcg 8400
   tagccatcag gcaacgacat gatgaattcg tgaatgttcg ctttgcgagc ggcatcctca 8460
   atcatctcct gcgttacttc agactcaggg ccagaccatc ccattagaat attctcacgt 8520
   agcgtgcctg aataaagcat tggttcttgc tggactaaag caatgtgtga tctcaatgca 8580
   ttcaggttat attcgcgtaa atctttccca tcgaaaagta cttgacctgc taatggatca 8640
   taaaatcttt ccaccagtcc aatagtagta gacttaccgc atccactggc tccaactaga 8700
   gcgatgtatt ggcccttttt gactgttaag ttgagatctt gtaaaactgg tacttgaggt 8760
   cgagtaggat atcggaaatt cacatgacgg aactcaatat ctcctctcac cgactcctcg 8820
   ggagcaacgt aaccttcctc actccataca tctatagaag gagtggcagt caagattctg 8880
   taaatgttac gcgctgcatc tttggctgag ttcatgtttg gagcatagct gaaaatttgg 8940
   ccagcggctt gagaacctgt aataatagcc atgaagacag tcatatatcc tgcgaccgaa 9000
   gcttcacctc gtctcattac agtgcttccc caccaaaaaa cgagggctac cacccagggt 9060
   gtcattcctt ccgagagtgc gtagtacaat gctgagcggg caatggcaat tctggagctg 9120
   aaaatctgag agtctactgt ctttgtgtat tttacgacca cgtctaactc acgagttaag 9180
   gactggactg tgcggacagc acttgtatac tcagatgcca tggagccact tcgttcgtaa 9240
   acttctctcg cacgatccga taattgggta agaacccaga ctctgacgaa gccacacacc 9300
   aacatgacag gaacaacaga cgtagccacg agtccaattc tccaattgaa aggtatacca 9360
   gtaactatgc cgccaatcaa ggtcaccaga ctctgttgaa tttgaccgag ggtggcccca 9420
   ctcaaaccct cgatcatttt agcttccttc gccaaaattg aggttagcgc acccggcgtg 9480
   ttgtttttgt ggtcgaagaa tgcaatatcc attcgcatca attggcggaa caaagctaat 9540
   ctgatatttt tgaccaactt atcagatgca agtgataaag cagctatagt gataaaagcc 9600
   gtcatgaatg aaatgcagcc tacgaaaaaa taccaccatc ccatgatatt caccacatgc 9660
   cgcatttttc cgtattcact gggaggtaga accatgcttc cagtggtttg gccagttatt 9720
   attgccattg caggatagca atagcccaaa ataatggagg ctaaactacc aatgagaatg 9780
   taaccccatt ctttcctatt cagcccccaa accagtttgg tattggtcat caacgtgcta 9840
   tgtggggggt tgcgcacacc agggatgtca ttttcttgat attcaggagg ttgagtggtc 9900
   tgagtacctg cactgtgaac actcaatgtg ctcacatcct tgggattgaa cttttcgttc 9960
   agtgagtcca gaggcgaaat gtctagagct tcaatatcga ggacctcaac gttagtgctc 10020
   tttgctttag ttactctttg agcatcaacc aaagctttat aaggcccttc tcgctgtatg 10080
   agctcattgt gagtaccctg ctctatgacg ttacctttag acatgacaac tatcttgttg 10140
   gcatccttga tcgtagagag tctgtgtgca acgactatag tggtacgacc ttcggccgct 10200
   ttgtcgagcg catcttgaac gataccttca gatttggtat ccagagcaga agtcgcttca 10260
   tcgagcagca gaattttagg gtctgagacg attgctcttg ctattgcaat gcgttgtttc 10320
   tgaccaccgc tgagaagaaa tcctcgatct ccaacattgg tttggatgcc ttctgagaga 10380
   gtctgaatga aatcccaggc attggcatct ttacaagctt gaatgatttt agcttcctta 10440
   acatgctcgt cagcgaactc aatgtcagtg ccaatcaaac catagctgat attctcatat 10500
   attgactctg aaaagagtac tggttcctgc tgaacataac caatttgttg acggagccat 10560
   cttgtgttca ggtcgctaat ctcctggcca tccagagtaa cgcttccttc gagaggtaaa 10620
   tagaacctct caagaatacc tacaattgta gacttccctg atcccgaggc acctaccagt 10680
   gccacagtag atccagcagg aacttcaagg ctaaaatcgg agaggaccaa aacgtctggg 10740
   cgactaggat atcggaactt gacatttttg agctcaattc tgccaacggc cttagtttgg 10800
   gggacaattc ctttatctat ggactggcca tcgatgactg ggacacgatc aatggcctca 10860
   ttgagaatgc tcgcggcagt gagacccttg acaagaaacc tcacgtttgg cgcgatattc 10920
   ccaagctgga agcttccaag taacatagct gtgattacaa ctattatctt tccaacgtca 10980
   gcactcccac taacgatttc tctggaaccc tgccacagag ctaaggcata cacccaaaaa 11040
   gtactagccc atatgcacgc taacatgacc cccaatgagt aactgctccg cttcgattcc 11100
   ttcacaacac gatcaagtac cttttcatac ttgacggcga gatgaggttg agcgccaaat 11160
   gctactgtag tcctgacagc actgagagcc tcctccgcaa cggtagctcc agactgcgaa 11220
   tatatcgcgt cagatctgag ctgatatttg gccatgaagg tggcgccagt tcccattgtg 11280
   attaccatga accctacagc actcaggagg atgcaagcca gtttccattg cgaagcaaaa 11340
   cttataacgg tggccgcaat gaaggaagct attccctgta cgacgtttcc aagcttgtcg 11400
   ctgatcgctt cctgaattga gttggtatcg ttaatgattc tggtgctgac ctcgccacca 11460
   cctagtttgt cgtaaaacgc gatattctgg cgaataacag cactcagata atgctttcgg 11520
   taacgtcctg ccaacacttc gcctctgtcc acaagcagga agctctcgag aaacgcactg 11580
   ccgagcatac caatgccaat atagacaaaa tagagagaca ggtgattcac cttatgctgg 11640
   aactcattgc ccttgaggtc atatgaagtg aagtctctga atgtgttgaa gatggcgccc 11700
   actactaacg tgaacattgg aagcgcggct ccatgcaccg ctgcaaaaaa aagcgcaagt 11760
   atctccaaga aaacgtcaag gggagtgcaa aatctgaaca acctgaaaaa gcttgtggcg 11820
   actctctttg tttcaagctg acttcgcaat acattggcct catgtggatc taacgcagag 11880
   agcttctcct cgagaagctt gtccttagtc tcgatgagtt tctcacgctt ctctacctgt 11940
   atatcatcca ccataagcca aaatcagaga gtgggacctg attcagaatc acacggaccc 12000
   gtatatataa caatcacttt ccaacaatat agcgagtatt aatatatttc cgggtaaggg 12060
   ttgttccgga cttatgcatt taatcacagg ttgcatcagc taaatatgtc agggccgacg 12120
   gcgtaaattt agaaggttag gtcaagatcc atcggtcagg ccaatggagc tctactatga 12180
   taggcagctg aagcgagaca agatatactt cagttgcgct ctctgaaaaa attattttgt 12240
   gattctcact cagtggatgt ggcgacacac ggaaccaata atctcgccgg aaaggcggct 12300
   gaacatcagt cttgcataag tgtgcaagtg gcctgagcac agcgtgcatt acccttacca 12360
   tacattcggg gcaagttaaa tccagcatta tataaacttg attgacacaa atgggcataa 12420
   aacaataaag tctcctatat ggccatcgag aaaccagtga tagttgcttg tgcctgccca 12480
   ctagcggggc acgtgggccc agtgctcagc ctggtccgcg gtctactcaa tagaggatat 12540
   gaggtgactt tcgtaacagg gaacgcattc aaggagaaag ttattgaggc aggatgcact 12600
   ttcgtccctc tccaaggacg agctgactac catgaataca atctccctga aatcgctcca 12660
   ggattgctca cgattcctcc aggccttgag cagaccggtt actcaatgaa tgagattttt 12720
   gtgaaggcga ttcctgagca gtacgatgca cttcaaactg ctctaaaaca ggttgaggct 12780
   gaaaataaat cagctgtggt gattggcgag accatgtttc taggggtgca tccgatatca 12840
   ctgggtgccc caggtctcaa gccccaaggc gtaatcacgt taggaactat tccgtgcatg 12900
   ctgaaagcag agaaggcgcc tggagttcct agtcttgagc caatgattga tactttagtg 12960
   cggcaacaag tatttcaacc aggaactgac tctgagaagg agatcatgaa gacgctcggg 13020
   gccacgaagg agcccgaatt tctcctggag aatatataca gcagccctga cagatttttg 13080
   caactgtgcc ctccatctct tgaatttcac ttgacttcgc ctcctcctgg cttctcgttc 13140
   gctggtagtg caccgcatgt aaagtctgct ggattagcaa ctccacctca cctgccgtct 13200
   tggtggcctg atgtgctgag tgcgaagcgt ctgattgttg ttacacaagg aacagcagcc 13260
   atcaactatg aagatctgct cattccagca ttgcaggcct ttgctgacga agaagacact 13320
   ctcgtagttg gtatattggg cgtcaaaggg gcgtcacttc ctgatagcgt taaagttcct 13380
   gcaaacgctc gaattgttga ttattttcct tacgatgagc tactaccgca tgcctctgtt 13440
   ttcatataca acggtggata cggaggtctg cagcacagtt tgagccatgg cgttcccgtc 13500
   atcatcggag gaggaatgtt ggtagacaag ccagctgttg cttcacgagc tgtatgggct 13560
   ggtgttggtt atgatcttca aaccttgcag gcaacttctg agctagtctc cacggccgtt 13620
   aaggaggtgt tggctactcc ctcgtatcac gagaaagcca tggcagtcaa gaaagagctt 13680
   gaaaaataca agtctcttga tattctagag tcggcaatta gtgaattagc ttcttaacct 13740
   ggctcttttt ctagatatgt ctgcgccctg ctcactgctt actggcctaa gctggtatta 13800
   cggaccttaa tcaagtatca ccccaaggca atcgagagtc ttatcgagtc tctaggtaga 13860
   tagatacacg ttttgatttt tcggcccact ttgtagaaaa atctcagtga tttcatggaa 13920
   ttcagttaca aatactaatc tgataaacca agaactacac tcggtgttga gagcagaatt 13980
   aaagggactt ggcgtctagc acaaaacgat acttgacgtc accactgtga acgcgcttcc 14040
   aagcttcggc gatatagctg tactcaatca gctcaacatc acaggtgatg ttattttcac 14100
   cacagaagtc cagcatctcc tgagtctctg gcaagccacc aatgtttgag taagtgatag 14160
   atttatttcc agccaaatga gaggtcagaa ccttgagggg tccaatttga ccaacaacaa 14220
   cgagacaccc accaatatca agggacttga ggtatggctc gaagtcgtgt tcaaagggaa 14280
   tggtgtcgat gatcaggtca aatgtgccag cgaccgcctc gagctcattc ggatcagagg 14340
   aagcaactac gcggctagca ccttgtgctt tcgctcctgc ggctttggcg tgactcctgc 14400
   tgaacagtgt gacttcagag cccatggctg aggcaaattt gatagccatg gaaccaaggc 14460
   ctccgagacc aactacaccg actctttttc caggtccggc gccgtgagcc ctcagaggag 14520
   agtaggtagt gataccagca cagagaaggg gcgcagaagc tgccaagtcg aggttggagg 14580
   ggattttgag cacaaactcc tcgcgagcaa gaatgtgttg cgaataccct cccttcgtga 14640
   cttccccgtt ctttccgctg gaattgtaag tttgagtgcg tgaaacacac caattttctt 14700
   tgcctaattt acagttcttg caagtacgac atgagtccac taagcagcca attccaacaa 14760
   tgtcgccagc ttggaacttc ttgacggccg ggccgacagc agtggccctt ccaataatct 14820
   catgcccacc aacaaaggga aattttgcat tgttccagtc gttgtgcgct gtatggagtt 14880
   cactgtgaca aattccacaa taaaggatct cgatgcttac gtcgttgggt cggggatcgc 14940
   gacgctcaat agtgccagga actgggtcgc tagttgtatc gtggactatg taggccttgc 15000
   aagttgaagg catcgtgaat tttgactgat ccgagcgcag tactctacgt ttagcttgaa 15060
   gtcgggagaa gggtccggat tagaagataa gcggcatcct gtgacaagca gtaaaaaaat 15120
   gcacccaaaa taaaagttgt gctaaggacc aagagttaga ttaaattcac tacctgatta 15180
   tgagctgttt agttttagaa ctttgttgct aaacaattat acgtggctat acaacctacc 15240
   caaaatttac aacgccgctt agctaatgac tacgcaaccc tactggatta ggctagggct 15300
   ccgagatagc gaaacgtggg gtagcgggcg acaggtcata tagagcccct accctactcg 15360
   gtgcaggtta ccgacggacg acatttggag tagtgatttt gactttccaa agatggaatt 15420
   tcctctgtag tgaaagatta ctgtatatat ttattggtcg catcgcttgc tcagtttgtg 15480
   atccaaccca gggttaatag tggtttaagc tgaactgcgg tgggaagccc agccggtgaa 15540
   aggagctttc tggagagcat acggcactaa tgagagcctc tgacaggctg cattcctttt 15600
   cccgcacgta cctgatatcc catcatgcgg gaccaggtta gggagtgggt tcagggttta 15660
   gatagtggag ctcattggta gctcaccagc gagctctgag tagatggctg tgtcacacat 15720
   tgaggcagaa gtttttctgt ctgaagtact gaagatttct tgctttggca acagtaatgg 15780
   ggccaggtcc gaaggctcgg caaacttaag ctcgaaatta gatgagcgta agattcactt 15840
   aacaacaaat tcgcgaagtc ctaggaagcg cgactgacag aggagtgttt cgttcaacaa 15900
   tttcgcgaag gattgcacta ctcaccaact catattaatt cagctaatgt ttctaatttt 15960
   caaaactagt acggaagtct gcagttagac agctcttgcg tttgaagaac ttaggcgcga 16020
   gatttctcag ctgtatctac acgtcttggg tcgacgcagc tgttggagcg aaccaacgca 16080
   caactaacaa caaatcaagt agactaggga tacaagatta aaatcatacg taaagcatca 16140
   tttatcatta ttgacaggca ctcaacaagc acaacggctc ggagatgaaa gcacactgct 16200
   ctctgcattt taaaagggac atctagatga ggagggcagc agcagcaata gcaccgacag 16260
   caacagggac ttggaggacc gaagcagcat taggggcagc tgacgcagtg cccttgctag 16320
   agccagaagc cttaggagtg ccagaactct tagagttgcc agaagcagaa gatttgccgg 16380
   atgcgctagc atcagcagca gaactcagag aagatgagga accggagtca gtggaggtcg 16440
   attttatggg agtgaacttg tagagcatgt tcttagaact cttgtcagtg acaaagacgt 16500
   ctccattggg ggcaacctcg atgtggttgg gagttgtgac gttgagctga gtgataatac 16560
   tatagtcttc aggatcaata acaaccacgg agtggcccgc acggcaggca acgtaaacaa 16620
   cgtcataaac gggatcgtaa cgagcgttga gaggacgtcc aggcatatcg atgctcttca 16680
   caaccttgcc ggacttgggg ttgacaataa cagtattgtt ggagccttgg ttcgtaacga 16740
   aaagttgctc acgtcgggag tcccaagcaa caccacttga aaacttgaca ttgtccccga 16800
   gatcgaagga tttgacagag tagtcgttga ggtcgatggc tgcggcaagg ggctgtttca 16860
   aagccaccgt gtagagtact ttgttgacct cgtcagcaac aagactcata ctgctggaga 16920
   aattcttgcc gagagactca gatatattga tactcttggc ggatttgtca gtggtgctag 16980
   catcgaatac ggctatgaca ctagacctcg cagaagagac gtaagcaagc ccagtgctct 17040
   ggtcaacata gacatcacgc ggatgcggct gaatgtcatc ggggtactga acaccaaggc 17100
   tgaggtcctt accattataa taggaaacag tgccctggcg ggtgttggta acccaaacac 17160
   ggttgttatc gtagtcgcta tcaacgccat aaactgcgta gcgttgggta acatttccag 17220
   tggtaccgat ggcaggctga acgtccctga caacagccag gctcttaggg tcaacctcga 17280
   taaggtcgga ctggttcaca gggggacgac caacagagtt ggtaaggaaa agcctgtcat 17340
   tggttctgtc ataagtgctt tggtagagac cgccgtactt actaaagtca gcgctttgag 17400
   tctcgtaaga gagggtgcga gcatcaatcc cgacggcgag gagaagaaca gcaagagagt 17460
   ggatagcaat cattagagct cagtaaaaac gctgttatgg tcaaaataac atttgtgaga 17520
   tagtttccct atttatattt ctcgagaaag agccgtttgc gaaaatgggc gccaggcata 17580
   attggccaag ggtaaatatg ggtcagggta tctttgggct cgggcggatt ctgcagatgg 17640
   cccagagaga ttttcatcat cgaggcaagt tcaaagctcg aaactggcca cattgagcac 17700
   cgtggtaaag attgaacgac tatatagtga tttcaattat gtcctgcatt agggcttggt 17760
   tttttttctg actgcagcag tgcctattga ggaattcgca atgagagagc cctacggtct 17820
   gtgctagatg taaaagatac gatcgagact tagatgcatc taccccagcc cttaccatct 17880
   tatatgaggt tgagagattt atttttgttt ttagagatga ttcttcagca aaccagaagg 17940
   gaatccggaa ggagttaggg ttaatgatcc agttagtgtt tgtagatatt atccagctcg 18000
   tagatgagaa gcg 18013
<210> 2
   <211> 1617
   <212> DNA
   <213> Candida bombicola
<400> 2
   atgttaatca aagacattat tctaactcca atgagtttat ccgctgttgc tggcttgttg     60
   ccactgctct tcgtagcttt cttagttcta cacgagccta tctggctcct atggtaccgc    120
   tatgcagcac gtaggcacaa gtgtagtatg cctcgcttca ttgagaaatc gttcccactg    180
   ggaatacaaa gaaccatgga catgatcaag acggccaagt catacacctt actggaagtt    240
   caatacgaca gagtcttcaa taagttcaaa gcacggacgt atcttcgaca agctcccctt    300
   caataccaaa tcttcacaat cgagccagaa aacattaaga caatcctggc aaccaaattc    360
   aatgattttg gtcttggagc acgtttccac acagtgggaa aagtgtttgg ccaagggata    420
   tttacactca gcggaaatgg atggaaacag tctcgatcga tgttgagacc tcagttcact    480
   aaagatcagg tttgcagaat tgatcagatt tccagtcatg ctgcggagtt aataaaggag    540
   atgaaccgtg caatgaaagt ggaccaattt attgatgttc aacattattt ccacaaactt    600
   acgctggata cagcgactga attcctattt ggggagtcct gcgagagctt gaaccctgag    660
   aatcagtcat gtattgtagc ccgtgatggt tcggagatta ctgccgaaca attcgtggag    720
   tcctacaact ttctactgaa ttacgctttc aaacggaccc tatcaagcaa agtctactgg    780
   ttgttcaact ctaaggaatt ccgagatcac aagaaacgtg ctcagtccta tattgactac    840
   tacgttgata aggctcttta cgccacatct ttcgctgctg agaactctat tgcagagaag    900
   gatgctgctg cagagtctag tggcatctat gtgttctcgc ttgagatggc taaagttacc    960
   cgagacccag tgacgatacg tgatcaaatt ttcaacattc tcattgctgg tagagataca   1020
   acagctgcta cgttgagctt cgctattcat ttccttgcca gaaatcctga cgtattcaac   1080
   aaactacgtg aggaggtcct cgatcatttt ggaaccaagg aggagcaaag gcctttatca   1140
   ttcgaacttc tgaagcaagc accttatttg aagcaagtta taaatgaagt cttgcgtctt   1200
   gcgccggtat tgccattgaa cttccgtact gctgtgagag atacaactct acccataggt   1260
   ggtggtcccg agcagaagga tccgatcttc gttcctaagg gcaccgcagt ttactattca   1320
   atttacatgg tccacaggga catcaagtat tggggtcctg acgcccacga attcaatccc   1380
   aatcgatggg agaacttgaa gctagataat gtgtgggcat tcttgccctt caatggcggt   1440
   ccccgaattt gtctcggcca acaattcgcc ctgacagagc tttcgctaac tctggtgaga   1500
   ctcttacagg agtattccaa gattgagatg ggtcccgact tcccagagtc ccctcgtttc   1560
   tcaacaacgc ttacagctca acacgctcct cccggtgtgg ttgtgcggtt ttcttaa      1617
<210> 3
   <211> 1392
   <212> DNA
   <213> Candida bombicola
<400> 3
   atgagccctt catcacacaa acccctgatt ctcgcttgcg gcttgcctct ttcaggccat      60
   ataatgcccg ttttgagtct ggtacacggc cttacggacg acggatacga agctactgtt     120
   gtgacaggca gagcgtttga acaaaaagtt cgagatgtgg gtgcagactt tgttccttta     180
   gaagggaacg cagattttga tgaccacacc ttagacgatc tggtcccggg ccgtaaagac     240
   atggccccaa gcttcgatcg tacagttcaa gatgtggagc acatgatggt agctactctt     300
   cctgagcagt ttgccgctat tcagagggct ttcaaaaagc tcagcgcaag cggccgccct     360
   gtcgttcttg tcagtgaagt gctgtttttc ggtgcacacc ctatcagcct cggtgctcct     420
   ggtttcaaac ccgctggctg gatttgttta ggggttttgc ctcttttgat ccgcagtgat     480
   cataccttag gacttgacaa cgacaggagc cccgaagcac atgcaaagaa actcgctatg     540
   aaccacgctc ttgagcacca aattttcgtt aaagccactg ctaagcacaa ggaaatctgc     600
   cgagagttag gttgcactga agatcccaaa tttatctggg agcacagtta cattgctgca     660
   gacaagttcc tgcagctgtg cccgccttct cttgagttca gcagagacca tctgcctagc     720
   aacttcaaat tcgccggctc aacgcccaag caccgaactc aattcacccc tccttcctgg     780
   tggggggatg ttctgagtgc caagcgagtc atcatggtca ctcaaggaac ttttgctgtc     840
   agttacaagc atcttattgt gcctactctt gaggccttga aggacgagcc tgacacttta     900
   acagtagcca tattgggccg ccgcggtgcc aagctaccgg atgatgttgt ggttcctgag     960
   aatgctcgcg tgatcgacta cttcaactac gatgctctac ttcctcacgt tgatgctctt    1020
   gtctacaatg gtggatatgg cggacttcag cacagcttaa gccactctgt tccagttgtt    1080
   attgctggtg actctgaaga caagccaatg gtggcatcga gagctgaggc cgctggcgtg    1140
   gcaattgatt tgaaaactgg cttgcctaca gtggagcaaa tcaaagaagc tgttgattcg    1200
   ataattggaa atccgaaatt ccacgaagcc tcgaagaagg ttcaaatgga gttggaaagc    1260
   cacaactcct tgaaaattct tgaggaaagc atcgaggaaa tcgccagcca tgactttggt    1320
   cttttgacca agagtgacga ggaaactgaa gatatacctg tcaaagggcc ggccttagcg    1380
   gtgagttctt ag                                                        1392
<210> 4
   <211> 780
   <212> **DNA**
   <213> Candida bombicola
<400> 4
   atggttgtaa actcctcgaa ggaccctcaa aacaaaggaa tgactcctag aaaagaaatt      60
   gaccaggaaa tggtctcttg ggccaaaaaa aacctcaaaa acacccctgg caatgaaaac     120
   tatgagaaga tggtctcagg agttccttac aatccatacg atccagatct tatgtttaga     180
   gccctggcta ctagtgagaa agttagggag ttcaatacca ttgcaagtga aagtcgtact     240
   tttgagtcaa atcacgctgc ttatatcaag aaggtcgaga ttctcaaaga cacttttggt     300
   caaacaaagg atattgtctg gctgaccgct ccattctcag ttgattttgg attcaacatc     360
   agcgtaggcg agcactttta cgccaacttc aacgtttgct tcttggactc ggctccaata     420
   atctttggtg atgaggtgat tgtagggccc aatacaacgt tcgtgactgc gactcatcct     480
   attagccccg agaaacgtgc gaggagaatt gtgtatgctc ttcctatcaa ggtggggaat     540
   aatgtatgga ttggtgcgaa tgtgactgtc ctgccgggtg ttacgattgg agatggctca     600
   acaattgcgg ctggtgctgt cgttcgagaa gatgttcctc ctcgtactgt ggtgggagga     660
   gtccctgcgc gaatcctcaa gcatattcca gaggaggatc ccgacgaggc tgaaggagag     720
   gaactggaat tccttcttcc agttgaaatg aacgtcaata ccgctaacca gaaggtctag     780
<210> 5
   <211> 3900
   <212> DNA
   <213> Candida bombicola
<400> 5
   atggtggatg atatacaggt agagaagcgt gagaaactca tcgagactaa ggacaagctt      60
   ctcgaggaga agctctctgc gttagatcca catgaggcca atgtattgcg aagtcagctt     120
   gaaacaaaga gagtcgccac aagctttttc aggttgttca gattttgcac tccccttgac 180
   gttttcttgg agatacttgc gctttttttt gcagcggtgc atggagccgc gcttccaatg 240
   ttcacgttag tagtgggcgc catcttcaac acattcagag acttcacttc atatgacctc 300
   aagggcaatg agttccagca taaggtgaat cacctgtctc tctattttgt ctatattggc 360
   attggtatgc tcggcagtgc gtttctcgag agcttcctgc ttgtggacag aggcgaagtg 420
   ttggcaggac gttaccgaaa gcattatctg agtgctgtta ttcgccagaa tatcgcgttt 480
   tacgacaaac taggtggtgg cgaggtcagc accagaatca ttaacgatac caactcaatt 540
   caggaagcga tcagcgacaa gcttggaaac gtcgtacagg gaatagcttc cttcattgcg 600
   gccaccgtta taagttttgc ttcgcaatgg aaactggctt gcatcctcct gagtgctgta 660
   gggttcatgg taatcacaat gggaactggc gccaccttca tggccaaata tcagctcaga 720
   tctgacgcga tatattcgca gtctggagct accgttgcgg aggaggctct cagtgctgtc 780
   aggactacag tagcatttgg cgctcaacct catctcgccg tcaagtatga aaaggtactt 840
   gatcgtgttg tgaaggaatc gaagcggagc agttactcat tgggggtcat gttagcgtgc 900
   atatgggcta gtactttttg ggtgtatgcc ttagctctgt ggcagggttc cagagaaatc 960
   gttagtggga gtgctgacgt tggaaagata atagttgtaa tcacagctat gttacttgga 1020
   agcttccagc ttgggaatat cgcgccaaac gtgaggtttc ttgtcaaggg tctcactgcc 1080
   gcgagcattc tcaatgaggc cattgatcgt gtcccagtca tcgatggcca gtccatagat 1140
   aaaggaattg tcccccaaac taaggccgtt ggcagaattg agctcaaaaa tgtcaagttc 1200
   cgatatccta gtcgcccaga cgttttggtc ctctccgatt ttagccttga agttcctgct 1260
   ggatctactg tggcactggt aggtgcctcg ggatcaggga agtctacaat tgtaggtatt 1320
   cttgagaggt tctatttacc tctcgaagga agcgttactc tggatggcca ggagattagc 1380
   gacctgaaca caagatggct ccgtcaacaa attggttatg ttcagcagga accagtactc 1440
   ttttcagagt caatatatga gaatatcagc tatggtttga ttggcactga cattgagttc 1500
   gctgacgagc atgttaagga agctaaaatc attcaagctt gtaaagatgc caatgcctgg 1560
   gatttcattc agactctctc agaaggcatc caaaccaatg ttggagatcg aggatttctt 1620
   ctcagcggtg gtcagaaaca acgcattgca atagcaagag caatcgtctc agaccctaaa 1680
   attctgctgc tcgatgaagc gacttctgct ctggatacca aatctgaagg tatcgttcaa 1740
   gatgcgctcg acaaagcggc cgaaggtcgt accactatag tcgttgcaca cagactctct 1800
   acgatcaagg atgccaacaa gatagttgtc atgtctaaag gtaacgtcat agagcagggt 1860
   actcacaatg agctcataca gcgagaaggg ccttataaag ctttggttga tgctcaaaga 1920
   gtaactaaag caaagagcac taacgttgag gtcctcgata ttgaagctct agacatttcg 1980
   cctctggact cactgaacga aaagttcaat cccaaggatg tgagcacatt gagtgttcac 2040
   agtgcaggta ctcagaccac tcaacctcct gaatatcaag aaaatgacat ccctggtgtg 2100
   cgcaaccccc cacatagcac gttgatgacc aataccaaac tggtttgggg gctgaatagg 2160
   aaagaatggg gttacattct cattggtagt ttagcctcca ttattttggg ctattgctat 2220
   cctgcaatgg caataataac tggccaaacc actggaagca tggttctacc tcccagtgaa 2280
   tacggaaaaa tgcggcatgt ggtgaatatc atgggatggt ggtatttttt cgtaggctgc 2340
   atttcattca tgacggcttt tatcactata gctgctttat cacttgcatc tgataagttg 2400
   gtcaaaaata tcagattagc tttgttccgc caattgatgc gaatggatat tgcattcttc 2460
   gaccacaaaa acaacacgcc gggtgcgcta acctcaattt tggcgaagga agctaaaatg 2520
   atcgagggtt tgagtggggc caccctcggt caaattcaac agagtctggt gaccttgatt 2580
   ggcggcatag ttactggtat acctttcaat tggagaattg gactcgtggc tacgtctgtt 2640
   gttcctgtca tgttggtgtg tggcttcgtc agagtctggg ttcttaccca attatcggat 2700
   cgtgcgagag aagtttacga acgaagtggc tccatggcat ctgagtatac aagtgctgtc 2760
   cgcacagtcc agtccttaac tcgtgagtta gacgtggtcg taaaatacac aaagacagta 2820
   gactctcaga ttttcagctc cagaattgcc attgcccgct cagcattgta ctacgcactc 2880
   tcggaaggaa tgacaccctg ggtggtagcc ctcgtttttt ggtggggaag cactgtaatg 2940
   agacgaggtg aagcttcggt cgcaggatat atgactgtct tcatggctat tattacaggt 3000
   tctcaagccg ctggccaaat tttcagctat gctccaaaca tgaactcagc caaagatgca 3060
   gcgcgtaaca tttacagaat cttgactgcc actccttcta tagatgtatg gagtgaggaa 3120
   ggttacgttg ctcccgagga gtcggtgaga ggagatattg agttccgtca tgtgaatttc 3180
   cgatatccta ctcgacctca agtaccagtt ttacaagatc tcaacttaac agtcaaaaag 3240
   ggccaataca tcgctctagt tggagccagt ggatgcggta agtctactac tattggactg 3300
   gtggaaagat tttatgatcc attagcaggt caagtacttt tcgatgggaa agatttacgc 3360
   gaatataacc tgaatgcatt gagatcacac attgctttag tccagcaaga accaatgctt 3420
   tattcaggca cgctacgtga gaatattcta atgggatggt ctggccctga gtctgaagta 3480
   acgcaggaga tgattgagga tgccgctcgc aaagcgaaca ttcacgaatt catcatgtcg 3540
   ttgcctgatg gctacgaaac gctcagcgga tctaggggat cgttgctatc tggggggcaa 3600
   aagcagcgaa ttgcaattgc aagggccctg atcagaaatc caaaggtact cctcctcgat 3660
   gaggccacct cagctctgga ttccgaatct gagaaagtag ttcaagcagc actcgacgca 3720
   gcagcgaagg gccgtactac aatcgccgtt gcgcatagat tatcaacaat tcagaaagca 3780
   gatgtcatat atgtgttctc aggagggcgc atcgtggagc agggcgacca tcagagcctc 3840
   cttgaactca atggatggta cgctgaattg gtgaacttgc aaggtctcgg agagatttga 3900
<210> 6
   <211> 1299
   <212> DNA
   <213> Candida bombicola
<400> 6
   atggccatcg agaaaccagt gatagttgct tgtgcctgcc cactagcggg gcacgtgggc     60
   ccagtgctca gcctggtccg cggtctactc aatagaggat atgaggtgac tttcgtaaca    120
   gggaacgcat tcaaggagaa agttattgag gcaggatgca ctttcgtccc tctccaagga    180
   cgagctgact accatgaata caatctccct gaaatcgctc caggattgct cacgattcct    240
   ccaggccttg agcagaccgg ttactcaatg aatgagattt ttgtgaaggc gattcctgag    300
   cagtacgatg cacttcaaac tgctctaaaa caggttgagg ctgaaaataa atcagctgtg    360
   gtgattggcg agaccatgtt tctaggggtg catccgatat cactgggtgc cccaggtctc    420
   aagccccaag gcgtaatcac gttaggaact attccgtgca tgctgaaagc agagaaggcg    480
   cctggagttc ctagtcttga gccaatgatt gatactttag tgcggcaaca agtatttcaa    540
   ccaggaactg actctgagaa ggagatcatg aagacgctcg gggccacgaa ggagcccgaa    600
   tttctcctgg agaatatata cagcagccct gacagatttt tgcaactgtg ccctccatct    660
   cttgaatttc acttgacttc gcctcctcct ggcttctcgt tcgctggtag tgcaccgcat    720
   gtaaagtctg ctggattagc aactccacct cacctgccgt cttggtggcc tgatgtgctg    780
   agtgcgaagc gtctgattgt tgttacacaa ggaacagcag ccatcaacta tgaagatctg    840
   ctcattccag cattgcaggc ctttgctgac gaagaagaca ctctcgtagt tggtatattg    900
   ggcgtcaaag gggcgtcact tcctgatagc gttaaagttc ctgcaaacgc tcgaattgtt    960
   gattattttc cttacgatga gctactaccg catgcctctg ttttcatata caacggtgga   1020
   tacggaggtc tgcagcacag tttgagccat ggcgttcccg tcatcatcgg aggaggaatg   1080
   ttggtagaca agccagctgt tgcttcacga gctgtatggg ctggtgttgg ttatgatctt   1140
   caaaccttgc aggcaacttc tgagctagtc tccacggccg ttaaggaggt gttggctact   1200
   ccctcgtatc acgagaaagc catggcagtc aagaaagagc ttgaaaaata caagtctctt   1260
   gatattctag agtcggcaat tagtgaatta gcttcttaa                          1299
<210> 7
   <211> 538
   <212> PRT
   <213> Candida bombicola
<400> 7
   Met Leu Ile Lys Asp Ile Ile Leu Thr Pro Met Ser Leu Ser Ala Val
   1               5                   10                  15
   Ala Gly Leu Leu Pro Leu Leu Phe Val Ala Phe Leu Val Leu His Glu
               20                  25                  30
   Pro Ile Trp Leu Leu Trp Tyr Arg Tyr Ala Ala Arg Arg His Lys Cys
           35                  40                  45
   Ser Met Pro Arg Phe Ile Glu Lys Ser Phe Pro Leu Gly Ile Gln Arg
       50                  55                  60
   Thr Met Asp Met Ile Lys Thr Ala Lys Ser Tyr Thr Leu Leu Glu Val
   65                  70                  75                  80
   Gln Tyr Asp Arg Val Phe Asn Lys Phe Lys Ala Arg Thr Tyr Leu Arg
                   85                  90                  95
   Gln Ala Pro Leu Gln Tyr Gln Ile Phe Thr Ile Glu Pro Glu Asn Ile
               100                 105                 110
   Lys Thr Ile Leu Ala Thr Lys Phe Asn Asp Phe Gly Leu Gly Ala Arg
           115                 120                 125
   Phe His Thr Val Gly Lys Val Phe Gly Gln Gly Ile Phe Thr Leu Ser
       130                 135                 140
   Gly Asn Gly Trp Lys Gln Ser Arg Ser Met Leu Arg Pro Gln Phe Thr
   145                 150                 155                 160
   Lys Asp Gln Val Cys Arg Ile Asp Gln Ile Ser Ser His Ala Ala Glu
                   165                 170                 175
   Leu Ile Lys Glu Met Asn Arg Ala Met Lys Val Asp Gln Phe Ile Asp
               180                 185                 190
   Val Gln His Tyr Phe His Lys Leu Thr Leu Asp Thr Ala Thr Glu Phe
           195                 200                 205
   Leu Phe Gly Glu Ser Cys Glu Ser Leu Asn Pro Glu Asn Gln Ser Cys
       210                 215                 220
   Ile Val Ala Arg Asp Gly Ser Glu Ile Thr Ala Glu Gln Phe Val Glu
   225                 230                 235                 240
   Ser Tyr Asn Phe Leu Leu Asn Tyr Ala Phe Lys Arg Thr Leu Ser Ser
                   245                 250                 255
   Lys Val Tyr Trp Leu Phe Asn Ser Lys Glu Phe Arg Asp His Lys Lys
               260                 265                 270
   Arg Ala Gln Ser Tyr Ile Asp Tyr Tyr Val Asp Lys Ala Leu Tyr Ala
           275                 280                 285
   Thr Ser Phe Ala Ala Glu Asn Ser Ile Ala Glu Lys Asp Ala Ala Ala
       290                 295                 300
   Glu Ser Ser Gly Ile Tyr Val Phe Ser Leu Glu Met Ala Lys Val Thr
   305                 310                 315                 320
   Arg Asp Pro Val Thr Ile Arg Asp Gln Ile Phe Asn Ile Leu Ile Ala
                   325                 330                 335
   Gly Arg Asp Thr Thr Ala Ala Thr Leu Ser Phe Ala Ile His Phe Leu
               340                 345                 350
   Ala Arg Asn Pro Asp Val Phe Asn Lys Leu Arg Glu Glu Val Leu Asp
           355                 360                 365
   His Phe Gly Thr Lys Glu Glu Gln Arg Pro Leu Ser Phe Glu Leu Leu
       370                 375                 380
   Lys Gln Ala Pro Tyr Leu Lys Gln Val Ile Asn Glu Val Leu Arg Leu
   385                 390                 395                 400
   Ala Pro Val Leu Pro Leu Asn Phe Arg Thr Ala Val Arg Asp Thr Thr
                   405                 410                 415
   Leu Pro Ile Gly Gly Gly Pro Glu Gln Lys Asp Pro Ile Phe Val Pro
               420                 425                 430
   Lys Gly Thr Ala Val Tyr Tyr Ser Ile Tyr Met Val His Arg Asp Ile
           435                 440                 445
   Lys Tyr Trp Gly Pro Asp Ala His Glu Phe Asn Pro Asn Arg Trp Glu
       450                 455                 460
   Asn Leu Lys Leu Asp Asn Val Trp Ala Phe Leu Pro Phe Asn Gly Gly
   465                 470                 475                 480
   Pro Arg Ile Cys Leu Gly Gln Gln Phe Ala Leu Thr Glu Leu Ser Leu
                   485                 490                 495
   Thr Leu Val Arg Leu Leu Gln Glu Tyr Ser Lys Ile Glu Met Gly Pro
               500                 505                 510
   Asp Phe Pro Glu Ser Pro Arg Phe Ser Thr Thr Leu Thr Ala Gln His
           515                 520                 525
   Ala Pro Pro Gly Val Val Val Arg Phe Ser
       530                 535
<210> 8
   <211> 463
   <212> PRT
   <213> Candida bombicola
<400> 8
   Met Ser Pro Ser Ser His Lys Pro Leu Ile Leu Ala Cys Gly Leu Pro
   1               5                   10                  15
   Leu Ser Gly His Ile Met Pro Val Leu Ser Leu Val His Gly Leu Thr
               20                  25                  30
   Asp Asp Gly Tyr Glu Ala Thr Val Val Thr Gly Arg Ala Phe Glu Gln
           35                  40                  45
   Lys Val Arg Asp Val Gly Ala Asp Phe Val Pro Leu Glu Gly Asn Ala
       50                  55                  60
   Asp Phe Asp Asp His Thr Leu Asp Asp Leu Val Pro Gly Arg Lys Asp
   65                  70                  75                  80
   Met Ala Pro Ser Phe Asp Arg Thr Val Gln Asp Val Glu His Met Met
                   85                  90                  95
   Val Ala Thr Leu Pro Glu Gln Phe Ala Ala Ile Gln Arg Ala Phe Lys
               100                 105                 110
   Lys Leu Ser Ala Ser Gly Arg Pro Val Val Leu Val Ser Glu Val Leu
           115                 120                 125
   Phe Phe Gly Ala His Pro Ile Ser Leu Gly Ala Pro Gly Phe Lys Pro
       130                 135                 140
   Ala Gly Trp Ile Cys Leu Gly Val Leu Pro Leu Leu Ile Arg Ser Asp
   145                 150                 155                 160
   His Thr Leu Gly Leu Asp Asn Asp Arg Ser Pro Glu Ala His Ala Lys
                   165                 170                 175
   Lys Leu Ala Met Asn His Ala Leu Glu His Gln Ile Phe Val Lys Ala
               180                 185                 190
   Thr Ala Lys His Lys Glu Ile Cys Arg Glu Leu Gly Cys Thr Glu Asp
           195                 200                 205
   Pro Lys Phe Ile Trp Glu His Ser Tyr Ile Ala Ala Asp Lys Phe Leu
       210                 215                 220
   Gln Leu Cys Pro Pro Ser Leu Glu Phe Ser Arg Asp His Leu Pro Ser
   225                 230                 235                 240
   Asn Phe Lys Phe Ala Gly Ser Thr Pro Lys His Arg Thr Gln Phe Thr
                   245                 250                 255
   Pro Pro Ser Trp Trp Gly Asp Val Leu Ser Ala Lys Arg Val Ile Met
               260                 265                 270
   Val Thr Gln Gly Thr Phe Ala Val Ser Tyr Lys His Leu Ile Val Pro
           275                 280                 285
   Thr Leu Glu Ala Leu Lys Asp Glu Pro Asp Thr Leu Thr Val Ala Ile
       290                 295                 300
   Leu Gly Arg Arg Gly Ala Lys Leu Pro Asp Asp Val Val Val Pro Glu
   305                 310                 315                 320
   Asn Ala Arg Val Ile Asp Tyr Phe Asn Tyr Asp Ala Leu Leu Pro His
                   325                 330                 335
   Val Asp Ala Leu Val Tyr Asn Gly Gly Tyr Gly Gly Leu Gln His Ser
               340                 345                 350
   Leu Ser His Ser Val Pro Val Val Ile Ala Gly Asp Ser Glu Asp Lys
           355                 360                 365
   Pro Met Val Ala Ser Arg Ala Glu Ala Ala Gly Val Ala Ile Asp Leu
       370                 375                 380
   Lys Thr Gly Leu Pro Thr Val Glu Gln Ile Lys Glu Ala Val Asp Ser
   385                 390                 395                 400
   Ile Ile Gly Asn Pro Lys Phe His Glu Ala Ser Lys Lys Val Gln Met
                   405                 410                 415
   Glu Leu Glu Ser His Asn Ser Leu Lys Ile Leu Glu Glu Ser Ile Glu
               420                 425                 430
   Glu Ile Ala Ser His Asp Phe Gly Leu Leu Thr Lys Ser Asp Glu Glu
           435                 440                 445
   Thr Glu Asp Ile Pro Val Lys Gly Pro Ala Leu Ala Val Ser Ser
       450                 455                 460
<210> 9
   <211> 259
   <212> PRT
   <213> Candida bombicola
<400> 9
   Met Val Val Asn Ser Ser Lys Asp Pro Gln Asn Lys Gly Met Thr Pro
   1               5                   10                  15
   Arg Lys Glu Ile Asp Gln Glu Met Val Ser Trp Ala Lys Lys Asn Leu
               20                  25                  30
   Lys Asn Thr Pro Gly Asn Glu Asn Tyr Glu Lys Met Val Ser Gly Val
           35                  40                  45
   Pro Tyr Asn Pro Tyr Asp Pro Asp Leu Met Phe Arg Ala Leu Ala Thr
       50                  55                  60
   Ser Glu Lys Val Arg Glu Phe Asn Thr Ile Ala Ser Glu Ser Arg Thr
   65                  70                  75                  80
   Phe Glu Ser Asn His Ala Ala Tyr Ile Lys Lys Val Glu Ile Leu Lys
                   85                  90                  95
   Asp Thr Phe Gly Gln Thr Lys Asp Ile Val Trp Leu Thr Ala Pro Phe
               100                 105                 110
   Ser Val Asp Phe Gly Phe Asn Ile Ser Val Gly Glu His Phe Tyr Ala
           115                 120                 125
   Asn Phe Asn Val Cys Phe Leu Asp Ser Ala Pro Ile Ile Phe Gly Asp
       130                 135                 140
   Glu Val Ile Val Gly Pro Asn Thr Thr Phe Val Thr Ala Thr His Pro
   145                 150                 155                 160
   Ile Ser Pro Glu Lys Arg Ala Arg Arg Ile Val Tyr Ala Leu Pro Ile
                   165                 170                 175
   Lys Val Gly Asn Asn Val Trp Ile Gly Ala Asn Val Thr Val Leu Pro
               180                 185                 190
   Gly Val Thr Ile Gly Asp Gly Ser Thr Ile Ala Ala Gly Ala Val Val
           195                 200                 205
   Arg Glu Asp Val Pro Pro Arg Thr Val Val Gly Gly Val Pro Ala Arg
       210                 215                 220
   Ile Leu Lys His Ile Pro Glu Glu Asp Pro Asp Glu Ala Glu Gly Glu
   225                 230                 235                 240
   Glu Leu Glu Phe Leu Leu Pro Val Glu Met Asn Val Asn Thr Ala Asn
                   245                 250                 255
   Gln Lys Val
<210> 10
   <211> 1299
   <212> PRT
   <213> Candida bombicola
<400> 10
   Met Val Asp Asp Ile Gln Val Glu Lys Arg Glu Lys Leu Ile Glu Thr
   1               5                   10                  15
   Lys Asp Lys Leu Leu Glu Glu Lys Leu Ser Ala Leu Asp Pro His Glu
               20                  25                  30
   Ala Asn Val Leu Arg Ser Gln Leu Glu Thr Lys Arg Val Ala Thr Ser
           35                  40                  45
   Phe Phe Arg Leu Phe Arg Phe Cys Thr Pro Leu Asp Val Phe Leu Glu
       50                  55                  60
   Ile Leu Ala Leu Phe Phe Ala Ala Val His Gly Ala Ala Leu Pro Met
   65                  70                  75                  80
   Phe Thr Leu Val Val Gly Ala Ile Phe Asn Thr Phe Arg Asp Phe Thr
                   85                  90                  95
   Ser Tyr Asp Leu Lys Gly Asn Glu Phe Gln His Lys Val Asn His Leu
               100                 105                 110
   Ser Leu Tyr Phe Val Tyr Ile Gly Ile Gly Met Leu Gly Ser Ala Phe
           115                 120                 125
   Leu Glu Ser Phe Leu Leu Val Asp Arg Gly Glu Val Leu Ala Gly Arg
       130                 135                 140
   Tyr Arg Lys His Tyr Leu Ser Ala Val Ile Arg Gln Asn Ile Ala Phe
   145                 150                 155                 160
   Tyr Asp Lys Leu Gly Gly Gly Glu Val Ser Thr Arg Ile Ile Asn Asp
                   165                 170                 175
   Thr Asn Ser Ile Gln Glu Ala Ile Ser Asp Lys Leu Gly Asn Val Val
               180                 185                 190
   Gln Gly Ile Ala Ser Phe Ile Ala Ala Thr Val Ile Ser Phe Ala Ser
           195                 200                 205
   Gln Trp Lys Leu Ala Cys Ile Leu Leu Ser Ala Val Gly Phe Met Val
       210                 215                 220
   Ile Thr Met Gly Thr Gly Ala Thr Phe Met Ala Lys Tyr Gln Leu Arg
   225                 230                 235                 240
   Ser Asp Ala Ile Tyr Ser Gln Ser Gly Ala Thr Val Ala Glu Glu Ala
   245 250 255
   Leu Ser Ala Val Arg Thr Thr Val Ala Phe Gly Ala Gln Pro His Leu
   260 265 270
   Ala Val Lys Tyr Glu Lys Val Leu Asp Arg Val Val Lys Glu Ser Lys
   275 280 285
   Arg Ser Ser Tyr Ser Leu Gly Val Met Leu Ala Cys Ile Trp Ala Ser
   290 295 300
   Thr Phe Trp Val Tyr Ala Leu Ala Leu Trp Gln Gly Ser Arg Glu Ile
   305 310 315 320
   Val Ser Gly Ser Ala Asp Val Gly Lys Ile Ile Val Val Ile Thr Ala
   325 330 335
   Met Leu Leu Gly Ser Phe Gln Leu Gly Asn Ile Ala Pro Asn Val Arg
   340 345 350
   Phe Leu Val Lys Gly Leu Thr Ala Ala Ser Ile Leu Asn Glu Ala Ile
   355 360 365
   Asp Arg Val Pro Val Ile Asp Gly Gln Ser Ile Asp Lys Gly Ile Val
   370 375 380
   Pro Gln Thr Lys Ala Val Gly Arg Ile Glu Leu Lys Asn Val Lys Phe
   385 390 395 400
   Arg Tyr Pro Ser Arg Pro Asp Val Leu Val Leu Ser Asp Phe Ser Leu
   405 410 415
   Glu Val Pro Ala Gly Ser Thr Val Ala Leu Val Gly Ala Ser Gly Ser
   420 425 430
   Gly Lys Ser Thr Ile Val Gly Ile Leu Glu Arg Phe Tyr Leu Pro Leu
   435 440 445
   Glu Gly Ser Val Thr Leu Asp Gly Gln Glu Ile Ser Asp Leu Asn Thr
   450 455 460
   Arg Trp Leu Arg Gln Gln Ile Gly Tyr Val Gln Gln Glu Pro Val Leu
   465 470 475 480
   Phe Ser Glu Ser Ile Tyr Glu Asn Ile Ser Tyr Gly Leu Ile Gly Thr
   485 490 495
   Asp Ile Glu Phe Ala Asp Glu His Val Lys Glu Ala Lys Ile Ile Gln
               500                 505                 510
   Ala Cys Lys Asp Ala Asn Ala Trp Asp Phe Ile Gln Thr Leu Ser Glu
           515                 520                 525
   Gly Ile Gln Thr Asn Val Gly Asp Arg Gly Phe Leu Leu Ser Gly Gly
       530                 535                 540
   Gln Lys Gln Arg Ile Ala Ile Ala Arg Ala Ile Val Ser Asp Pro Lys
   545                 550                 555                 560
   Ile Leu Leu Leu Asp Glu Ala Thr Ser Ala Leu Asp Thr Lys Ser Glu
                   565                 570                 575
   Gly Ile Val Gln Asp Ala Leu Asp Lys Ala Ala Glu Gly Arg Thr Thr
               580                 585                 590
   Ile Val Val Ala His Arg Leu Ser Thr Ile Lys Asp Ala Asn Lys Ile
           595                 600                 605
   Val Val Met Ser Lys Gly Asn Val Ile Glu Gln Gly Thr His Asn Glu
       610                 615                 620
   Leu Ile Gln Arg Glu Gly Pro Tyr Lys Ala Leu Val Asp Ala Gln Arg
   625                 630                 635                 640
   Val Thr Lys Ala Lys Ser Thr Asn Val Glu Val Leu Asp Ile Glu Ala
                   645                 650                 655
   Leu Asp Ile Ser Pro Leu Asp Ser Leu Asn Glu Lys Phe Asn Pro Lys
               660                 665                 670
   Asp Val Ser Thr Leu Ser Val His Ser Ala Gly Thr Gln Thr Thr Gln
           675                 680                 685
   Pro Pro Glu Tyr Gln Glu Asn Asp Ile Pro Gly Val Arg Asn Pro Pro
       690                 695                 700
   His Ser Thr Leu Met Thr Asn Thr Lys Leu Val Trp Gly Leu Asn Arg
   705                 710                 715                 720
   Lys Glu Trp Gly Tyr Ile Leu Ile Gly Ser Leu Ala Ser Ile Ile Leu
                   725                 730                 735
   Gly Tyr Cys Tyr Pro Ala Met Ala Ile Ile Thr Gly Gln Thr Thr Gly
               740                 745                 750
   Ser Met Val Leu Pro Pro Ser Glu Tyr Gly Lys Met Arg His Val Val
   755 760 765
   Asn Ile Met Gly Trp Trp Tyr Phe Phe Val Gly Cys Ile Ser Phe Met
   770 775 780
   Thr Ala Phe Ile Thr Ile Ala Ala Leu Ser Leu Ala Ser Asp Lys Leu
   785 790 795 800
   Val Lys Asn Ile Arg Leu Ala Leu Phe Arg Gln Leu Met Arg Met Asp
   805 810 815
   Ile Ala Phe Phe Asp His Lys Asn Asn Thr Pro Gly Ala Leu Thr Ser
   820 825 830
   Ile Leu Ala Lys Glu Ala Lys Met Ile Glu Gly Leu Ser Gly Ala Thr
   835 840 845
   Leu Gly Gln Ile Gln Gln Ser Leu Val Thr Leu Ile Gly Gly Ile Val
   850 855 860
   Thr Gly Ile Pro Phe Asn Trp Arg Ile Gly Leu Val Ala Thr Ser Val
   865 870 875 880
   Val Pro Val Met Leu Val Cys Gly Phe Val Arg Val Trp Val Leu Thr
   885 890 895
   Gln Leu Ser Asp Arg Ala Arg Glu Val Tyr Glu Arg Ser Gly Ser Met
   900 905 910
   Ala Ser Glu Tyr Thr Ser Ala Val Arg Thr Val Gln Ser Leu Thr Arg
   915 920 925
   Glu Leu Asp Val Val Val Lys Tyr Thr Lys Thr Val Asp Ser Gln Ile
   930 935 940
   Phe Ser Ser Arg Ile Ala Ile Ala Arg Ser Ala Leu Tyr Tyr Ala Leu
   945 950 955 960
   Ser Glu Gly Met Thr Pro Trp Val Val Ala Leu Val Phe Trp Trp Gly
   965 970 975
   Ser Thr Val Met Arg Arg Gly Glu Ala Ser Val Ala Gly Tyr Met Thr
   980 985 990
   Val Phe Met Ala Ile Ile Thr Gly Ser Gln Ala Ala Gly Gln Ile Phe
   995 1000 1005
   Ser Tyr Ala Pro Asn Met Asn Ser Ala Lys Asp Ala Ala Arg Asn
       1010                1015                1020
   Ile Tyr  Arg Ile Leu Thr Ala  Thr Pro Ser Ile Asp  Val Trp Ser
       1025                1030                1035
   Glu Glu  Gly Tyr Val Ala Pro  Glu Glu Ser Val Arg  Gly Asp Ile
       1040                1045                1050
   Glu Phe  Arg His Val Asn Phe  Arg Tyr Pro Thr Arg  Pro Gln Val
       1055                1060                1065
   Pro Val  Leu Gln Asp Leu Asn  Leu Thr Val Lys Lys  Gly Gln Tyr
       1070                1075                1080
   Ile Ala  Leu Val Gly Ala Ser  Gly Cys Gly Lys Ser  Thr Thr Ile
       1085                1090                1095
   Gly Leu  Val Glu Arg Phe Tyr  Asp Pro Leu Ala Gly  Gln Val Leu
       1100                1105                1110
   Phe Asp  Gly Lys Asp Leu Arg  Glu Tyr Asn Leu Asn  Ala Leu Arg
       1115                1120                1125
   Ser His  Ile Ala Leu Val Gln  Gln Glu Pro Met Leu  Tyr Ser Gly
       1130                1135                1140
   Thr Leu  Arg Glu Asn Ile Leu  Met Gly Trp Ser Gly  Pro Glu Ser
       1145                1150                1155
   Glu Val  Thr Gln Glu Met Ile  Glu Asp Ala Ala Arg  Lys Ala Asn
       1160                1165                1170
   Ile His  Glu Phe Ile Met Ser  Leu Pro Asp Gly Tyr  Glu Thr Leu
       1175                1180                1185
   Ser Gly  Ser Arg Gly Ser Leu  Leu Ser Gly Gly Gln  Lys Gln Arg
       1190                1195                1200
   Ile Ala  Ile Ala Arg Ala Leu  Ile Arg Asn Pro Lys  Val Leu Leu
       1205                1210                1215
   Leu Asp  Glu Ala Thr Ser Ala  Leu Asp Ser Glu Ser  Glu Lys Val
       1220                1225                1230
   Val Gln  Ala Ala Leu Asp Ala  Ala Ala Lys Gly Arg  Thr Thr Ile
       1235                1240                1245
   Ala Val  Ala His Arg Leu Ser  Thr Ile Gln Lys Ala  Asp Val Ile
       1250                1255                1260
   Tyr Val  Phe Ser Gly Gly Arg  Ile Val Glu Gln Gly  Asp His Gln
       1265                 1270                 1275
   Ser Leu  Leu Glu Leu Asn Gly  Trp Tyr Ala Glu Leu  Val Asn Leu
       1280                 1285                 1290
   Gln Gly  Leu Gly Glu Ile
       1295
<210> 11
   <211> 432
   <212> PRT
   <213> Candida bombicola
<400> 11
   Met Ala Ile Glu Lys Pro Val Ile Val Ala Cys Ala Cys Pro Leu Ala
   1               5                   10                  15
   Gly His Val Gly Pro Val Leu Ser Leu Val Arg Gly Leu Leu Asn Arg
               20                  25                  30
   Gly Tyr Glu Val Thr Phe Val Thr Gly Asn Ala Phe Lys Glu Lys Val
           35                  40                  45
   Ile Glu Ala Gly Cys Thr Phe Val Pro Leu Gln Gly Arg Ala Asp Tyr
       50                  55                  60
   His Glu Tyr Asn Leu Pro Glu Ile Ala Pro Gly Leu Leu Thr Ile Pro
   65                  70                  75                  80
   Pro Gly Leu Glu Gln Thr Gly Tyr Ser Met Asn Glu Ile Phe Val Lys
                   85                  90                  95
   Ala Ile Pro Glu Gln Tyr Asp Ala Leu Gln Thr Ala Leu Lys Gln Val
               100                 105                 110
   Glu Ala Glu Asn Lys Ser Ala Val Val Ile Gly Glu Thr Met Phe Leu
           115                 120                 125
   Gly Val His Pro Ile Ser Leu Gly Ala Pro Gly Leu Lys Pro Gln Gly
       130                 135                 140
   Val Ile Thr Leu Gly Thr Ile Pro Cys Met Leu Lys Ala Glu Lys Ala
   145                 150                 155                 160
   Pro Gly Val Pro Ser Leu Glu Pro Met Ile Asp Thr Leu Val Arg Gln
                   165                 170                 175
   Gln Val Phe Gln Pro Gly Thr Asp Ser Glu Lys Glu Ile Met Lys Thr
               180                 185                 190
   Leu Gly Ala Thr Lys Glu Pro Glu Phe Leu Leu Glu Asn Ile Tyr Ser
           195                 200                 205
   Ser Pro Asp Arg Phe Leu Gln Leu Cys Pro Pro Ser Leu Glu Phe His
       210                 215                 220
   Leu Thr Ser Pro Pro Pro Gly Phe Ser Phe Ala Gly Ser Ala Pro His
   225                 230                 235                 240
   Val Lys Ser Ala Gly Leu Ala Thr Pro Pro His Leu Pro Ser Trp Trp
                   245                 250                 255
   Pro Asp Val Leu Ser Ala Lys Arg Leu Ile Val Val Thr Gln Gly Thr
               260                 265                 270
   Ala Ala Ile Asn Tyr Glu Asp Leu Leu Ile Pro Ala Leu Gln Ala Phe
           275                 280                 285
   Ala Asp Glu Glu Asp Thr Leu Val Val Gly Ile Leu Gly Val Lys Gly
       290                 295                 300
   Ala Ser Leu Pro Asp Ser Val Lys Val Pro Ala Asn Ala Arg Ile Val
   305                 310                 315                 320
   Asp Tyr Phe Pro Tyr Asp Glu Leu Leu Pro His Ala Ser Val Phe Ile
                   325                 330                 335
   Tyr Asn Gly Gly Tyr Gly Gly Leu Gln His Ser Leu Ser His Gly Val
               340                 345                 350
   Pro Val Ile Ile Gly Gly Gly Met Leu Val Asp Lys Pro Ala Val Ala
           355                 360                 365
   Ser Arg Ala Val Trp Ala Gly Val Gly Tyr Asp Leu Gln Thr Leu Gln
       370                 375                 380
   Ala Thr Ser Glu Leu Val Ser Thr Ala Val Lys Glu Val Leu Ala Thr
   385                 390                 395                 400
   Pro Ser Tyr His Glu Lys Ala Met Ala Val Lys Lys Glu Leu Glu Lys
                   405                 410                 415
   Tyr Lys Ser Leu Asp Ile Leu Glu Ser Ala Ile Ser Glu Leu Ala Ser
               420                 425                 430
<210> 12
   <211> 4143
   <212> DNA
   <213> Artificial
<220>
   <223> IntegrationConstruct
<400> 12
   aattgttcga tggatagctt tggagtctgt cccatcatga tacgaaaagc gtgaagctcc 60
   tctgacaatc aaaactttgt ttcaatgggg tgtaggatgg accccggatc caaacgaccg 120
   cgagtcaaaa aacctacggg tgcatttacc cgtagttgat ctggaaagtc gagatcaact 180
   ttttgtagtt tagttacatt catttcacgg tcgaaaaact cacacacaac gattgcagta 240
   tatttaccaa aatcgtctga agagaagcat ctgattgaga gttcaccatg acgaatccca 300
   taaacgacta ctccactgga cacaccgaca gacgccctgg ggatagtgaa actgaatttg 360
   tcggtataat ggcccgtctc acaggccggg cagaacactt tcatgtcctt tcgcaggtct 420
   cgacattgga caagtatgtt gtcgtgggtg acgacaaatt ggtcctcatc cttgaataag 480
   atgctccctt tgttctcagg aactggcacc attccattat gggcgaataa tttctgctca 540
   tcttcgggac tgatgccata ttcttctaac agaagacggc gctcacatgg gacctggtgc 600
   tctcgccggc ctctcaaatc gccggtgcat ctccacacgc aaattcacgg gtgtataccc 660
   ctgatcaaac gtatcttgcg cgttctgtta ttcattggag cgagggcccg atcctgtcct 720
   atcaaatgat ttcatgtggg aataatccat caattgttct ggattgaggt atacttcgag 780
   ctgtaaagat gtcgcttcta tgtcaagaat agtcggttaa acgcactcct tcaagattta 840
   catgatttac atgattcttc ataaagagca taaataaaga actgcagcca ttcttgagta 900
   aagtgctcag aataataaaa aggttgccac aggttgagtt aacatgggtt gattgaacca 960
   attaaggagg gaacgtttct tccatgggag gctaagaaac ttaataactt cgtataatgt 1020
   atgctatacg aagttattaa ttaactgacg ggcggatagt acaggctttg ccaaaagcct 1080
   ataaggctaa agaaagtaaa caagtgaggt tgaaccatga tggcagtgtt cgaattctga 1140
   tcaatgaagt acactgcgaa gggaatcccc gaaacggcga acaaaaagaa catcagagga 1200
   ggaacgccct cgcaatcccg aacataccag tttcgcagaa cctggggtat caactggatg 1260
   caccagcata ctgttcccac tgttgccaat gctgtagacg ctccattgtt gtcagtcatt 1320
   ttagcatttt acagtaacca actccaaaaa acagcccgct ctgctgggaa gacttcgcaa 1380
   ttatttatcc actactgctg cggttatata cttctcgatc tcagtctcgg ttataattgc 1440
   cgcttgacag cctggagaaa ttcggatact ccacgtgata attgccatag ggcataattt 1500
   tcgaaacagc tcgcaacgat ctcggctagt tttccccttt tttgacccat atcgacgctg 1560
   agactcactc acttgatgcc taccgttagg gtaaattttt caagcctgca gaatatcgcg 1620
   ggacgcagtc tcctgcacgc gcgtgacttc atcttactta catcaaacag cccgattaat 1680
   ttgaaaagtc ctagctgatc gagggcacgg gcactactgt agagaaataa tatgaagctg 1740
   agctatgagg agcgccgaga gaggctgccg gctgtagcag cccggctatt cgacatcatt 1800
   gtgagcaagc aaacaaatct ttgcgcaagc ttggatgtgc gaactacctc tgagttactg 1860
   agtatcctgg accgcattgg accttacatt tgtatggtta agacccacat tgacataatt 1920
   gacgacttcg aatacgacac aactgtcagc ggtttgaaac agctttcaac gaagcacaat 1980
   tttctcattt ttgaagaccg aaagttcgca gacatcggtt ccactgttaa ggcccaatat 2040
   gcaggtggag tgtttaagat cgctcaatgg gctgatataa caaatgctca cggtgttcct 2100
   gggccgggaa ttgtgagcgg actagaagag gctgcgaagg aaactacgga tgaacctcgc 2160
   ggccttgtca tgcttgcaga actgagttcg aagggcacac tggctcacgg cgaatactcg 2220
   caagcgacag tagacatcgc tcgcagtaac cgcgcatttg tgtttggttt catcgctcag 2280
   caaaaagtcg gaaagccaga ggaagactgg gtcattatga ctcctggggt gggcctggac 2340
   gacaaaggtg atggattggg gcagcagtat cgtactgtgg acgacgtcat agagaccggc 2400
   acagacgtta ttatcgtcgg acgcgggctc tatagcaagg gacgagatcc tgtgcacgaa 2460
   gctcagcgtt accaaaaggc gggctggaat gcatatctga gaaaagttca gtcaagatga 2520
   ttttctcaaa cagttccttc aatgcaactt gcacatgaat acctataaaa tctgattaaa 2580
   ttaccataaa aggtacagat taaaatatat atgccttcaa tggcatcctt cgcgattctg 2640
   attcgtcagc acacttcaac cttcctacta tgagtgacag tgatgatgat ctgctggcat 2700
   tggccgacgt tggctccgac tccgaagagg aaatctcgct gccgtcgccg ccaagcaatg 2760
   aggtcgtcaa tccctatcct ctagaaggca aatatctcga tgctgaagac agggcgaagt 2820
   tggacgcgct gccagagatt gagcgagaag agatcttgta tgaccgagct caggagatgc 2880
   agcggtacga ggagagaagg tatcttgctc agcgaaggaa gcagatgacg cgggttgctg 2940
   acgaggacga agccccctcc gccaagcgtc aacggggtac aacaggcgtc tcttcgggta 3000
   cgaagtcatc tcttgaggca ttaaagaaac gaagggccca gcagtctcgg aagtcctcac 3060
   gccatggagt tgatgacgat gtgtatagtg acgatgatgt taattaataa cttcgtataa 3120
   tgtatgctat acgaagttat atatgtactt ttcaatatga taaacggaga aataacgccc 3180
   ggctctatat gcaagctgca tcaaccctaa tatatattag cgagtttctc atgcaggctg 3240
   tagtttgagt cgctgtaacc tcagcctcaa gactcttaca ccataggtag agtttcgtca 3300
   ctgggaaact cagttactat ctaaaccaaa ctgtgctaat gctcaaacct atcactcaga 3360
   atttagattg aatcaatcta agtctgttga gaaacagata tgcatcaggg gcacagacta 3420
   aaagctgctc tcagcgagta cccttacctc ttgagaaccc tcaaaattta cccagcctgc 3480
   agcatatcat gcaccatggt taaattcgga aatgaattta ccggtggcct tgaaccacgt 3540
   tcctccaatt atttaaggca ataacctgcc actctcttga tttgattaag aaagactttc 3600
   aatttagctt ctccctacga atattcaatg agcccttcat cacacaaacc cctgattctc 3660
   gcttgcggct tgcctctttc aggccatata atgcccgttt tgagtctggt acacggcctt 3720
   acggacgacg gatacgaagc tactgttgtg acaggcagag cgtttgaaca aaaagttcga 3780
   gatgtgggtg cagactttgt tcctttagaa gggaacgcag attttgatga ccacacctta 3840
   gacgatctgg tcccgggccg taaagacatg gccccaagct tcgatcgtac agttcaagat 3900
   gtggagcaca tgatggtagc tactcttcct gagcagtttg ccgctattca gagggctttc 3960
   aaaaagctca gcgcaagcgg ccgccctgtc gttcttgtca gtgaagtgct gtttttcggt 4020
   gcacacccta tcagcctcgg tgctcctggt ttcaaacccg ctggctggat ttgtttaggg 4080
   gttttgcctc ttttgatccg cagtgatcat accttaggac ttgacaacga caggagcccc 4140
   gaa 4143
<210> 13
   <211> 4143
   <212> DNA
   <213> Artificial
<220>
   <223> IntegrationConstruct
<400> 13
   gaaatctgat caattctgca aacctgatct ttagtgaact gaggtctcaa catcgatcga      60
   gactgtttcc atccatttcc gctgagtgta aatatccctt ggccaaacac ttttcccact     120
   gtgtggaaac gtgctccaag accaaaatca ttgaatttgg ttgccaggat tgtcttaatg     180
   ttttctggct cgattgtgaa gatttggtat tgaaggggag cttgtcgaag atacgtccgt     240
   gctttgaact tattgaagac tctgtcgtat tgaacttcca gtaaggtgta tgacttggcc     300
   gtcttgatca tgtccatggt tctttgtatt cccagtggga acgatttctc aatgaagcga     360
   ggcatactac acttgtgcct acgtgctgca tagcggtacc ataggagcca gataggctcg     420
   tgtagaacta agaaagctac gaagagcagt ggcaacaagc cagcaacagc ggataaactc     480
   attggagtta gaataatgtc tttgattaac atatatgtac ttttcaatat gataaacgga     540
   gaaataacgc ccggctctat atgcaagctg catcaaccct aatatatatt agcgagtttc     600
   tcatgcaggc tgtagtttga gtcgctgtaa cctcagcctc aagactctta caccataggt     660
   agagtttcgt cactgggaaa ctcagttact atctaaacca aactgtgcta atgctcaaac     720
   ctatcactca gaatttagat tgaatcaatc taagtctgtt gagaaacaga tatgcatcag     780
   gggcacagac taaaagctgc tctcagcgag tacccttacc tcttgagaac cctcaaaatt     840
   tacccagcct gcagcatatc atgcaccatg gttaaattcg gaaatgaatt taccggtggc     900
   cttgaaccac gttcctccaa ttatttaagg caataacctg ccactctctt gatttgatta     960
   agaaagactt tcaatttagc ttctccctac gaatattcaa taacttcgta taatgtatgc    1020
   tatacgaagt tattaattaa ctgacgggcg gatagtacag gctttgccaa aagcctataa    1080
   ggctaaagaa agtaaacaag tgaggttgaa ccatgatggc agtgttcgaa ttctgatcaa    1140
   tgaagtacac tgcgaaggga atccccgaaa cggcgaacaa aaagaacatc agaggaggaa 1200
   cgccctcgca atcccgaaca taccagtttc gcagaacctg gggtatcaac tggatgcacc 1260
   agcatactgt tcccactgtt gccaatgctg tagacgctcc attgttgtca gtcattttag 1320
   cattttacag taaccaactc caaaaaacag cccgctctgc tgggaagact tcgcaattat 1380
   ttatccacta ctgctgcggt tatatacttc tcgatctcag tctcggttat aattgccgct 1440
   tgacagcctg gagaaattcg gatactccac gtgataattg ccatagggca taattttcga 1500
   aacagctcgc aacgatctcg gctagttttc cccttttttg acccatatcg acgctgagac 1560
   tcactcactt gatgcctacc gttagggtaa atttttcaag cctgcagaat atcgcgggac 1620
   gcagtctcct gcacgcgcgt gacttcatct tacttacatc aaacagcccg attaatttga 1680
   aaagtcctag ctgatcgagg gcacgggcac tactgtagag aaataatatg aagctgagct 1740
   atgaggagcg ccgagagagg ctgccggctg tagcagcccg gctattcgac atcattgtga 1800
   gcaagcaaac aaatctttgc gcaagcttgg atgtgcgaac tacctctgag ttactgagta 1860
   tcctggaccg cattggacct tacatttgta tggttaagac ccacattgac ataattgacg 1920
   acttcgaata cgacacaact gtcagcggtt tgaaacagct ttcaacgaag cacaattttc 1980
   tcatttttga agaccgaaag ttcgcagaca tcggttccac tgttaaggcc caatatgcag 2040
   gtggagtgtt taagatcgct caatgggctg atataacaaa tgctcacggt gttcctgggc 2100
   cgggaattgt gagcggacta gaagaggctg cgaaggaaac tacggatgaa cctcgcggcc 2160
   ttgtcatgct tgcagaactg agttcgaagg gcacactggc tcacggcgaa tactcgcaag 2220
   cgacagtaga catcgctcgc agtaaccgcg catttgtgtt tggtttcatc gctcagcaaa 2280
   aagtcggaaa gccagaggaa gactgggtca ttatgactcc tggggtgggc ctggacgaca 2340
   aaggtgatgg attggggcag cagtatcgta ctgtggacga cgtcatagag accggcacag 2400
   acgttattat cgtcggacgc gggctctata gcaagggacg agatcctgtg cacgaagctc 2460
   agcgttacca aaaggcgggc tggaatgcat atctgagaaa agttcagtca agatgatttt 2520
   ctcaaacagt tccttcaatg caacttgcac atgaatacct ataaaatctg attaaattac 2580
   cataaaaggt acagattaaa atatatatgc cttcaatggc atccttcgcg attctgattc 2640
   gtcagcacac ttcaaccttc ctactatgag tgacagtgat gatgatctgc tggcattggc 2700
   cgacgttggc tccgactccg aagaggaaat ctcgctgccg tcgccgccaa gcaatgaggt 2760
   cgtcaatccc tatcctctag aaggcaaata tctcgatgct gaagacaggg cgaagttgga 2820
   cgcgctgcca gagattgagc gagaagagat cttgtatgac cgagctcagg agatgcagcg 2880
   gtacgaggag agaaggtatc ttgctcagcg aaggaagcag atgacgcggg ttgctgacga 2940
   ggacgaagcc ccctccgcca agcgtcaacg gggtacaaca ggcgtctctt cgggtacgaa 3000
   gtcatctctt gaggcattaa agaaacgaag ggcccagcag tctcggaagt cctcacgcca 3060
   tggagttgat gacgatgtgt atagtgacga tgatgttaat taataacttc gtataatgta   3120
   tgctatacga agttattaga atcgtacgat caaatcagat cagggaagag aggtagggtt   3180
   ttttttattt atgtctttgt ttttattgat tgaaatttac aatacaacaa ccatcaaatt   3240
   aatttgaaca aacaacaaca cacacacaca ctgcaacttt caaaaaaata agtaaaagga   3300
   agagaggagt ttgccaatat atttaccttc ttctaattct gttatttttt ttaattgttt   3360
   tgtggaaaga aagaagaaaa ggctgtcatg aatttagttt acctagacct tctggttagc   3420
   ggtattgacg ttcatttcaa ctggaagaag gaattccagt tcctctcctt cagcctcgtc   3480
   gggatcctcc tctggaatat gcttgaggat tcgcgcaggg actcctccca ccacagtacg   3540
   aggaggaaca tcttctcgaa cgacagcacc agccgcaatt gttgagccat ctccaatcgt   3600
   aacacccggc aggacagtca cattcgcacc aatccataca ttattcccca ccttgatagg   3660
   aagagcatac acaattctcc tcgcacgttt ctcggggcta ataggatgag tcgcagtcac   3720
   gaacgttgta ttgggcccta caatcacctc atcaccaaag attattggag ccgagtccaa   3780
   gaagcaaacg ttgaagttgg cgtaaaagtg ctcgcctacg ctgatgttga atccaaaatc   3840
   aactgagaat ggagcggtca gccagacaat atcctttgtt tgaccaaaag tgtctttgag   3900
   aatctcgacc ttcttgatat aagcagcgtg atttgactca aaagtacgac tttcacttgc   3960
   aatggtattg aactccctaa ctttctcact agtagccagg gctctaaaca taagatctgg   4020
   atcgtatgga ttgtaaggaa ctcctgagac catcttctca tagttttcat tgccaggggt   4080
   gtttttgagg ttttttttgg cccaagagac catttcctgg tcaatttctt ttctaggagt   4140
   cat                                                                 4143
<210> 14
   <211> 4140
   <212> DNA
   <213> Artificial
<220>
   <223> IntegrationConstruct
<400> 14
   tgcagacaag ttcctgcagc tgtgcccgcc ttctcttgag ttcagcagag accatctgcc      60
   tagcaacttc aaattcgccg gctcaacgcc caagcaccga actcaattca cccctccttc     120
   ctggtggggg gatgttctga gtgccaagcg agtcatcatg gtcactcaag gaacttttgc     180
   tgtcagttac aagcatctta ttgtgcctac tcttgaggcc ttgaaggacg agcctgacac     240
   tttaacagta gccatattgg gccgccgcgg tgccaagcta ccggatgatg ttgtggttcc     300
   tgagaatgct cgcgtgatcg actacttcaa ctacgatgct ctacttcctc acgttgatgc     360
   tcttgtctac aatggtggat atggcggact tcagcacagc ttaagccact ctgttccagt     420
   tgttattgct ggtgactctg aagacaagcc aatggtggca tcgagagctg aggccgctgg     480
   cgtggcaatt gatttgaaaa ctggcttgcc tacagtggag caaatcaaag aagctgttga     540
   ttcgataatt ggaaatccga aattccacga agcctcgaag aaggttcaaa tggagttgga 600
   aagccacaac tccttgaaaa ttcttgagga aagcatcgag gaaatcgcca gccatgactt 660
   tggtcttttg accaagagtg acgaggaaac tgaagatata cctgtcaaag ggccggcctt 720
   agcggtgagt tcttagaatc gtacgatcaa atcagatcag ggaagagagg tagggttttt 780
   tttatttatg tctttgtttt tattgattga aatttacaat acaacaacca tcaaattaat 840
   ttgaacaaac aacaacacac acacacactg caactttcaa aaaaataagt aaaaggaaga 900
   gaggagtttg ccaatatatt taccttcttc taattctgtt atttttttta attgttttgt 960
   ggaaagaaag aagaaaaggc tgtcatgaat ttagtttacc taataacttc gtataatgta 1020
   tgctatacga agttattaat taactgacgg gcggatagta caggctttgc caaaagccta 1080
   taaggctaaa gaaagtaaac aagtgaggtt gaaccatgat ggcagtgttc gaattctgat 1140
   caatgaagta cactgcgaag ggaatccccg aaacggcgaa caaaaagaac atcagaggag 1200
   gaacgccctc gcaatcccga acataccagt ttcgcagaac ctggggtatc aactggatgc 1260
   accagcatac tgttcccact gttgccaatg ctgtagacgc tccattgttg tcagtcattt 1320
   tagcatttta cagtaaccaa ctccaaaaaa cagcccgctc tgctgggaag acttcgcaat 1380
   tatttatcca ctactgctgc ggttatatac ttctcgatct cagtctcggt tataattgcc 1440
   gcttgacagc ctggagaaat tcggatactc cacgtgataa ttgccatagg gcataatttt 1500
   cgaaacagct cgcaacgatc tcggctagtt ttcccctttt ttgacccata tcgacgctga 1560
   gactcactca cttgatgcct accgttaggg taaatttttc aagcctgcag aatatcgcgg 1620
   gacgcagtct cctgcacgcg cgtgacttca tcttacttac atcaaacagc ccgattaatt 1680
   tgaaaagtcc tagctgatcg agggcacggg cactactgta gagaaataat atgaagctga 1740
   gctatgagga gcgccgagag aggctgccgg ctgtagcagc ccggctattc gacatcattg 1800
   tgagcaagca aacaaatctt tgcgcaagct tggatgtgcg aactacctct gagttactga 1860
   gtatcctgga ccgcattgga ccttacattt gtatggttaa gacccacatt gacataattg 1920
   acgacttcga atacgacaca actgtcagcg gtttgaaaca gctttcaacg aagcacaatt 1980
   ttctcatttt tgaagaccga aagttcgcag acatcggttc cactgttaag gcccaatatg 2040
   caggtggagt gtttaagatc gctcaatggg ctgatataac aaatgctcac ggtgttcctg 2100
   ggccgggaat tgtgagcgga ctagaagagg ctgcgaagga aactacggat gaacctcgcg 2160
   gccttgtcat gcttgcagaa ctgagttcga agggcacact ggctcacggc gaatactcgc 2220
   aagcgacagt agacatcgct cgcagtaacc gcgcatttgt gtttggtttc atcgctcagc 2280
   aaaaagtcgg aaagccagag gaagactggg tcattatgac tcctggggtg ggcctggacg 2340
   acaaaggtga tggattgggg cagcagtatc gtactgtgga cgacgtcata gagaccggca 2400
   cagacgttat tatcgtcgga cgcgggctct atagcaaggg acgagatcct gtgcacgaag 2460
   ctcagcgtta ccaaaaggcg ggctggaatg catatctgag aaaagttcag tcaagatgat 2520
   tttctcaaac agttccttca atgcaacttg cacatgaata cctataaaat ctgattaaat 2580
   taccataaaa ggtacagatt aaaatatata tgccttcaat ggcatccttc gcgattctga 2640
   ttcgtcagca cacttcaacc ttcctactat gagtgacagt gatgatgatc tgctggcatt 2700
   ggccgacgtt ggctccgact ccgaagagga aatctcgctg ccgtcgccgc caagcaatga 2760
   ggtcgtcaat ccctatcctc tagaaggcaa atatctcgat gctgaagaca gggcgaagtt 2820
   ggacgcgctg ccagagattg agcgagaaga gatcttgtat gaccgagctc aggagatgca 2880
   gcggtacgag gagagaaggt atcttgctca gcgaaggaag cagatgacgc gggttgctga 2940
   cgaggacgaa gccccctccg ccaagcgtca acggggtaca acaggcgtct cttcgggtac 3000
   gaagtcatct cttgaggcat taaagaaacg aagggcccag cagtctcgga agtcctcacg 3060
   ccatggagtt gatgacgatg tgtatagtga cgatgatgtt aattaataac ttcgtataat 3120
   gtatgctata cgaagttatt gaattctaga atgtgaggtg gaatgaggca aggaaggagg 3180
   aacgtattga gttgtacctt aagatatctc aaagtgctta tctccgacta ccggaatatg 3240
   ctccgggtaa tgcaagtcag tgtgcatatg ggtaaggtga tgcaagctaa ccctcagggc 3300
   atatctaatt cgcgtgaggg ttattattgg tctacattac ctcagtcata gcccgtcaaa 3360
   gcaaaagccc aaaatcagca cgaaatccca gagatagatt gttgctgtct cttcaagtac 3420
   tacgacagtt ccctatatct acagattatc gtcacgagtg aattatgcag gataggtgac 3480
   tcaggggtca taatcagagg aatccaatgt gctatttcaa ttaacgagtc cctttaatca 3540
   gacaatgtat ggtgactcag gggccataac tagagaaatt cgatatgcta tttcaattaa 3600
   tgagtgcctt taatcaaata atgtatgcaa gcagtggcca aaaataaatg aacgtcaaat 3660
   ctctccgaga ccttgcaagt tcaccaattc agcgtaccat ccattgagtt caaggaggct 3720
   ctgatggtcg ccctgctcca cgatgcgccc tcctgagaac acatatatga catctgcttt 3780
   ctgaattgtt gataatctat gcgcaacggc gattgtagta cggcccttcg ctgctgcgtc 3840
   gagtgctgct tgaactactt tctcagattc ggaatccaga gctgaggtgg cctcatcgag 3900
   gaggagtacc tttggatttc tgatcagggc ccttgcaatt gcaattcgct gcttttgccc 3960
   cccagatagc aacgatcccc tagatccgct gagcgtttcg tagccatcag gcaacgacat 4020
   gatgaattcg tgaatgttcg ctttgcgagc ggcatcctca atcatctcct gcgttacttc 4080
   agactcaggg ccagaccatc ccattagaat attctcacgt agcgtgcctg aataaagcat 4140
<210> 15
   <211> 4130
   <212> DNA
   <213> Artificial
<220>
   <223> IntegrationConstruct
<400> 15
   ggatgagtcg cagtcacgaa cgttgtattg ggccctacaa tcacctcatc accaaagatt 60
   attggagccg agtccaagaa gcaaacgttg aagttggcgt aaaagtgctc gcctacgctg 120
   atgttgaatc caaaatcaac tgagaatgga gcggtcagcc agacaatatc ctttgtttga 180
   ccaaaagtgt ctttgagaat ctcgaccttc ttgatataag cagcgtgatt tgactcaaaa 240
   gtacgacttt cacttgcaat ggtattgaac tccctaactt tctcactagt agccagggct 300
   ctaaacataa gatctggatc gtatggattg taaggaactc ctgagaccat cttctcatag 360
   ttttcattgc caggggtgtt tttgaggttt tttttggccc aagagaccat ttcctggtca 420
   atttcttttc taggagtcat tcctttgttt tgagggtcct tcgaggagtt tacaaccatt 480
   gaattctaga atgtgaggtg gaatgaggca aggaaggagg aacgtattga gttgtacctt 540
   aagatatctc aaagtgctta tctccgacta ccggaatatg ctccgggtaa tgcaagtcag 600
   tgtgcatatg ggtaaggtga tgcaagctaa ccctcagggc atatctaatt cgcgtgaggg 660
   ttattattgg tctacattac ctcagtcata gcccgtcaaa gcaaaagccc aaaatcagca 720
   cgaaatccca gagatagatt gttgctgtct cttcaagtac tacgacagtt ccctatatct 780
   acagattatc gtcacgagtg aattatgcag gataggtgac tcaggggtca taatcagagg 840
   aatccaatgt gctatttcaa ttaacgagtc cctttaatca gacaatgtat ggtgactcag 900
   gggccataac tagagaaatt cgatatgcta tttcaattaa tgagtgcctt taatcaaata 960
   atgtatgcaa gcagtggcca aaaataaatg aacgtcaata acttcgtata atgtatgcta 1020
   tacgaagtta ttaattaact gacgggcgga tagtacaggc tttgccaaaa gcctataagg 1080
   ctaaagaaag taaacaagtg aggttgaacc atgatggcag tgttcgaatt ctgatcaatg 1140
   aagtacactg cgaagggaat ccccgaaacg gcgaacaaaa agaacatcag aggaggaacg 1200
   ccctcgcaat cccgaacata ccagtttcgc agaacctggg gtatcaactg gatgcaccag 1260
   catactgttc ccactgttgc caatgctgta gacgctccat tgttgtcagt cattttagca 1320
   ttttacagta accaactcca aaaaacagcc cgctctgctg ggaagacttc gcaattattt 1380
   atccactact gctgcggtta tatacttctc gatctcagtc tcggttataa ttgccgcttg 1440
   acagcctgga gaaattcgga tactccacgt gataattgcc atagggcata attttcgaaa 1500
   cagctcgcaa cgatctcggc tagttttccc cttttttgac ccatatcgac gctgagactc 1560
   actcacttga tgcctaccgt tagggtaaat ttttcaagcc tgcagaatat cgcgggacgc 1620
   agtctcctgc acgcgcgtga cttcatctta cttacatcaa acagcccgat taatttgaaa 1680
   agtcctagct gatcgagggc acgggcacta ctgtagagaa ataatatgaa gctgagctat 1740
   gaggagcgcc gagagaggct gccggctgta gcagcccggc tattcgacat cattgtgagc 1800
   aagcaaacaa atctttgcgc aagcttggat gtgcgaacta cctctgagtt actgagtatc 1860
   ctggaccgca ttggacctta catttgtatg gttaagaccc acattgacat aattgacgac 1920
   ttcgaatacg acacaactgt cagcggtttg aaacagcttt caacgaagca caattttctc 1980
   atttttgaag accgaaagtt cgcagacatc ggttccactg ttaaggccca atatgcaggt 2040
   ggagtgttta agatcgctca atgggctgat ataacaaatg ctcacggtgt tcctgggccg 2100
   ggaattgtga gcggactaga agaggctgcg aaggaaacta cggatgaacc tcgcggcctt 2160
   gtcatgcttg cagaactgag ttcgaagggc acactggctc acggcgaata ctcgcaagcg 2220
   acagtagaca tcgctcgcag taaccgcgca tttgtgtttg gtttcatcgc tcagcaaaaa 2280
   gtcggaaagc cagaggaaga ctgggtcatt atgactcctg gggtgggcct ggacgacaaa 2340
   ggtgatggat tggggcagca gtatcgtact gtggacgacg tcatagagac cggcacagac 2400
   gttattatcg tcggacgcgg gctctatagc aagggacgag atcctgtgca cgaagctcag 2460
   cgttaccaaa aggcgggctg gaatgcatat ctgagaaaag ttcagtcaag atgattttct 2520
   caaacagttc cttcaatgca acttgcacat gaatacctat aaaatctgat taaattacca 2580
   taaaaggtac agattaaaat atatatgcct tcaatggcat ccttcgcgat tctgattcgt 2640
   cagcacactt caaccttcct actatgagtg acagtgatga tgatctgctg gcattggccg 2700
   acgttggctc cgactccgaa gaggaaatct cgctgccgtc gccgccaagc aatgaggtcg 2760
   tcaatcccta tcctctagaa ggcaaatatc tcgatgctga agacagggcg aagttggacg 2820
   cgctgccaga gattgagcga gaagagatct tgtatgaccg agctcaggag atgcagcggt 2880
   acgaggagag aaggtatctt gctcagcgaa ggaagcagat gacgcgggtt gctgacgagg 2940
   acgaagcccc ctccgccaag cgtcaacggg gtacaacagg cgtctcttcg ggtacgaagt 3000
   catctcttga ggcattaaag aaacgaaggg cccagcagtc tcggaagtcc tcacgccatg 3060
   gagttgatga cgatgtgtat agtgacgatg atgttaatta ataacttcgt ataatgtatg 3120
   ctatacgaag ttataagcca aaatcagaga gtgggacctg attcagaatc acacggaccc 3180
   gtatatataa caatcacttt ccaacaatat agcgagtatt aatatatttc cgggtaaggg 3240
   ttgttccgga cttatgcatt taatcacagg ttgcatcagc taaatatgtc agggccgacg 3300
   gcgtaaattt agaaggttag gtcaagatcc atcggtcagg ccaatggagc tctactatga 3360
   taggcagctg aagcgagaca agatatactt cagttgcgct ctctgaaaaa attattttgt 3420
   gattctcact cagtggatgt ggcgacacac ggaaccaata atctcgccgg aaaggcggct 3480
   gaacatcagt cttgcataag tgtgcaagtg gcctgagcac agcgtgcatt acccttacca 3540
   tacattcggg gcaagttaaa tccagcatta tataaacttg attgacacaa atgggcataa 3600
   aacaataaag tctcctatat ggccatcgag aaaccagtga tagttgcttg tgcctgccca 3660
   ctagcggggc acgtgggccc agtgctcagc ctggtccgcg gtctactcaa tagaggatat 3720
   gaggtgactt tcgtaacagg gaacgcattc aaggagaaag ttattgaggc aggatgcact 3780
   ttcgtccctc tccaaggacg agctgactac catgaataca atctccctga aatcgctcca 3840
   ggattgctca cgattcctcc aggccttgag cagaccggtt actcaatgaa tgagattttt 3900
   gtgaaggcga ttcctgagca gtacgatgca cttcaaactg ctctaaaaca ggttgaggct 3960
   gaaaataaat cagctgtggt gattggcgag accatgtttc taggggtgca tccgatatca 4020
   ctgggtgccc caggtctcaa gccccaaggc gtaatcacgt taggaactat tccgtgcatg 4080
   ctgaaagcag agaaggcgcc tggagttcct agtcttgagc caatgattga 4130
<210> 16
   <211> 4141
   <212> DNA
   <213> Artificial
<220>
   <223> IntegrationConstruct
<400> 16
   attctggtgc tgacctcgcc accacctagt ttgtcgtaaa acgcgatatt ctggcgaata      60
   acagcactca gataatgctt tcggtaacgt cctgccaaca cttcgcctct gtccacaagc     120
   aggaagctct cgagaaacgc actgccgagc ataccaatgc caatatagac aaaatagaga     180
   gacaggtgat tcaccttatg ctggaactca ttgcccttga ggtcatatga agtgaagtct     240
   ctgaatgtgt tgaagatggc gcccactact aacgtgaaca ttggaagcgc ggctccatgc     300
   accgctgcaa aaaaaagcgc aagtatctcc aagaaaacgt caaggggagt gcaaaatctg     360
   aacaacctga aaaagcttgt ggcgactctc tttgtttcaa gctgacttcg caatacattg     420
   gcctcatgtg gatctaacgc agagagcttc tcctcgagaa gcttgtcctt agtctcgatg     480
   agtttctcac gcttctctac ctgtatatca tccaccataa gccaaaatca gagagtggga     540
   cctgattcag aatcacacgg acccgtatat ataacaatca ctttccaaca atatagcgag     600
   tattaatata tttccgggta agggttgttc cggacttatg catttaatca caggttgcat     660
   cagctaaata tgtcagggcc gacggcgtaa atttagaagg ttaggtcaag atccatcggt     720
   caggccaatg gagctctact atgataggca gctgaagcga gacaagatat acttcagttg     780
   cgctctctga aaaaattatt ttgtgattct cactcagtgg atgtggcgac acacggaacc     840
   aataatctcg ccggaaaggc ggctgaacat cagtcttgca taagtgtgca agtggcctga     900
   gcacagcgtg cattaccctt accatacatt cggggcaagt taaatccagc attatataaa     960
   cttgattgac acaaatgggc ataaaacaat aaagtctcct atataacttc gtataatgta    1020
   tgctatacga agttattaat taactgacgg gcggatagta caggctttgc caaaagccta    1080
   taaggctaaa gaaagtaaac aagtgaggtt gaaccatgat ggcagtgttc gaattctgat    1140
   caatgaagta cactgcgaag ggaatccccg aaacggcgaa caaaaagaac atcagaggag    1200
   gaacgccctc gcaatcccga acataccagt ttcgcagaac ctggggtatc aactggatgc    1260
   accagcatac tgttcccact gttgccaatg ctgtagacgc tccattgttg tcagtcattt    1320
   tagcatttta cagtaaccaa ctccaaaaaa cagcccgctc tgctgggaag acttcgcaat    1380
   tatttatcca ctactgctgc ggttatatac ttctcgatct cagtctcggt tataattgcc 1440
   gcttgacagc ctggagaaat tcggatactc cacgtgataa ttgccatagg gcataatttt 1500
   cgaaacagct cgcaacgatc tcggctagtt ttcccctttt ttgacccata tcgacgctga 1560
   gactcactca cttgatgcct accgttaggg taaatttttc aagcctgcag aatatcgcgg 1620
   gacgcagtct cctgcacgcg cgtgacttca tcttacttac atcaaacagc ccgattaatt 1680
   tgaaaagtcc tagctgatcg agggcacggg cactactgta gagaaataat atgaagctga 1740
   gctatgagga gcgccgagag aggctgccgg ctgtagcagc ccggctattc gacatcattg 1800
   tgagcaagca aacaaatctt tgcgcaagct tggatgtgcg aactacctct gagttactga 1860
   gtatcctgga ccgcattgga ccttacattt gtatggttaa gacccacatt gacataattg 1920
   acgacttcga atacgacaca actgtcagcg gtttgaaaca gctttcaacg aagcacaatt 1980
   ttctcatttt tgaagaccga aagttcgcag acatcggttc cactgttaag gcccaatatg 2040
   caggtggagt gtttaagatc gctcaatggg ctgatataac aaatgctcac ggtgttcctg 2100
   ggccgggaat tgtgagcgga ctagaagagg ctgcgaagga aactacggat gaacctcgcg 2160
   gccttgtcat gcttgcagaa ctgagttcga agggcacact ggctcacggc gaatactcgc 2220
   aagcgacagt agacatcgct cgcagtaacc gcgcatttgt gtttggtttc atcgctcagc 2280
   aaaaagtcgg aaagccagag gaagactggg tcattatgac tcctggggtg ggcctggacg 2340
   acaaaggtga tggattgggg cagcagtatc gtactgtgga cgacgtcata gagaccggca 2400
   cagacgttat tatcgtcgga cgcgggctct atagcaaggg acgagatcct gtgcacgaag 2460
   ctcagcgtta ccaaaaggcg ggctggaatg catatctgag aaaagttcag tcaagatgat 2520
   tttctcaaac agttccttca atgcaacttg cacatgaata cctataaaat ctgattaaat 2580
   taccataaaa ggtacagatt aaaatatata tgccttcaat ggcatccttc gcgattctga 2640
   ttcgtcagca cacttcaacc ttcctactat gagtgacagt gatgatgatc tgctggcatt 2700
   ggccgacgtt ggctccgact ccgaagagga aatctcgctg ccgtcgccgc caagcaatga 2760
   ggtcgtcaat ccctatcctc tagaaggcaa atatctcgat gctgaagaca gggcgaagtt 2820
   ggacgcgctg ccagagattg agcgagaaga gatcttgtat gaccgagctc aggagatgca 2880
   gcggtacgag gagagaaggt atcttgctca gcgaaggaag cagatgacgc gggttgctga 2940
   cgaggacgaa gccccctccg ccaagcgtca acggggtaca acaggcgtct cttcgggtac 3000
   gaagtcatct cttgaggcat taaagaaacg aagggcccag cagtctcgga agtcctcacg 3060
   ccatggagtt gatgacgatg tgtatagtga cgatgatgtt aattaataac ttcgtataat 3120
   gtatgctata cgaagttatt aacctggctc tttttctaga tatgtctgcg ccctgctcac 3180
   tgcttactgg cctaagctgg tattacggac cttaatcaag tatcacccca aggcaatcga 3240
   gagtcttatc gagtctctag gtagatagat acacgttttg atttttcggc ccactttgta 3300
   gaaaaatctc agtgatttca tggaattcag ttacaaatac taatctgata aaccaagaac 3360
   tacactcggt gttgagagca gaattaaagg gacttggcgt ctagcacaaa acgatacttg 3420
   acgtcaccac tgtgaacgcg cttccaagct tcggcgatat agctgtactc aatcagctca 3480
   acatcacagg tgatgttatt ttcaccacag aagtccagca tctcctgagt ctctggcaag 3540
   ccaccaatgt ttgagtaagt gatagattta tttccagcca aatgagaggt cagaaccttg 3600
   aggggtccaa tttgaccaac aacaacgaga cacccaccaa tatcaaggga cttgaggtat 3660
   ggctcgaagt cgtgttcaaa gggaatggtg tcgatgatca ggtcaaatgt gccagcgacc 3720
   gcctcgagct cattcggatc agaggaagca actacgcggc tagcaccttg tgctttcgct 3780
   cctgcggctt tggcgtgact cctgctgaac agtgtgactt cagagcccat ggctgaggca 3840
   aatttgatag ccatggaacc aaggcctccg agaccaacta caccgactct ttttccaggt 3900
   ccggcgccgt gagccctcag aggagagtag gtagtgatac cagcacagag aaggggcgca 3960
   gaagctgcca agtcgaggtt ggaggggatt ttgagcacaa actcctcgcg agcaagaatg 4020
   tgttgcgaat accctccctt cgtgacttcc ccgttctttc cgctggaatt gtaagtttga 4080
   gtgcgtgaaa cacaccaatt ttctttgcct aatttacagt tcttgcaagt acgacatgag 4140
   t 4141
<210> 17
   <211> 27
   <212> DNA
   <213> Artificial
<220>
   <223> Primer
<400> 17
   aattgttcga tggatagctt tggagtc 27
<210> 18
   <211> 21
   <212> DNA
   <213> Artificial
<220>
   <223> Primer
<400> 18
   ttcggggctc ctgtcgttgt c 21
<210> 19
   <211> 26
   <212> DNA
   <213> Artificial
<220>
   <223> Primer
<400> 19
   gaaatctgat caattctgca aacctg 26
<210> 20
   <211> 27
   <212> DNA
   <213> Artificial
<220>
   <223> Primer
<400> 20
   atgactccta gaaaagaaat tgaccag 27
<210> 21
   <211> 22
   <212> DNA
   <213> Artificial
<220>
   <223> Primer
<400> 21
   tgcagacaag ttcctgcagc tg 22
<210> 22
   <211> 23
   <212> DNA
   <213> Artificial
<220>
   <223> Primer
<400> 22
   atgctttatt caggcacgct acg 23
<210> 23
   <211> 21
   <212> DNA
   <213> Artificial
<220>
   <223> Primer
<400> 23
   ggatgagtcg cagtcacgaa c 21
<210> 24
   <211> 26
   <212> DNA
   <213> Artificial
<220>
   <223> Primer
<400> 24
   tcaatcattg gctcaagact aggaac 26
<210> 25
   <211> 22
   <212> DNA
   <213> Artificial
<220>
   <223> Primer
<400> 25
   attctggtgc tgacctcgcc ac 22
<210> 26
   <211> 25
   <212> DNA
   <213> Artificial
<220>
   <223> Primer
<400> 26
   actcatgtcg tacttgcaag aactg 25
<210> 27
   <211> 27
   <212> DNA
   <213> Artificial
<220>
   <223> Primer
<400> 27
   gtgtcgactc gccaaattcc atcggag 27
<210> 28
   <211> 28
   <212> DNA
   <213> Artificial
<220>
   <223> Primer
<400> 28
   ggttcatagc gagtttcttt gcatgtgc 28
<210> 29
   <211> 28
   <212> DNA
   <213> Artificial
<220>
   <223> Primer
<400> 29
   ctcctttatt aactccgcag catgactg 28
<210> 30
   <211> 27
   <212> DNA
   <213> Artificial
<220>
   <223> Primer
<400> 30
   ctcctcgaag gaccctcaaa acaaagg 27
<210> 31
   <211> 30
   <212> DNA
   <213> Artificial
<220>
   <223> Primer
<400> 31
   caaatttatc tgggagcaca gttacattgc 30
<210> 32
   <211> 28
   <212> DNA
   <213> Artificial
<220>
   <223> Primer
<400> 32
   cacacattgc tttagtccag caagaacc 28
<210> 33
   <211> 25
   <212> DNA
   <213> Artificial
<220>
   <223> Primer
<400> 33
   attctcctcg cacgtttctc ggggc 25
<210> 34
   <211> 28
   <212> DNA
   <213> Artificial
<220>
   <223> Primer
<400> 34
   ggttgaaata cttgttgccg cactaaag 28
<210> 35
   <211> 31
   <212> DNA
   <213> Artificial
<220>
   <223> Primer
<400> 35
   cgcttcctga attgagttgg tatcgttaat g 31
<210> 36
   <211> 28
   <212> DNA
   <213> Artificial
<220>
   <223> Primer
<400> 36
   gacattgttg gaattggctg cttagtgg 28
<210> 37
   <211> 3107
   <212> DNA
   <213> Artificial
<220>
   <223> ExpressionCassette
<400> 37
   acaaacgacc ccgccccacc cctcacacgg ccttaccagc ccaggaagca atggcccgaa 60
   cctcgtgggc taccgcactc cgtttggaaa cccaatagga actgcagcag cagggaactc 120
   agctgctact ccagctggaa accctctagg gaaggtaaga gcagactctt caacgagcct 180
   tactactcag ggacagcgaa gggtccgcgt gcatgtccag ggcgacacat ttctcatttt 240
   ggtgccaccg gacctgaagt ttgagcatct ttccaatcgt gttgagcgca agctccgact 300
   atgtgggaaa atgccgcctt caggccaggc aggctcactc atttttgaat acatggatga 360
   agacgaggac cgcgtgcgac tggagagcga cgaggaccta agtgtggcgt ttgaggctgt 420
   gcccgaccac catgagctgt ccgtctacgt caaaaactga cgattatgat ctaatgatat 480
   ttaaaagata tgtaaaacgg ttattttttg gacctgcgcc ctaaaatggg actttgtcaa 540
   aaaaagaacg gcctcctgcg cgatggagag caatcaagaa ttcggagttc cgatgcgaat 600
   ccatcaagaa aacggcccct aggcaatcta aaaccgtggc cgacatacta taagtcaatt 660
   ccgctgtaca aataacaagc gatcaatcca taatctgagg ctcatttcat acggactttt 720
   ctaagttcac ataattctat gatgcatact aacaaatacg atgcacaaat gggtacaagg 780
   cctaaagagg gccacaatcg cgatttactc gatacggcaa atcagttcca caagtaattc 840
   gctatcgtcg gtgttgttat acacctctcg gcttgagtca atatcgagca tgcaaggttg 900
   acgcattctg gggaaatgta tccacgtgat cgccgatatc ggagcggata cgctgtgtag 960
   tcttcagttg taagatttct tatacagcga cgcaaccatc atgtctgtgc aaacgaaaac 1020
   aattgttctt cttcctggag accactgtgg cccagaagtc gttgccgaag cagtgaaagt 1080
   actcaaagcc gtggaaactg ctttaccatc ggttaccttc gagtttcagc accatttgat 1140
   tggcggtgct gccatagatg ctgctggtgt tcccattacg gaagagactc ttgctgcctc 1200
   tagaaaggct gacgctgttt tgcttggtgc tgtaggaggg cccaagtggg gcactggctc 1260
   agtgagaccc gaacagggtc tcctcaagat tcgcaaggag cttcaattgt acgcgaatct 1320
   gcgtccctgt aacatcattg ctccaaagtt tgccaagctc agtcctctga aggaggagaa 1380
   tgttttggga accgacatta tgattgtacg agaactcaca ggtggaatct acttcggaga   1440
   tcgcgaagaa gccgatatga gcacggccga ccctcatgcc acagatactg agaagtacag   1500
   cgttagtgaa attacgcgca tcgctcgtat ggcaggcttt ttggctctgc aggcccaacc   1560
   tccgctacct gtttggagct tggacaaggc caatgtgctt gcttccagcc gtttgtggcg   1620
   cgaaaccgtc accaaggtgt tcaaagagga attccctcag ctcaaattgg agcatcagct   1680
   cattgattcg gcggccatga ttttggtgaa gaaccctcga cagctcaatg gtgtcgttat   1740
   caccaccaac atgttcggag acattttcag cgacgaggcg agtgttattc ctggctctct   1800
   gggtctgcta ccctcagctt cgctcagtgg actgcctgac acaaactctg cctttggtct   1860
   gtacgagcct tgtcacggct ctgctcccga cctcgctgct aacaaggcaa atccagtcgc   1920
   taccattctc agcgcagcaa tgatgcttcg tctttcacta ggtcttcctg aagctgctga   1980
   tgctgttgag aaagctgttt ccaacgtttt gaactcagtc gcggccacgg cagacattgg   2040
   tggaacagcc tccaccacag aggtaggcga tgcaattgcc gcagagacgt tgaagcttct   2100
   caaatagtct gctataaatt gacggagttt cgtacagtgc gctcgtacag tgcgctgcca   2160
   aatacaattt agtgtagcca gattggatgg ttgaattgct cttcacggtt gcacgctatt   2220
   ggcaaaaaag agagagccgc tctgaactgg ttcatccgca gctgaccttc gaaactcttt   2280
   aatatttaat aatattgcag caaaatctat agcttatgcc acatctatac ggaagaggta   2340
   ttcaacatta gagcttgtgt cgcccattct ctacacgagc ccacgcatca gcagtgaggg   2400
   gcttgtagct cgtgccctct aaccagtaga ttgtttgtcc tgctggggcg ggaatctgct   2460
   ggtttcggaa ttctttcttc tgaactttgt tgttgccggt gatggtgacg gtgtcgacga   2520
   acttaatgaa tatcggcacg gcatagcgtg gcagcctttc caaaagatgc ttgccgagtt   2580
   tatccatatc cagctgtttt ctaggattgt tgagcttgat cacagcaaat ccggcacgac   2640
   cctcatgctt gggaacctgc acacctacac agacacacag atcgactcca ccgaagtcca   2700
   caactgcttc ctcgacttcg tttgtgctaa cgttctcgct cttccatcga aacgtatccc   2760
   cgagtcgatc aacaaagtag acgctatgat ctttatcagc cctcagaagg tctccgctgc   2820
   gcacccaggc atctcccttc ttgaaaacat caaacacaag cttctcatcc gtggctgatt   2880
   ggttgccgac atagccctgg aaatcgagtt tgatattctt cgggtcgagt ttgaaaagga   2940
   attcacccgg ctcgtccgag tgtgtctcac ggcacaggcc ggttttggga tcgcgccata   3000
   aatcctgcgt gtcaacatca atcgcggcga tgttccacct ggtacgatgc agcacgcggg   3060
   tggccacagt accataatgg ccacatgcac caacaccata tgcacct                 3107
<210> 38
   <211> 45
   <212> DNA
   <213> Artificial
<220>
   <223> Primer
<400> 38
   atatatatac atatgttaat caaagacatt attctaactc caatg 45
<210> 39
   <211> 42
   <212> DNA
   <213> Artificial
<220>
   <223> Primer
<400> 39
   atatatggcc ggccaactta agaaaaccgc acaaccacac cg 42
<210> 40
   <211> 39
   <212> DNA
   <213> Artificial
<220>
   <223> Primer
<400> 40
   atatatatac atatgagccc ttcatcacac aaacccctg 39
<210> 41
   <211> 39
   <212> DNA
   <213> Artificial
<220>
   <223> Primer
<400> 41
   atatatggcc ggccattcta agaactcacc gctaaggcc 39
<210> 42
   <211> 38
   <212> DNA
   <213> Artificial
<220>
   <223> Primer
<400> 42
   atatatatac atatggttgt aaactcctcg aaggaccc 38
<210> 43
   <211> 42
   <212> DNA
   <213> Artificial
<220>
   <223> Primer
<400> 43
   atatatggcc ggcctaccta gaccttctgg ttagcggtat tg 42
<210> 44
   <211> 40
   <212> DNA
   <213> Artificial
<220>
   <223> Primer
<400> 44
   atatatatac atatggtgga tgatatacag gtagagaagc 40
<210> 45
   <211> 42
   <212> DNA
   <213> Artificial
<220>
   <223> Primer
<400> 45
   atatatggcc ggccacgtca aatctctccg agaccttgca ag 42
<210> 46
   <211> 40
   <212> DNA
   <213> Artificial
<220>
   <223> Primer
<400> 46
   atatatatac atatggccat cgagaaacca gtgatagttg 40
<210> 47
   <211> 45
   <212> DNA
   <213> Artificial
<220>
   <223> Primer
<400> 47
   atatatggcc ggccaggtta agaagctaat tcactaattg ccgac 45
<210> 48
   <211> 23
   <212> DNA
   <213> Artificial
<220>
   <223> Primer
<400> 48
   ggacctgcgc cctaaaatgg gac 23
<210> 49
   <211> 30
   <212> DNA
   <213> Artificial
<220>
   <223> Primer
<400> 49
   atcctagaaa acagctggat atggataaac 30
<210> 50
   <211> 23
   <212> DNA
   <213> Artificial
<220>
   <223> Primer
<400> 50
   gtgcccgacc accatgagct gtc 23
<210> 51
   <211> 23
   <212> DNA
   <213> Artificial
<220>
   <223> Primer
<400> 51
   cccaagcatg agggtcgtgc cgg 23
<210> 52
   <211> 1572
   <212> DNA
   <213> Candida bombicola
<220>
   <221> CDS
   <222> (1)..(1572)
<400> 52
   atg att ctt tat gct gtg ctg ggc gca ttc gcc gcc ttc ttg ctt tac        48
   Met Ile Leu Tyr Ala Val Leu Gly Ala Phe Ala Ala Phe Leu Leu Tyr
   1               5                   10                  15
   atg gat gta ctt tac cct ttc gtg att tac cct ctg aga gcg cga tgg        96
   Met Asp Val Leu Tyr Pro Phe Val Ile Tyr Pro Leu Arg Ala Arg Trp
               20                  25                  30
   cac aaa tgt ggt tac atc cct aga gat ttg agc tgg cca ttg ggg att       144
   His Lys Cys Gly Tyr Ile Pro Arg Asp Leu Ser Trp Pro Leu Gly Ile
           35                  40                  45
   cca ctc acc ctg gta gtt ctc tcg aag ttg agg aaa gat atg ctg ctg       192
   Pro Leu Thr Leu Val Val Leu Ser Lys Leu Arg Lys Asp Met Leu Leu
       50                  55                  60
   caa ttc atg gca gcg caa gac ctt agt cgc cct tac aag aca tcc tta       240
   Gln Phe Met Ala Ala Gln Asp Leu Ser Arg Pro Tyr Lys Thr Ser Leu
   65                  70                  75                  80
   cgt caa ttt ctg ggt aaa tgg gta atc gcc act aga gat cct gag aac       288
   Arg Gln Phe Leu Gly Lys Trp Val Ile Ala Thr Arg Asp Pro Glu Asn
                   85                  90                  95
   atc aag gct gtt cta tcc acc aag ttc aat gac ttc tcg ctg aaa gaa      336
   Ile Lys Ala Val Leu Ser Thr Lys Phe Asn Asp Phe Ser Leu Lys Glu
               100                 105                 110
   aga ggg aat agg atg agg cat gta atc ggt gat gga att ttt acc caa      384
   Arg Gly Asn Arg Met Arg His Val Ile Gly Asp Gly Ile Phe Thr Gln
           115                 120                 125
   gat ggc gca cca tgg aag cac tcg cga gat atg ctc agg cct cag ttc      432
   Asp Gly Ala Pro Trp Lys His Ser Arg Asp Met Leu Arg Pro Gln Phe
       130                 135                 140
   acc aag gat caa atc agc cga gtg gaa ttg ttg agc cac cac atc gac      480
   Thr Lys Asp Gln Ile Ser Arg Val Glu Leu Leu Ser His His Ile Asp
   145                 150                 155                 160
   gtt ttg att cgt gaa atc agg aag tcg gga ggt aac gtc gag ttg caa      528
   Val Leu Ile Arg Glu Ile Arg Lys Ser Gly Gly Asn Val Glu Leu Gln
                   165                 170                 175
   cgt tta ttc cac ctc atg act atg gac acc gcc act cac ttt cta ttc      576
   Arg Leu Phe His Leu Met Thr Met Asp Thr Ala Thr His Phe Leu Phe
               180                 185                 190
   ggc gag tcc gtt ggc tcg ttg gag gtc agt ggc gaa agc aag ggc att      624
   Gly Glu Ser Val Gly Ser Leu Glu Val Ser Gly Glu Ser Lys Gly Ile
           195                 200                 205
   gag atc acc gac cca aag act gga gag att gtg aac acc gtt gat ttt      672
   Glu Ile Thr Asp Pro Lys Thr Gly Glu Ile Val Asn Thr Val Asp Phe
       210                 215                 220
   gtt gag tct tat act ttt gca aac aag ttt gct ctc aag aag att atc      720
   Val Glu Ser Tyr Thr Phe Ala Asn Lys Phe Ala Leu Lys Lys Ile Ile
   225                 230                 235                 240
   ctc aac gac ttg gag ttt tta gcc gac ttg acg gag ccc tcg tat aag      768
   Leu Asn Asp Leu Glu Phe Leu Ala Asp Leu Thr Glu Pro Ser Tyr Lys
                   245                 250                 255
   tgg cat ctg cgc cgt gtc cac aca gtc atg gat cac tac gtt cag ctg      816
   Trp His Leu Arg Arg Val His Thr Val Met Asp His Tyr Val Gln Leu
               260                 265                 270
   gct ttg aag gct act gag aag tat gat cct gat gat gat agc gag aag      864
   Ala Leu Lys Ala Thr Glu Lys Tyr Asp Pro Asp Asp Asp Ser Glu Lys
           275                 280                 285
   gga gaa tac tac ttt agc cat gag ctg gcg aaa ctc acg aga gac ccc      912
   Gly Glu Tyr Tyr Phe Ser His Glu Leu Ala Lys Leu Thr Arg Asp Pro
       290                 295                 300
   ttg tcg ttg aga gat cag ctt ttc aat att ctc att gct ggc cgc gac      960
   Leu Ser Leu Arg Asp Gln Leu Phe Asn Ile Leu Ile Ala Gly Arg Asp
   305                 310                 315                 320
   act acc gca gca act ttg tcc tat gcc ttc cac tat cta acg aag aat     1008
   Thr Thr Ala Ala Thr Leu Ser Tyr Ala Phe His Tyr Leu Thr Lys Asn
                   325                 330                 335
   ccc gct atc tac gcc aag gtc cgc gaa gat gtg ctc acg gtc ttc cct     1056
   Pro Ala Ile Tyr Ala Lys Val Arg Glu Asp Val Leu Thr Val Phe Pro
               340                 345                 350
   aat gga gac gca tca ttg gcg act tac gag gac ttg cga aag gct aag      1104
   Asn Gly Asp Ala Ser Leu Ala Thr Tyr Glu Asp Leu Arg Lys Ala Lys
           355                 360                 365
   tat ctc caa atg gtg atc aag gag gta ttg cgt ctt gcg cct gcg gtt      1152
   Tyr Leu Gln Met Val Ile Lys Glu Val Leu Arg Leu Ala Pro Ala Val
       370                 375                 380
   ccc ttg aac acg cgt gcc gcg gtt cgt gac aca tat ctg cca cgg ggc      1200
   Pro Leu Asn Thr Arg Ala Ala Val Arg Asp Thr Tyr Leu Pro Arg Gly
   385                 390                 395                 400
   gga ggc cca gcc gga aac ctg ccc gtt ttt gtt ccc aag ggc act gct      1248
   Gly Gly Pro Ala Gly Asn Leu Pro Val Phe Val Pro Lys Gly Thr Ala
                   405                 410                 415
   gtc aac tac cct aca tat att ttg cac cgc gat cca gat atc tat ggt      1296
   Val Asn Tyr Pro Thr Tyr Ile Leu His Arg Asp Pro Asp Ile Tyr Gly
               420                 425                 430
   gcc gac gcg tac gag ttc aac ccc gag aga tgg agg cct gag aat aag      1344
   Ala Asp Ala Tyr Glu Phe Asn Pro Glu Arg Trp Arg Pro Glu Asn Lys
           435                 440                 445
   ctt ccg aat agc cca atg tac tct tgg gga tac att ccc ttc aat ggt      1392
   Leu Pro Asn Ser Pro Met Tyr Ser Trp Gly Tyr Ile Pro Phe Asn Gly
       450                 455                 460
   ggc cct cgc atc tgc att gga cag cag ttc gcc ttg act gag atc gct      1440
   Gly Pro Arg Ile Cys Ile Gly Gln Gln Phe Ala Leu Thr Glu Ile Ala
   465                 470                 475                 480
   ttg acg atg atc aag ctg gtt ctg gaa ttt gag agg ctg gag cct gcc      1488
   Leu Thr Met Ile Lys Leu Val Leu Glu Phe Glu Arg Leu Glu Pro Ala
                   485                 490                 495
   gac gac ttt gag ccc aat ctt caa gac aag tcc tct tta act gtc atg      1536
   Asp Asp Phe Glu Pro Asn Leu Gln Asp Lys Ser Ser Leu Thr Val Met
               500                 505                 510
   gtc gga ggg tcg ggc gtc cga gtg aaa ctg agt taa                      1572
   Val Gly Gly Ser Gly Val Arg Val Lys Leu Ser
           515                 520
<210> 53
   <211> 523
   <212> PRT
   <213> Candida bombicola
<400> 53
   Met Ile Leu Tyr Ala Val Leu Gly Ala Phe Ala Ala Phe Leu Leu Tyr
   1               5                   10                  15
   Met Asp Val Leu Tyr Pro Phe Val Ile Tyr Pro Leu Arg Ala Arg Trp
               20                  25                  30
   His Lys Cys Gly Tyr Ile Pro Arg Asp Leu Ser Trp Pro Leu Gly Ile
           35                  40                  45
   Pro Leu Thr Leu Val Val Leu Ser Lys Leu Arg Lys Asp Met Leu Leu
       50                  55                  60
   Gln Phe Met Ala Ala Gln Asp Leu Ser Arg Pro Tyr Lys Thr Ser Leu
   65                  70                  75                  80
   Arg Gln Phe Leu Gly Lys Trp Val Ile Ala Thr Arg Asp Pro Glu Asn
                   85                  90                  95
   Ile Lys Ala Val Leu Ser Thr Lys Phe Asn Asp Phe Ser Leu Lys Glu
               100                 105                 110
   Arg Gly Asn Arg Met Arg His Val Ile Gly Asp Gly Ile Phe Thr Gln
           115                 120                 125
   Asp Gly Ala Pro Trp Lys His Ser Arg Asp Met Leu Arg Pro Gln Phe
       130                 135                 140
   Thr Lys Asp Gln Ile Ser Arg Val Glu Leu Leu Ser His His Ile Asp
   145                 150                 155                 160
   Val Leu Ile Arg Glu Ile Arg Lys Ser Gly Gly Asn Val Glu Leu Gln
                   165                 170                 175
   Arg Leu Phe His Leu Met Thr Met Asp Thr Ala Thr His Phe Leu Phe
               180                 185                 190
   Gly Glu Ser Val Gly Ser Leu Glu Val Ser Gly Glu Ser Lys Gly Ile
           195                 200                 205
   Glu Ile Thr Asp Pro Lys Thr Gly Glu Ile Val Asn Thr Val Asp Phe
       210                 215                 220
   Val Glu Ser Tyr Thr Phe Ala Asn Lys Phe Ala Leu Lys Lys Ile Ile
   225                 230                 235                 240
   Leu Asn Asp Leu Glu Phe Leu Ala Asp Leu Thr Glu Pro Ser Tyr Lys
                   245                 250                 255
   Trp His Leu Arg Arg Val His Thr Val Met Asp His Tyr Val Gln Leu
               260                 265                 270
   Ala Leu Lys Ala Thr Glu Lys Tyr Asp Pro Asp Asp Asp Ser Glu Lys
           275                 280                 285
   Gly Glu Tyr Tyr Phe Ser His Glu Leu Ala Lys Leu Thr Arg Asp Pro
       290                 295                 300
   Leu Ser Leu Arg Asp Gln Leu Phe Asn Ile Leu Ile Ala Gly Arg Asp
   305                 310                 315                 320
   Thr Thr Ala Ala Thr Leu Ser Tyr Ala Phe His Tyr Leu Thr Lys Asn
                   325                 330                 335
   Pro Ala Ile Tyr Ala Lys Val Arg Glu Asp Val Leu Thr Val Phe Pro
               340                 345                 350
   Asn Gly Asp Ala Ser Leu Ala Thr Tyr Glu Asp Leu Arg Lys Ala Lys
           355                 360                 365
   Tyr Leu Gln Met Val Ile Lys Glu Val Leu Arg Leu Ala Pro Ala Val
       370                 375                 380
   Pro Leu Asn Thr Arg Ala Ala Val Arg Asp Thr Tyr Leu Pro Arg Gly
   385                 390                 395                 400
   Gly Gly Pro Ala Gly Asn Leu Pro Val Phe Val Pro Lys Gly Thr Ala
                   405                 410                 415
   Val Asn Tyr Pro Thr Tyr Ile Leu His Arg Asp Pro Asp Ile Tyr Gly
               420                 425                 430
   Ala Asp Ala Tyr Glu Phe Asn Pro Glu Arg Trp Arg Pro Glu Asn Lys
           435                 440                 445
   Leu Pro Asn Ser Pro Met Tyr Ser Trp Gly Tyr Ile Pro Phe Asn Gly
       450                 455                 460
   Gly Pro Arg Ile Cys Ile Gly Gln Gln Phe Ala Leu Thr Glu Ile Ala
   465                 470                 475                 480
   Leu Thr Met Ile Lys Leu Val Leu Glu Phe Glu Arg Leu Glu Pro Ala
                   485                 490                 495
   Asp Asp Phe Glu Pro Asn Leu Gln Asp Lys Ser Ser Leu Thr Val Met
               500                 505                 510
   Val Gly Gly Ser Gly Val Arg Val Lys Leu Ser
           515                 520
<210> 54
   <211> 1671
   <212> DNA
   <213> Candida bombicola
<220>
   <221> CDS
   <222> (1)..(1671)
<400> 54
   atg agg ccc ctg ttg cgg gaa caa gac aca tca cac cca gag cta ttg       48
   Met Arg Pro Leu Leu Arg Glu Gln Asp Thr Ser His Pro Glu Leu Leu
   1               5                   10                  15
   ttg gca agc aat act att ttt aac ccc ctt tcc aag agt gtc caa act       96
   Leu Ala Ser Asn Thr Ile Phe Asn Pro Leu Ser Lys Ser Val Gln Thr
               20                  25                  30
   gtt caa tac ggc ctc atg aac att aat ttc tct gac gtg ctc gtg cta      144
   Val Gln Tyr Gly Leu Met Asn Ile Asn Phe Ser Asp Val Leu Val Leu
           35                  40                  45
   gga ggc atc agc gtg agc ttt ttg ctc gcc tac cag gcg att tac ttt      192
   Gly Gly Ile Ser Val Ser Phe Leu Leu Ala Tyr Gln Ala Ile Tyr Phe
       50                  55                  60
   tat ttc att tac tcg cca cga gcc aaa aag ctc ggt tgc gct ctt cca      240
   Tyr Phe Ile Tyr Ser Pro Arg Ala Lys Lys Leu Gly Cys Ala Leu Pro
   65                  70                  75                  80
   ccg gtc ttc ttc tct ttc cca ctc gga ata ccg gag gtc ata cgt ctt      288
   Pro Val Phe Phe Ser Phe Pro Leu Gly Ile Pro Glu Val Ile Arg Leu
                   85                  90                  95
   gtg aac gcc tgg ttc aac gat gat ctc ctt gag tat ttc acc ttc aaa      336
   Val Asn Ala Trp Phe Asn Asp Asp Leu Leu Glu Tyr Phe Thr Phe Lys
               100                 105                 110
   ttc gag gag ttc cag cgc aaa acc gga ttc caa tca gtc gct ggg caa      384
   Phe Glu Glu Phe Gln Arg Lys Thr Gly Phe Gln Ser Val Ala Gly Gln
           115                 120                 125
   cta tgg att ggg act att gag ccc gag aac atc aag act atg ctc gct      432
   Leu Trp Ile Gly Thr Ile Glu Pro Glu Asn Ile Lys Thr Met Leu Ala
       130                 135                 140
   act tca ttt aaa gac tac tcc cta ggc ttc cgt tac gag gcc atg tac      480
   Thr Ser Phe Lys Asp Tyr Ser Leu Gly Phe Arg Tyr Glu Ala Met Tyr
   145                 150                 155                 160
   ggc ctt ctc gga aat ggc att ttc act ctc agt ggt gag ggc tgg aag      528
   Gly Leu Leu Gly Asn Gly Ile Phe Thr Leu Ser Gly Glu Gly Trp Lys
                   165                 170                 175
   cac agc cgc gct ttg ttg cgt ccg caa ttt agt cgt gag caa gtc tct      576
   His Ser Arg Ala Leu Leu Arg Pro Gln Phe Ser Arg Glu Gln Val Ser
               180                 185                 190
   cac ctt gaa tca atg cgc aca cac atc aat atg ttg atc aac aac cac      624
   His Leu Glu Ser Met Arg Thr His Ile Asn Met Leu Ile Asn Asn His
           195                 200                 205
   ttc aag ggt ggc aaa gtc gtc gat gct cag gtt ttg ttc cac aat cta      672
   Phe Lys Gly Gly Lys Val Val Asp Ala Gln Val Leu Phe His Asn Leu
       210                 215                 220
   acc att gat act gct acc gaa ttc cta ttc gga gag agc acc aac act      720
   Thr Ile Asp Thr Ala Thr Glu Phe Leu Phe Gly Glu Ser Thr Asn Thr
   225                 230                 235                 240
   ctt gac cct gct ctt gct cag cat gga ttc cct gga cct aag ggt ctt      768
   Leu Asp Pro Ala Leu Ala Gln His Gly Phe Pro Gly Pro Lys Gly Leu
                   245                 250                 255
   gta acc ggt gag cag ttt gct gag gct ttt acc tct gct ctc gaa ttg      816
   Val Thr Gly Glu Gln Phe Ala Glu Ala Phe Thr Ser Ala Leu Glu Leu
               260                 265                 270
   ctt tct gtg cga gtt atg gcc ggc gcc gca tgg ttc ctc gtt tgg acc      864
   Leu Ser Val Arg Val Met Ala Gly Ala Ala Trp Phe Leu Val Trp Thr
           275                 280                 285
   ccc aaa ttc tgg cgc tca tgc aaa gtc tgc cac aac ttc att gat tac      912
   Pro Lys Phe Trp Arg Ser Cys Lys Val Cys His Asn Phe Ile Asp Tyr
       290                 295                 300
   ttc gtt ttc aag gct ctg gcc act cct atg gag aag gac cag gaa gct      960
   Phe Val Phe Lys Ala Leu Ala Thr Pro Met Glu Lys Asp Gln Glu Ala
   305                 310                 315                 320
   gat cgc tac gtc ttt att cga gaa ctc aca aag gag acc tct gac cca     1008
   Asp Arg Tyr Val Phe Ile Arg Glu Leu Thr Lys Glu Thr Ser Asp Pro
                   325                 330                 335
   cgg gtc atc cgc gac cag gcc ctc aac atc ctc ttg gct ggt cgt gat     1056
   Arg Val Ile Arg Asp Gln Ala Leu Asn Ile Leu Leu Ala Gly Arg Asp
               340                 345                 350
   acc act gcg gca ctt ctc agc ttc acc acc tac tac ctt ggt gcc tac     1104
   Thr Thr Ala Ala Leu Leu Ser Phe Thr Thr Tyr Tyr Leu Gly Ala Tyr
           355                 360                 365
   cct gag gtc tac gat gag ctt cgc gag gct gtt att gcg gac ttc ggc     1152
   Pro Glu Val Tyr Asp Glu Leu Arg Glu Ala Val Ile Ala Asp Phe Gly
       370                 375                 380
   aag gaa gat gct gag ccc cct acg ttt gag cag ctt aag cag tgc aag     1200
   Lys Glu Asp Ala Glu Pro Pro Thr Phe Glu Gln Leu Lys Gln Cys Lys
   385                 390                 395                 400
   gtg cta cag aac gtc att cgg gaa gtt ttg cga ttg cac ccg aat gtg     1248
   Val Leu Gln Asn Val Ile Arg Glu Val Leu Arg Leu His Pro Asn Val
                   405                 410                 415
   ccc ctc aac ttc cgc gag gcc att acc gat act aag ttc ccc aca gga     1296
   Pro Leu Asn Phe Arg Glu Ala Ile Thr Asp Thr Lys Phe Pro Thr Gly
               420                 425                 430
   ggc ggc ccg aat gga gac cag ccc gtt ttc gtt ccc aag gga cag aaa     1344
   Gly Gly Pro Asn Gly Asp Gln Pro Val Phe Val Pro Lys Gly Gln Lys
           435                 440                 445
   gtg ttt tac gcc acc tac gtc atg cag cga aat gag ggt ctc tgg ggt     1392
   Val Phe Tyr Ala Thr Tyr Val Met Gln Arg Asn Glu Gly Leu Trp Gly
       450                 455                 460
   cct gac tcc aca aca ttc cgc cct gac cgc tgg aac gag tca aga gag     1440
   Pro Asp Ser Thr Thr Phe Arg Pro Asp Arg Trp Asn Glu Ser Arg Glu
   465                 470                 475                 480
   gcc atc gca tcc gga tgg gac tac att cct ttc aac ggc ggc cct cgt     1488
   Ala Ile Ala Ser Gly Trp Asp Tyr Ile Pro Phe Asn Gly Gly Pro Arg
                   485                 490                 495
   att tgc ctg ggt cag cag ttc gct ctc aca gag gcg agc tac acg ctc     1536
   Ile Cys Leu Gly Gln Gln Phe Ala Leu Thr Glu Ala Ser Tyr Thr Leu
               500                 505                 510
   gtg cgt atc tgc caa gag ttc tcc agg att gag gtt ctc cac cct gat     1584
   Val Arg Ile Cys Gln Glu Phe Ser Arg Ile Glu Val Leu His Pro Asp
           515                 520                 525
   gtt att acc tcc agg aac gtg atg aaa cag cgc atg cgt ttg acc aac      1632
   Val Ile Thr Ser Arg Asn Val Met Lys Gln Arg Met Arg Leu Thr Asn
       530                 535                 540
   tct tcc agc ggc ggc gtc ata gcg aag ttc att cgc tag                  1671
   Ser Ser Ser Gly Gly Val Ile Ala Lys Phe Ile Arg
   545                 550                 555
<210> 55
   <211> 556
   <212> PRT
   <213> Candida bombicola
<400> 55
   Met Arg Pro Leu Leu Arg Glu Gln Asp Thr Ser His Pro Glu Leu Leu
   1               5                   10                  15
   Leu Ala Ser Asn Thr Ile Phe Asn Pro Leu Ser Lys Ser Val Gln Thr
               20                  25                  30
   Val Gln Tyr Gly Leu Met Asn Ile Asn Phe Ser Asp Val Leu Val Leu
           35                  40                  45
   Gly Gly Ile Ser Val Ser Phe Leu Leu Ala Tyr Gln Ala Ile Tyr Phe
       50                  55                  60
   Tyr Phe Ile Tyr Ser Pro Arg Ala Lys Lys Leu Gly Cys Ala Leu Pro
   65                  70                  75                  80
   Pro Val Phe Phe Ser Phe Pro Leu Gly Ile Pro Glu Val Ile Arg Leu
                   85                  90                  95
   Val Asn Ala Trp Phe Asn Asp Asp Leu Leu Glu Tyr Phe Thr Phe Lys
               100                 105                 110
   Phe Glu Glu Phe Gln Arg Lys Thr Gly Phe Gln Ser Val Ala Gly Gln
           115                 120                 125
   Leu Trp Ile Gly Thr Ile Glu Pro Glu Asn Ile Lys Thr Met Leu Ala
       130                 135                 140
   Thr Ser Phe Lys Asp Tyr Ser Leu Gly Phe Arg Tyr Glu Ala Met Tyr
   145                 150                 155                 160
   Gly Leu Leu Gly Asn Gly Ile Phe Thr Leu Ser Gly Glu Gly Trp Lys
                   165                 170                 175
   His Ser Arg Ala Leu Leu Arg Pro Gln Phe Ser Arg Glu Gln Val Ser
               180                 185                 190
   His Leu Glu Ser Met Arg Thr His Ile Asn Met Leu Ile Asn Asn His
           195                 200                 205
   Phe Lys Gly Gly Lys Val Val Asp Ala Gln Val Leu Phe His Asn Leu
       210                 215                 220
   Thr Ile Asp Thr Ala Thr Glu Phe Leu Phe Gly Glu Ser Thr Asn Thr
   225                 230                 235                 240
   Leu Asp Pro Ala Leu Ala Gln His Gly Phe Pro Gly Pro Lys Gly Leu
                   245                 250                 255
   Val Thr Gly Glu Gln Phe Ala Glu Ala Phe Thr Ser Ala Leu Glu Leu
               260                 265                 270
   Leu Ser Val Arg Val Met Ala Gly Ala Ala Trp Phe Leu Val Trp Thr
           275                 280                 285
   Pro Lys Phe Trp Arg Ser Cys Lys Val Cys His Asn Phe Ile Asp Tyr
       290                 295                 300
   Phe Val Phe Lys Ala Leu Ala Thr Pro Met Glu Lys Asp Gln Glu Ala
   305                 310                 315                 320
   Asp Arg Tyr Val Phe Ile Arg Glu Leu Thr Lys Glu Thr Ser Asp Pro
                   325                 330                 335
   Arg Val Ile Arg Asp Gln Ala Leu Asn Ile Leu Leu Ala Gly Arg Asp
               340                 345                 350
   Thr Thr Ala Ala Leu Leu Ser Phe Thr Thr Tyr Tyr Leu Gly Ala Tyr
           355                 360                 365
   Pro Glu Val Tyr Asp Glu Leu Arg Glu Ala Val Ile Ala Asp Phe Gly
       370                 375                 380
   Lys Glu Asp Ala Glu Pro Pro Thr Phe Glu Gln Leu Lys Gln Cys Lys
   385                 390                 395                 400
   Val Leu Gln Asn Val Ile Arg Glu Val Leu Arg Leu His Pro Asn Val
                   405                 410                 415
   Pro Leu Asn Phe Arg Glu Ala Ile Thr Asp Thr Lys Phe Pro Thr Gly
               420                 425                 430
   Gly Gly Pro Asn Gly Asp Gln Pro Val Phe Val Pro Lys Gly Gln Lys
           435                 440                 445
   Val Phe Tyr Ala Thr Tyr Val Met Gln Arg Asn Glu Gly Leu Trp Gly
       450                 455                 460
   Pro Asp Ser Thr Thr Phe Arg Pro Asp Arg Trp Asn Glu Ser Arg Glu
   465                 470                 475                 480
   Ala Ile Ala Ser Gly Trp Asp Tyr Ile Pro Phe Asn Gly Gly Pro Arg
                   485                 490                 495
   Ile Cys Leu Gly Gln Gln Phe Ala Leu Thr Glu Ala Ser Tyr Thr Leu
               500                 505                 510
   Val Arg Ile Cys Gln Glu Phe Ser Arg Ile Glu Val Leu His Pro Asp
           515                 520                 525
   Val Ile Thr Ser Arg Asn Val Met Lys Gln Arg Met Arg Leu Thr Asn
       530                 535                 540
   Ser Ser Ser Gly Gly Val Ile Ala Lys Phe Ile Arg
   545                 550                 555
<210> 56
   <211> 1560
   <212> DNA
   <213> Candida bombicola
<220>
   <221> CDS
   <222> (1)..(1560)
<400> 56
   atg att att gat ctt tca gac gcg ctg ata ata gga ggc atc gcc ctg      48
   Met Ile Ile Asp Leu Ser Asp Ala Leu Ile Ile Gly Gly Ile Ala Leu
   1               5                   10                  15
   tgc ttc ttg ctc tcc tac cag gcg atc tac ttt tac ttt att tac tcg      96
   Cys Phe Leu Leu Ser Tyr Gln Ala Ile Tyr Phe Tyr Phe Ile Tyr Ser
               20                  25                  30
   cca cgg gcc aag aag ctt gga tgc gct cct cct ctc att gtg cac gct     144
   Pro Arg Ala Lys Lys Leu Gly Cys Ala Pro Pro Leu Ile Val His Ala
           35                  40                  45
   ttc cca ctg ggt ttg ccg aca att ttc gga ctt ata aga gct tgg cgc     192
   Phe Pro Leu Gly Leu Pro Thr Ile Phe Gly Leu Ile Arg Ala Trp Arg
       50                  55                  60
   aac gac gat ctt ctc cag tac ttg agc gac aac ttc gct aga atc agg     240
   Asn Asp Asp Leu Leu Gln Tyr Leu Ser Asp Asn Phe Ala Arg Ile Arg
   65                  70                  75                  80
   acc aga acc gga atg caa gta atg gcc ggt cag ctg tgg ctc aac acc     288
   Thr Arg Thr Gly Met Gln Val Met Ala Gly Gln Leu Trp Leu Asn Thr
                   85                  90                  95
   att gag cca gaa aac atc aag gcc atg ctt gcc act tcg ttc aag gat     336
   Ile Glu Pro Glu Asn Ile Lys Ala Met Leu Ala Thr Ser Phe Lys Asp
               100                 105                 110
   ttc tcg ctt ggg ttc cgc tat gaa gtc atg cat ggc ctc ctc gga gat      384
   Phe Ser Leu Gly Phe Arg Tyr Glu Val Met His Gly Leu Leu Gly Asp
           115                 120                 125
   ggt atc ttc act ctc agt ggt gag ggc tgg aaa cac agc cgt gcc ttg      432
   Gly Ile Phe Thr Leu Ser Gly Glu Gly Trp Lys His Ser Arg Ala Leu
       130                 135                 140
   cta cgt cca cag ttc agc cgt gag caa gtc tct cac ttg gac tca atg      480
   Leu Arg Pro Gln Phe Ser Arg Glu Gln Val Ser His Leu Asp Ser Met
   145                 150                 155                 160
   cgc aca cac atc aat ttg atg atc aac aac cac ttc aaa ggt ggc cag      528
   Arg Thr His Ile Asn Leu Met Ile Asn Asn His Phe Lys Gly Gly Gln
                   165                 170                 175
   gtc gtc gac gct cag gtt cta tac cat aac ctg aca atc gac act gcc      576
   Val Val Asp Ala Gln Val Leu Tyr His Asn Leu Thr Ile Asp Thr Ala
               180                 185                 190
   act gaa ttc ctg ttc ggt gag agc acc aac act ctt gac cct gtt ctt      624
   Thr Glu Phe Leu Phe Gly Glu Ser Thr Asn Thr Leu Asp Pro Val Leu
           195                 200                 205
   gca cag cag gga cta ccg ggt cct agg ggc gtt gtt act ggt gag cag      672
   Ala Gln Gln Gly Leu Pro Gly Pro Arg Gly Val Val Thr Gly Glu Gln
       210                 215                 220
   ttc gct aac gct ttc acc tac gct caa gag ttg ctc agt att cga gtc      720
   Phe Ala Asn Ala Phe Thr Tyr Ala Gln Glu Leu Leu Ser Ile Arg Val
   225                 230                 235                 240
   atg gcc ggc tca gca tgg ttc ctc gtc tgg act cct aag ttc agg cgc      768
   Met Ala Gly Ser Ala Trp Phe Leu Val Trp Thr Pro Lys Phe Arg Arg
                   245                 250                 255
   tcg tgc aag gtg tgc cac aac ttt att gac tac ttc gtc ttt aag gct      816
   Ser Cys Lys Val Cys His Asn Phe Ile Asp Tyr Phe Val Phe Lys Ala
               260                 265                 270
   ctg gcc act cct atg gag aaa gac cag gag gct gat cgc tat gta ttc      864
   Leu Ala Thr Pro Met Glu Lys Asp Gln Glu Ala Asp Arg Tyr Val Phe
           275                 280                 285
   atc cga gaa ctc act aag gag act tct gac cca aag gtt ata cgt gac      912
   Ile Arg Glu Leu Thr Lys Glu Thr Ser Asp Pro Lys Val Ile Arg Asp
       290                 295                 300
   cag gct ctc aac atc ctt tta gct ggc cgc gat acc act gca gca ctc      960
   Gln Ala Leu Asn Ile Leu Leu Ala Gly Arg Asp Thr Thr Ala Ala Leu
   305                 310                 315                 320
   ctc agc ttc acc act tac tac ctt ggc gca tat cct gag gtc tac gac     1008
   Leu Ser Phe Thr Thr Tyr Tyr Leu Gly Ala Tyr Pro Glu Val Tyr Asp
                   325                 330                 335
   gag ctt cgc gag gca gtt ctt gca gac ttc ggc cct gcc gat tct gag     1056
   Glu Leu Arg Glu Ala Val Leu Ala Asp Phe Gly Pro Ala Asp Ser Glu
               340                 345                 350
   ccc cct acc ttt gag agg ctc aag cag tgc aag gtg ttg cag aat gtc     1104
   Pro Pro Thr Phe Glu Arg Leu Lys Gln Cys Lys Val Leu Gln Asn Val
           355                 360                 365
   atc cgc gag gtt ctg cga ttg cac ccg aat gtg ccc ctc aac ttc cgc      1152
   Ile Arg Glu Val Leu Arg Leu His Pro Asn Val Pro Leu Asn Phe Arg
       370                 375                 380
   cag gcc atc gtt gat act aag ttc cct act ggt ggt ggc ccg aat aga      1200
   Gln Ala Ile Val Asp Thr Lys Phe Pro Thr Gly Gly Gly Pro Asn Arg
   385                 390                 395                 400
   gac cag ccc atc ttt gtt cca aaa gga cag aag gtg ttc tac tcc acg      1248
   Asp Gln Pro Ile Phe Val Pro Lys Gly Gln Lys Val Phe Tyr Ser Thr
                   405                 410                 415
   tac gtc atg cag cga agc aag gac atc tgg ggc gct gac tcc aca tcg      1296
   Tyr Val Met Gln Arg Ser Lys Asp Ile Trp Gly Ala Asp Ser Thr Ser
               420                 425                 430
   ttc cga cca gaa cgc tgg aac gag ccc aga gaa gct ctt gca tca ggt      1344
   Phe Arg Pro Glu Arg Trp Asn Glu Pro Arg Glu Ala Leu Ala Ser Gly
           435                 440                 445
   tgg gat tac att cct ttc aat ggt ggc cct cgc att tgt atc ggt cag      1392
   Trp Asp Tyr Ile Pro Phe Asn Gly Gly Pro Arg Ile Cys Ile Gly Gln
       450                 455                 460
   cag ttc gct ctc act gag gct agc tac acg ctt gtc cgt att tgc cag      1440
   Gln Phe Ala Leu Thr Glu Ala Ser Tyr Thr Leu Val Arg Ile Cys Gln
   465                 470                 475                 480
   gag ttt acc aga att gag gtt ctt cat ccc gat gtc att act tct agg      1488
   Glu Phe Thr Arg Ile Glu Val Leu His Pro Asp Val Ile Thr Ser Arg
                   485                 490                 495
   aaa gag atg aag cag cgc atg cgc ttg acc aac tcg gct agc ggt ggc      1536
   Lys Glu Met Lys Gln Arg Met Arg Leu Thr Asn Ser Ala Ser Gly Gly
               500                 505                 510
   gtg atg gcg aga ttc att cgt tag                                      1560
   Val Met Ala Arg Phe Ile Arg
           515
<210> 57
   <211> 519
   <212> PRT
   <213> Candida bombicola
<400> 57
   Met Ile Ile Asp Leu Ser Asp Ala Leu Ile Ile Gly Gly Ile Ala Leu
   1               5                   10                  15
   Cys Phe Leu Leu Ser Tyr Gln Ala Ile Tyr Phe Tyr Phe Ile Tyr Ser
               20                  25                  30
   Pro Arg Ala Lys Lys Leu Gly Cys Ala Pro Pro Leu Ile Val His Ala
           35                  40                  45
   Phe Pro Leu Gly Leu Pro Thr Ile Phe Gly Leu Ile Arg Ala Trp Arg
       50                  55                  60
   Asn Asp Asp Leu Leu Gln Tyr Leu Ser Asp Asn Phe Ala Arg Ile Arg
   65 70 75 80
   Thr Arg Thr Gly Met Gln Val Met Ala Gly Gln Leu Trp Leu Asn Thr
   85 90 95
   Ile Glu Pro Glu Asn Ile Lys Ala Met Leu Ala Thr Ser Phe Lys Asp
   100 105 110
   Phe Ser Leu Gly Phe Arg Tyr Glu Val Met His Gly Leu Leu Gly Asp
   115 120 125
   Gly Ile Phe Thr Leu Ser Gly Glu Gly Trp Lys His Ser Arg Ala Leu
   130 135 140
   Leu Arg Pro Gln Phe Ser Arg Glu Gln Val Ser His Leu Asp Ser Met
   145 150 155 160
   Arg Thr His Ile Asn Leu Met Ile Asn Asn His Phe Lys Gly Gly Gln
   165 170 175
   Val Val Asp Ala Gln Val Leu Tyr His Asn Leu Thr Ile Asp Thr Ala
   180 185 190
   Thr Glu Phe Leu Phe Gly Glu Ser Thr Asn Thr Leu Asp Pro Val Leu
   195 200 205
   Ala Gln Gln Gly Leu Pro Gly Pro Arg Gly Val Val Thr Gly Glu Gln
   210 215 220
   Phe Ala Asn Ala Phe Thr Tyr Ala Gln Glu Leu Leu Ser Ile Arg Val
   225 230 235 240
   Met Ala Gly Ser Ala Trp Phe Leu Val Trp Thr Pro Lys Phe Arg Arg
   245 250 255
   Ser Cys Lys Val Cys His Asn Phe Ile Asp Tyr Phe Val Phe Lys Ala
   260 265 270
   Leu Ala Thr Pro Met Glu Lys Asp Gln Glu Ala Asp Arg Tyr Val Phe
   275 280 285
   Ile Arg Glu Leu Thr Lys Glu Thr Ser Asp Pro Lys Val Ile Arg Asp
   290 295 300
   Gln Ala Leu Asn Ile Leu Leu Ala Gly Arg Asp Thr Thr Ala Ala Leu
   305 310 315 320
   Leu Ser Phe Thr Thr Tyr Tyr Leu Gly Ala Tyr Pro Glu Val Tyr Asp
                   325                 330                 335
   Glu Leu Arg Glu Ala Val Leu Ala Asp Phe Gly Pro Ala Asp Ser Glu
               340                 345                 350
   Pro Pro Thr Phe Glu Arg Leu Lys Gln Cys Lys Val Leu Gln Asn Val
           355                 360                 365
   Ile Arg Glu Val Leu Arg Leu His Pro Asn Val Pro Leu Asn Phe Arg
       370                 375                 380
   Gln Ala Ile Val Asp Thr Lys Phe Pro Thr Gly Gly Gly Pro Asn Arg
   385                 390                 395                 400
   Asp Gln Pro Ile Phe Val Pro Lys Gly Gln Lys Val Phe Tyr Ser Thr
                   405                 410                 415
   Tyr Val Met Gln Arg Ser Lys Asp Ile Trp Gly Ala Asp Ser Thr Ser
               420                 425                 430
   Phe Arg Pro Glu Arg Trp Asn Glu Pro Arg Glu Ala Leu Ala Ser Gly
           435                 440                 445
   Trp Asp Tyr Ile Pro Phe Asn Gly Gly Pro Arg Ile Cys Ile Gly Gln
       450                 455                 460
   Gln Phe Ala Leu Thr Glu Ala Ser Tyr Thr Leu Val Arg Ile Cys Gln
   465                 470                 475                 480
   Glu Phe Thr Arg Ile Glu Val Leu His Pro Asp Val Ile Thr Ser Arg
                   485                 490                 495
   Lys Glu Met Lys Gln Arg Met Arg Leu Thr Asn Ser Ala Ser Gly Gly
               500                 505                 510
   Val Met Ala Arg Phe Ile Arg
           515
<210> 58
   <211> 1572
   <212> DNA
   <213> Candida bombicola
<220>
   <221> CDS
   <222> (1)..(1572)
<400> 58
   atg att ttt tat gct gtg ctt ggc gct gtg gtc acc ttc tta ctt tac        48
   Met Ile Phe Tyr Ala Val Leu Gly Ala Val Val Thr Phe Leu Leu Tyr
   1               5                   10                  15
   gta gat gtg atc tac cct ttc gtg ata tat cct tta aaa gca cga tgg       96
   Val Asp Val Ile Tyr Pro Phe Val Ile Tyr Pro Leu Lys Ala Arg Trp
               20                  25                  30
   cac aaa tgt ggc tcc gta cct cga gag ctt agc tgg cca ttg ggg att      144
   His Lys Cys Gly Ser Val Pro Arg Glu Leu Ser Trp Pro Leu Gly Ile
           35                  40                  45
   cca acc acc ata gga gtt ttt tcg aac ata aag aag gat cta cat ctt      192
   Pro Thr Thr Ile Gly Val Phe Ser Asn Ile Lys Lys Asp Leu His Leu
       50                  55                  60
   caa gtc ctg gca gcg tac gac ctc agc cgg tct tat aag aca agc ttg      240
   Gln Val Leu Ala Ala Tyr Asp Leu Ser Arg Ser Tyr Lys Thr Ser Leu
   65                  70                  75                  80
   cgt caa agt ctc ggc aca tgg gta gtt gct acg cgg gat cct gag aac      288
   Arg Gln Ser Leu Gly Thr Trp Val Val Ala Thr Arg Asp Pro Glu Asn
                   85                  90                  95
   atc aag gcc gtt ttg tct acc aag ttc aat gac ttt tca ctg aaa gag      336
   Ile Lys Ala Val Leu Ser Thr Lys Phe Asn Asp Phe Ser Leu Lys Glu
               100                 105                 110
   aga gga att cgg tta agg cat gta att ggt gat ggt atc ttt acc caa      384
   Arg Gly Ile Arg Leu Arg His Val Ile Gly Asp Gly Ile Phe Thr Gln
           115                 120                 125
   gat ggt gca ccg tgg aag cac tcg cga gat atg ctc aga cct caa ttc      432
   Asp Gly Ala Pro Trp Lys His Ser Arg Asp Met Leu Arg Pro Gln Phe
       130                 135                 140
   agt agg gaa caa atc agc cgc gtg gag gtg ttg agt cac cac atc gat      480
   Ser Arg Glu Gln Ile Ser Arg Val Glu Val Leu Ser His His Ile Asp
   145                 150                 155                 160
   gtt ttg att cgt gag atc aaa aag tcg gga ggt aat gtt gag ttg caa      528
   Val Leu Ile Arg Glu Ile Lys Lys Ser Gly Gly Asn Val Glu Leu Gln
                   165                 170                 175
   cga cta ttc cac ctc atg act atg gac acc gcc aca cag ttt ctt ttc      576
   Arg Leu Phe His Leu Met Thr Met Asp Thr Ala Thr Gln Phe Leu Phe
               180                 185                 190
   ggc gaa tca att ggc tcg cta gaa gtc agt ggc gac agc aag ggc att      624
   Gly Glu Ser Ile Gly Ser Leu Glu Val Ser Gly Asp Ser Lys Gly Ile
           195                 200                 205
   gag att act gac cca aat act gga gat att gtg agt acc gtt gac ttc      672
   Glu Ile Thr Asp Pro Asn Thr Gly Asp Ile Val Ser Thr Val Asp Phe
       210                 215                 220
   gtt gag tct tat act ttc aca aac aga ttt gct atg aag aag gta ttc      720
   Val Glu Ser Tyr Thr Phe Thr Asn Arg Phe Ala Met Lys Lys Val Phe
   225                 230                 235                 240
   ctg aac aaa tgg gaa ttc ttg gca aac ttg tcg aac ccc tca tat gag      768
   Leu Asn Lys Trp Glu Phe Leu Ala Asn Leu Ser Asn Pro Ser Tyr Glu
                   245                 250                 255
   agg cat atg cgg cgt gtc cac aca gtc ctg gat cac tac gtt cag ctg      816
   Arg His Met Arg Arg Val His Thr Val Leu Asp His Tyr Val Gln Leu
               260                 265                 270
   gct ttg aag gct act gag aag tat gat cct gaa gat gac agc gag aaa      864
   Ala Leu Lys Ala Thr Glu Lys Tyr Asp Pro Glu Asp Asp Ser Glu Lys
           275                 280                 285
   gga gaa tac tac ttt agc cat gag ctg gct aaa ctc acg aga gac ccc      912
   Gly Glu Tyr Tyr Phe Ser His Glu Leu Ala Lys Leu Thr Arg Asp Pro
       290                 295                 300
   ttg tcg ttg cgc aat cag ctt ttt aat atc ctg att gct ggc cgc gac      960
   Leu Ser Leu Arg Asn Gln Leu Phe Asn Ile Leu Ile Ala Gly Arg Asp
   305                 310                 315                 320
   act acc gca gca aca ttg tcc tat gcc ttc cat tac tta acg aag aac     1008
   Thr Thr Ala Ala Thr Leu Ser Tyr Ala Phe His Tyr Leu Thr Lys Asn
                   325                 330                 335
   cca gcc atc tac gcc aag gtt cgc gaa gat gtg ctc acc gtc ttc ccc     1056
   Pro Ala Ile Tyr Ala Lys Val Arg Glu Asp Val Leu Thr Val Phe Pro
               340                 345                 350
   gat gga gac gcc tca ttg gcg acc ttt gag gac ttg cga aag gcc aag     1104
   Asp Gly Asp Ala Ser Leu Ala Thr Phe Glu Asp Leu Arg Lys Ala Lys
           355                 360                 365
   tat ctc caa atg gta atc aag gag gta ttg cgc ctt gcg cct gcg gtt     1152
   Tyr Leu Gln Met Val Ile Lys Glu Val Leu Arg Leu Ala Pro Ala Val
       370                 375                 380
   ccc aca aat tcg cgt act gcg gtt cgt gac acc tat ctg cca cgg ggt     1200
   Pro Thr Asn Ser Arg Thr Ala Val Arg Asp Thr Tyr Leu Pro Arg Gly
   385                 390                 395                 400
   gga ggc cca gct gga aac cta ccc gtt ttc gtt ccc aag ggc act att     1248
   Gly Gly Pro Ala Gly Asn Leu Pro Val Phe Val Pro Lys Gly Thr Ile
                   405                 410                 415
   atc agg tat cct gca tat atc ttg cac cgc gat cct gat ata tat ggt     1296
   Ile Arg Tyr Pro Ala Tyr Ile Leu His Arg Asp Pro Asp Ile Tyr Gly
               420                 425                 430
   gcc gac tcg tat gac ttc aac cct gag agg tgg aga ccc gag aat aag     1344
   Ala Asp Ser Tyr Asp Phe Asn Pro Glu Arg Trp Arg Pro Glu Asn Lys
           435                 440                 445
   ctc cca ggt agc cca atg tac tca tgg ggc tat att ccc ttt aat ggc     1392
   Leu Pro Gly Ser Pro Met Tyr Ser Trp Gly Tyr Ile Pro Phe Asn Gly
       450                 455                 460
   ggc cct cgc att tgc gtt gga cag cag ttt gcc ttg act gaa atc gct     1440
   Gly Pro Arg Ile Cys Val Gly Gln Gln Phe Ala Leu Thr Glu Ile Ala
   465                 470                 475                 480
   ttg aca atg atc aag ctg gtt ttg gaa ttt gag agg ctg gag cct gct     1488
   Leu Thr Met Ile Lys Leu Val Leu Glu Phe Glu Arg Leu Glu Pro Ala
                   485                 490                 495
   gat gac ttt gag ccc aat ctt cga gat agg acc tca tta act tcc atg     1536
   Asp Asp Phe Glu Pro Asn Leu Arg Asp Arg Thr Ser Leu Thr Ser Met
               500                 505                 510
   gtc gga ggg tcg ggc gtc cga gta aaa ctg agt taa                     1572
   Val Gly Gly Ser Gly Val Arg Val Lys Leu Ser
           515                 520
<210> 59
   <211> 523
   <212> PRT
   <213> Candida bombicola
<400> 59
   Met Ile Phe Tyr Ala Val Leu Gly Ala Val Val Thr Phe Leu Leu Tyr
   1               5                   10                  15
   Val Asp Val Ile Tyr Pro Phe Val Ile Tyr Pro Leu Lys Ala Arg Trp
               20                  25                  30
   His Lys Cys Gly Ser Val Pro Arg Glu Leu Ser Trp Pro Leu Gly Ile
           35                  40                  45
   Pro Thr Thr Ile Gly Val Phe Ser Asn Ile Lys Lys Asp Leu His Leu
       50                  55                  60
   Gln Val Leu Ala Ala Tyr Asp Leu Ser Arg Ser Tyr Lys Thr Ser Leu
   65                  70                  75                  80
   Arg Gln Ser Leu Gly Thr Trp Val Val Ala Thr Arg Asp Pro Glu Asn
                   85                  90                  95
   Ile Lys Ala Val Leu Ser Thr Lys Phe Asn Asp Phe Ser Leu Lys Glu
               100                 105                 110
   Arg Gly Ile Arg Leu Arg His Val Ile Gly Asp Gly Ile Phe Thr Gln
           115                 120                 125
   Asp Gly Ala Pro Trp Lys His Ser Arg Asp Met Leu Arg Pro Gln Phe
       130                 135                 140
   Ser Arg Glu Gln Ile Ser Arg Val Glu Val Leu Ser His His Ile Asp
   145                 150                 155                 160
   Val Leu Ile Arg Glu Ile Lys Lys Ser Gly Gly Asn Val Glu Leu Gln
                   165                 170                 175
   Arg Leu Phe His Leu Met Thr Met Asp Thr Ala Thr Gln Phe Leu Phe
               180                 185                 190
   Gly Glu Ser Ile Gly Ser Leu Glu Val Ser Gly Asp Ser Lys Gly Ile
           195                 200                 205
   Glu Ile Thr Asp Pro Asn Thr Gly Asp Ile Val Ser Thr Val Asp Phe
       210                 215                 220
   Val Glu Ser Tyr Thr Phe Thr Asn Arg Phe Ala Met Lys Lys Val Phe
   225                 230                 235                 240
   Leu Asn Lys Trp Glu Phe Leu Ala Asn Leu Ser Asn Pro Ser Tyr Glu
                   245                 250                 255
   Arg His Met Arg Arg Val His Thr Val Leu Asp His Tyr Val Gln Leu
               260                 265                 270
   Ala Leu Lys Ala Thr Glu Lys Tyr Asp Pro Glu Asp Asp Ser Glu Lys
           275                 280                 285
   Gly Glu Tyr Tyr Phe Ser His Glu Leu Ala Lys Leu Thr Arg Asp Pro
       290                 295                 300
   Leu Ser Leu Arg Asn Gln Leu Phe Asn Ile Leu Ile Ala Gly Arg Asp
   305                 310                 315                 320
   Thr Thr Ala Ala Thr Leu Ser Tyr Ala Phe His Tyr Leu Thr Lys Asn
                   325                 330                 335
   Pro Ala Ile Tyr Ala Lys Val Arg Glu Asp Val Leu Thr Val Phe Pro
               340                 345                 350
   Asp Gly Asp Ala Ser Leu Ala Thr Phe Glu Asp Leu Arg Lys Ala Lys
           355                 360                 365
   Tyr Leu Gln Met Val Ile Lys Glu Val Leu Arg Leu Ala Pro Ala Val
       370                 375                 380
   Pro Thr Asn Ser Arg Thr Ala Val Arg Asp Thr Tyr Leu Pro Arg Gly
   385                 390                 395                 400
   Gly Gly Pro Ala Gly Asn Leu Pro Val Phe Val Pro Lys Gly Thr Ile
                   405                 410                 415
   Ile Arg Tyr Pro Ala Tyr Ile Leu His Arg Asp Pro Asp Ile Tyr Gly
               420                 425                 430
   Ala Asp Ser Tyr Asp Phe Asn Pro Glu Arg Trp Arg Pro Glu Asn Lys
           435                 440                 445
   Leu Pro Gly Ser Pro Met Tyr Ser Trp Gly Tyr Ile Pro Phe Asn Gly
       450                 455                 460
   Gly Pro Arg Ile Cys Val Gly Gln Gln Phe Ala Leu Thr Glu Ile Ala
   465                 470                 475                 480
   Leu Thr Met Ile Lys Leu Val Leu Glu Phe Glu Arg Leu Glu Pro Ala
                   485                 490                 495
   Asp Asp Phe Glu Pro Asn Leu Arg Asp Arg Thr Ser Leu Thr Ser Met
               500                 505                 510
   Val Gly Gly Ser Gly Val Arg Val Lys Leu Ser
           515                 520
<210> 60
   <211> 1572
   <212> DNA
   <213> Candida bombicola
<220>
   <221> CDS
   <222> (1)..(1572)
<400> 60
   atg att ttt tat gct gtg ctt ggc act gtg gtc gcc ttc tta ctt tac      48
   Met Ile Phe Tyr Ala Val Leu Gly Thr Val Val Ala Phe Leu Leu Tyr
   1               5                   10                  15
   gta gat gtg atc tac cct ttc gtg ata tat cct tta aag gca cga tgg      96
   Val Asp Val Ile Tyr Pro Phe Val Ile Tyr Pro Leu Lys Ala Arg Trp
               20                  25                  30
   cac aaa tgt ggc ttc gtc cct cga gag ctg agc tgg cca ttg ggg att      144
   His Lys Cys Gly Phe Val Pro Arg Glu Leu Ser Trp Pro Leu Gly Ile
           35                  40                  45
   cca gac acc ata gca gtt ttt tcg agg ata aag aag gat cta cat ctt      192
   Pro Asp Thr Ile Ala Val Phe Ser Arg Ile Lys Lys Asp Leu His Leu
       50                  55                  60
   caa ttc ctg gca gcg cac gac ctc agc cgg tct tat aag aca agc ttg      240
   Gln Phe Leu Ala Ala His Asp Leu Ser Arg Ser Tyr Lys Thr Ser Leu
   65                  70                  75                  80
   cgt caa act ctc ggc aca tgg gta gtt gat acg cga gat cct gag aat      288
   Arg Gln Thr Leu Gly Thr Trp Val Val Asp Thr Arg Asp Pro Glu Asn
                   85                  90                  95
   atc aag gcc gtt ttg tct acc aag ttc aat gac ttt tca ctg aaa gat      336
   Ile Lys Ala Val Leu Ser Thr Lys Phe Asn Asp Phe Ser Leu Lys Asp
               100                 105                 110
   aga gga att cgg tta agg caa gta att ggt gat ggt att ttt acc caa      384
   Arg Gly Ile Arg Leu Arg Gln Val Ile Gly Asp Gly Ile Phe Thr Gln
           115                 120                 125
   gat ggt gca ccg tgg aag cac tcg cga gat atg ctc aga cct caa ttc      432
   Asp Gly Ala Pro Trp Lys His Ser Arg Asp Met Leu Arg Pro Gln Phe
       130                 135                 140
   agt agg gaa caa att agc cgc gtg gag gtg ttg agt cac cac atc gat      480
   Ser Arg Glu Gln Ile Ser Arg Val Glu Val Leu Ser His His Ile Asp
   145                 150                 155                 160
   gtt ttg att cgt gag atc aaa aag tcg gga ggt aat gtt gag ttg caa      528
   Val Leu Ile Arg Glu Ile Lys Lys Ser Gly Gly Asn Val Glu Leu Gln
                   165                 170                 175
   cga cta ttc cac ctc atg act atg gac act gct aca cag ttt ctt ttc      576
   Arg Leu Phe His Leu Met Thr Met Asp Thr Ala Thr Gln Phe Leu Phe
               180                 185                 190
   ggc gaa tca att ggc tcg cta gaa gtc agt ggc gac agc aag ggc att      624
   Gly Glu Ser Ile Gly Ser Leu Glu Val Ser Gly Asp Ser Lys Gly Ile
           195                 200                 205
   gag att act gac cca aat act gga gat att gtg aat acc gtt gac ttc      672
   Glu Ile Thr Asp Pro Asn Thr Gly Asp Ile Val Asn Thr Val Asp Phe
       210                 215                 220
   gtt gag tct tat act ttt gca aac aga ttt gct atg aaa aag ata tta      720
   Val Glu Ser Tyr Thr Phe Ala Asn Arg Phe Ala Met Lys Lys Ile Leu
   225                 230                 235                 240
   ctg aac aaa tgg gaa ttc gtg gta aac ttg tcg aac ccc tca tat gag      768
   Leu Asn Lys Trp Glu Phe Val Val Asn Leu Ser Asn Pro Ser Tyr Glu
                   245                 250                 255
   agg cat atg cga cgt gtc cac aca gtc ctg gat cac tac gtt cag ctg      816
   Arg His Met Arg Arg Val His Thr Val Leu Asp His Tyr Val Gln Leu
               260                 265                 270
   gct ttg aag gct act gag aag tat gat cct gaa gat gac tgc gag aaa      864
   Ala Leu Lys Ala Thr Glu Lys Tyr Asp Pro Glu Asp Asp Cys Glu Lys
           275                 280                 285
   gga gaa tac tac ttt agc cat gag ctg gct aaa ctc acg aga gac ccc      912
   Gly Glu Tyr Tyr Phe Ser His Glu Leu Ala Lys Leu Thr Arg Asp Pro
       290                 295                 300
   ttg tgc ttg cgc aat cag ctt ttt aat atc ctg att gct ggc cgc gac      960
   Leu Cys Leu Arg Asn Gln Leu Phe Asn Ile Leu Ile Ala Gly Arg Asp
   305                 310                 315                 320
   act acc gca gca aca ttg gcc tat gcc ttc cat tac ttg acg aag aac     1008
   Thr Thr Ala Ala Thr Leu Ala Tyr Ala Phe His Tyr Leu Thr Lys Asn
                   325                 330                 335
   cca gcc atc tac gcc aag gtg cgc gaa gat gtg ctc acc gtc ttc ccc     1056
   Pro Ala Ile Tyr Ala Lys Val Arg Glu Asp Val Leu Thr Val Phe Pro
               340                 345                 350
   aat gga gat gcc tca ttg gcg acc ttt gag gac ttg cga aag gcc aag     1104
   Asn Gly Asp Ala Ser Leu Ala Thr Phe Glu Asp Leu Arg Lys Ala Lys
           355                 360                 365
   tat ctc caa atg gta atc aag gag gta ttg cgc ctt gcg cct gtg gtt     1152
   Tyr Leu Gln Met Val Ile Lys Glu Val Leu Arg Leu Ala Pro Val Val
       370                 375                 380
   ccc aca aat tcg cgt act gcg gtt cgt gac acc tat ctg cca cgg ggt     1200
   Pro Thr Asn Ser Arg Thr Ala Val Arg Asp Thr Tyr Leu Pro Arg Gly
   385                 390                 395                 400
   gga ggc cca gct gga aac cta ccc gtt ttc gtt ccc aag ggc aca aat     1248
   Gly Gly Pro Ala Gly Asn Leu Pro Val Phe Val Pro Lys Gly Thr Asn
                   405                 410                 415
   gtc agg tat tct gca tat gtc ttg cac cgc gat cct gat ata tat ggt     1296
   Val Arg Tyr Ser Ala Tyr Val Leu His Arg Asp Pro Asp Ile Tyr Gly
               420                 425                 430
   gcc gac tcg tat gac ttc aac cct gag agg tgg aga ccc gag aat aag     1344
   Ala Asp Ser Tyr Asp Phe Asn Pro Glu Arg Trp Arg Pro Glu Asn Lys
           435                 440                 445
   ctc cca ggt agc cca atg tac tca tgg ggc tat att ccc ttt aat ggc      1392
   Leu Pro Gly Ser Pro Met Tyr Ser Trp Gly Tyr Ile Pro Phe Asn Gly
       450                 455                 460
   ggc cct cgc att tgc gtt gga cag cag ttt gcc ttg act gaa ttc gct      1440
   Gly Pro Arg Ile Cys Val Gly Gln Gln Phe Ala Leu Thr Glu Phe Ala
   465                 470                 475                 480
   ttg aca atg atc aag ctg gtt tta gaa ttt gag agg ctg gag cct gct      1488
   Leu Thr Met Ile Lys Leu Val Leu Glu Phe Glu Arg Leu Glu Pro Ala
                   485                 490                 495
   gat gac ttt gag ccc aat ctt cta gat agg acc tca tta act gcc atg      1536
   Asp Asp Phe Glu Pro Asn Leu Leu Asp Arg Thr Ser Leu Thr Ala Met
               500                 505                 510
   gtc gga ggg tcg ggc gtc cga gta aaa ctg agt taa                      1572
   Val Gly Gly Ser Gly Val Arg Val Lys Leu Ser
           515                 520
<210> 61
   <211> 523
   <212> PRT
   <213> Candida bombicola
<400> 61
   Met Ile Phe Tyr Ala Val Leu Gly Thr Val Val Ala Phe Leu Leu Tyr
   1               5                   10                  15
   Val Asp Val Ile Tyr Pro Phe Val Ile Tyr Pro Leu Lys Ala Arg Trp
               20                  25                  30
   His Lys Cys Gly Phe Val Pro Arg Glu Leu Ser Trp Pro Leu Gly Ile
           35                  40                  45
   Pro Asp Thr Ile Ala Val Phe Ser Arg Ile Lys Lys Asp Leu His Leu
       50                  55                  60
   Gln Phe Leu Ala Ala His Asp Leu Ser Arg Ser Tyr Lys Thr Ser Leu
   65                  70                  75                  80
   Arg Gln Thr Leu Gly Thr Trp Val Val Asp Thr Arg Asp Pro Glu Asn
                   85                  90                  95
   Ile Lys Ala Val Leu Ser Thr Lys Phe Asn Asp Phe Ser Leu Lys Asp
               100                 105                 110
   Arg Gly Ile Arg Leu Arg Gln Val Ile Gly Asp Gly Ile Phe Thr Gln
           115                 120                 125
   Asp Gly Ala Pro Trp Lys His Ser Arg Asp Met Leu Arg Pro Gln Phe
       130                 135                 140
   Ser Arg Glu Gln Ile Ser Arg Val Glu Val Leu Ser His His Ile Asp
   145 150 155 160
   Val Leu Ile Arg Glu Ile Lys Lys Ser Gly Gly Asn Val Glu Leu Gln
   165 170 175
   Arg Leu Phe His Leu Met Thr Met Asp Thr Ala Thr Gln Phe Leu Phe
   180 185 190
   Gly Glu Ser Ile Gly Ser Leu Glu Val Ser Gly Asp Ser Lys Gly Ile
   195 200 205
   Glu Ile Thr Asp Pro Asn Thr Gly Asp Ile Val Asn Thr Val Asp Phe
   210 215 220
   Val Glu Ser Tyr Thr Phe Ala Asn Arg Phe Ala Met Lys Lys Ile Leu
   225 230 235 240
   Leu Asn Lys Trp Glu Phe Val Val Asn Leu Ser Asn Pro Ser Tyr Glu
   245 250 255
   Arg His Met Arg Arg Val His Thr Val Leu Asp His Tyr Val Gln Leu
   260 265 270
   Ala Leu Lys Ala Thr Glu Lys Tyr Asp Pro Glu Asp Asp Cys Glu Lys
   275 280 285
   Gly Glu Tyr Tyr Phe Ser His Glu Leu Ala Lys Leu Thr Arg Asp Pro
   290 295 300
   Leu Cys Leu Arg Asn Gln Leu Phe Asn Ile Leu Ile Ala Gly Arg Asp
   305 310 315 320
   Thr Thr Ala Ala Thr Leu Ala Tyr Ala Phe His Tyr Leu Thr Lys Asn
   325 330 335
   Pro Ala Ile Tyr Ala Lys Val Arg Glu Asp Val Leu Thr Val Phe Pro
   340 345 350
   Asn Gly Asp Ala Ser Leu Ala Thr Phe Glu Asp Leu Arg Lys Ala Lys
   355 360 365
   Tyr Leu Gln Met Val Ile Lys Glu Val Leu Arg Leu Ala Pro Val Val
   370 375 380
   Pro Thr Asn Ser Arg Thr Ala Val Arg Asp Thr Tyr Leu Pro Arg Gly
   385 390 395 400
   Gly Gly Pro Ala Gly Asn Leu Pro Val Phe Val Pro Lys Gly Thr Asn
                   405                 410                 415
   Val Arg Tyr Ser Ala Tyr Val Leu His Arg Asp Pro Asp Ile Tyr Gly
               420                 425                 430
   Ala Asp Ser Tyr Asp Phe Asn Pro Glu Arg Trp Arg Pro Glu Asn Lys
           435                 440                 445
   Leu Pro Gly Ser Pro Met Tyr Ser Trp Gly Tyr Ile Pro Phe Asn Gly
       450                 455                 460
   Gly Pro Arg Ile Cys Val Gly Gln Gln Phe Ala Leu Thr Glu Phe Ala
   465                 470                 475                 480
   Leu Thr Met Ile Lys Leu Val Leu Glu Phe Glu Arg Leu Glu Pro Ala
                   485                 490                 495
   Asp Asp Phe Glu Pro Asn Leu Leu Asp Arg Thr Ser Leu Thr Ala Met
               500                 505                 510
   Val Gly Gly Ser Gly Val Arg Val Lys Leu Ser
           515                 520
<210> 62
   <211> 1206
   <212> DNA
   <213> Candida bombicola
<220>
   <221> CDS
   <222> (1)..(1206)
<400> 62
   atg ttt gcg aaa gct tta tgg gag gat gat gtt ttg gag tac gcc tgc        48
   Met Phe Ala Lys Ala Leu Trp Glu Asp Asp Val Leu Glu Tyr Ala Cys
   1               5                   10                  15
   cgc agg ttt gca ggc atg aag gtc aga act ggg ctt caa act gtc gct        96
   Arg Arg Phe Ala Gly Met Lys Val Arg Thr Gly Leu Gln Thr Val Ala
               20                  25                  30
   ggc cag cta tgg ata gca act atc gag ccg gag aac atc aag acc gta       144
   Gly Gln Leu Trp Ile Ala Thr Ile Glu Pro Glu Asn Ile Lys Thr Val
           35                  40                  45
   ctt gcc acc tcg ttc aat gac tac tcc ctt ggc ttc cgt tat aat gcc       192
   Leu Ala Thr Ser Phe Asn Asp Tyr Ser Leu Gly Phe Arg Tyr Asn Ala
       50                  55                  60
   cta tac ggc ctt ctc gga aat ggt att ttc acc ctt agt ggt gat ggc       240
   Leu Tyr Gly Leu Leu Gly Asn Gly Ile Phe Thr Leu Ser Gly Asp Gly
   65                  70                  75                  80
   tgg aag cac agt cgt gct ttg ttg cgt ccg cag ttc agt cgt gag caa       288
   Trp Lys His Ser Arg Ala Leu Leu Arg Pro Gln Phe Ser Arg Glu Gln
                   85                  90                  95
   gtt tct cac ttg gac tcc atg cgt aca cac atc aac ttg atg atc aac      336
   Val Ser His Leu Asp Ser Met Arg Thr His Ile Asn Leu Met Ile Asn
               100                 105                 110
   aac cat ttc aaa ggc ggc cac gtc gtt gac gca cag gct cga tac cac      384
   Asn His Phe Lys Gly Gly His Val Val Asp Ala Gln Ala Arg Tyr His
           115                 120                 125
   aat ttg acc atc gat act gcg act gaa ttc ctt ttc ggt gag agc act      432
   Asn Leu Thr Ile Asp Thr Ala Thr Glu Phe Leu Phe Gly Glu Ser Thr
       130                 135                 140
   aac aca ctc gac cct gtt ctt gca cag caa gga ctc cct ggt cct aag      480
   Asn Thr Leu Asp Pro Val Leu Ala Gln Gln Gly Leu Pro Gly Pro Lys
   145                 150                 155                 160
   ggc acc gtt acc gga gag cag ttt gct gaa gct ttc acc tcc gct ctt      528
   Gly Thr Val Thr Gly Glu Gln Phe Ala Glu Ala Phe Thr Ser Ala Leu
                   165                 170                 175
   caa gtg ctg agt gtc cga gtt atg gcc ggc tcc gca tgg ttc ctc att      576
   Gln Val Leu Ser Val Arg Val Met Ala Gly Ser Ala Trp Phe Leu Ile
               180                 185                 190
   tgg act cct aaa ttc tgg cgc tcg tgc aag gtg tgc cac aac ttc att      624
   Trp Thr Pro Lys Phe Trp Arg Ser Cys Lys Val Cys His Asn Phe Ile
           195                 200                 205
   gac tac ttc gta tac aag gcc ttg gcc act ccg atg gag aag ggc caa      672
   Asp Tyr Phe Val Tyr Lys Ala Leu Ala Thr Pro Met Glu Lys Gly Gln
       210                 215                 220
   gag gct gat cgc tat gtt ttt att cga gag ctc aca aag gag act tct      720
   Glu Ala Asp Arg Tyr Val Phe Ile Arg Glu Leu Thr Lys Glu Thr Ser
   225                 230                 235                 240
   gac cca aga gtc atc cgt gac cag gct cta aat atc ctg ctg gct ggt      768
   Asp Pro Arg Val Ile Arg Asp Gln Ala Leu Asn Ile Leu Leu Ala Gly
                   245                 250                 255
   cgt gat acc act gcg gca ctc ctc atc att gcg gac ttt ggc tct gag      816
   Arg Asp Thr Thr Ala Ala Leu Leu Ile Ile Ala Asp Phe Gly Ser Glu
               260                 265                 270
   gac gct gag ccc cct acc ttt gag cag ctc aag cag tgc aag gta ctg      864
   Asp Ala Glu Pro Pro Thr Phe Glu Gln Leu Lys Gln Cys Lys Val Leu
           275                 280                 285
   cag aat gtc att cgc gag gtt tta cgt ttg cac cct aat gtg ccg ctc      912
   Gln Asn Val Ile Arg Glu Val Leu Arg Leu His Pro Asn Val Pro Leu
       290                 295                 300
   aac ttc cgc cag gct ata act gat act aag ctc ccc act ggt ggt ggc      960
   Asn Phe Arg Gln Ala Ile Thr Asp Thr Lys Leu Pro Thr Gly Gly Gly
   305                 310                 315                 320
   ccg aac aga gac cag cct gtc ttt gtt cca aag gga cag aaa gtg ttc     1008
   Pro Asn Arg Asp Gln Pro Val Phe Val Pro Lys Gly Gln Lys Val Phe
                   325                 330                 335
   tac gcc acc tac gtc atg cag cga gat ccg gaa ata tgg ggc ccc gac     1056
   Tyr Ala Thr Tyr Val Met Gln Arg Asp Pro Glu Ile Trp Gly Pro Asp
               340                 345                 350
   tct aca agc ttc cgc cct gat cga tgg aat gag ccg aga gag gct ctt      1104
   Ser Thr Ser Phe Arg Pro Asp Arg Trp Asn Glu Pro Arg Glu Ala Leu
           355                 360                 365
   gca tca ggt tgg gat tat att cct ttc aat ggc ggc cct cgc att tgt      1152
   Ala Ser Gly Trp Asp Tyr Ile Pro Phe Asn Gly Gly Pro Arg Ile Cys
       370                 375                 380
   atc ggt cag cag ttc gct ctc act gag gct agc tac aca ctt gtc cgt      1200
   Ile Gly Gln Gln Phe Ala Leu Thr Glu Ala Ser Tyr Thr Leu Val Arg
   385                 390                 395                 400
   atc tag                                                              1206
   Ile
<210> 63
   <211> 401
   <212> PRT
   <213> Candida bombicola
<400> 63
   Met Phe Ala Lys Ala Leu Trp Glu Asp Asp Val Leu Glu Tyr Ala Cys
   1               5                   10                  15
   Arg Arg Phe Ala Gly Met Lys Val Arg Thr Gly Leu Gln Thr Val Ala
               20                  25                  30
   Gly Gln Leu Trp Ile Ala Thr Ile Glu Pro Glu Asn Ile Lys Thr Val
           35                  40                  45
   Leu Ala Thr Ser Phe Asn Asp Tyr Ser Leu Gly Phe Arg Tyr Asn Ala
       50                  55                  60
   Leu Tyr Gly Leu Leu Gly Asn Gly Ile Phe Thr Leu Ser Gly Asp Gly
   65                  70                  75                  80
   Trp Lys His Ser Arg Ala Leu Leu Arg Pro Gln Phe Ser Arg Glu Gln
                   85                  90                  95
   Val Ser His Leu Asp Ser Met Arg Thr His Ile Asn Leu Met Ile Asn
               100                 105                 110
   Asn His Phe Lys Gly Gly His Val Val Asp Ala Gln Ala Arg Tyr His
           115                 120                 125
   Asn Leu Thr Ile Asp Thr Ala Thr Glu Phe Leu Phe Gly Glu Ser Thr
       130                 135                 140
   Asn Thr Leu Asp Pro Val Leu Ala Gln Gln Gly Leu Pro Gly Pro Lys
   145                 150                 155                 160
   Gly Thr Val Thr Gly Glu Gln Phe Ala Glu Ala Phe Thr Ser Ala Leu
                   165                 170                 175
   Gln Val Leu Ser Val Arg Val Met Ala Gly Ser Ala Trp Phe Leu Ile
               180                 185                 190
   Trp Thr Pro Lys Phe Trp Arg Ser Cys Lys Val Cys His Asn Phe Ile
           195                 200                 205
   Asp Tyr Phe Val Tyr Lys Ala Leu Ala Thr Pro Met Glu Lys Gly Gln
       210                 215                 220
   Glu Ala Asp Arg Tyr Val Phe Ile Arg Glu Leu Thr Lys Glu Thr Ser
   225                 230                 235                 240
   Asp Pro Arg Val Ile Arg Asp Gln Ala Leu Asn Ile Leu Leu Ala Gly
                   245                 250                 255
   Arg Asp Thr Thr Ala Ala Leu Leu Ile Ile Ala Asp Phe Gly Ser Glu
               260                 265                 270
   Asp Ala Glu Pro Pro Thr Phe Glu Gln Leu Lys Gln Cys Lys Val Leu
           275                 280                 285
   Gln Asn Val Ile Arg Glu Val Leu Arg Leu His Pro Asn Val Pro Leu
       290                 295                 300
   Asn Phe Arg Gln Ala Ile Thr Asp Thr Lys Leu Pro Thr Gly Gly Gly
   305                 310                 315                 320
   Pro Asn Arg Asp Gln Pro Val Phe Val Pro Lys Gly Gln Lys Val Phe
                   325                 330                 335
   Tyr Ala Thr Tyr Val Met Gln Arg Asp Pro Glu Ile Trp Gly Pro Asp
               340                 345                 350
   Ser Thr Ser Phe Arg Pro Asp Arg Trp Asn Glu Pro Arg Glu Ala Leu
           355                 360                 365
   Ala Ser Gly Trp Asp Tyr Ile Pro Phe Asn Gly Gly Pro Arg Ile Cys
       370                 375                 380
   Ile Gly Gln Gln Phe Ala Leu Thr Glu Ala Ser Tyr Thr Leu Val Arg
   385                 390                 395                 400
   Ile
<210> 64
   <211> 6084
   <212> DNA
   <213> Artificial
<220>
   <223> Vector
<400> 64
   ctaaattgta agcgttaata ttttgttaaa attcgcgtta aatttttgtt aaatcagctc 60
   attttttaac caataggccg aaatcggcaa aatcccttat aaatcaaaag aatagaccga 120
   gatagggttg agtggccgct acagggcgct cccattcgcc attcaggctg cgcaactgtt 180
   gggaagggcg tttcggtgcg ggcctcttcg ctattacgcc agctggcgaa agggggatgt 240
   gctgcaaggc gattaagttg ggtaacgcca gggttttccc agtcacgacg ttgtaaaacg 300
   acggccagtg agcgcgacgt aatacgactc actatagggc gaattggcgg aaggccgtca 360
   aggcctaggc gcgcctgcag gatcctagaa aacagctgga tatggataaa ctcggcaagc 420
   atcttttgga aaggctgcca cgctatgccg tgccgatatt cattaagttc gtcgacaccg 480
   tcaccatcac cggcaacaac aaagttcaga agaaagaatt ccgaaaccag cagattcccg 540
   ccccagcagg acaaacaatc tactggttag agggcacgag ctacaagccc ctcactgctg 600
   atgcgtgggc tcgtgtagag aatgggcgac acaagctcta atgttgaata cctcttccgt 660
   atagatgtgg cataagctat agattttgct gcaatattat taaatattaa agagtttcga 720
   aggtcagctg cggatgaacc agttcagagc ggctctctct tttttgccaa tagcgtgcaa 780
   ccgtgaagag caattcaacc atccaatctg gctacactaa attgtatttg gcagcgcact 840
   gtacgagcgc actgtacgaa actccgtcaa tttatagcag aacgcgtgcg atcgcgggcc 900
   ccagcgatat gacgagaatc aggcaataat agcttaagct gaagtgtttt tagatttagt 960
   tcggagtgcg cttctcaaaa gtgctgggat caacaagttt ttaacatggg ttttgattta 1020
   tattgtttta tatgagcgcc tcagatatgc gctgacagcc tattaggaga aatggccggc 1080
   ctacaagtcc atatgtgtag agttgttttt gttgttaagt ctttctttaa gagcttgacc 1140
   gactataacc gttcaacggc gcattatata ctttgggtat cggccagtgc tgacaactca 1200
   cacgttgcga ccccttaccc agaagcatac ccagcgcgat gtcgatcgtg ttatatcgta 1260
   gacgcacacc ctgcaatgac gggtaggctc taaatcggga tgcgaaaaag aggttgcctt 1320
   gctttttgcc ctggtagatg gcatgctgag cgtgcgcttg ccgcctaatt tttgtgtgtc 1380
   gcctgctatt tattgctgaa gctagcccgc cgcatctttc cccaaggctt cgattgctcg 1440
   tattggggca gggattggta ctcaaccttg cagatgagac tccagcaaca acgtcgtact 1500
   gcttagcgat cgcacatgtt tcatcatcgt cactatacac atcgtcatca actccatggc 1560
   gtgaggactt ccgagactgc tgggcccttc gtttctttaa tgcctcaaga gatgacttcg 1620
   tacccgaaga gacgcctgtt gtaccccgtt gacgcttggc ggagggggct tcgtcctcgt 1680
   cagcaacccg cgtcatctgc ttccttcgct gagcaagata ccttctctcc tcgtaccgct 1740
   gcatctcctg agctcggtca tacaagatct cttctcgctc aatctctggc agcgcgtcca 1800
   acttcgccct gtcttcagca tcgagatatt tgccttctag aggataggga ttgacgacct 1860
   cattgcttgg cggcgacggc agcgagattt cctcttcgga gtcggagcca acgtcggcca 1920
   atgccagcag atcatcatca ctgtcactca tagtaggaag gttgaagtgt gctgacgaat 1980
   cagaatcgcg aaggatgcca ttgaaggcat atatatttta atctgtacct tttatggtaa 2040
   tttaatcaga ttttataggt attcatgtgc aagttgcatt gaaggaactg tttgagaaaa 2100
   tcatcttgac tgaacttttc tcagatatgc attccagccc gccttttggt aacgctgagc 2160
   ttcgtgcaca ggatctcgtc ccttgctata gagcccgcgt ccgacgataa taacgtctgt 2220
   gccggtctct atgacgtcgt ccacagtacg atactgctgc cccaatccat cacctttgtc 2280
   gtccaggccc accccaggag tcataatgac ccagtcttcc tctggctttc cgactttttg 2340
   ctgagcgatg aaaccaaaca caaatgcgcg gttactgcga gcgatgtcta ctgtcgcttg 2400
   cgagtattcg ccgtgagcca gtgtgccctt cgaactcagt tctgcaagca tgacaaggcc 2460
   gcgaggttca tccgtagttt ccttcgcagc ctcttctagt ccgctcacaa ttcccggccc 2520
   aggaacaccg tgagcatttg ttatatcagc ccattgagcg atcttaaaca ctccacctgc 2580
   atattgggcc ttaacagtgg aaccgatgtc tgcgaacttt cggtcttcaa aaatgagaaa 2640
   attgtgcttc gttgaaagct gtttcaaacc gctgacagtt gtgtcgtatt cgaagtcgtc 2700
   aattatgtca atgtgggtct taaccataca aatgtaaggt ccaatgcggt ccaggatact 2760
   cagtaactca gaggtagttc gcacatccaa gcttgcgcaa agatttgttt gcttgctcac 2820
   aatgatgtcg aatagccggg ctgctacagc cggcagcctc tctcggcgct cctcatagct 2880
   cagcttcata ttatttctct acagtagtgc ccgtgccctc gatcagctag gacttttcaa 2940
   attaatcggg ctgtttgatg taagtaagat gaagtcacgc gcgtgcagga gactgcgtcc 3000
   cgcgatattc tgcaggcttg aaaaatttac cctaacggta ggcatcaagt gagtgagtct 3060
   cagcgtcgat atgggtcaaa aaaggggaaa actagccgag atcgttgcga gctgtttcga 3120
   aaattatgcc ctatggcaat tatcacgtgg agtatccgaa tttctccagg ctgtcaagcg 3180
   gcaattataa ccgagactga gatcgagaag tatataaccg cagcagtagt ggataaataa 3240
   ttgcgaagtc ttcccagcag agcgggctgt tttttggagt tggttactgt aaaatgctaa 3300
   aatgactgac aacaatggag cgtctacagc attggcaaca gtgggaacag tatgctggtg 3360
   catccagttg ataccccagg ttctgcgaaa ctggtatgtt cgggattgcg agggcgttcc 3420
   tcctctgatg ttctttttgt tcgccgtttc ggggattccc ttcgcagtgt acttcattga 3480
   tcagaattcg aacactgcca tcatggttca acctcacttg tttactttct ttagccttat 3540
   aggcttttgg caaagcctgt actatccgcc cgtcagacca gcacgggccg tcacatgtat 3600
   ggttgcgtcg ctgtataaga aatcttacaa ctgaagacta cacagcgtat ccgctccgat 3660
   atcggcgatc acgtggatac atttccccag aatgcgtcaa ccttgcatgc tcgatattga 3720
   ctcaagccga gaggtgtata acaacaccga cgatagcgaa ttacttgtgg aactgatttg 3780
   ccgtatcgag taaatcgcga ttgtggccct ctttaggcct tgtacccatt tgtgcatcgt 3840
   atttgttagt atgcatcata gaattatgtg aacttagaaa agtccgtatg aaatgagcct 3900
   cagattatgg attgatcgct tgttatttgt acagcggaat tgacttatag tatgtcggcc 3960
   acggttttag attgcctagg ggccgttttc ttgatggatt cgcatcggaa ctccgaattc 4020
   ttgattgctc tccatcgcgc aggaggccgt tctttttttg acaaagtccc attttagggc 4080
   gcaggtccaa aaaataagcg gccgcttaat taactggcct catgggcctt ccgctcactg 4140
   cccgctttcc agtcgggaaa cctgtcgtgc cagctgcatt aacatggtca tagctgtttc 4200
   cttgcgtatt gggcgctctc cgcttcctcg ctcactgact cgctgcgctc ggtcgttcgg 4260
   gtaaagcctg gggtgcctaa tgagcaaaag gccagcaaaa ggccaggaac cgtaaaaagg 4320
   ccgcgttgct ggcgtttttc cataggctcc gcccccctga cgagcatcac aaaaatcgac 4380
   gctcaagtca gaggtggcga aacccgacag gactataaag ataccaggcg tttccccctg 4440
   gaagctccct cgtgcgctct cctgttccga ccctgccgct taccggatac ctgtccgcct 4500
   ttctcccttc gggaagcgtg gcgctttctc atagctcacg ctgtaggtat ctcagttcgg 4560
   tgtaggtcgt tcgctccaag ctgggctgtg tgcacgaacc ccccgttcag cccgaccgct 4620
   gcgccttatc cggtaactat cgtcttgagt ccaacccggt aagacacgac ttatcgccac 4680
   tggcagcagc cactggtaac aggattagca gagcgaggta tgtaggcggt gctacagagt 4740
   tcttgaagtg gtggcctaac tacggctaca ctagaagaac agtatttggt atctgcgctc 4800
   tgctgaagcc agttaccttc ggaaaaagag ttggtagctc ttgatccggc aaacaaacca 4860
   ccgctggtag cggtggtttt tttgtttgca agcagcagat tacgcgcaga aaaaaaggat 4920
   ctcaagaaga tcctttgatc ttttctacgg ggtctgacgc tcagtggaac gaaaactcac 4980
   gttaagggat tttggtcatg agattatcaa aaaggatctt cacctagatc cttttaaatt 5040
   aaaaatgaag ttttaaatca atctaaagta tatatgagta aacttggtct gacagttacc 5100
   aatgcttaat cagtgaggca cctatctcag cgatctgtct atttcgttca tccatagttg 5160
   cctgactccc cgtcgtgtag ataactacga tacgggaggg cttaccatct ggccccagtg 5220
   ctgcaatgat accgcgagaa ccacgctcac cggctccaga tttatcagca ataaaccagc 5280
   cagccggaag ggccgagcgc agaagtggtc ctgcaacttt atccgcctcc atccagtcta 5340
   ttaattgttg ccgggaagct agagtaagta gttcgccagt taatagtttg cgcaacgttg 5400
   ttgccattgc tacaggcatc gtggtgtcac gctcgtcgtt tggtatggct tcattcagct 5460
   ccggttccca acgatcaagg cgagttacat gatcccccat gttgtgcaaa aaagcggtta 5520
   gctccttcgg tcctccgatc gttgtcagaa gtaagttggc cgcagtgtta tcactcatgg 5580
   ttatggcagc actgcataat tctcttactg tcatgccatc cgtaagatgc ttttctgtga 5640
   ctggtgagta ctcaaccaag tcattctgag aatagtgtat gcggcgaccg agttgctctt 5700
   gcccggcgtc aatacgggat aataccgcgc cacatagcag aactttaaaa gtgctcatca 5760
   ttggaaaacg ttcttcgggg cgaaaactct caaggatctt accgctgttg agatccagtt 5820
   cgatgtaacc cactcgtgca cccaactgat cttcagcatc ttttactttc accagcgttt 5880
   ctgggtgagc aaaaacagga aggcaaaatg ccgcaaaaaa gggaataagg gcgacacgga 5940
   aatgttgaat actcatactc ttcctttttc aatattattg aagcatttat cagggttatt 6000
   gtctcatgag cggatacata tttgaatgta tttagaaaaa taaacaaata ggggttccgc 6060
   gcacatttcc ccgaaaagtg ccac 6084
<210> 65
   <211> 7693
   <212> DNA
   <213> Artificial
<220>
   <223> Vector
<400> 65
   ctaaattgta agcgttaata ttttgttaaa attcgcgtta aatttttgtt aaatcagctc      60
   attttttaac caataggccg aaatcggcaa aatcccttat aaatcaaaag aatagaccga      120
   gatagggttg agtggccgct acagggcgct cccattcgcc attcaggctg cgcaactgtt     180
   gggaagggcg tttcggtgcg ggcctcttcg ctattacgcc agctggcgaa agggggatgt     240
   gctgcaaggc gattaagttg ggtaacgcca gggttttccc agtcacgacg ttgtaaaacg     300
   acggccagtg agcgcgacgt aatacgactc actatagggc gaattggcgg aaggccgtca     360
   aggcctaggc gcgcctgcag gatcctagaa aacagctgga tatggataaa ctcggcaagc     420
   atcttttgga aaggctgcca cgctatgccg tgccgatatt cattaagttc gtcgacaccg     480
   tcaccatcac cggcaacaac aaagttcaga agaaagaatt ccgaaaccag cagattcccg     540
   ccccagcagg acaaacaatc tactggttag agggcacgag ctacaagccc ctcactgctg     600
   atgcgtgggc tcgtgtagag aatgggcgac acaagctcta atgttgaata cctcttccgt     660
   atagatgtgg cataagctat agattttgct gcaatattat taaatattaa agagtttcga     720
   aggtcagctg cggatgaacc agttcagagc ggctctctct tttttgccaa tagcgtgcaa     780
   ccgtgaagag caattcaacc atccaatctg gctacactaa attgtatttg gcagcgcact     840
   gtacgagcgc actgtacgaa actccgtcaa tttatagcag aacgcgtgcg atcgcgggcc     900
   ccagcgatat gacgagaatc aggcaataat agcttaagct gaagtgtttt tagatttagt     960
   tcggagtgcg cttctcaaaa gtgctgggat caacaagttt ttaacatggg ttttgattta    1020
   tattgtttta tatgagcgcc tcagatatgc gctgacagcc tattaggaga aatggccggc    1080
   caacttaaga aaaccgcaca accacaccgg gaggagcgtg ttgagctgta agcgttgttg    1140
   agaaacgagg ggactctggg aagtcgggac ccatctcaat cttggaatac tcctgtaaga 1200
   gtctcaccag agttagcgaa agctctgtca gggcgaattg ttggccgaga caaattcggg 1260
   gaccgccatt gaagggcaag aatgcccaca cattatctag cttcaagttc tcccatcgat 1320
   tgggattgaa ttcgtgggcg tcaggacccc aatacttgat gtccctgtgg accatgtaaa 1380
   ttgaatagta aactgcggtg cccttaggaa cgaagatcgg atccttctgc tcgggaccac 1440
   cacctatggg tagagttgta tctctcacag cagtacggaa gttcaatggc aataccggcg 1500
   caagacgcaa gacttcattt ataacttgct tcaaataagg tgcttgcttc agaagttcga 1560
   atgataaagg cctttgctcc tccttggttc caaaatgatc gaggacctcc tcacgtagtt 1620
   tgttgaatac gtcaggattt ctggcaagga aatgaatagc gaagctcaac gtagcagctg 1680
   ttgtatctct accagcaatg agaatgttga aaatttgatc acgtatcgtc actgggtctc 1740
   gggtaacttt agccatctca agcgagaaca catagatgcc actagactct gcagcagcat 1800
   ccttctctgc aatagagttc tcagcagcga aagatgtggc gtaaagagcc ttatcaacgt 1860
   agtagtcaat ataggactga gcacgtttct tgtgatctcg gaattcctta gagttgaaca 1920
   accagtagac tttgcttgat agggtccgtt tgaaagcgta attcagtaga aagttgtagg 1980
   actccacgaa ttgttcggca gtaatctccg aaccatcacg ggctacaata catgactgat 2040
   tctcagggtt caagctctcg caggactccc caaataggaa ttcagtcgct gtatccagcg 2100
   taagtttgtg gaaataatgt tgaacatcaa taaattggtc cactttcatt gcacggttca 2160
   tctcctttat taactccgca gcatgactgg aaatctgatc aattctgcaa acctgatctt 2220
   tagtgaactg aggtctcaac atcgatcgag actgtttcca tccatttccg ctgagtgtaa 2280
   atatcccttg gccaaacact tttcccactg tgtggaaacg tgctccaaga ccaaaatcat 2340
   tgaatttggt tgccaggatt gtcttaatgt tttctggctc gattgtgaag atttggtatt 2400
   gaaggggagc ttgtcgaaga tacgtccgtg ctttgaactt attgaagact ctgtcgtatt 2460
   gaacttccag taaggtgtat gacttggccg tcttgatcat gtccatggtt ctttgtattc 2520
   ccagtgggaa cgatttctca atgaagcgag gcatactaca cttgtgccta cgtgctgcat 2580
   agcggtacca taggagccag ataggctcgt gtagaactaa gaaagctacg aagagcagtg 2640
   gcaacaagcc agcaacagcg gataaactca ttggagttag aataatgtct ttgattaaca 2700
   tatgtgtaga gttgtttttg ttgttaagtc tttctttaag agcttgaccg actataaccg 2760
   ttcaacggcg cattatatac tttgggtatc ggccagtgct gacaactcac acgttgcgac 2820
   cccttaccca gaagcatacc cagcgcgatg tcgatcgtgt tatatcgtag acgcacaccc 2880
   tgcaatgacg ggtaggctct aaatcgggat gcgaaaaaga ggttgccttg ctttttgccc 2940
   tggtagatgg catgctgagc gtgcgcttgc cgcctaattt ttgtgtgtcg cctgctattt 3000
   attgctgaag ctagcccgcc gcatctttcc ccaaggcttc gattgctcgt attggggcag 3060
   ggattggtac tcaaccttgc agatgagact ccagcaacaa cgtcgtactg cttagcgatc 3120
   gcacatgttt catcatcgtc actatacaca tcgtcatcaa ctccatggcg tgaggacttc 3180
   cgagactgct gggcccttcg tttctttaat gcctcaagag atgacttcgt acccgaagag 3240
   acgcctgttg taccccgttg acgcttggcg gagggggctt cgtcctcgtc agcaacccgc 3300
   gtcatctgct tccttcgctg agcaagatac cttctctcct cgtaccgctg catctcctga 3360
   gctcggtcat acaagatctc ttctcgctca atctctggca gcgcgtccaa cttcgccctg 3420
   tcttcagcat cgagatattt gccttctaga ggatagggat tgacgacctc attgcttggc 3480
   ggcgacggca gcgagatttc ctcttcggag tcggagccaa cgtcggccaa tgccagcaga 3540
   tcatcatcac tgtcactcat agtaggaagg ttgaagtgtg ctgacgaatc agaatcgcga 3600
   aggatgccat tgaaggcata tatattttaa tctgtacctt ttatggtaat ttaatcagat 3660
   tttataggta ttcatgtgca agttgcattg aaggaactgt ttgagaaaat catcttgact 3720
   gaacttttct cagatatgca ttccagcccg ccttttggta acgctgagct tcgtgcacag 3780
   gatctcgtcc cttgctatag agcccgcgtc cgacgataat aacgtctgtg ccggtctcta 3840
   tgacgtcgtc cacagtacga tactgctgcc ccaatccatc acctttgtcg tccaggccca 3900
   ccccaggagt cataatgacc cagtcttcct ctggctttcc gactttttgc tgagcgatga 3960
   aaccaaacac aaatgcgcgg ttactgcgag cgatgtctac tgtcgcttgc gagtattcgc 4020
   cgtgagccag tgtgcccttc gaactcagtt ctgcaagcat gacaaggccg cgaggttcat 4080
   ccgtagtttc cttcgcagcc tcttctagtc cgctcacaat tcccggccca ggaacaccgt 4140
   gagcatttgt tatatcagcc cattgagcga tcttaaacac tccacctgca tattgggcct 4200
   taacagtgga accgatgtct gcgaactttc ggtcttcaaa aatgagaaaa ttgtgcttcg 4260
   ttgaaagctg tttcaaaccg ctgacagttg tgtcgtattc gaagtcgtca attatgtcaa 4320
   tgtgggtctt aaccatacaa atgtaaggtc caatgcggtc caggatactc agtaactcag 4380
   aggtagttcg cacatccaag cttgcgcaaa gatttgtttg cttgctcaca atgatgtcga 4440
   atagccgggc tgctacagcc ggcagcctct ctcggcgctc ctcatagctc agcttcatat 4500
   tatttctcta cagtagtgcc cgtgccctcg atcagctagg acttttcaaa ttaatcgggc 4560
   tgtttgatgt aagtaagatg aagtcacgcg cgtgcaggag actgcgtccc gcgatattct 4620
   gcaggcttga aaaatttacc ctaacggtag gcatcaagtg agtgagtctc agcgtcgata 4680
   tgggtcaaaa aaggggaaaa ctagccgaga tcgttgcgag ctgtttcgaa aattatgccc 4740
   tatggcaatt atcacgtgga gtatccgaat ttctccaggc tgtcaagcgg caattataac 4800
   cgagactgag atcgagaagt atataaccgc agcagtagtg gataaataat tgcgaagtct 4860
   tcccagcaga gcgggctgtt ttttggagtt ggttactgta aaatgctaaa atgactgaca 4920
   acaatggagc gtctacagca ttggcaacag tgggaacagt atgctggtgc atccagttga 4980
   taccccaggt tctgcgaaac tggtatgttc gggattgcga gggcgttcct cctctgatgt 5040
   tctttttgtt cgccgtttcg gggattccct tcgcagtgta cttcattgat cagaattcga 5100
   acactgccat catggttcaa cctcacttgt ttactttctt tagccttata ggcttttggc 5160
   aaagcctgta ctatccgccc gtcagaccag cacgggccgt cacatgtatg gttgcgtcgc 5220
   tgtataagaa atcttacaac tgaagactac acagcgtatc cgctccgata tcggcgatca 5280
   cgtggataca tttccccaga atgcgtcaac cttgcatgct cgatattgac tcaagccgag 5340
   aggtgtataa caacaccgac gatagcgaat tacttgtgga actgatttgc cgtatcgagt 5400
   aaatcgcgat tgtggccctc tttaggcctt gtacccattt gtgcatcgta tttgttagta 5460
   tgcatcatag aattatgtga acttagaaaa gtccgtatga aatgagcctc agattatgga 5520
   ttgatcgctt gttatttgta cagcggaatt gacttatagt atgtcggcca cggttttaga 5580
   ttgcctaggg gccgttttct tgatggattc gcatcggaac tccgaattct tgattgctct 5640
   ccatcgcgca ggaggccgtt ctttttttga caaagtccca ttttagggcg caggtccaaa 5700
   aaataagcgg ccgcttaatt aactggcctc atgggccttc cgctcactgc ccgctttcca 5760
   gtcgggaaac ctgtcgtgcc agctgcatta acatggtcat agctgtttcc ttgcgtattg 5820
   ggcgctctcc gcttcctcgc tcactgactc gctgcgctcg gtcgttcggg taaagcctgg 5880
   ggtgcctaat gagcaaaagg ccagcaaaag gccaggaacc gtaaaaaggc cgcgttgctg 5940
   gcgtttttcc ataggctccg cccccctgac gagcatcaca aaaatcgacg ctcaagtcag 6000
   aggtggcgaa acccgacagg actataaaga taccaggcgt ttccccctgg aagctccctc 6060
   gtgcgctctc ctgttccgac cctgccgctt accggatacc tgtccgcctt tctcccttcg 6120
   ggaagcgtgg cgctttctca tagctcacgc tgtaggtatc tcagttcggt gtaggtcgtt 6180
   cgctccaagc tgggctgtgt gcacgaaccc cccgttcagc ccgaccgctg cgccttatcc 6240
   ggtaactatc gtcttgagtc caacccggta agacacgact tatcgccact ggcagcagcc 6300
   actggtaaca ggattagcag agcgaggtat gtaggcggtg ctacagagtt cttgaagtgg 6360
   tggcctaact acggctacac tagaagaaca gtatttggta tctgcgctct gctgaagcca 6420
   gttaccttcg gaaaaagagt tggtagctct tgatccggca aacaaaccac cgctggtagc 6480
   ggtggttttt ttgtttgcaa gcagcagatt acgcgcagaa aaaaaggatc tcaagaagat 6540
   cctttgatct tttctacggg gtctgacgct cagtggaacg aaaactcacg ttaagggatt 6600
   ttggtcatga gattatcaaa aaggatcttc acctagatcc ttttaaatta aaaatgaagt 6660
   tttaaatcaa tctaaagtat atatgagtaa acttggtctg acagttacca atgcttaatc 6720
   agtgaggcac ctatctcagc gatctgtcta tttcgttcat ccatagttgc ctgactcccc 6780
   gtcgtgtaga taactacgat acgggagggc ttaccatctg gccccagtgc tgcaatgata 6840
   ccgcgagaac cacgctcacc ggctccagat ttatcagcaa taaaccagcc agccggaagg 6900
   gccgagcgca gaagtggtcc tgcaacttta tccgcctcca tccagtctat taattgttgc 6960
   cgggaagcta gagtaagtag ttcgccagtt aatagtttgc gcaacgttgt tgccattgct 7020
   acaggcatcg tggtgtcacg ctcgtcgttt ggtatggctt cattcagctc cggttcccaa 7080
   cgatcaaggc gagttacatg atcccccatg ttgtgcaaaa aagcggttag ctccttcggt 7140
   cctccgatcg ttgtcagaag taagttggcc gcagtgttat cactcatggt tatggcagca 7200
   ctgcataatt ctcttactgt catgccatcc gtaagatgct tttctgtgac tggtgagtac 7260
   tcaaccaagt cattctgaga atagtgtatg cggcgaccga gttgctcttg cccggcgtca 7320
   atacgggata ataccgcgcc acatagcaga actttaaaag tgctcatcat tggaaaacgt 7380
   tcttcggggc gaaaactctc aaggatctta ccgctgttga gatccagttc gatgtaaccc 7440
   actcgtgcac ccaactgatc ttcagcatct tttactttca ccagcgtttc tgggtgagca 7500
   aaaacaggaa ggcaaaatgc cgcaaaaaag ggaataaggg cgacacggaa atgttgaata 7560
   ctcatactct tcctttttca atattattga agcatttatc agggttattg tctcatgagc 7620
   ggatacatat ttgaatgtat ttagaaaaat aaacaaatag gggttccgcg cacatttccc 7680
   cgaaaagtgc cac 7693
<210> 66
   <211> 7465
   <212> DNA
   <213> Artificial
<220>
   <223> Vector
<400> 66
   ctaaattgta agcgttaata ttttgttaaa attcgcgtta aatttttgtt aaatcagctc     60
   attttttaac caataggccg aaatcggcaa aatcccttat aaatcaaaag aatagaccga    120
   gatagggttg agtggccgct acagggcgct cccattcgcc attcaggctg cgcaactgtt    180
   gggaagggcg tttcggtgcg ggcctcttcg ctattacgcc agctggcgaa agggggatgt    240
   gctgcaaggc gattaagttg ggtaacgcca gggttttccc agtcacgacg ttgtaaaacg    300
   acggccagtg agcgcgacgt aatacgactc actatagggc gaattggcgg aaggccgtca    360
   aggcctaggc gcgcctgcag gatcctagaa aacagctgga tatggataaa ctcggcaagc    420
   atcttttgga aaggctgcca cgctatgccg tgccgatatt cattaagttc gtcgacaccg    480
   tcaccatcac cggcaacaac aaagttcaga agaaagaatt ccgaaaccag cagattcccg    540
   ccccagcagg acaaacaatc tactggttag agggcacgag ctacaagccc ctcactgctg    600
   atgcgtgggc tcgtgtagag aatgggcgac acaagctcta atgttgaata cctcttccgt    660
   atagatgtgg cataagctat agattttgct gcaatattat taaatattaa agagtttcga    720
   aggtcagctg cggatgaacc agttcagagc ggctctctct tttttgccaa tagcgtgcaa    780
   ccgtgaagag caattcaacc atccaatctg gctacactaa attgtatttg gcagcgcact    840
   gtacgagcgc actgtacgaa actccgtcaa tttatagcag aacgcgtgcg atcgcgggcc    900
   ccagcgatat gacgagaatc aggcaataat agcttaagct gaagtgtttt tagatttagt 960
   tcggagtgcg cttctcaaaa gtgctgggat caacaagttt ttaacatggg ttttgattta 1020
   tattgtttta tatgagcgcc tcagatatgc gctgacagcc tattaggaga aatggccggc 1080
   cctaagaact caccgctaag gccggacctt tgacaggtat atcttcagtt tcctcgtcac 1140
   tcttggtcaa aagaccaaag tcatggctgg cgatttcctc gatgctttcc tcaagaattt 1200
   tcaaggagtt gtggctttcc aactccattt gaaccttctt cgaggcttcg tggaatttcg 1260
   gatttccaat tatcgaatca acagcttctt tgatttgctc cactgtaggc aagccagttt 1320
   tcaaatcaat tgccacgcca gcggcctcag ctctcgatgc caccattggc ttgtcttcag 1380
   agtcaccagc aataacaact ggaacagagt ggcttaagct gtgctgaagt ccgccatatc 1440
   caccattgta gacaagagca tcaacgtgag gaagtagagc atcgtagttg aagtagtcga 1500
   tcacgcgagc attctcagga accacaacat catccggtag cttggcaccg cggcggccca 1560
   atatggctac tgttaaagtg tcaggctcgt ccttcaaggc ctcaagagta ggcacaataa 1620
   gatgcttgta actgacagca aaagttcctt gagtgaccat gatgactcgc ttggcactca 1680
   gaacatcccc ccaccaggaa ggaggggtga attgagttcg gtgcttgggc gttgagccgg 1740
   cgaatttgaa gttgctaggc agatggtctc tgctgaactc aagagaaggc gggcacagct 1800
   gcaggaactt gtctgcagca atgtaactgt gctcccagat aaatttggga tcttcagtgc 1860
   aacctaactc tcggcagatt tccttgtgct tagcagtggc tttaacgaaa atttggtgct 1920
   caagagcgtg gttcatagcg agtttctttg catgtgcttc ggggctcctg tcgttgtcaa 1980
   gtcctaaggt atgatcactg cggatcaaaa gaggcaaaac ccctaaacaa atccagccag 2040
   cgggtttgaa accaggagca ccgaggctga tagggtgtgc accgaaaaac agcacttcac 2100
   tgacaagaac gacagggcga ccgcttgcgc tgagcttttt gaaagccctc tgaatagcgg 2160
   caaactgctc aggaagagta gctaccatca tgtgctccac atcttgaact gtacgatcga 2220
   agcttggggc catgtcttta cggcccggga ccagatcgtc taaggtgtgg tcatcaaaat 2280
   ctgcgttccc ttctaaagga acaaagtctg cacccacatc tcgaactttt tgttcaaacg 2340
   ctctgcctgt cacaacagta gcttcgtatc cgtcgtccgt aaggccgtgt accagactca 2400
   aaacgggcat tatatggcct gaaagaggca agccgcaagc gagaatcagg ggtttgtgtg 2460
   atgaagggct catatgtgta gagttgtttt tgttgttaag tctttcttta agagcttgac 2520
   cgactataac cgttcaacgg cgcattatat actttgggta tcggccagtg ctgacaactc 2580
   acacgttgcg accccttacc cagaagcata cccagcgcga tgtcgatcgt gttatatcgt 2640
   agacgcacac cctgcaatga cgggtaggct ctaaatcggg atgcgaaaaa gaggttgcct 2700
   tgctttttgc cctggtagat ggcatgctga gcgtgcgctt gccgcctaat ttttgtgtgt 2760
   cgcctgctat ttattgctga agctagcccg ccgcatcttt ccccaaggct tcgattgctc 2820
   gtattggggc agggattggt actcaacctt gcagatgaga ctccagcaac aacgtcgtac 2880
   tgcttagcga tcgcacatgt ttcatcatcg tcactataca catcgtcatc aactccatgg 2940
   cgtgaggact tccgagactg ctgggccctt cgtttcttta atgcctcaag agatgacttc 3000
   gtacccgaag agacgcctgt tgtaccccgt tgacgcttgg cggagggggc ttcgtcctcg 3060
   tcagcaaccc gcgtcatctg cttccttcgc tgagcaagat accttctctc ctcgtaccgc 3120
   tgcatctcct gagctcggtc atacaagatc tcttctcgct caatctctgg cagcgcgtcc 3180
   aacttcgccc tgtcttcagc atcgagatat ttgccttcta gaggataggg attgacgacc 3240
   tcattgcttg gcggcgacgg cagcgagatt tcctcttcgg agtcggagcc aacgtcggcc 3300
   aatgccagca gatcatcatc actgtcactc atagtaggaa ggttgaagtg tgctgacgaa 3360
   tcagaatcgc gaaggatgcc attgaaggca tatatatttt aatctgtacc ttttatggta 3420
   atttaatcag attttatagg tattcatgtg caagttgcat tgaaggaact gtttgagaaa 3480
   atcatcttga ctgaactttt ctcagatatg cattccagcc cgccttttgg taacgctgag 3540
   cttcgtgcac aggatctcgt cccttgctat agagcccgcg tccgacgata ataacgtctg 3600
   tgccggtctc tatgacgtcg tccacagtac gatactgctg ccccaatcca tcacctttgt 3660
   cgtccaggcc caccccagga gtcataatga cccagtcttc ctctggcttt ccgacttttt 3720
   gctgagcgat gaaaccaaac acaaatgcgc ggttactgcg agcgatgtct actgtcgctt 3780
   gcgagtattc gccgtgagcc agtgtgccct tcgaactcag ttctgcaagc atgacaaggc 3840
   cgcgaggttc atccgtagtt tccttcgcag cctcttctag tccgctcaca attcccggcc 3900
   caggaacacc gtgagcattt gttatatcag cccattgagc gatcttaaac actccacctg 3960
   catattgggc cttaacagtg gaaccgatgt ctgcgaactt tcggtcttca aaaatgagaa 4020
   aattgtgctt cgttgaaagc tgtttcaaac cgctgacagt tgtgtcgtat tcgaagtcgt 4080
   caattatgtc aatgtgggtc ttaaccatac aaatgtaagg tccaatgcgg tccaggatac 4140
   tcagtaactc agaggtagtt cgcacatcca agcttgcgca aagatttgtt tgcttgctca 4200
   caatgatgtc gaatagccgg gctgctacag ccggcagcct ctctcggcgc tcctcatagc 4260
   tcagcttcat attatttctc tacagtagtg cccgtgccct cgatcagcta ggacttttca 4320
   aattaatcgg gctgtttgat gtaagtaaga tgaagtcacg cgcgtgcagg agactgcgtc 4380
   ccgcgatatt ctgcaggctt gaaaaattta ccctaacggt aggcatcaag tgagtgagtc 4440
   tcagcgtcga tatgggtcaa aaaaggggaa aactagccga gatcgttgcg agctgtttcg 4500
   aaaattatgc cctatggcaa ttatcacgtg gagtatccga atttctccag gctgtcaagc 4560
   ggcaattata accgagactg agatcgagaa gtatataacc gcagcagtag tggataaata 4620
   attgcgaagt cttcccagca gagcgggctg ttttttggag ttggttactg taaaatgcta 4680
   aaatgactga caacaatgga gcgtctacag cattggcaac agtgggaaca gtatgctggt 4740
   gcatccagtt gataccccag gttctgcgaa actggtatgt tcgggattgc gagggcgttc 4800
   ctcctctgat gttctttttg ttcgccgttt cggggattcc cttcgcagtg tacttcattg 4860
   atcagaattc gaacactgcc atcatggttc aacctcactt gtttactttc tttagcctta 4920
   taggcttttg gcaaagcctg tactatccgc ccgtcagacc agcacgggcc gtcacatgta 4980
   tggttgcgtc gctgtataag aaatcttaca actgaagact acacagcgta tccgctccga 5040
   tatcggcgat cacgtggata catttcccca gaatgcgtca accttgcatg ctcgatattg 5100
   actcaagccg agaggtgtat aacaacaccg acgatagcga attacttgtg gaactgattt 5160
   gccgtatcga gtaaatcgcg attgtggccc tctttaggcc ttgtacccat ttgtgcatcg 5220
   tatttgttag tatgcatcat agaattatgt gaacttagaa aagtccgtat gaaatgagcc 5280
   tcagattatg gattgatcgc ttgttatttg tacagcggaa ttgacttata gtatgtcggc 5340
   cacggtttta gattgcctag gggccgtttt cttgatggat tcgcatcgga actccgaatt 5400
   cttgattgct ctccatcgcg caggaggccg ttcttttttt gacaaagtcc cattttaggg 5460
   cgcaggtcca aaaaataagc ggccgcttaa ttaactggcc tcatgggcct tccgctcact 5520
   gcccgctttc cagtcgggaa acctgtcgtg ccagctgcat taacatggtc atagctgttt 5580
   ccttgcgtat tgggcgctct ccgcttcctc gctcactgac tcgctgcgct cggtcgttcg 5640
   ggtaaagcct ggggtgccta atgagcaaaa ggccagcaaa aggccaggaa ccgtaaaaag 5700
   gccgcgttgc tggcgttttt ccataggctc cgcccccctg acgagcatca caaaaatcga 5760
   cgctcaagtc agaggtggcg aaacccgaca ggactataaa gataccaggc gtttccccct 5820
   ggaagctccc tcgtgcgctc tcctgttccg accctgccgc ttaccggata cctgtccgcc 5880
   tttctccctt cgggaagcgt ggcgctttct catagctcac gctgtaggta tctcagttcg 5940
   gtgtaggtcg ttcgctccaa gctgggctgt gtgcacgaac cccccgttca gcccgaccgc 6000
   tgcgccttat ccggtaacta tcgtcttgag tccaacccgg taagacacga cttatcgcca 6060
   ctggcagcag ccactggtaa caggattagc agagcgaggt atgtaggcgg tgctacagag 6120
   ttcttgaagt ggtggcctaa ctacggctac actagaagaa cagtatttgg tatctgcgct 6180
   ctgctgaagc cagttacctt cggaaaaaga gttggtagct cttgatccgg caaacaaacc 6240
   accgctggta gcggtggttt ttttgtttgc aagcagcaga ttacgcgcag aaaaaaagga 6300
   tctcaagaag atcctttgat cttttctacg gggtctgacg ctcagtggaa cgaaaactca 6360
   cgttaaggga ttttggtcat gagattatca aaaaggatct tcacctagat ccttttaaat 6420
   taaaaatgaa gttttaaatc aatctaaagt atatatgagt aaacttggtc tgacagttac 6480
   caatgcttaa tcagtgaggc acctatctca gcgatctgtc tatttcgttc atccatagtt 6540
   gcctgactcc ccgtcgtgta gataactacg atacgggagg gcttaccatc tggccccagt 6600
   gctgcaatga taccgcgaga cccacgctca ccggctccag atttatcagc aataaaccag 6660
   ccagccggaa gggccgagcg cagaagtggt cctgcaactt tatccgcctc catccagtct 6720
   attaattgtt gccgggaagc tagagtaagt agttcgccag ttaatagttt gcgcaacgtt 6780
   gttgccattg ctacaggcat cgtggtgtca cgctcgtcgt ttggtatggc ttcattcagc 6840
   tccggttccc aacgatcaag gcgagttaca tgatccccca tgttgtgcaa aaaagcggtt 6900
   agctccttcg gtcctccgat cgttgtcaga agtaagttgg ccgcagtgtt atcactcatg 6960
   gttatggcag cactgcataa ttctcttact gtcatgccat ccgtaagatg cttttctgtg 7020
   actggtgagt actcaaccaa gtcattctga gaatagtgta tgcggcgacc gagttgctct 7080
   tgcccggcgt caatacggga taataccgcg ccacatagca gaactttaaa agtgctcatc 7140
   attggaaaac gttcttcggg gcgaaaactc tcaaggatct taccgctgtt gagatccagt 7200
   tcgatgtaac ccactcgtgc acccaactga tcttcagcat cttttacttt caccagcgtt 7260
   tctgggtgag caaaaacagg aaggcaaaat gccgcaaaaa agggaataag ggcgacacgg 7320
   aaatgttgaa tactcatact cttccttttt caatattatt gaagcattta tcagggttat 7380
   tgtctcatga gcggatacat atttgaatgt atttagaaaa ataaacaaat aggggttccg 7440
   cgcacatttc cccgaaaagt gccac 7465
<210> 67
   <211> 6856
   <212> DNA
   <213> Artificial
<220>
   <223> Vector
<400> 67
   ctaaattgta agcgttaata ttttgttaaa attcgcgtta aatttttgtt aaatcagctc      60
   attttttaac caataggccg aaatcggcaa aatcccttat aaatcaaaag aatagaccga      120
   gatagggttg agtggccgct acagggcgct cccattcgcc attcaggctg cgcaactgtt     180
   gggaagggcg tttcggtgcg ggcctcttcg ctattacgcc agctggcgaa agggggatgt     240
   gctgcaaggc gattaagttg ggtaacgcca gggttttccc agtcacgacg ttgtaaaacg     300
   acggccagtg agcgcgacgt aatacgactc actatagggc gaattggcgg aaggccgtca     360
   aggcctaggc gcgcctgcag gatcctagaa aacagctgga tatggataaa ctcggcaagc     420
   atcttttgga aaggctgcca cgctatgccg tgccgatatt cattaagttc gtcgacaccg     480
   tcaccatcac cggcaacaac aaagttcaga agaaagaatt ccgaaaccag cagattcccg     540
   ccccagcagg acaaacaatc tactggttag agggcacgag ctacaagccc ctcactgctg     600
   atgcgtgggc tcgtgtagag aatgggcgac acaagctcta atgttgaata cctcttccgt     660
   atagatgtgg cataagctat agattttgct gcaatattat taaatattaa agagtttcga     720
   aggtcagctg cggatgaacc agttcagagc ggctctctct tttttgccaa tagcgtgcaa     780
   ccgtgaagag caattcaacc atccaatctg gctacactaa attgtatttg gcagcgcact     840
   gtacgagcgc actgtacgaa actccgtcaa tttatagcag aacgcgtgcg atcgcgggcc     900
   ccagcgatat gacgagaatc aggcaataat agcttaagct gaagtgtttt tagatttagt 960
   tcggagtgcg cttctcaaaa gtgctgggat caacaagttt ttaacatggg ttttgattta 1020
   tattgtttta tatgagcgcc tcagatatgc gctgacagcc tattaggaga aatggccggc 1080
   ctacctagac cttctggtta gcggtattga cgttcatttc aactggaaga aggaattcca 1140
   gttcctctcc ttcagcctcg tcgggatcct cctctggaat atgcttgagg attcgcgcag 1200
   ggactcctcc caccacagta cgaggaggaa catcttctcg aacgacagca ccagccgcaa 1260
   ttgttgagcc atctccaatc gtaacacccg gcaggacagt cacattcgca ccaatccata 1320
   cattattccc caccttgata ggaagagcat acacaattct cctcgcacgt ttctcggggc 1380
   taataggatg agtcgcagtc acgaacgttg tattgggccc tacaatcacc tcatcaccaa 1440
   agattattgg agccgagtcc aagaagcaaa cgttgaagtt ggcgtaaaag tgctcgccta 1500
   cgctgatgtt gaatccaaaa tcaactgaga atggagcggt cagccagaca atatcctttg 1560
   tttgaccaaa agtgtctttg agaatctcga ccttcttgat ataagcagcg tgatttgact 1620
   caaaagtacg actttcactt gcaatggtat tgaactccct aactttctca ctagtagcca 1680
   gggctctaaa cataagatct ggatcgtatg gattgtaagg aactcctgag accatcttct 1740
   catagttttc attgccaggg gtgtttttga ggtttttttt ggcccaagag accatttcct 1800
   ggtcaatttc ttttctagga gtcattcctt tgttttgagg gtccttcgag gagtttacaa 1860
   ccatatgtgt agagttgttt ttgttgttaa gtctttcttt aagagcttga ccgactataa 1920
   ccgttcaacg gcgcattata tactttgggt atcggccagt gctgacaact cacacgttgc 1980
   gaccccttac ccagaagcat acccagcgcg atgtcgatcg tgttatatcg tagacgcaca 2040
   ccctgcaatg acgggtaggc tctaaatcgg gatgcgaaaa agaggttgcc ttgctttttg 2100
   ccctggtaga tggcatgctg agcgtgcgct tgccgcctaa tttttgtgtg tcgcctgcta 2160
   tttattgctg aagctagccc gccgcatctt tccccaaggc ttcgattgct cgtattgggg 2220
   cagggattgg tactcaacct tgcagatgag actccagcaa caacgtcgta ctgcttagcg 2280
   atcgcacatg tttcatcatc gtcactatac acatcgtcat caactccatg gcgtgaggac 2340
   ttccgagact gctgggccct tcgtttcttt aatgcctcaa gagatgactt cgtacccgaa 2400
   gagacgcctg ttgtaccccg ttgacgcttg gcggaggggg cttcgtcctc gtcagcaacc 2460
   cgcgtcatct gcttccttcg ctgagcaaga taccttctct cctcgtaccg ctgcatctcc 2520
   tgagctcggt catacaagat ctcttctcgc tcaatctctg gcagcgcgtc caacttcgcc 2580
   ctgtcttcag catcgagata tttgccttct agaggatagg gattgacgac ctcattgctt 2640
   ggcggcgacg gcagcgagat ttcctcttcg gagtcggagc caacgtcggc caatgccagc 2700
   agatcatcat cactgtcact catagtagga aggttgaagt gtgctgacga atcagaatcg 2760
   cgaaggatgc cattgaaggc atatatattt taatctgtac cttttatggt aatttaatca 2820
   gattttatag gtattcatgt gcaagttgca ttgaaggaac tgtttgagaa aatcatcttg 2880
   actgaacttt tctcagatat gcattccagc ccgccttttg gtaacgctga gcttcgtgca 2940
   caggatctcg tcccttgcta tagagcccgc gtccgacgat aataacgtct gtgccggtct 3000
   ctatgacgtc gtccacagta cgatactgct gccccaatcc atcacctttg tcgtccaggc 3060
   ccaccccagg agtcataatg acccagtctt cctctggctt tccgactttt tgctgagcga 3120
   tgaaaccaaa cacaaatgcg cggttactgc gagcgatgtc tactgtcgct tgcgagtatt 3180
   cgccgtgagc cagtgtgccc ttcgaactca gttctgcaag catgacaagg ccgcgaggtt 3240
   catccgtagt ttccttcgca gcctcttcta gtccgctcac aattcccggc ccaggaacac 3300
   cgtgagcatt tgttatatca gcccattgag cgatcttaaa cactccacct gcatattggg 3360
   ccttaacagt ggaaccgatg tctgcgaact ttcggtcttc aaaaatgaga aaattgtgct 3420
   tcgttgaaag ctgtttcaaa ccgctgacag ttgtgtcgta ttcgaagtcg tcaattatgt 3480
   caatgtgggt cttaaccata caaatgtaag gtccaatgcg gtccaggata ctcagtaact 3540
   cagaggtagt tcgcacatcc aagcttgcgc aaagatttgt ttgcttgctc acaatgatgt 3600
   cgaatagccg ggctgctaca gccggcagcc tctctcggcg ctcctcatag ctcagcttca 3660
   tattatttct ctacagtagt gcccgtgccc tcgatcagct aggacttttc aaattaatcg 3720
   ggctgtttga tgtaagtaag atgaagtcac gcgcgtgcag gagactgcgt cccgcgatat 3780
   tctgcaggct tgaaaaattt accctaacgg taggcatcaa gtgagtgagt ctcagcgtcg 3840
   atatgggtca aaaaagggga aaactagccg agatcgttgc gagctgtttc gaaaattatg 3900
   ccctatggca attatcacgt ggagtatccg aatttctcca ggctgtcaag cggcaattat 3960
   aaccgagact gagatcgaga agtatataac cgcagcagta gtggataaat aattgcgaag 4020
   tcttcccagc agagcgggct gtttttggga gttggttact gtaaaatgct aaaatgactg 4080
   acaacaatgg agcgtctaca gcattggcaa cagtgggaac agtatgctgg tgcatccagt 4140
   tgatacccca ggttctgcga aactggtatg ttcgggattg cgagggcgtt cctcctctga 4200
   tgttcttttt gttcgccgtt tcggggattc ccttcgcagt gtacttcatt gatcagaatt 4260
   cgaacactgc catcatggtt caacctcact tgtttacttt ctttagcctt ataggctttt 4320
   ggcaaagcct gtactatccg cccgtcagac cagcacgggc cgtcacatgt atggttgcgt 4380
   cgctgtataa gaaatcttac aactgaagac tacacagcgt atccgctccg atatcggcga 4440
   tcacgtggat acatttcccc agaatgcgtc aaccttgcat gctcgatatt gactcaagcc 4500
   gagaggtgta taacaacacc gacgatagcg aattacttgt ggaactgatt tgccgtatcg 4560
   agtaaatcgc gattgtggcc ctctttaggc cttgtaccca tttgtgcatc gtatttgtta 4620
   gtatgcatca tagaattatg tgaacttaga aaagtccgta tgaaatgagc ctcagattat 4680
   ggattgatcg cttgttattt gtacagcgga attgacttat agtatgtcgg ccacggtttt 4740
   agattgccta ggggccgttt tcttgatgga ttcgcatcgg aactccgaat tcttgattgc 4800
   tctccatcgc gcaggaggcc gttctttttt tgacaaagtc ccattttagg gcgcaggtcc 4860
   aaaaaataag cggccgctta attaactggc ctcatgggcc ttccgctcac tgcccgcttt 4920
   ccagtcggga aacctgtcgt gccagctgca ttaacatggt catagctgtt tccttgcgta 4980
   ttgggcgctc tccgcttcct cgctcactga ctcgctgcgc tcggtcgttc gggtaaagcc 5040
   tggggtgcct aatgagcaaa aggccagcaa aaggccagga accgtaaaaa ggccgcgttg 5100
   ctggcgtttt tccataggct ccgcccccct gacgagcatc acaaaaatcg acgctcaagt 5160
   cagaggtggc gaaacccgac aggactataa agataccagg cgtttccccc tggaagctcc 5220
   ctcgtgcgct ctcctgttcc gaccctgccg cttaccggat acctgtccgc ctttctccct 5280
   tcgggaagcg tggcgctttc tcatagctca cgctgtaggt atctcagttc ggtgtaggtc 5340
   gttcgctcca agctgggctg tgtgcacgaa ccccccgttc agcccgaccg ctgcgcctta 5400
   tccggtaact atcgtcttga gtccaacccg gtaagacacg acttatcgcc actggcagca 5460
   gccactggta acaggattag cagagcgagg tatgtaggcg gtgctacaga gttcttgaag 5520
   tggtggccta actacggcta cactagaaga acagtatttg gtatctgcgc tctgctgaag 5580
   ccagttacct tcggaaaaag agttggtagc tcttgatccg gcaaacaaac caccgctggt 5640
   agcggtggtt tttttgtttg caagcagcag attacgcgca gaaaaaaagg atctcaagaa 5700
   gatcctttga tcttttctac ggggtctgac gctcagtgga acgaaaactc acgttaaggg 5760
   attttggtca tgagattatc aaaaaggatc ttcacctaga tccttttaaa ttaaaaatga 5820
   agttttaaat caatctaaag tatatatgag taaacttggt ctgacagtta ccaatgctta 5880
   atcagtgagg cacctatctc agcgatctgt ctatttcgtt catccatagt tgcctgactc 5940
   cccgtcgtgt agataactac gatacgggag ggcttaccat ctggccccag tgctgcaatg 6000
   ataccgcgag aaccacgctc accggctcca gatttatcag caataaacca gccagccgga 6060
   agggccgagc gcagaagtgg tcctgcaact ttatccgcct ccatccagtc tattaattgt 6120
   tgccgggaag ctagagtaag tagttcgcca gttaatagtt tgcgcaacgt tgttgccatt 6180
   gctacaggca tcgtggtgtc acgctcgtcg tttggtatgg cttcattcag ctccggttcc 6240
   caacgatcaa ggcgagttac atgatccccc atgttgtgca aaaaagcggt tagctccttc 6300
   ggtcctccga tcgttgtcag aagtaagttg gccgcagtgt tatcactcat ggttatggca 6360
   gcactgcata attctcttac tgtcatgcca tccgtaagat gcttttctgt gactggtgag 6420
   tactcaacca agtcattctg agaatagtgt atgcggcgac cgagttgctc ttgcccggcg 6480
   tcaatacggg ataataccgc gccacatagc agaactttaa aagtgctcat cattggaaaa 6540
   cgttcttcgg ggcgaaaact ctcaaggatc ttaccgctgt tgagatccag ttcgatgtaa 6600
   cccactcgtg cacccaactg atcttcagca tcttttactt tcaccagcgt ttctgggtga 6660
   gcaaaaacag gaaggcaaaa tgccgcaaaa aagggaataa gggcgacacg gaaatgttga 6720
   atactcatac tcttcctttt tcaatattat tgaagcattt atcagggtta ttgtctcatg 6780
   agcggataca tatttgaatg tatttagaaa aataaacaaa taggggttcc gcgcacattt 6840
   ccccgaaaag tgccac 6856
<210> 68
   <211> 9973
   <212> DNA
   <213> Artificial
<220>
   <223> Vector
<400> 68
   ctaaattgta agcgttaata ttttgttaaa attcgcgtta aatttttgtt aaatcagctc 60
   attttttaac caataggccg aaatcggcaa aatcccttat aaatcaaaag aatagaccga 120
   gatagggttg agtggccgct acagggcgct cccattcgcc attcaggctg cgcaactgtt 180
   gggaagggcg tttcggtgcg ggcctcttcg ctattacgcc agctggcgaa agggggatgt 240
   gctgcaaggc gattaagttg ggtaacgcca gggttttccc agtcacgacg ttgtaaaacg 300
   acggccagtg agcgcgacgt aatacgactc actatagggc gaattggcgg aaggccgtca 360
   aggcctaggc gcgcctgcag gatcctagaa aacagctgga tatggataaa ctcggcaagc 420
   atcttttgga aaggctgcca cgctatgccg tgccgatatt cattaagttc gtcgacaccg 480
   tcaccatcac cggcaacaac aaagttcaga agaaagaatt ccgaaaccag cagattcccg 540
   ccccagcagg acaaacaatc tactggttag agggcacgag ctacaagccc ctcactgctg 600
   atgcgtgggc tcgtgtagag aatgggcgac acaagctcta atgttgaata cctcttccgt 660
   atagatgtgg cataagctat agattttgct gcaatattat taaatattaa agagtttcga 720
   aggtcagctg cggatgaacc agttcagagc ggctctctct tttttgccaa tagcgtgcaa 780
   ccgtgaagag caattcaacc atccaatctg gctacactaa attgtatttg gcagcgcact 840
   gtacgagcgc actgtacgaa actccgtcaa tttatagcag aacgcgtgcg atcgcgggcc 900
   ccagcgatat gacgagaatc aggcaataat agcttaagct gaagtgtttt tagatttagt 960
   tcggagtgcg cttctcaaaa gtgctgggat caacaagttt ttaacatggg ttttgattta 1020
   tattgtttta tatgagcgcc tcagatatgc gctgacagcc tattaggaga aatggccggc 1080
   ctcaaatctc tccgagacct tgcaagttca ccaattcagc gtaccatcca ttgagttcaa 1140
   ggaggctctg atggtcgccc tgctccacga tgcgccctcc tgagaacaca tatatgacat 1200
   ctgctttctg aattgttgat aatctatgcg caacggcgat tgtagtacgg cccttcgctg 1260
   ctgcgtcgag tgctgcttga actactttct cagattcgga atccagagct gaggtggcct 1320
   catcgaggag gagtaccttt ggatttctga tcagggccct tgcaattgca attcgctgct 1380
   tttgcccccc agatagcaac gatcccctag atccgctgag cgtttcgtag ccatcaggca 1440
   acgacatgat gaattcgtga atgttcgctt tgcgagcggc atcctcaatc atctcctgcg 1500
   ttacttcaga ctcagggcca gaccatccca ttagaatatt ctcacgtagc gtgcctgaat 1560
   aaagcattgg ttcttgctgg actaaagcaa tgtgtgatct caatgcattc aggttatatt 1620
   cgcgtaaatc tttcccatcg aaaagtactt gacctgctaa tggatcataa aatctttcca 1680
   ccagtccaat agtagtagac ttaccgcatc cactggctcc aactagagcg atgtattggc 1740
   cctttttgac tgttaagttg agatcttgta aaactggtac ttgaggtcga gtaggatatc 1800
   ggaaattcac atgacggaac tcaatatctc ctctcaccga ctcctcggga gcaacgtaac 1860
   cttcctcact ccatacatct atagaaggag tggcagtcaa gattctgtaa atgttacgcg 1920
   ctgcatcttt ggctgagttc atgtttggag catagctgaa aatttggcca gcggcttgag 1980
   aacctgtaat aatagccatg aagacagtca tatatcctgc gaccgaagct tcacctcgtc 2040
   tcattacagt gcttccccac caaaaaacga gggctaccac ccagggtgtc attccttccg 2100
   agagtgcgta gtacaatgct gagcgggcaa tggcaattct ggagctgaaa atctgagagt 2160
   ctactgtctt tgtgtatttt acgaccacgt ctaactcacg agttaaggac tggactgtgc 2220
   ggacagcact tgtatactca gatgccatgg agccacttcg ttcgtaaact tctctcgcac 2280
   gatccgataa ttgggtaaga acccagactc tgacgaagcc acacaccaac atgacaggaa 2340
   caacagacgt agccacgagt ccaattctcc aattgaaagg tataccagta actatgccgc 2400
   caatcaaggt caccagactc tgttgaattt gaccgagggt ggccccactc aaaccctcga 2460
   tcattttagc ttccttcgcc aaaattgagg ttagcgcacc cggcgtgttg tttttgtggt 2520
   cgaagaatgc aatatccatt cgcatcaatt ggcggaacaa agctaatctg atatttttga 2580
   ccaacttatc agatgcaagt gataaagcag ctatagtgat aaaagccgtc atgaatgaaa 2640
   tgcagcctac gaaaaaatac caccatccca tgatattcac cacatgccgc atttttccgt 2700
   attcactggg aggtagaacc atgcttccag tggtttggcc agttattatt gccattgcag 2760
   gatagcaata gcccaaaata atggaggcta aactaccaat gagaatgtaa ccccattctt 2820
   tcctattcag cccccaaacc agtttggtat tggtcatcaa cgtgctatgt ggggggttgc 2880
   gcacaccagg gatgtcattt tcttgatatt caggaggttg agtggtctga gtacctgcac 2940
   tgtgaacact caatgtgctc acatccttgg gattgaactt ttcgttcagt gagtccagag 3000
   gcgaaatgtc tagagcttca atatcgagga cctcaacgtt agtgctcttt gctttagtta 3060
   ctctttgagc atcaaccaaa gctttataag gcccttctcg ctgtatgagc tcattgtgag 3120
   taccctgctc tatgacgtta cctttagaca tgacaactat cttgttggca tccttgatcg 3180
   tagagagtct gtgtgcaacg actatagtgg tacgaccttc ggccgctttg tcgagcgcat 3240
   cttgaacgat accttcagat ttggtatcca gagcagaagt cgcttcatcg agcagcagaa 3300
   ttttagggtc tgagacgatt gctcttgcta ttgcaatgcg ttgtttctga ccaccgctga 3360
   gaagaaatcc tcgatctcca acattggttt ggatgccttc tgagagagtc tgaatgaaat 3420
   cccaggcatt ggcatcttta caagcttgaa tgattttagc ttccttaaca tgctcgtcag 3480
   cgaactcaat gtcagtgcca atcaaaccat agctgatatt ctcatatatt gactctgaaa 3540
   agagtactgg ttcctgctga acataaccaa tttgttgacg gagccatctt gtgttcaggt 3600
   cgctaatctc ctggccatcc agagtaacgc ttccttcgag aggtaaatag aacctctcaa 3660
   gaatacctac aattgtagac ttccctgatc ccgaggcacc taccagtgcc acagtagatc 3720
   cagcaggaac ttcaaggcta aaatcggaga ggaccaaaac gtctgggcga ctaggatatc 3780
   ggaacttgac atttttgagc tcaattctgc caacggcctt agtttggggg acaattcctt 3840
   tatctatgga ctggccatcg atgactggga cacgatcaat ggcctcattg agaatgctcg 3900
   cggcagtgag acccttgaca agaaacctca cgtttggcgc gatattccca agctggaagc 3960
   ttccaagtaa catagctgtg attacaacta ttatctttcc aacgtcagca ctcccactaa 4020
   cgatttctct ggaaccctgc cacagagcta aggcatacac ccaaaaagta ctagcccaaa 4080
   tgcacgctaa catgaccccc aatgagtaac tgctccgctt cgattccttc acaacacgat 4140
   caagtacctt ttcatacttg acggcgagat gaggttgagc gccaaatgct actgtagtcc 4200
   tgacagcact gagagcctcc tccgcaacgg tagctccaga ctgcgaatat atcgcgtcag 4260
   atctgagctg atatttggcc atgaaggtgg cgccagttcc cattgtgatt accatgaacc 4320
   ctacagcact caggaggatg caagccagtt tccattgcga agcaaaactt ataacggtgg 4380
   ccgcaatgaa ggaagctatt ccctgtacga cgtttccaag cttgtcgctg atcgcttcct 4440
   gaattgagtt ggtatcgtta atgattctgg tgctgacctc gccaccacct agtttgtcgt 4500
   aaaacgcgat attctggcga ataacagcac tcagataatg ctttcggtaa cgtcctgcca 4560
   acacttcgcc tctgtccaca agcaggaagc tctcgagaaa cgcactgccg agcataccaa 4620
   tgccaatata gacaaaatag agagacaggt gattcacctt atgctggaac tcattgccct 4680
   tgaggtcata gctagtgaag tctctgaatg tgttgaagat ggcgcccact actaacgtga 4740
   acattggaag cgcggctcca tgcaccgctg caaaaaaaag cgcaagtatc tccaagaaaa 4800
   cgtcaagggg agtgcaaaat ctgaacaacc tgaaaaagct tgtggcgact ctctttgttt 4860
   caagctgact tcgcaataca ttggcctcat gtggatctaa cgcagagagc ttctcctcga 4920
   gaagcttgtc cttagtctcg atgagtttct cacgcttctc tacctgtata tcatccacca 4980
   tatgtgtaga gttgtttttg ttgttaagtc tttctttaag agcttgaccg actataaccg 5040
   ttcaacggcg cattatatac tttgggtatc ggccagtgct gacaactcac acgttgcgac 5100
   cccttaccca gaagcatacc cagcgcgatg tcgatcgtgt tatatcgtag acgcacaccc 5160
   tgcaatgacg ggtaggctct aaatcgggat gcgaaaaaga ggttgccttg ctttttgccc 5220
   tggtagatgg catgctgagc gtgcgcttgc cgcctaattt ttgtgtgtcg cctgctattt 5280
   attgctgaag ctagcccgcc gcatctttcc ccaaggcttc gattgctcgt attggggcag 5340
   ggattggtac tcaaccttgc agatgagact ccagcaacaa cgtcgtactg cttagcgatc 5400
   gcacatgttt catcatcgtc actatacaca tcgtcatcaa ctccatggcg tgaggacttc 5460
   cgagactgct gggcccttcg tttctttaat gcctcaagag atgacttcgt acccgaagag 5520
   acgcctgttg taccccgttg acgcttggcg gagggggctt cgtcctcgtc agcaacccgc 5580
   gtcatctgct tccttcgctg agcaagatac cttctctcct cgtaccgctg catctcctga 5640
   gctcggtcat acaagatctc ttctcgctca atctctggca gcgcgtccaa cttcgccctg 5700
   tcttcagcat cgagatattt gccttctaga ggatagggat tgacgacctc attgcttggc 5760
   ggcgacggca gcgagatttc ctcttcggag tcggagccaa cgtcggccaa tgccagcaga 5820
   tcatcatcac tgtcactcat agtaggaagg ttgaagtgtg ctgacgaatc agaatcgcga 5880
   aggatgccat tgaaggcata tatattttaa tctgtacctt ttatggtaat ttaatcagat 5940
   tttataggta ttcatgtgca agttgcattg aaggaactgt ttgagaaaat catcttgact 6000
   gaacttttct cagatatgca ttccagcccg ccttttggta acgctgagct tcgtgcacag 6060
   gatctcgtcc cttgctatag agcccgcgtc cgacgataat aacgtctgtg ccggtctcta 6120
   tgacgtcgtc cacagtacga tactgctgcc ccaatccatc acctttgtcg tccaggccca 6180
   ccccaggagt cataatgacc cagtcttcct ctggctttcc gactttttgc tgagcgatga 6240
   aaccaaacac aaatgcgcgg ttactgcgag cgatgtctac tgtcgcttgc gagtattcgc 6300
   cgtgagccag tgtgcccttc gaactcagtt ctgcaagcat gacaaggccg cgaggttcat 6360
   ccgtagtttc cttcgcagcc tcttctagtc cgctcacaat tcccggccca ggaacaccgt 6420
   gagcatttgt tatatcagcc cattgagcga tcttaaacac tccacctgca tattgggcct 6480
   taacagtgga accgatgtct gcgaactttc ggtcttcaaa aatgagaaaa ttgtgcttcg 6540
   ttgaaagctg tttcaaaccg ctgacagttg tgtcgtattc gaagtcgtca attatgtcaa 6600
   tgtgggtctt aaccatacaa atgtaaggtc caatgcggtc caggatactc agtaactcag 6660
   aggtagttcg cacatccaag cttgcgcaaa gatttgtttg cttgctcaca atgatgtcga 6720
   atagccgggc tgctacagcc ggcagcctct ctcggcgctc ctcatagctc agcttcatat 6780
   tatttctcta cagtagtgcc cgtgccctcg atcagctagg acttttcaaa ttaatcgggc 6840
   tgtttgatgt aagtaagatg aagtcacgcg cgtgcaggag actgcgtccc gcgatattct 6900
   gcaggcttga aaaatttacc ctaacggtag gcatcaagtg agtgagtctc agcgtcgata 6960
   tgggtcaaaa aaggggaaaa ctagccgaga tcgttgcgag ctgtttcgaa aattatgccc 7020
   tatggcaatt atcacgtgga gtatccgaat ttctccaggc tgtcaagcgg caattataac 7080
   cgagactgag atcgagaagt atataaccgc agcagtagtg gataaataat tgcgaagtct 7140
   tcccagcaga gcgggctgtt ttttggagtt ggttactgta aaatgctaaa atgactgaca 7200
   acaatggagc gtctacagca ttggcaacag tgggaacagt atgctggtgc atccagttga 7260
   taccccaggt tctgcgaaac tggtatgttc gggattgcga gggcgttcct cctctgatgt 7320
   tctttttgtt cgccgtttcg gggattccct tcgcagtgta cttcattgat cagaattcga 7380
   acactgccat catggttcaa cctcacttgt ttactttctt tagccttata ggcttttggc 7440
   aaagcctgta ctatccgccc gtcagaccag cacgggccgt cacatgtatg gttgcgtcgc 7500
   tgtataagaa atcttacaac tgaagactac acagcgtatc cgctccgata tcggcgatca 7560
   cgtggataca tttccccaga atgcgtcaac cttgcatgct cgatattgac tcaagccgag 7620
   aggtgtataa caacaccgac gatagcgaat tacttgtgga actgatttgc cgtatcgagt 7680
   aaatcgcgat tgtggccctc tttaggcctt gtacccattt gtgcatcgta tttgttagta 7740
   tgcatcatag aattatgtga acttagaaaa gtccgtatga aatgagcctc agattatgga 7800
   ttgatcgctt gttatttgta cagcggaatt gacttatagt atgtcggcca cggttttaga 7860
   ttgcctaggg gccgttttct tgatggattc gcatcggaac tccgaattct tgattgctct 7920
   ccatcgcgca ggaggccgtt ctttttttga caaagtccca ttttagggcg caggtccaaa 7980
   aaataagcgg ccgcttaatt aactggcctc atgggccttc cgctcactgc ccgctttcca 8040
   gtcgggaaac ctgtcgtgcc agctgcatta acatggtcat agctgtttcc ttgcgtattg 8100
   ggcgctctcc gcttcctcgc tcactgactc gctgcgctcg gtcgttcggg taaagcctgg 8160
   ggtgcctaat gagcaaaagg ccagcaaaag gccaggaacc gtaaaaaggc cgcgttgctg 8220
   gcgtttttcc ataggctccg cccccctgac gagcatcaca aaaatcgacg ctcaagtcag 8280
   aggtggcgaa acccgacagg actataaaga taccaggcgt ttccccctgg aagctccctc 8340
   gtgcgctctc ctgttccgac cctgccgctt accggatacc tgtccgcctt tctcccttcg 8400
   ggaagcgtgg cgctttctca tagctcacgc tgtaggtatc tcagttcggt gtaggtcgtt 8460
   cgctccaagc tgggctgtgt gcacgaaccc cccgttcagc ccgaccgctg cgccttatcc 8520
   ggtaactatc gtcttgagtc caacccggta agacacgact tatcgccact ggcagcagcc 8580
   actggtaaca ggattagcag agcgaggtat gtaggcggtg ctacagagtt cttgaagtgg 8640
   tggcctaact acggctacac tagaagaaca gtatttggta tctgcgctct gctgaagcca 8700
   gttaccttcg gaaaaagagt tggtagctct tgatccggca aacaaaccac cgctggtagc 8760
   ggtggttttt ttgtttgcaa gcagcagatt acgcgcagaa aaaaaggatc tcaagaagat 8820
   cctttgatct tttctacggg gtctgacgct cagtggaacg aaaactcacg ttaagggatt 8880
   ttggtcatga gattatcaaa aaggatcttc acctagatcc ttttaaatta aaaatgaagt 8940
   tttaaatcaa tctaaagtat atatgagtaa acttggtctg acagttacca atgcttaatc 9000
   agtgaggcac ctatctcagc gatctgtcta tttcgttcat ccatagttgc ctgactcccc 9060
   gtcgtgtaga taactacgat acgggagggc ttaccatctg gccccagtgc tgcaatgata 9120
   ccgcgagaac cacgctcacc ggctccagat ttatcagcaa taaaccagcc agccggaagg 9180
   gccgagcgca gaagtggtcc tgcaacttta tccgcctcca tccagtctat taattgttgc 9240
   cgggaagcta gagtaagtag ttcgccagtt aatagtttgc gcaacgttgt tgccattgct 9300
   acaggcatcg tggtgtcacg ctcgtcgttt ggtatggctt cattcagctc cggttcccaa 9360
   cgatcaaggc gagttacatg atcccccatg ttgtgcaaaa aagcggttag ctccttcggt 9420
   cctccgatcg ttgtcagaag taagttggcc gcagtgttat cactcatggt tatggcagca 9480
   ctgcataatt ctcttactgt catgccatcc gtaagatgct tttctgtgac tggtgagtac 9540
   tcaaccaagt cattctgaga atagtgtatg cggcgaccga gttgctcttg cccggcgtca 9600
   atacgggata ataccgcgcc acatagcaga actttaaaag tgctcatcat tggaaaacgt 9660
   tcttcggggc gaaaactctc aaggatctta ccgctgttga gatccagttc gatgtaaccc 9720
   actcgtgcac ccaactgatc ttcagcatct tttactttca ccagcgtttc tgggtgagca 9780
   aaaacaggaa ggcaaaatgc cgcaaaaaag ggaataaggg cgacacggaa atgttgaata 9840
   ctcatactct tcctttttca atattattga agcatttatc agggttattg tctcatgagc 9900
   ggatacatat ttgaatgtat ttagaaaaat aaacaaatag gggttccgcg cacatttccc 9960
   cgaaaagtgc cac 9973
<210> 69
   <211> 7375
   <212> DNA
   <213> Artificial
<220>
   <223> Vector
<400> 69
   ctaaattgta agcgttaata ttttgttaaa attcgcgtta aatttttgtt aaatcagctc      60
   attttttaac caataggccg aaatcggcaa aatcccttat aaatcaaaag aatagaccga    120
   gatagggttg agtggccgct acagggcgct cccattcgcc attcaggctg cgcaactgtt    180
   gggaagggcg tttcggtgcg ggcctcttcg ctattacgcc agctggcgaa agggggatgt    240
   gctgcaaggc gattaagttg ggtaacgcca gggttttccc agtcacgacg ttgtaaaacg    300
   acggccagtg agcgcgacgt aatacgactc actatagggc gaattggcgg aaggccgtca    360
   aggcctaggc gcgcctgcag gatcctagaa aacagctgga tatggataaa ctcggcaagc    420
   atcttttgga aaggctgcca cgctatgccg tgccgatatt cattaagttc gtcgacaccg    480
   tcaccatcac cggcaacaac aaagttcaga agaaagaatt ccgaaaccag cagattcccg    540
   ccccagcagg acaaacaatc tactggttag agggcacgag ctacaagccc ctcactgctg    600
   atgcgtgggc tcgtgtagag aatgggcgac acaagctcta atgttgaata cctcttccgt    660
   atagatgtgg cataagctat agattttgct gcaatattat taaatattaa agagtttcga    720
   aggtcagctg cggatgaacc agttcagagc ggctctctct tttttgccaa tagcgtgcaa    780
   ccgtgaagag caattcaacc atccaatctg gctacactaa attgtatttg gcagcgcact    840
   gtacgagcgc actgtacgaa actccgtcaa tttatagcag aacgcgtgcg atcgcgggcc    900
   ccagcgatat gacgagaatc aggcaataat agcttaagct gaagtgtttt tagatttagt 960
   tcggagtgcg cttctcaaaa gtgctgggat caacaagttt ttaacatggg ttttgattta 1020
   tattgtttta tatgagcgcc tcagatatgc gctgacagcc tattaggaga aatggccggc 1080
   caggttaaga agctaattca ctaattgccg actctagaat atcaagagac ttgtattttt 1140
   caagctcttt cttgactgcc atggctttct cgtgatacga gggagtagcc aacacctcct 1200
   taacggccgt ggagactagc tcagaagttg cctgcaaggt ttgaagatca taaccaacac 1260
   cagcccatac agctcgtgaa gcaacagctg gcttgtctac caacattcct cctccgatga 1320
   tgacgggaac gccatggctc aaactgtgct gcagacctcc gtatccaccg ttgtatatga 1380
   aaacagaggc atgcggtagt agctcatcgt aaggaaaata atcaacaatt cgagcgtttg 1440
   caggaacttt aacgctatca ggaagtgacg cccctttgac gcccaatata ccaactacga 1500
   gagtgtcttc ttcgtcagca aaggcctgca atgctggaat gagcagatct tcatagttga 1560
   tggctgctgt tccttgtgta acaacaatca gacgcttcgc actcagcaca tcaggccacc 1620
   aagacggcag gtgaggtgga gttgctaatc cagcagactt tacatgcggt gcactaccag 1680
   cgaacgagaa gccaggagga ggcgaagtca agtgaaattc aagagatgga gggcacagtt 1740
   gcaaaaatct gtcagggctg ctgtatatat tctccaggag aaattcgggc tccttcgtgg 1800
   ccccgagcgt cttcatgatc tccttctcag agtcagttcc tggttgaaat acttgttgcc 1860
   gcactaaagt atcaatcatt ggctcaagac taggaactcc aggcgccttc tctgctttca 1920
   gcatgcacgg aatagttcct aacgtgatta cgccttgggg cttgagacct ggggcaccca 1980
   gtgatatcgg atgcacccct agaaacatgg tctcgccaat caccacagct gatttatttt 2040
   cagcctcaac ctgttttaga gcagtttgaa gtgcatcgta ctgctcagga atcgccttca 2100
   caaaaatctc attcattgag taaccggtct gctcaaggcc tggaggaatc gtgagcaatc 2160
   ctggagcgat ttcagggaga ttgtattcat ggtagtcagc tcgtccttgg agagggacga 2220
   aagtgcatcc tgcctcaata actttctcct tgaatgcgtt ccctgttacg aaagtcacct 2280
   catatcctct attgagtaga ccgcggacca ggctgagcac tgggcccacg tgccccgcta 2340
   gtgggcaggc acaagcaact atcactggtt tctcgatggc catatgtgta gagttgtttt 2400
   tgttgttaag tctttcttta agagcttgac cgactataac cgttcaacgg cgcattatat 2460
   actttgggta tcggccagtg ctgacaactc acacgttgcg accccttacc cagaagcata 2520
   cccagcgcga tgtcgatcgt gttatatcgt agacgcacac cctgcaatga cgggtaggct 2580
   ctaaatcggg atgcgaaaaa gaggttgcct tgctttttgc cctggtagat ggcatgctga 2640
   gcgtgcgctt gccgcctaat ttttgtgtgt cgcctgctat ttattgctga agctagcccg 2700
   ccgcatcttt ccccaaggct tcgattgctc gtattggggc agggattggt actcaacctt 2760
   gcagatgaga ctccagcaac aacgtcgtac tgcttagcga tcgcacatgt ttcatcatcg 2820
   tcactataca catcgtcatc aactccatgg cgtgaggact tccgagactg ctgggccctt 2880
   cgtttcttta atgcctcaag agatgacttc gtacccgaag agacgcctgt tgtaccccgt 2940
   tgacgcttgg cggagggggc ttcgtcctcg tcagcaaccc gcgtcatctg cttccttcgc 3000
   tgagcaagat accttctctc ctcgtaccgc tgcatctcct gagctcggtc atacaagatc 3060
   tcttctcgct caatctctgg cagcgcgtcc aacttcgccc tgtcttcagc atcgagatat 3120
   ttgccttcta gaggataggg attgacgacc tcattgcttg gcggcgacgg cagcgagatt 3180
   tcctcttcgg agtcggagcc aacgtcggcc aatgccagca gatcatcatc actgtcactc 3240
   atagtaggaa ggttgaagtg tgctgacgaa tcagaatcgc gaaggatgcc attgaaggca 3300
   tatatatttt aatctgtacc ttttatggta atttaatcag attttatagg tattcatgtg 3360
   caagttgcat tgaaggaact gtttgagaaa atcatcttga ctgaactttt ctcagatatg 3420
   cattccagcc cgccttttgg taacgctgag cttcgtgcac aggatctcgt cccttgctat 3480
   agagcccgcg tccgacgata ataacgtctg tgccggtctc tatgacgtcg tccacagtac 3540
   gatactgctg ccccaatcca tcacctttgt cgtccaggcc caccccagga gtcataatga 3600
   cccagtcttc ctctggcttt ccgacttttt gctgagcgat gaaaccaaac acaaatgcgc 3660
   ggttactgcg agcgatgtct actgtcgctt gcgagtattc gccgtgagcc agtgtgccct 3720
   tcgaactcag ttctgcaagc atgacaaggc cgcgaggttc atccgtagtt tccttcgcag 3780
   cctcttctag tccgctcaca attcccggcc caggaacacc gtgagcattt gttatatcag 3840
   cccattgagc gatcttaaac actccacctg catattgggc cttaacagtg gaaccgatgt 3900
   ctgcgaactt tcggtcttca aaaatgagaa aattgtgctt cgttgaaagc tgtttcaaac 3960
   cgctgacagt tgtgtcgtat tcgaagtcgt caattatgtc aatgtgggtc ttaaccatac 4020
   aaatgtaagg tccaatgcgg tccaggatac tcagtaactc agaggtagtt cgcacatcca 4080
   agcttgcgca aagatttgtt tgcttgctca caatgatgtc gaatagccgg gctgctacag 4140
   ccggcagcct ctctcggcgc tcctcatagc tcagcttcat attatttctc tacagtagtg 4200
   cccgtgccct cgatcagcta ggacttttca aattaatcgg gctgtttgat gtaagtaaga 4260
   tgaagtcacg cgcgtgcagg agactgcgtc ccgcgatatt ctgcaggctt gaaaaattta 4320
   ccctaacggt aggcatcaag tgagtgagtc tcagcgtcga tatgggtcaa aaaaggggaa 4380
   aactagccga gatcgttgcg agctgtttcg aaaattatgc cctatggcaa ttatcacgtg 4440
   gagtatccga atttctccag gctgtcaagc ggcaattata accgagactg agatcgagaa 4500
   gtatataacc gcagcagtag tggataaata attgcgaagt cttcccagca gagcgggctg 4560
   ttttttggag ttggttactg taaaatgcta aaatgactga caacaatgga gcgtctacag 4620
   cattggcaac agtgggaaca gtatgctggt gcatccagtt gataccccag gttctgcgaa 4680
   actggtatgt tcgggattgc gagggcgttc ctcctctgat gttctttttg ttcgccgttt 4740
   cggggattcc cttcgcagtg tacttcattg atcagaattc gaacactgcc atcatggttc 4800
   aacctcactt gtttactttc tttagcctta taggcttttg gcaaagcctg tactatccgc 4860
   ccgtcagacc agcacgggcc gtcacatgta tggttgcgtc gctgtataag aaatcttaca 4920
   actgaagact acacagcgta tccgctccga tatcggcgat cacgtggata catttcccca 4980
   gaatgcgtca accttgcatg ctcgatattg actcaagccg agaggtgtat aacaacaccg 5040
   acgatagcga attacttgtg gaactgattt gccgtatcga gtaaatcgcg attgtggccc 5100
   tctttaggcc ttgtacccat ttgtgcatcg tatttgttag tatgcatcat agaattatgt 5160
   gaacttagaa aagtccgtat gaaatgagcc tcagattatg gattgatcgc ttgttatttg 5220
   tacagcggaa ttgacttata gtatgtcggc cacggtttta gattgcctag gggccgtttt 5280
   cttgatggat tcgcatcgga actccgaatt cttgattgct ctccatcgcg caggaggccg 5340
   ttcttttttt gacaaagtcc cattttaggg cgcaggtcca aaaaataagc ggccgcttaa 5400
   ttaactggcc tcatgggcct tccgctcact gcccgctttc cagtcgggaa acctgtcgtg 5460
   ccagctgcat taacatggtc atagctgttt ccttgcgtat tgggcgctct ccgcttcctc 5520
   gctcactgac tcgctgcgct cggtcgttcg ggtaaagcct ggggtgccta atgagcaaaa 5580
   ggccagcaaa aggccaggaa ccgtaaaaag gccgcgttgc tggcgttttt ccataggctc 5640
   cgcccccctg acgagcatca caaaaatcga cgctcaagtc agaggtggcg aaacccgaca 5700
   ggactataaa gataccaggc gtttccccct ggaagctccc tcgtgcgctc tcctgttccg 5760
   accctgccgc ttaccggata cctgtccgcc tttctccctt cgggaagcgt ggcgctttct 5820
   catagctcac gctgtaggta tctcagttcg gtgtaggtcg ttcgctccaa gctgggctgt 5880
   gtgcacgaac cccccgttca gcccgaccgc tgcgccttat ccggtaacta tcgtcttgag 5940
   tccaacccgg taagacacga cttatcgcca ctggcagcag ccactggtaa caggattagc 6000
   agagcgaggt atgtaggcgg tgctacagag ttcttgaagt ggtggcctaa ctacggctac 6060
   actagaagaa cagtatttgg tatctgcgct ctgctgaagc cagttacctt cggaaaaaga 6120
   gttggtagct cttgatccgg caaacaaacc accgctggta gcggtggttt ttttgtttgc 6180
   aagcagcaga ttacgcgcag aaaaaaagga tctcaagaag atcctttgat cttttctacg 6240
   gggtctgacg ctcagtggaa cgaaaactca cgttaaggga ttttggtcat gagattatca 6300
   aaaaggatct tcacctagat ccttttaaat taaaaatgaa gttttaaatc aatctaaagt 6360
   atatatgagt aaacttggtc tgacagttac caatgcttaa tcagtgaggc acctatctca 6420
   gcgatctgtc tatttcgttc atccatagtt gcctgactcc ccgtcgtgta gataactacg 6480
   atacgggagg gcttaccatc tggccccagt gctgcaatga taccgcgaga accacgctca 6540
   ccggctccag atttatcagc aataaaccag ccagccggaa gggccgagcg cagaagtggt 6600
   cctgcaactt tatccgcctc catccagtct attaattgtt gccgggaagc tagagtaagt 6660
   agttcgccag ttaatagttt gcgcaacgtt gttgccattg ctacaggcat cgtggtgtca 6720
   cgctcgtcgt ttggtatggc ttcattcagc tccggttccc aacgatcaag gcgagttaca 6780
   tgatccccca tgttgtgcaa aaaagcggtt agctccttcg gtcctccgat cgttgtcaga   6840
   agtaagttgg ccgcagtgtt atcactcatg gttatggcag cactgcataa ttctcttact   6900
   gtcatgccat ccgtaagatg cttttctgtg actggtgagt actcaaccaa gtcattctga   6960
   gaatagtgta tgcggcgacc gagttgctct tgcccggcgt caatacggga taataccgcg   7020
   ccacatagca gaactttaaa agtgctcatc attggaaaac gttcttcggg gcgaaaactc   7080
   tcaaggatct taccgctgtt gagatccagt tcgatgtaac ccactcgtgc acccaactga   7140
   tcttcagcat cttttacttt caccagcgtt tctgggtgag caaaaacagg aaggcaaaat   7200
   gccgcaaaaa agggaataag ggcgacacgg aaatgttgaa tactcatact cttccttttt   7260
   caatattatt gaagcattta tcagggttat tgtctcatga gcggatacat atttgaatgt   7320
   atttagaaaa ataaacaaat aggggttccg cgcacatttc cccgaaaagt gccac        7375
<210> 70
   <211> 5316
   <212> DNA
   <213> Artificial
<220>
   <223> koCasssette
<400> 70
   ggacctgcgc cctaaaatgg gactttgtca aaaaaagaac ggcctcctgc gcgatggaga     60
   gcaatcaaga attcggagtt ccgatgcgaa tccatcaaga aaacggcccc taggcaatct    120
   aaaaccgtgg ccgacatact ataagtcaat tccgctgtac aaataacaag cgatcaatcc    180
   ataatctgag gctcatttca tacggacttt tctaagttca cataattcta tgatgcatac    240
   taacaaatac gatgcacaaa tgggtacaag gcctaaagag ggccacaatc gcgatttact    300
   cgatacggca aatcagttcc acaagtaatt cgctatcgtc ggtgttgtta tacacctctc    360
   ggcttgagtc aatatcgagc atgcaaggtt gacgcattct ggggaaatgt atccacgtga    420
   tcgccgatat cggagcggat acgctgtgta gtcttcagtt gtaagatttc ttatacagcg    480
   acgcaaccat acatgtgacg gcccgtgctg gtctgacggg cggatagtac aggctttgcc    540
   aaaagcctat aaggctaaag aaagtaaaca agtgaggttg aaccatgatg gcagtgttcg    600
   aattctgatc aatgaagtac actgcgaagg gaatccccga aacggcgaac aaaaagaaca    660
   tcagaggagg aacgccctcg caatcccgaa cataccagtt tcgcagaacc tggggtatca    720
   actggatgca ccagcatact gttcccactg ttgccaatgc tgtagacgct ccattgttgt    780
   cagtcatttt agcattttac agtaaccaac tccaaaaaac agcccgctct gctgggaaga    840
   cttcgcaatt atttatccac tactgctgcg gttatatact tctcgatctc agtctcggtt    900
   ataattgccg cttgacagcc tggagaaatt cggatactcc acgtgataat tgccataggg    960
   cataattttc gaaacagctc gcaacgatct cggctagttt tccccttttt tgacccatat   1020
   cgacgctgag actcactcac ttgatgccta ccgttagggt aaatttttca agcctgcaga   1080
   atatcgcggg acgcagtctc ctgcacgcgc gtgacttcat cttacttaca tcaaacagcc   1140
   cgattaattt gaaaagtcct agctgatcga gggcacgggc actactgtag agaaataata   1200
   tgaagctgag ctatgaggag cgccgagaga ggctgccggc tgtagcagcc cggctattcg   1260
   acatcattgt gagcaagcaa acaaatcttt gcgcaagctt ggatgtgcga actacctctg   1320
   agttactgag tatcctggac cgcattggac cttacatttg tatggttaag acccacattg   1380
   acataattga cgacttcgaa tacgacacaa ctgtcagcgg tttgaaacag ctttcaacga   1440
   agcacaattt tctcattttt gaagaccgaa agttcgcaga catcggttcc actgttaagg   1500
   cccaatatgc aggtggagtg tttaagatcg ctcaatgggc tgatataaca aatgctcacg   1560
   gtgttcctgg gccgggaatt gtgagcggac tagaagaggc tgcgaaggaa actacggatg   1620
   aacctcgcgg ccttgtcatg cttgcagaac tgagttcgaa gggcacactg gctcacggcg   1680
   aatactcgca agcgacagta gacatcgctc gcagtaaccg cgcatttgtg tttggtttca   1740
   tcgctcagca aaaagtcgga aagccagagg aagactgggt cattatgact cctggggtgg   1800
   gcctggacga caaaggtgat ggattggggc agcagtatcg tactgtggac gacgtcatag   1860
   agaccggcac agacgttatt atcgtcggac gcgggctcta tagcaaggga cgagatcctg   1920
   tgcacgaagc tcagcgttac caaaaggcgg gctggaatgc atatctgaga aaagttcagt   1980
   caagatgatt ttctcaaaca gttccttcaa tgcaacttgc acatgaatac ctataaaatc   2040
   tgattaaatt accataaaag gtacagatta aaatatatat gccttcaatg gcatccttcg   2100
   cgattctgat tcgtcagcac acttcaacct tcctactatg agtgacagtg atgatgatct   2160
   gctggcattg gccgacgttg gctccgactc cgaagaggaa atctcgctgc cgtcgccgcc   2220
   aagcaatgag gtcgtcaatc cctatcctct agaaggcaaa tatctcgatg ctgaagacag   2280
   ggcgaagttg gacgcgctgc cagagattga gcgagaagag atcttgtatg accgagctca   2340
   ggagatgcag cggtacgagg agagaaggta tcttgctcag cgaaggaagc agatgacgcg   2400
   ggttgctgac gaggacgaag ccccctccgc caagcgtcaa cggggtacaa caggcgtctc   2460
   ttcgggtacg aagtcatctc ttgaggcatt aaagaaacga agggcccagc agtctcggaa   2520
   gtcctcacgc catggagttg atgacgatgt gtatagtgac gatgatgaaa catgtgcgat   2580
   cgctaagcag tacgacgttg ttgctggagt ctcatctgca aggttgagta ccaatccctg   2640
   ccccaatacg agcaatcgaa gccttgggga aagatgcggc gggctagctt cagcaataaa   2700
   tagcaggcga cacacaaaaa ttaggcggca agcgcacgct cagcatgcca tctaccaggg   2760
   caaaaagcaa ggcaacctct ttttcgcatc ccgatttaga gcctacccgt cattgcaggg   2820
   tgtgcgtcta cgatataaca cgatcgacat cgcgctgggt atgcttctgg gtaaggggtc   2880
   gcaacgtgtg agttgtcagc actggccgat acccaaagta tataatgcgc cgttgaacgg   2940
   ttatagtcgg tcaagctctt aaagaaagac ttaacaacaa aaacaactct acacatatgt   3000
   taatcaaaga cattattcta actccaatga gtttatccgc tgttgctggc ttgttgccac 3060
   tgctcttcgt agctttctta gttctacacg agcctatctg gctcctatgg taccgctatg 3120
   cagcacgtag gcacaagtgt agtatgcctc gcttcattga gaaatcgttc ccactgggaa 3180
   tacaaagaac catggacatg atcaagacgg ccaagtcata caccttactg gaagttcaat 3240
   acgacagagt cttcaataag ttcaaagcac ggacgtatct tcgacaagct ccccttcaat 3300
   accaaatctt cacaatcgag ccagaaaaca ttaagacaat cctggcaacc aaattcaatg 3360
   attttggtct tggagcacgt ttccacacag tgggaaaagt gtttggccaa gggatattta 3420
   cactcagcgg aaatggatgg aaacagtctc gatcgatgtt gagacctcag ttcactaaag 3480
   atcaggtttg cagaattgat cagatttcca gtcatgctgc ggagttaata aaggagatga 3540
   accgtgcaat gaaagtggac caatttattg atgttcaaca ttatttccac aaacttacgc 3600
   tggatacagc gactgaattc ctatttgggg agtcctgcga gagcttgaac cctgagaatc 3660
   agtcatgtat tgtagcccgt gatggttcgg agattactgc cgaacaattc gtggagtcct 3720
   acaactttct actgaattac gctttcaaac ggaccctatc aagcaaagtc tactggttgt 3780
   tcaactctaa ggaattccga gatcacaaga aacgtgctca gtcctatatt gactactacg 3840
   ttgataaggc tctttacgcc acatctttcg ctgctgagaa ctctattgca gagaaggatg 3900
   ctgctgcaga gtctagtggc atctatgtgt tctcgcttga gatggctaaa gttacccgag 3960
   acccagtgac gatacgtgat caaattttca acattctcat tgctggtaga gatacaacag 4020
   ctgctacgtt gagcttcgct attcatttcc ttgccagaaa tcctgacgta ttcaacaaac 4080
   tacgtgagga ggtcctcgat cattttggaa ccaaggagga gcaaaggcct ttatcattcg 4140
   aacttctgaa gcaagcacct tatttgaagc aagttataaa tgaagtcttg cgtcttgcgc 4200
   cggtattgcc attgaacttc cgtactgctg tgagagatac aactctaccc ataggtggtg 4260
   gtcccgagca gaaggatccg atcttcgttc ctaagggcac cgcagtttac tattcaattt 4320
   acatggtcca cagggacatc aagtattggg gtcctgacgc ccacgaattc aatcccaatc 4380
   gatgggagaa cttgaagcta gataatgtgt gggcattctt gcccttcaat ggcggtcccc 4440
   gaatttgtct cggccaacaa ttcgccctga cagagctttc gctaactctg gtgagactct 4500
   tacaggagta ttccaagatt gagatgggtc ccgacttccc agagtcccct cgtttctcaa 4560
   caacgcttac agctcaacac gctcctcccg gtgtggttgt gcggttttct taagttggcc 4620
   ggccatttct cctaataggc tgtcagcgca tatctgaggc gctcatataa aacaatataa 4680
   atcaaaaccc atgttaaaaa cttgttgatc ccagcacttt tgagaagcgc actccgaact 4740
   aaatctaaaa acacttcagc ttaagctatt attgcctgat tctcgtcata tcgctggggc 4800
   ccgcgatcgc acgcgttctg ctataaattg acggagtttc gtacagtgcg ctcgtacagt 4860
   gcgctgccaa atacaattta gtgtagccag attggatggt tgaattgctc ttcacggttg 4920
   cacgctattg gcaaaaaaga gagagccgct ctgaactggt tcatccgcag ctgaccttcg 4980
   aaactcttta atatttaata atattgcagc aaaatctata gcttatgcca catctatacg 5040
   gaagaggtat tcaacattag agcttgtgtc gcccattctc tacacgagcc cacgcatcag 5100
   cagtgagggg cttgtagctc gtgccctcta accagtagat tgtttgtcct gctggggcgg 5160
   gaatctgctg gtttcggaat tctttcttct gaactttgtt gttgccggtg atggtgacgg 5220
   tgtcgacgaa cttaatgaat atcggcacgg catagcgtgg cagcctttcc aaaagatgct 5280
   tgccgagttt atccatatcc agctgttttc taggat 5316
<210> 71
   <211> 5088
   <212> DNA
   <213> Artificial
<220>
   <223> koCassette
<400> 71
   ggacctgcgc cctaaaatgg gactttgtca aaaaaagaac ggcctcctgc gcgatggaga      60
   gcaatcaaga attcggagtt ccgatgcgaa tccatcaaga aaacggcccc taggcaatct     120
   aaaaccgtgg ccgacatact ataagtcaat tccgctgtac aaataacaag cgatcaatcc     180
   ataatctgag gctcatttca tacggacttt tctaagttca cataattcta tgatgcatac     240
   taacaaatac gatgcacaaa tgggtacaag gcctaaagag ggccacaatc gcgatttact     300
   cgatacggca aatcagttcc acaagtaatt cgctatcgtc ggtgttgtta tacacctctc     360
   ggcttgagtc aatatcgagc atgcaaggtt gacgcattct ggggaaatgt atccacgtga     420
   tcgccgatat cggagcggat acgctgtgta gtcttcagtt gtaagatttc ttatacagcg     480
   acgcaaccat acatgtgacg gcccgtgctg gtctgacggg cggatagtac aggctttgcc     540
   aaaagcctat aaggctaaag aaagtaaaca agtgaggttg aaccatgatg gcagtgttcg     600
   aattctgatc aatgaagtac actgcgaagg gaatccccga aacggcgaac aaaaagaaca     660
   tcagaggagg aacgccctcg caatcccgaa cataccagtt tcgcagaacc tggggtatca     720
   actggatgca ccagcatact gttcccactg ttgccaatgc tgtagacgct ccattgttgt     780
   cagtcatttt agcattttac agtaaccaac tccaaaaaac agcccgctct gctgggaaga     840
   cttcgcaatt atttatccac tactgctgcg gttatatact tctcgatctc agtctcggtt     900
   ataattgccg cttgacagcc tggagaaatt cggatactcc acgtgataat tgccataggg     960
   cataattttc gaaacagctc gcaacgatct cggctagttt tccccttttt tgacccatat    1020
   cgacgctgag actcactcac ttgatgccta ccgttagggt aaatttttca agcctgcaga    1080
   atatcgcggg acgcagtctc ctgcacgcgc gtgacttcat cttacttaca tcaaacagcc    1140
   cgattaattt gaaaagtcct agctgatcga gggcacgggc actactgtag agaaataata    1200
   tgaagctgag ctatgaggag cgccgagaga ggctgccggc tgtagcagcc cggctattcg    1260
   acatcattgt gagcaagcaa acaaatcttt gcgcaagctt ggatgtgcga actacctctg 1320
   agttactgag tatcctggac cgcattggac cttacatttg tatggttaag acccacattg 1380
   acataattga cgacttcgaa tacgacacaa ctgtcagcgg tttgaaacag ctttcaacga 1440
   agcacaattt tctcattttt gaagaccgaa agttcgcaga catcggttcc actgttaagg 1500
   cccaatatgc aggtggagtg tttaagatcg ctcaatgggc tgatataaca aatgctcacg 1560
   gtgttcctgg gccgggaatt gtgagcggac tagaagaggc tgcgaaggaa actacggatg 1620
   aacctcgcgg ccttgtcatg cttgcagaac tgagttcgaa gggcacactg gctcacggcg 1680
   aatactcgca agcgacagta gacatcgctc gcagtaaccg cgcatttgtg tttggtttca 1740
   tcgctcagca aaaagtcgga aagccagagg aagactgggt cattatgact cctggggtgg 1800
   gcctggacga caaaggtgat ggattggggc agcagtatcg tactgtggac gacgtcatag 1860
   agaccggcac agacgttatt atcgtcggac gcgggctcta tagcaaggga cgagatcctg 1920
   tgcacgaagc tcagcgttac caaaaggcgg gctggaatgc atatctgaga aaagttcagt 1980
   caagatgatt ttctcaaaca gttccttcaa tgcaacttgc acatgaatac ctataaaatc 2040
   tgattaaatt accataaaag gtacagatta aaatatatat gccttcaatg gcatccttcg 2100
   cgattctgat tcgtcagcac acttcaacct tcctactatg agtgacagtg atgatgatct 2160
   gctggcattg gccgacgttg gctccgactc cgaagaggaa atctcgctgc cgtcgccgcc 2220
   aagcaatgag gtcgtcaatc cctatcctct agaaggcaaa tatctcgatg ctgaagacag 2280
   ggcgaagttg gacgcgctgc cagagattga gcgagaagag atcttgtatg accgagctca 2340
   ggagatgcag cggtacgagg agagaaggta tcttgctcag cgaaggaagc agatgacgcg 2400
   ggttgctgac gaggacgaag ccccctccgc caagcgtcaa cggggtacaa caggcgtctc 2460
   ttcgggtacg aagtcatctc ttgaggcatt aaagaaacga agggcccagc agtctcggaa 2520
   gtcctcacgc catggagttg atgacgatgt gtatagtgac gatgatgaaa catgtgcgat 2580
   cgctaagcag tacgacgttg ttgctggagt ctcatctgca aggttgagta ccaatccctg 2640
   ccccaatacg agcaatcgaa gccttgggga aagatgcggc gggctagctt cagcaataaa 2700
   tagcaggcga cacacaaaaa ttaggcggca agcgcacgct cagcatgcca tctaccaggg 2760
   caaaaagcaa ggcaacctct ttttcgcatc ccgatttaga gcctacccgt cattgcaggg 2820
   tgtgcgtcta cgatataaca cgatcgacat cgcgctgggt atgcttctgg gtaaggggtc 2880
   gcaacgtgtg agttgtcagc actggccgat acccaaagta tataatgcgc cgttgaacgg 2940
   ttatagtcgg tcaagctctt aaagaaagac ttaacaacaa aaacaactct acacatatga 3000
   gcccttcatc acacaaaccc ctgattctcg cttgcggctt gcctctttca ggccatataa 3060
   tgcccgtttt gagtctggta cacggcctta cggacgacgg atacgaagct actgttgtga 3120
   caggcagagc gtttgaacaa aaagttcgag atgtgggtgc agactttgtt cctttagaag 3180
   ggaacgcaga ttttgatgac cacaccttag acgatctggt cccgggccgt aaagacatgg 3240
   ccccaagctt cgatcgtaca gttcaagatg tggagcacat gatggtagct actcttcctg 3300
   agcagtttgc cgctattcag agggctttca aaaagctcag cgcaagcggt cgccctgtcg 3360
   ttcttgtcag tgaagtgctg tttttcggtg cacaccctat cagcctcggt gctcctggtt 3420
   tcaaacccgc tggctggatt tgtttagggg ttttgcctct tttgatccgc agtgatcata 3480
   ccttaggact tgacaacgac aggagccccg aagcacatgc aaagaaactc gctatgaacc 3540
   acgctcttga gcaccaaatt ttcgttaaag ccactgctaa gcacaaggaa atctgccgag 3600
   agttaggttg cactgaagat cccaaattta tctgggagca cagttacatt gctgcagaca 3660
   agttcctgca gctgtgcccg ccttctcttg agttcagcag agaccatctg cctagcaact 3720
   tcaaattcgc cggctcaacg cccaagcacc gaactcaatt cacccctcct tcctggtggg 3780
   gggatgttct gagtgccaag cgagtcatca tggtcactca aggaactttt gctgtcagtt 3840
   acaagcatct tattgtgcct actcttgagg ccttgaagga cgagcctgac actttaacag 3900
   tagccatatt gggccgccgc ggtgccaagc taccggatga tgttgtggtt cctgagaatg 3960
   ctcgcgtgat cgactacttc aactacgatg ctctacttcc tcacgttgat gctcttgtct 4020
   acaatggtgg atatggcgga cttcagcaca gcttaagcca ctctgttcca gttgttattg 4080
   ctggtgactc tgaagacaag ccaatggtgg catcgagagc tgaggccgct ggcgtggcaa 4140
   ttgatttgaa aactggcttg cctacagtgg agcaaatcaa agaagctgtt gattcgataa 4200
   ttggaaatcc gaaattccac gaagcctcga agaaggttca aatggagttg gaaagccaca 4260
   actccttgaa aattcttgag gaaagcatcg aggaaatcgc cagccatgac tttggtcttt 4320
   tgaccaagag tgacgaggaa actgaagata tacctgtcaa aggtccggcc ttagcggtga 4380
   gttcttaggg ccggccattt ctcctaatag gctgtcagcg catatctgag gcgctcatat 4440
   aaaacaatat aaatcaaaac ccatgttaaa aacttgttga tcccagcact tttgagaagc 4500
   gcactccgaa ctaaatctaa aaacacttca gcttaagcta ttattgcctg attctcgtca 4560
   tatcgctggg gcccgcgatc gcacgcgttc tgctataaat tgacggagtt tcgtacagtg 4620
   cgctcgtaca gtgcgctgcc aaatacaatt tagtgtagcc agattggatg gttgaattgc 4680
   tcttcacggt tgcacgctat tggcaaaaaa gagagagccg ctctgaactg gttcatccgc 4740
   agctgacctt cgaaactctt taatatttaa taatattgca gcaaaatcta tagcttatgc 4800
   cacatctata cggaagaggt attcaacatt agagcttgtg tcgcccattc tctacacgag 4860
   cccacgcatc agcagtgagg ggcttgtagc tcgtgccctc taaccagtag attgtttgtc 4920
   ctgctggggc gggaatctgc tggtttcgga attctttctt ctgaactttg ttgttgccgg 4980
   tgatggtgac ggtgtcgacg aacttaatga atatcggcac ggcatagcgt ggcagccttt 5040
   ccaaaagatg cttgccgagt ttatccatat ccagctgttt tctaggat 5088
<210> 72
   <211> 4479
   <212> DNA
   <213> Artificial
<220>
   <223> koCassette
<400> 72
   ggacctgcgc cctaaaatgg gactttgtca aaaaaagaac ggcctcctgc gcgatggaga      60
   gcaatcaaga attcggagtt ccgatgcgaa tccatcaaga aaacggcccc taggcaatct     120
   aaaaccgtgg ccgacatact ataagtcaat tccgctgtac aaataacaag cgatcaatcc     180
   ataatctgag gctcatttca tacggacttt tctaagttca cataattcta tgatgcatac     240
   taacaaatac gatgcacaaa tgggtacaag gcctaaagag ggccacaatc gcgatttact     300
   cgatacggca aatcagttcc acaagtaatt cgctatcgtc ggtgttgtta tacacctctc     360
   ggcttgagtc aatatcgagc atgcaaggtt gacgcattct ggggaaatgt atccacgtga     420
   tcgccgatat cggagcggat acgctgtgta gtcttcagtt gtaagatttc ttatacagcg     480
   acgcaaccat acatgtgacg gcccgtgctg gtctgacggg cggatagtac aggctttgcc     540
   aaaagcctat aaggctaaag aaagtaaaca agtgaggttg aaccatgatg gcagtgttcg     600
   aattctgatc aatgaagtac actgcgaagg gaatccccga aacggcgaac aaaaagaaca     660
   tcagaggagg aacgccctcg caatcccgaa cataccagtt tcgcagaacc tggggtatca     720
   actggatgca ccagcatact gttcccactg ttgccaatgc tgtagacgct ccattgttgt     780
   cagtcatttt agcattttac agtaaccaac tccaaaaaac agcccgctct gctgggaaga     840
   cttcgcaatt atttatccac tactgctgcg gttatatact tctcgatctc agtctcggtt     900
   ataattgccg cttgacagcc tggagaaatt cggatactcc acgtgataat tgccataggg     960
   cataattttc gaaacagctc gcaacgatct cggctagttt tccccttttt tgacccatat    1020
   cgacgctgag actcactcac ttgatgccta ccgttagggt aaatttttca agcctgcaga    1080
   atatcgcggg acgcagtctc ctgcacgcgc gtgacttcat cttacttaca tcaaacagcc    1140
   cgattaattt gaaaagtcct agctgatcga gggcacgggc actactgtag agaaataata    1200
   tgaagctgag ctatgaggag cgccgagaga ggctgccggc tgtagcagcc cggctattcg    1260
   acatcattgt gagcaagcaa acaaatcttt gcgcaagctt ggatgtgcga actacctctg    1320
   agttactgag tatcctggac cgcattggac cttacatttg tatggttaag acccacattg    1380
   acataattga cgacttcgaa tacgacacaa ctgtcagcgg tttgaaacag ctttcaacga    1440
   agcacaattt tctcattttt gaagaccgaa agttcgcaga catcggttcc actgttaagg    1500
   cccaatatgc aggtggagtg tttaagatcg ctcaatgggc tgatataaca aatgctcacg    1560
   gtgttcctgg gccgggaatt gtgagcggac tagaagaggc tgcgaaggaa actacggatg    1620
   aacctcgcgg ccttgtcatg cttgcagaac tgagttcgaa gggcacactg gctcacggcg    1680
   aatactcgca agcgacagta gacatcgctc gcagtaaccg cgcatttgtg tttggtttca 1740
   tcgctcagca aaaagtcgga aagccagagg aagactgggt cattatgact cctggggtgg 1800
   gcctggacga caaaggtgat ggattggggc agcagtatcg tactgtggac gacgtcatag 1860
   agaccggcac agacgttatt atcgtcggac gcgggctcta tagcaaggga cgagatcctg 1920
   tgcacgaagc tcagcgttac caaaaggcgg gctggaatgc atatctgaga aaagttcagt 1980
   caagatgatt ttctcaaaca gttccttcaa tgcaacttgc acatgaatac ctataaaatc 2040
   tgattaaatt accataaaag gtacagatta aaatatatat gccttcaatg gcatccttcg 2100
   cgattctgat tcgtcagcac acttcaacct tcctactatg agtgacagtg atgatgatct 2160
   gctggcattg gccgacgttg gctccgactc cgaagaggaa atctcgctgc cgtcgccgcc 2220
   aagcaatgag gtcgtcaatc cctatcctct agaaggcaaa tatctcgatg ctgaagacag 2280
   ggcgaagttg gacgcgctgc cagagattga gcgagaagag atcttgtatg accgagctca 2340
   ggagatgcag cggtacgagg agagaaggta tcttgctcag cgaaggaagc agatgacgcg 2400
   ggttgctgac gaggacgaag ccccctccgc caagcgtcaa cggggtacaa caggcgtctc 2460
   ttcgggtacg aagtcatctc ttgaggcatt aaagaaacga agggcccagc agtctcggaa 2520
   gtcctcacgc catggagttg atgacgatgt gtatagtgac gatgatgaaa catgtgcgat 2580
   cgctaagcag tacgacgttg ttgctggagt ctcatctgca aggttgagta ccaatccctg 2640
   ccccaatacg agcaatcgaa gccttgggga aagatgcggc gggctagctt cagcaataaa 2700
   tagcaggcga cacacaaaaa ttaggcggca agcgcacgct cagcatgcca tctaccaggg 2760
   caaaaagcaa ggcaacctct ttttcgcatc ccgatttaga gcctacccgt cattgcaggg 2820
   tgtgcgtcta cgatataaca cgatcgacat cgcgctgggt atgcttctgg gtaaggggtc 2880
   gcaacgtgtg agttgtcagc actggccgat acccaaagta tataatgcgc cgttgaacgg 2940
   ttatagtcgg tcaagctctt aaagaaagac ttaacaacaa aaacaactct acacatatgg 3000
   ttgtaaactc ctcgaaggac cctcaaaaca aaggaatgac tcctagaaaa gaaattgacc 3060
   aggaaatggt ctcttgggcc aaaaaaaacc tcaaaaacac ccctggcaat gaaaactatg 3120
   agaagatggt ctcaggagtt ccttacaatc catacgatcc agatcttatg tttagagccc 3180
   tggctactag tgagaaagtt agggagttca ataccattgc aagtgaaagt cgtacttttg 3240
   agtcaaatca cgctgcttat atcaagaagg tcgagattct caaagacact tttggtcaaa 3300
   caaaggatat tgtctggctg accgctccat tctcagttga ttttggattc aacatcagcg 3360
   taggcgagca cttttacgcc aacttcaacg tttgcttctt ggactcggct ccaataatct 3420
   ttggtgatga ggtgattgta gggcccaata caacgttcgt gactgcgact catcctatta 3480
   gccccgagaa acgtgcgagg agaattgtgt atgctcttcc tatcaaggtg gggaataatg 3540
   tatggattgg tgcgaatgtg actgtcctgc cgggtgttac gattggagat ggctcaacaa 3600
   ttgcggctgg tgctgtcgtt cgagaagatg ttcctcctcg tactgtggtg ggaggagtcc 3660
   ctgcgcgaat cctcaagcat attccagagg aggatcccga cgaggctgaa ggagaggaac 3720
   tggaattcct tcttccagtt gaaatgaacg tcaataccgc taaccagaag gtctaggtag 3780
   gccggccatt tctcctaata ggctgtcagc gcatatctga ggcgctcata taaaacaata 3840
   taaatcaaaa cccatgttaa aaacttgttg atcccagcac ttttgagaag cgcactccga 3900
   actaaatcta aaaacacttc agcttaagct attattgcct gattctcgtc atatcgctgg 3960
   ggcccgcgat cgcacgcgtt ctgctataaa ttgacggagt ttcgtacagt gcgctcgtac 4020
   agtgcgctgc caaatacaat ttagtgtagc cagattggat ggttgaattg ctcttcacgg 4080
   ttgcacgcta ttggcaaaaa agagagagcc gctctgaact ggttcatccg cagctgacct 4140
   tcgaaactct ttaatattta ataatattgc agcaaaatct atagcttatg ccacatctat 4200
   acggaagagg tattcaacat tagagcttgt gtcgcccatt ctctacacga gcccacgcat 4260
   cagcagtgag gggcttgtag ctcgtgccct ctaaccagta gattgtttgt cctgctgggg 4320
   cgggaatctg ctggtttcgg aattctttct tctgaacttt gttgttgccg gtgatggtga 4380
   cggtgtcgac gaacttaatg aatatcggca cggcatagcg tggcagcctt tccaaaagat 4440
   gcttgccgag tttatccata tccagctgtt ttctaggat 4479
<210> 73
   <211> 7596
   <212> DNA
   <213> Artificial
<220>
   <223> koCassette
<400> 73
   ggacctgcgc cctaaaatgg gactttgtca aaaaaagaac ggcctcctgc gcgatggaga     60
   gcaatcaaga attcggagtt ccgatgcgaa tccatcaaga aaacggcccc taggcaatct    120
   aaaaccgtgg ccgacatact ataagtcaat tccgctgtac aaataacaag cgatcaatcc    180
   ataatctgag gctcatttca tacggacttt tctaagttca cataattcta tgatgcatac    240
   taacaaatac gatgcacaaa tgggtacaag gcctaaagag ggccacaatc gcgatttact    300
   cgatacggca aatcagttcc acaagtaatt cgctatcgtc ggtgttgtta tacacctctc    360
   ggcttgagtc aatatcgagc atgcaaggtt gacgcattct ggggaaatgt atccacgtga    420
   tcgccgatat cggagcggat acgctgtgta gtcttcagtt gtaagatttc ttatacagcg    480
   acgcaaccat acatgtgacg gcccgtgctg gtctgacggg cggatagtac aggctttgcc    540
   aaaagcctat aaggctaaag aaagtaaaca agtgaggttg aaccatgatg gcagtgttcg    600
   aattctgatc aatgaagtac actgcgaagg gaatccccga aacggcgaac aaaaagaaca    660
   tcagaggagg aacgccctcg caatcccgaa cataccagtt tcgcagaacc tggggtatca    720
   actggatgca ccagcatact gttcccactg ttgccaatgc tgtagacgct ccattgttgt    780
   cagtcatttt agcattttac agtaaccaac tccaaaaaac agcccgctct gctgggaaga 840
   cttcgcaatt atttatccac tactgctgcg gttatatact tctcgatctc agtctcggtt 900
   ataattgccg cttgacagcc tggagaaatt cggatactcc acgtgataat tgccataggg 960
   cataattttc gaaacagctc gcaacgatct cggctagttt tccccttttt tgacccatat 1020
   cgacgctgag actcactcac ttgatgccta ccgttagggt aaatttttca agcctgcaga 1080
   atatcgcggg acgcagtctc ctgcacgcgc gtgacttcat cttacttaca tcaaacagcc 1140
   cgattaattt gaaaagtcct agctgatcga gggcacgggc actactgtag agaaataata 1200
   tgaagctgag ctatgaggag cgccgagaga ggctgccggc tgtagcagcc cggctattcg 1260
   acatcattgt gagcaagcaa acaaatcttt gcgcaagctt ggatgtgcga actacctctg 1320
   agttactgag tatcctggac cgcattggac cttacatttg tatggttaag acccacattg 1380
   acataattga cgacttcgaa tacgacacaa ctgtcagcgg tttgaaacag ctttcaacga 1440
   agcacaattt tctcattttt gaagaccgaa agttcgcaga catcggttcc actgttaagg 1500
   cccaatatgc aggtggagtg tttaagatcg ctcaatgggc tgatataaca aatgctcacg 1560
   gtgttcctgg gccgggaatt gtgagcggac tagaagaggc tgcgaaggaa actacggatg 1620
   aacctcgcgg ccttgtcatg cttgcagaac tgagttcgaa gggcacactg gctcacggcg 1680
   aatactcgca agcgacagta gacatcgctc gcagtaaccg cgcatttgtg tttggtttca 1740
   tcgctcagca aaaagtcgga aagccagagg aagactgggt cattatgact cctggggtgg 1800
   gcctggacga caaaggtgat ggattggggc agcagtatcg tactgtggac gacgtcatag 1860
   agaccggcac agacgttatt atcgtcggac gcgggctcta tagcaaggga cgagatcctg 1920
   tgcacgaagc tcagcgttac caaaaggcgg gctggaatgc atatctgaga aaagttcagt 1980
   caagatgatt ttctcaaaca gttccttcaa tgcaacttgc acatgaatac ctataaaatc 2040
   tgattaaatt accataaaag gtacagatta aaatatatat gccttcaatg gcatccttcg 2100
   cgattctgat tcgtcagcac acttcaacct tcctactatg agtgacagtg atgatgatct 2160
   gctggcattg gccgacgttg gctccgactc cgaagaggaa atctcgctgc cgtcgccgcc 2220
   aagcaatgag gtcgtcaatc cctatcctct agaaggcaaa tatctcgatg ctgaagacag 2280
   ggcgaagttg gacgcgctgc cagagattga gcgagaagag atcttgtatg accgagctca 2340
   ggagatgcag cggtacgagg agagaaggta tcttgctcag cgaaggaagc agatgacgcg 2400
   ggttgctgac gaggacgaag ccccctccgc caagcgtcaa cggggtacaa caggcgtctc 2460
   ttcgggtacg aagtcatctc ttgaggcatt aaagaaacga agggcccagc agtctcggaa 2520
   gtcctcacgc catggagttg atgacgatgt gtatagtgac gatgatgaaa catgtgcgat 2580
   cgctaagcag tacgacgttg ttgctggagt ctcatctgca aggttgagta ccaatccctg 2640
   ccccaatacg agcaatcgaa gccttgggga aagatgcggc gggctagctt cagcaataaa 2700
   tagcaggcga cacacaaaaa ttaggcggca agcgcacgct cagcatgcca tctaccaggg 2760
   caaaaagcaa ggcaacctct ttttcgcatc ccgatttaga gcctacccgt cattgcaggg 2820
   tgtgcgtcta cgatataaca cgatcgacat cgcgctgggt atgcttctgg gtaaggggtc 2880
   gcaacgtgtg agttgtcagc actggccgat acccaaagta tataatgcgc cgttgaacgg 2940
   ttatagtcgg tcaagctctt aaagaaagac ttaacaacaa aaacaactct acacatatgg 3000
   tggatgatat acaggtagag aagcgtgaga aactcatcga gactaaggac aagcttctcg 3060
   aggagaagct ctctgcgtta gatccacatg aggccaatgt attgcgaagt cagcttgaaa 3120
   caaagagagt cgccacaagc tttttcaggt tgttcagatt ttgcactccc cttgacgttt 3180
   tcttggagat acttgcgctt ttttttgcag cggtgcatgg agccgcgctt ccaatgttca 3240
   cgttagtagt gggcgccatc ttcaacacat tcagagactt cactagctat gacctcaagg 3300
   gcaatgagtt ccagcataag gtgaatcacc tgtctctcta ttttgtctat attggcattg 3360
   gtatgctcgg cagtgcgttt ctcgagagct tcctgcttgt ggacagaggc gaagtgttgg 3420
   caggacgtta ccgaaagcat tatctgagtg ctgttattcg ccagaatatc gcgttttacg 3480
   acaaactagg tggtggcgag gtcagcacca gaatcattaa cgataccaac tcaattcagg 3540
   aagcgatcag cgacaagctt ggaaacgtcg tacagggaat agcttccttc attgcggcca 3600
   ccgttataag ttttgcttcg caatggaaac tggcttgcat cctcctgagt gctgtagggt 3660
   tcatggtaat cacaatggga actggcgcca ccttcatggc caaatatcag ctcagatctg 3720
   acgcgatata ttcgcagtct ggagctaccg ttgcggagga ggctctcagt gctgtcagga 3780
   ctacagtagc atttggcgct caacctcatc tcgccgtcaa gtatgaaaag gtacttgatc 3840
   gtgttgtgaa ggaatcgaag cggagcagtt actcattggg ggtcatgtta gcgtgcattt 3900
   gggctagtac tttttgggtg tatgccttag ctctgtggca gggttccaga gaaatcgtta 3960
   gtgggagtgc tgacgttgga aagataatag ttgtaatcac agctatgtta cttggaagct 4020
   tccagcttgg gaatatcgcg ccaaacgtga ggtttcttgt caagggtctc actgccgcga 4080
   gcattctcaa tgaggccatt gatcgtgtcc cagtcatcga tggccagtcc atagataaag 4140
   gaattgtccc ccaaactaag gccgttggca gaattgagct caaaaatgtc aagttccgat 4200
   atcctagtcg cccagacgtt ttggtcctct ccgattttag ccttgaagtt cctgctggat 4260
   ctactgtggc actggtaggt gcctcgggat cagggaagtc tacaattgta ggtattcttg 4320
   agaggttcta tttacctctc gaaggaagcg ttactctgga tggccaggag attagcgacc 4380
   tgaacacaag atggctccgt caacaaattg gttatgttca gcaggaacca gtactctttt 4440
   cagagtcaat atatgagaat atcagctatg gtttgattgg cactgacatt gagttcgctg 4500
   acgagcatgt taaggaagct aaaatcattc aagcttgtaa agatgccaat gcctgggatt 4560
   tcattcagac tctctcagaa ggcatccaaa ccaatgttgg agatcgagga tttcttctca 4620
   gcggtggtca gaaacaacgc attgcaatag caagagcaat cgtctcagac cctaaaattc 4680
   tgctgctcga tgaagcgact tctgctctgg ataccaaatc tgaaggtatc gttcaagatg 4740
   cgctcgacaa agcggccgaa ggtcgtacca ctatagtcgt tgcacacaga ctctctacga 4800
   tcaaggatgc caacaagata gttgtcatgt ctaaaggtaa cgtcatagag cagggtactc 4860
   acaatgagct catacagcga gaagggcctt ataaagcttt ggttgatgct caaagagtaa 4920
   ctaaagcaaa gagcactaac gttgaggtcc tcgatattga agctctagac atttcgcctc 4980
   tggactcact gaacgaaaag ttcaatccca aggatgtgag cacattgagt gttcacagtg 5040
   caggtactca gaccactcaa cctcctgaat atcaagaaaa tgacatccct ggtgtgcgca 5100
   accccccaca tagcacgttg atgaccaata ccaaactggt ttgggggctg aataggaaag 5160
   aatggggtta cattctcatt ggtagtttag cctccattat tttgggctat tgctatcctg 5220
   caatggcaat aataactggc caaaccactg gaagcatggt tctacctccc agtgaatacg 5280
   gaaaaatgcg gcatgtggtg aatatcatgg gatggtggta ttttttcgta ggctgcattt 5340
   cattcatgac ggcttttatc actatagctg ctttatcact tgcatctgat aagttggtca 5400
   aaaatatcag attagctttg ttccgccaat tgatgcgaat ggatattgca ttcttcgacc 5460
   acaaaaacaa cacgccgggt gcgctaacct caattttggc gaaggaagct aaaatgatcg 5520
   agggtttgag tggggccacc ctcggtcaaa ttcaacagag tctggtgacc ttgattggcg 5580
   gcatagttac tggtatacct ttcaattgga gaattggact cgtggctacg tctgttgttc 5640
   ctgtcatgtt ggtgtgtggc ttcgtcagag tctgggttct tacccaatta tcggatcgtg 5700
   cgagagaagt ttacgaacga agtggctcca tggcatctga gtatacaagt gctgtccgca 5760
   cagtccagtc cttaactcgt gagttagacg tggtcgtaaa atacacaaag acagtagact 5820
   ctcagatttt cagctccaga attgccattg cccgctcagc attgtactac gcactctcgg 5880
   aaggaatgac accctgggtg gtagccctcg ttttttggtg gggaagcact gtaatgagac 5940
   gaggtgaagc ttcggtcgca ggatatatga ctgtcttcat ggctattatt acaggttctc 6000
   aagccgctgg ccaaattttc agctatgctc caaacatgaa ctcagccaaa gatgcagcgc 6060
   gtaacattta cagaatcttg actgccactc cttctataga tgtatggagt gaggaaggtt 6120
   acgttgctcc cgaggagtcg gtgagaggag atattgagtt ccgtcatgtg aatttccgat 6180
   atcctactcg acctcaagta ccagttttac aagatctcaa cttaacagtc aaaaagggcc 6240
   aatacatcgc tctagttgga gccagtggat gcggtaagtc tactactatt ggactggtgg 6300
   aaagatttta tgatccatta gcaggtcaag tacttttcga tgggaaagat ttacgcgaat 6360
   ataacctgaa tgcattgaga tcacacattg ctttagtcca gcaagaacca atgctttatt 6420
   caggcacgct acgtgagaat attctaatgg gatggtctgg ccctgagtct gaagtaacgc 6480
   aggagatgat tgaggatgcc gctcgcaaag cgaacattca cgaattcatc atgtcgttgc 6540
   ctgatggcta cgaaacgctc agcggatcta ggggatcgtt gctatctggg gggcaaaagc 6600
   agcgaattgc aattgcaagg gccctgatca gaaatccaaa ggtactcctc ctcgatgagg 6660
   ccacctcagc tctggattcc gaatctgaga aagtagttca agcagcactc gacgcagcag   6720
   cgaagggccg tactacaatc gccgttgcgc atagattatc aacaattcag aaagcagatg   6780
   tcatatatgt gttctcagga gggcgcatcg tggagcaggg cgaccatcag agcctccttg   6840
   aactcaatgg atggtacgct gaattggtga acttgcaagg tctcggagag atttgaggcc   6900
   ggccatttct cctaataggc tgtcagcgca tatctgaggc gctcatataa aacaatataa   6960
   atcaaaaccc atgttaaaaa cttgttgatc ccagcacttt tgagaagcgc actccgaact   7020
   aaatctaaaa acacttcagc ttaagctatt attgcctgat tctcgtcata tcgctggggc   7080
   ccgcgatcgc acgcgttctg ctataaattg acggagtttc gtacagtgcg ctcgtacagt   7140
   gcgctgccaa atacaattta gtgtagccag attggatggt tgaattgctc ttcacggttg   7200
   cacgctattg gcaaaaaaga gagagccgct ctgaactggt tcatccgcag ctgaccttcg   7260
   aaactcttta atatttaata atattgcagc aaaatctata gcttatgcca catctatacg   7320
   gaagaggtat tcaacattag agcttgtgtc gcccattctc tacacgagcc cacgcatcag   7380
   cagtgagggg cttgtagctc gtgccctcta accagtagat tgtttgtcct gctggggcgg   7440
   gaatctgctg gtttcggaat tctttcttct gaactttgtt gttgccggtg atggtgacgg   7500
   tgtcgacgaa cttaatgaat atcggcacgg catagcgtgg cagcctttcc aaaagatgct   7560
   tgccgagttt atccatatcc agctgttttc taggat                              7596
<210> 74
   <211> 4998
   <212> DNA
   <213> Artificial
<220>
   <223> koCassette
<400> 74
   ggacctgcgc cctaaaatgg gactttgtca aaaaaagaac ggcctcctgc gcgatggaga 60
   gcaatcaaga attcggagtt ccgatgcgaa tccatcaaga aaacggcccc taggcaatct 120
   aaaaccgtgg ccgacatact ataagtcaat tccgctgtac aaataacaag cgatcaatcc 180
   ataatctgag gctcatttca tacggacttt tctaagttca cataattcta tgatgcatac 240
   taacaaatac gatgcacaaa tgggtacaag gcctaaagag ggccacaatc gcgatttact 300
   cgatacggca aatcagttcc acaagtaatt cgctatcgtc ggtgttgtta tacacctctc 360
   ggcttgagtc aatatcgagc atgcaaggtt gacgcattct ggggaaatgt atccacgtga 420
   tcgccgatat cggagcggat acgctgtgta gtcttcagtt gtaagatttc ttatacagcg 480
   acgcaaccat acatgtgacg gcccgtgctg gtctgacggg cggatagtac aggctttgcc 540
   aaaagcctat aaggctaaag aaagtaaaca agtgaggttg aaccatgatg gcagtgttcg 600
   aattctgatc aatgaagtac actgcgaagg gaatccccga aacggcgaac aaaaagaaca 660
   tcagaggagg aacgccctcg caatcccgaa cataccagtt tcgcagaacc tggggtatca 720
   actggatgca ccagcatact gttcccactg ttgccaatgc tgtagacgct ccattgttgt 780
   cagtcatttt agcattttac agtaaccaac tccaaaaaac agcccgctct gctgggaaga 840
   cttcgcaatt atttatccac tactgctgcg gttatatact tctcgatctc agtctcggtt 900
   ataattgccg cttgacagcc tggagaaatt cggatactcc acgtgataat tgccataggg 960
   cataattttc gaaacagctc gcaacgatct cggctagttt tccccttttt tgacccatat 1020
   cgacgctgag actcactcac ttgatgccta ccgttagggt aaatttttca agcctgcaga 1080
   atatcgcggg acgcagtctc ctgcacgcgc gtgacttcat cttacttaca tcaaacagcc 1140
   cgattaattt gaaaagtcct agctgatcga gggcacgggc actactgtag agaaataata 1200
   tgaagctgag ctatgaggag cgccgagaga ggctgccggc tgtagcagcc cggctattcg 1260
   acatcattgt gagcaagcaa acaaatcttt gcgcaagctt ggatgtgcga actacctctg 1320
   agttactgag tatcctggac cgcattggac cttacatttg tatggttaag acccacattg 1380
   acataattga cgacttcgaa tacgacacaa ctgtcagcgg tttgaaacag ctttcaacga 1440
   agcacaattt tctcattttt gaagaccgaa agttcgcaga catcggttcc actgttaagg 1500
   cccaatatgc aggtggagtg tttaagatcg ctcaatgggc tgatataaca aatgctcacg 1560
   gtgttcctgg gccgggaatt gtgagcggac tagaagaggc tgcgaaggaa actacggatg 1620
   aacctcgcgg ccttgtcatg cttgcagaac tgagttcgaa gggcacactg gctcacggcg 1680
   aatactcgca agcgacagta gacatcgctc gcagtaaccg cgcatttgtg tttggtttca 1740
   tcgctcagca aaaagtcgga aagccagagg aagactgggt cattatgact cctggggtgg 1800
   gcctggacga caaaggtgat ggattggggc agcagtatcg tactgtggac gacgtcatag 1860
   agaccggcac agacgttatt atcgtcggac gcgggctcta tagcaaggga cgagatcctg 1920
   tgcacgaagc tcagcgttac caaaaggcgg gctggaatgc atatctgaga aaagttcagt 1980
   caagatgatt ttctcaaaca gttccttcaa tgcaacttgc acatgaatac ctataaaatc 2040
   tgattaaatt accataaaag gtacagatta aaatatatat gccttcaatg gcatccttcg 2100
   cgattctgat tcgtcagcac acttcaacct tcctactatg agtgacagtg atgatgatct 2160
   gctggcattg gccgacgttg gctccgactc cgaagaggaa atctcgctgc cgtcgccgcc 2220
   aagcaatgag gtcgtcaatc cctatcctct agaaggcaaa tatctcgatg ctgaagacag 2280
   ggcgaagttg gacgcgctgc cagagattga gcgagaagag atcttgtatg accgagctca 2340
   ggagatgcag cggtacgagg agagaaggta tcttgctcag cgaaggaagc agatgacgcg 2400
   ggttgctgac gaggacgaag ccccctccgc caagcgtcaa cggggtacaa caggcgtctc 2460
   ttcgggtacg aagtcatctc ttgaggcatt aaagaaacga agggcccagc agtctcggaa 2520
   gtcctcacgc catggagttg atgacgatgt gtatagtgac gatgatgaaa catgtgcgat 2580
   cgctaagcag tacgacgttg ttgctggagt ctcatctgca aggttgagta ccaatccctg 2640
   ccccaatacg agcaatcgaa gccttgggga aagatgcggc gggctagctt cagcaataaa 2700
   tagcaggcga cacacaaaaa ttaggcggca agcgcacgct cagcatgcca tctaccaggg 2760
   caaaaagcaa ggcaacctct ttttcgcatc ccgatttaga gcctacccgt cattgcaggg 2820
   tgtgcgtcta cgatataaca cgatcgacat cgcgctgggt atgcttctgg gtaaggggtc 2880
   gcaacgtgtg agttgtcagc actggccgat acccaaagta tataatgcgc cgttgaacgg 2940
   ttatagtcgg tcaagctctt aaagaaagac ttaacaacaa aaacaactct acacatatgg 3000
   ccatcgagaa accagtgata gttgcttgtg cctgcccact agcggggcac gtgggcccag 3060
   tgctcagcct ggtccgcggt ctactcaata gaggatatga ggtgactttc gtaacaggga 3120
   acgcattcaa ggagaaagtt attgaggcag gatgcacttt cgtccctctc caaggacgag 3180
   ctgactacca tgaatacaat ctccctgaaa tcgctccagg attgctcacg attcctccag 3240
   gccttgagca gaccggttac tcaatgaatg agatttttgt gaaggcgatt cctgagcagt 3300
   acgatgcact tcaaactgct ctaaaacagg ttgaggctga aaataaatca gctgtggtga 3360
   ttggcgagac catgtttcta ggggtgcatc cgatatcact gggtgcccca ggtctcaagc 3420
   cccaaggcgt aatcacgtta ggaactattc cgtgcatgct gaaagcagag aaggcgcctg 3480
   gagttcctag tcttgagcca atgattgata ctttagtgcg gcaacaagta tttcaaccag 3540
   gaactgactc tgagaaggag atcatgaaga cgctcggggc cacgaaggag cccgaatttc 3600
   tcctggagaa tatatacagc agccctgaca gatttttgca actgtgccct ccatctcttg 3660
   aatttcactt gacttcgcct cctcctggct tctcgttcgc tggtagtgca ccgcatgtaa 3720
   agtctgctgg attagcaact ccacctcacc tgccgtcttg gtggcctgat gtgctgagtg 3780
   cgaagcgtct gattgttgtt acacaaggaa cagcagccat caactatgaa gatctgctca 3840
   ttccagcatt gcaggccttt gctgacgaag aagacactct cgtagttggt atattgggcg 3900
   tcaaaggggc gtcacttcct gatagcgtta aagttcctgc aaacgctcga attgttgatt 3960
   attttcctta cgatgagcta ctaccgcatg cctctgtttt catatacaac ggtggatacg 4020
   gaggtctgca gcacagtttg agccatggcg ttcccgtcat catcggagga ggaatgttgg 4080
   tagacaagcc agctgttgct tcacgagctg tatgggctgg tgttggttat gatcttcaaa 4140
   ccttgcaggc aacttctgag ctagtctcca cggccgttaa ggaggtgttg gctactccct 4200
   cgtatcacga gaaagccatg gcagtcaaga aagagcttga aaaatacaag tctcttgata 4260
   ttctagagtc ggcaattagt gaattagctt cttaacctgg ccggccattt ctcctaatag 4320
   gctgtcagcg catatctgag gcgctcatat aaaacaatat aaatcaaaac ccatgttaaa 4380
   aacttgttga tcccagcact tttgagaagc gcactccgaa ctaaatctaa aaacacttca 4440
   gcttaagcta ttattgcctg attctcgtca tatcgctggg gcccgcgatc gcacgcgttc 4500
   tgctataaat tgacggagtt tcgtacagtg cgctcgtaca gtgcgctgcc aaatacaatt 4560
   tagtgtagcc agattggatg gttgaattgc tcttcacggt tgcacgctat tggcaaaaaa 4620
   gagagagccg ctctgaactg gttcatccgc agctgacctt cgaaactctt taatatttaa 4680
   taatattgca gcaaaatcta tagcttatgc cacatctata cggaagaggt attcaacatt 4740
   agagcttgtg tcgcccattc tctacacgag cccacgcatc agcagtgagg ggcttgtagc 4800
   tcgtgccctc taaccagtag attgtttgtc ctgctggggc gggaatctgc tggtttcgga 4860
   attctttctt ctgaactttg ttgttgccgg tgatggtgac ggtgtcgacg aacttaatga 4920
   atatcggcac ggcatagcgt ggcagccttt ccaaaagatg cttgccgagt ttatccatat 4980
   ccagctgttt tctaggat 4998
<210> 75
   <211> 3732
   <212> DNA
   <213> Artificial
<220>
   <223> IntegrationCassette
<400> 75
   gcggccgctt attttttgga cctgcgccct aaaatgggac tttgtcaaaa aaagaacggc    60
   ctcctgcgcg atggagagca atcaagaatt cggagttccg atgcgaatcc atcaagaaaa   120
   cggcccctag gcaatctaaa accgtggccg acatactata agtcaattcc gctgtacaaa   180
   taacaagcga tcaatccata atctgaggct catttcatac ggacttttct aagttcacat   240
   aattctatga tgcatactaa caaatacgat gcacaaatgg gtacaaggcc taaagagggc   300
   cacaatcgcg atttactcga tacggcaaat cagttccaca agtaattcgc tatcgtcggt   360
   gttgttatac acctctcggc ttgagtcaat atcgagcatg caaggttgac gcattctggg   420
   gaaatgtatc cacgtgatcg ccgatatcgg agcggatacg ctgtgtagtc ttcagttgta   480
   agatttctta tacagcgacg caaccataca tgtgacggcc cgtgctggtc tgacgggcgg   540
   atagtacagg ctttgccaaa agcctataag gctaaagaaa gtaaacaagt gaggttgaac   600
   catgatggca gtgttcgaat tctgatcaat gaagtacact gcgaagggaa tccccgaaac   660
   ggcgaacaaa aagaacatca gaggaggaac gccctcgcaa tcccgaacat accagtttcg   720
   cagaacctgg ggtatcaact ggatgcacca gcatactgtt cccactgttg ccaatgctgt   780
   agacgctcca ttgttgtcag tcattttagc attttacagt aaccaactcc aaaaaacagc   840
   ccgctctgct gggaagactt cgcaattatt tatccactac tgctgcggtt atatacttct   900
   cgatctcagt ctcggttata attgccgctt gacagcctgg agaaattcgg atactccacg   960
   tgataattgc catagggcat aattttcgaa acagctcgca acgatctcgg ctagttttcc  1020
   ccttttttga cccatatcga cgctgagact cactcacttg atgcctaccg ttagggtaaa  1080
   tttttcaagc ctgcagaata tcgcgggacg cagtctcctg cacgcgcgtg acttcatctt  1140
   acttacatca aacagcccga ttaatttgaa aagtcctagc tgatcgaggg cacgggcact  1200
   actgtagaga aataatatga agctgagcta tgaggagcgc cgagagaggc tgccggctgt 1260
   agcagcccgg ctattcgaca tcattgtgag caagcaaaca aatctttgcg caagcttgga 1320
   tgtgcgaact acctctgagt tactgagtat cctggaccgc attggacctt acatttgtat 1380
   ggttaagacc cacattgaca taattgacga cttcgaatac gacacaactg tcagcggttt 1440
   gaaacagctt tcaacgaagc acaattttct catttttgaa gaccgaaagt tcgcagacat 1500
   cggttccact gttaaggccc aatatgcagg tggagtgttt aagatcgctc aatgggctga 1560
   tataacaaat gctcacggtg ttcctgggcc gggaattgtg agcggactag aagaggctgc 1620
   gaaggaaact acggatgaac ctcgcggcct tgtcatgctt gcagaactga gttcgaaggg 1680
   cacactggct cacggcgaat actcgcaagc gacagtagac atcgctcgca gtaaccgcgc 1740
   atttgtgttt ggtttcatcg ctcagcaaaa agtcggaaag ccagaggaag actgggtcat 1800
   tatgactcct ggggtgggcc tggacgacaa aggtgatgga ttggggcagc agtatcgtac 1860
   tgtggacgac gtcatagaga ccggcacaga cgttattatc gtcggacgcg ggctctatag 1920
   caagggacga gatcctgtgc acgaagctca gcgttaccaa aaggcgggct ggaatgcata 1980
   tctgagaaaa gttcagtcaa gatgattttc tcaaacagtt ccttcaatgc aacttgcaca 2040
   tgaataccta taaaatctga ttaaattacc ataaaaggta cagattaaaa tatatatgcc 2100
   ttcaatggca tccttcgcga ttctgattcg tcagcacact tcaaccttcc tactatgagt 2160
   gacagtgatg atgatctgct ggcattggcc gacgttggct ccgactccga agaggaaatc 2220
   tcgctgccgt cgccgccaag caatgaggtc gtcaatccct atcctctaga aggcaaatat 2280
   ctcgatgctg aagacagggc gaagttggac gcgctgccag agattgagcg agaagagatc 2340
   ttgtatgacc gagctcagga gatgcagcgg tacgaggaga gaaggtatct tgctcagcga 2400
   aggaagcaga tgacgcgggt tgctgacgag gacgaagccc cctccgccaa gcgtcaacgg 2460
   ggtacaacag gcgtctcttc gggtacgaag tcatctcttg aggcattaaa gaaacgaagg 2520
   gcccagcagt ctcggaagtc ctcacgccat ggagttgatg acgatgtgta tagtgacgat 2580
   gatgaaacat gtgcgatcgc taagcagtac gacgttgttg ctggagtctc atctgcaagg 2640
   ttgagtacca atccctgccc caatacgagc aatcgaagcc ttggggaaag atgcggcggg 2700
   ctagcttcag caataaatag caggcgacac acaaaaatta ggcggcaagc gcacgctcag 2760
   catgccatct accagggcaa aaagcaaggc aacctctttt tcgcatcccg atttagagcc 2820
   tacccgtcat tgcagggtgt gcgtctacga tataacacga tcgacatcgc gctgggtatg 2880
   cttctgggta aggggtcgca acgtgtgagt tgtcagcact ggccgatacc caaagtatat 2940
   aatgcgccgt tgaacggtta tagtcggtca agctcttaaa gaaagactta acaacaaaaa 3000
   caactctaca catatggact tgtaggccgg ccatttctcc taataggctg tcagcgcata 3060
   tctgaggcgc tcatataaaa caatataaat caaaacccat gttaaaaact tgttgatccc 3120
   agcacttttg agaagcgcac tccgaactaa atctaaaaac acttcagctt aagctattat 3180
   tgcctgattc tcgtcatatc gctggggccc gcgatcgcac gcgttctgct ataaattgac 3240
   ggagtttcgt acagtgcgct cgtacagtgc gctgccaaat acaatttagt gtagccagat 3300
   tggatggttg aattgctctt cacggttgca cgctattggc aaaaaagaga gagccgctct 3360
   gaactggttc atccgcagct gaccttcgaa actctttaat atttaataat attgcagcaa 3420
   aatctatagc ttatgccaca tctatacgga agaggtattc aacattagag cttgtgtcgc 3480
   ccattctcta cacgagccca cgcatcagca gtgaggggct tgtagctcgt gccctctaac 3540
   cagtagattg tttgtcctgc tggggcggga atctgctggt ttcggaattc tttcttctga 3600
   actttgttgt tgccggtgat ggtgacggtg tcgacgaact taatgaatat cggcacggca 3660
   tagcgtggca gcctttccaa aagatgcttg ccgagtttat ccatatccag ctgttttcta 3720
   ggatcctgca gg 3732
<210> 76
   <211> 50
   <212> DNA
   <213> Artificial
<220>
   <223> Primer
<400> 76
   aaacgtctca gatgcaccac caccaccacc acatggttgt aaactcctcg 50
<210> 77
   <211> 30
   <212> DNA
   <213> Artificial
<220>
   <223> Primer
<400> 77
   aaaggcgcgc cctagacctt ctggttagcg 30
<210> 78
   <211> 6700
   <212> DNA
   <213> Artificial
<220>
   <223> Vector
<400> 78
   tggcgaatgg gacgcgccct gtagcggcgc attaagcgcg gcgggtgtgg tggttacgcg     60
   cagcgtgacc gctacacttg ccagcgccct agcgcccgct cctttcgctt tcttcccttc    120
   ctttctcgcc acgttcgccg gctttccccg tcaagctcta aatcgggggc tccctttagg    180
   gttccgattt agtgctttac ggcacctcga ccccaaaaaa cttgattagg gtgatggttc    240
   acgtagtggg ccatcgccct gatagacggt ttttcgccct ttgacgttgg agtccacgtt    300
   ctttaatagt ggactcttgt tccaaactgg aacaacactc aaccctatct cggtctattc    360
   ttttgattta taagggattt tgccgatttc ggcctattgg ttaaaaaatg agctgattta    420
   acaaaaattt aacgcgaatt ttaacaaaat attaacgttt acaatttcag gtggcacttt    480
   tcggggaaat gtgcgcggaa cccctatttg tttatttttc taaatacatt caaatatgta    540
   tccgctcatg aattaattct tagaaaaact catcgagcat caaatgaaac tgcaatttat    600
   tcatatcagg attatcaata ccatattttt gaaaaagccg tttctgtaat gaaggagaaa    660
   actcaccgag gcagttccat aggatggcaa gatcctggta tcggtctgcg attccgactc    720
   gtccaacatc aatacaacct attaatttcc cctcgtcaaa aataaggtta tcaagtgaga    780
   aatcaccatg agtgacgact gaatccggtg agaatggcaa aagtttatgc atttctttcc    840
   agacttgttc aacaggccag ccattacgct cgtcatcaaa atcactcgca tcaaccaaac    900
   cgttattcat tcgtgattgc gcctgagcga gacgaaatac gcgatcgctg ttaaaaggac    960
   aattacaaac aggaatcgaa tgcaaccggc gcaggaacac tgccagcgca tcaacaatat   1020
   tttcacctga atcaggatat tcttctaata cctggaatgc tgttttcccg gggatcgcag   1080
   tggtgagtaa ccatgcatca tcaggagtac ggataaaatg cttgatggtc ggaagaggca   1140
   taaattccgt cagccagttt agtctgacca tctcatctgt aacatcattg gcaacgctac   1200
   ctttgccatg tttcagaaac aactctggcg catcgggctt cccatacaat cgatagattg   1260
   tcgcacctga ttgcccgaca ttatcgcgag cccatttata cccatataaa tcagcatcca   1320
   tgttggaatt taatcgcggc ctagagcaag acgtttcccg ttgaatatgg ctcataacac   1380
   cccttgtatt actgtttatg taagcagaca gttttattgt tcatgaccaa aatcccttaa   1440
   cgtgagtttt cgttccactg agcgtcagac cccgtagaaa agatcaaagg atcttcttga   1500
   gatccttttt ttctgcgcgt aatctgctgc ttgcaaacaa aaaaaccacc gctaccagcg   1560
   gtggtttgtt tgccggatca agagctacca actctttttc cgaaggtaac tggcttcagc   1620
   agagcgcaga taccaaatac tgtccttcta gtgtagccgt agttaggcca ccacttcaag   1680
   aactctgtag caccgcctac atacctcgct ctgctaatcc tgttaccagt ggctgctgcc   1740
   agtggcgata agtcgtgtct taccgggttg gactcaagac gatagttacc ggataaggcg   1800
   cagcggtcgg gctgaacggg gggttcgtgc acacagccca gcttggagcg aacgacctac   1860
   accgaactga gatacctaca gcgtgagcta tgagaaagcg ccacgcttcc cgaagggaga   1920
   aaggcggaca ggtatccggt aagcggcagg gtcggaacag gagagcgcac gagggagctt   1980
   ccagggggaa acgcctggta tctttatagt cctgtcgggt ttcgccacct ctgacttgag   2040
   cgtcgatttt tgtgatgctc gtcagggggg cggagcctat ggaaaaacgc cagcaacgcg   2100
   gcctttttac ggttcctggc cttttgctgg ccttttgctc acatgttctt tcctgcgtta   2160
   tcccctgatt ctgtggataa ccgtattacc gcctttgagt gagctgatac cgctcgccgc   2220
   agccgaacga ccgagcgcag cgagtcagtg agcgaggaag cggaagagcg cctgatgcgg 2280
   tattttctcc ttacgcatct gtgcggtatt tcacaccgca tatatggtgc actctcagta 2340
   caatctgctc tgatgccgca tagttaagcc agtatacact ccgctatcgc tacgtgactg 2400
   ggtcatggct gcgccccgac acccgccaac acccgctgac gcgccctgac gggcttgtct 2460
   gctcccggca tccgcttaca gacaagctgt gaccgtctcc gggagctgca tgtgtcagag 2520
   gttttcaccg tcatcaccga aacgcgcgag gcagctgcgg taaagctcat cagcgtggtc 2580
   gtgaagcgat tcacagatgt ctgcctgttc atccgcgtcc agctcgttga gtttctccag 2640
   aagcgttaat gtctggcttc tgataaagcg ggccatgtta agggcggttt tttcctgttt 2700
   ggtcactgat gcctccgtgt aagggggatt tctgttcatg ggggtaatga taccgatgaa 2760
   acgagagagg atgctcacga tacgggttac tgatgatgaa catgcccggt tactggaacg 2820
   ttgtgagggt aaacaactgg cggtatggat gcggcgggac cagagaaaaa tcactcaggg 2880
   tcaatgccag cgcttcgtta atacagatgt aggtgttcca cagggtagcc agcagcatcc 2940
   tgcgatgcag atccggaaca taatggtgca gggcgctgac ttccgcgttt ccagacttta 3000
   cgaaacacgg aaaccgaaga ccattcatgt tgttgctcag gtcgcagacg ttttgcagca 3060
   gcagtcgctt cacgttcgct cgcgtatcgg tgattcattc tgctaaccag taaggcaacc 3120
   ccgccagcct agccgggtcc tcaacgacag gagcacgatc atgcgcaccc gtggggccgc 3180
   catgccggcg ataatggcct gcttctcgcc gaaacgtttg gtggcgggac cagtgacgaa 3240
   ggcttgagcg agggcgtgca agattccgaa taccgcaagc gacaggccga tcatcgtcgc 3300
   gctccagcga aagcggtcct cgccgaaaat gacccagagc gctgccggca cctgtcctac 3360
   gagttgcatg ataaagaaga cagtcataag tgcggcgacg atagtcatgc cccgcgccca 3420
   ccggaaggag ctgactgggt tgaaggctct caagggcatc ggtcgagatc ccggtgccta 3480
   atgagtgagc taacttacat taattgcgtt gcgctcactg cccgctttcc agtcgggaaa 3540
   cctgtcgtgc cagctgcatt aatgaatcgg ccaacgcgcg gggagaggcg gtttgcgtat 3600
   tgggcgccag ggtggttttt cttttcacca gtgagacggg caacagctga ttgcccttca 3660
   ccgcctggcc ctgagagagt tgcagcaagc ggtccacgct ggtttgcccc agcaggcgaa 3720
   aatcctgttt gatggtggtt aacggcggga tataacatga gctgtcttcg gtatcgtcgt 3780
   atcccactac cgagatatcc gcaccaacgc gcagcccgga ctcggtaatg gcgcgcattg 3840
   cgcccagcgc catctgatcg ttggcaacca gcatcgcagt gggaacgatg ccctcattca 3900
   gcatttgcat ggtttgttga aaaccggaca tggcactcca gtcgccttcc cgttccgcta 3960
   tcggctgaat ttgattgcga gtgagatatt tatgccagcc agccagacgc agacgcgccg 4020
   agacagaact taatgggccc gctaacagcg cgatttgctg gtgacccaat gcgaccagat 4080
   gctccacgcc cagtcgcgta ccgtcttcat gggagaaaat aatactgttg atgggtgtct 4140
   ggtcagagac atcaagaaat aacgccggaa cattagtgca ggcagcttcc acagcaatgg 4200
   catcctggtc atccagcgga tagttaatga tcagcccact gacgcgttgc gcgagaagat 4260
   tgtgcaccgc cgctttacag gcttcgacgc cgcttcgttc taccatcgac accaccacgc 4320
   tggcacccag ttgatcggcg cgagatttaa tcgccgcgac aatttgcgac ggcgcgtgca 4380
   gggccagact ggaggtggca acgccaatca gcaacgactg tttgcccgcc agttgttgtg 4440
   ccacgcggtt gggaatgtaa ttcagctccg ccatcgccgc ttccactttt tcccgcgttt 4500
   tcgcagaaac gtggctggcc tggttcacca cgcgggaaac ggtctgataa gagacaccgg 4560
   catactctgc gacatcgtat aacgttactg gtttcacatt caccaccctg aattgactct 4620
   cttccgggcg ctatcatgcc ataccgcgaa aggttttgcg ccattcgatg gtgtccggga 4680
   tctcgacgct ctcccttatg cgactcctgc attaggaagc agcccagtag taggttgagg 4740
   ccgttgagca ccgccgccgc aaggaatggt gcatgcaagg agatggcgcc caacagtccc 4800
   ccggccacgg ggcctgccac catacccacg ccgaaacaag cgctcatgag cccgaagtgg 4860
   cgagcccgat cttccccatc ggtgatgtcg gcgatatagg cgccagcaac cgcacctgtg 4920
   gcgccggtga tgccggccac gatgcgtccg gcgtagagga tcgagatctc gatcccgcga 4980
   aattaatacg actcactata ggggaattgt gagcggataa caattcccct ctagaaataa 5040
   ttttgtttaa ctttaagaag gagatatacg atgcaccacc accaccacca catgagccct 5100
   tcatcacaca aacccctgat tctcgcttgc ggcttgcctc tttcaggcca tataatgccc 5160
   gttttgagtc tggtacacgg ccttacggac gacggatacg aagctactgt tgtgacaggc 5220
   agagcgtttg aacaaaaagt tcgagatgtg ggtgcagact ttgttccttt agaagggaac 5280
   gcagattttg atgaccacac cttagacgat ctggtcccgg gccgtaaaga catggcccca 5340
   agcttcgatc gtacagttca agatgtggag cacatgatgg tagctactct tcctgagcag 5400
   tttgccgcta ttcagagggc tttcaaaaag ctcagcgcaa gcggtcgccc tgtcgttctt 5460
   gtcagtgaag tgctgttttt cggtgcacac cctatcagcc tcggtgctcc tggtttcaaa 5520
   cccgctggct ggatttgttt aggggttttg cctcttttga tccgcagtga tcatacctta 5580
   ggacttgaca acgacaggag ccccgaagca catgcaaaga aactcgctat gaaccacgct 5640
   cttgagcacc aaattttcgt taaagccact gctaagcaca aggaaatctg ccgagagtta 5700
   ggttgcactg aagatcccaa atttatctgg gagcacagtt acattgctgc agacaagttc 5760
   ctgcagctgt gcccgccttc tcttgagttc agcagagacc atctgcctag caacttcaaa 5820
   ttcgccggct caacgcccaa gcaccgaact caattcaccc ctccttcctg gtggggggat 5880
   gttctgagtg ccaagcgagt catcatggtc actcaaggaa cttttgctgt cagttacaag 5940
   catcttattg tgcctactct tgaggccttg aaggacgagc ctgacacttt aacagtagcc 6000
   atattgggcc gccgcggtgc caagctaccg gatgatgttg tggttcctga gaatgctcgc 6060
   gtgatcgact acttcaacta cgatgctcta cttcctcacg ttgatgctct tgtctacaat 6120
   ggtggatatg gcggacttca gcacagctta agccactctg ttccagttgt tattgctggt 6180
   gactctgaag acaagccaat ggtggcatcg agagctgagg ccgctggcgt ggcaattgat 6240
   ttgaaaactg gcttgcctac agtggagcaa atcaaagaag ctgttgattc gataattgga 6300
   aatccgaaat tccacgaagc ctcgaagaag gttcaaatgg agttggaaag ccacaactcc 6360
   ttgaaaattc ttgaggaaag catcgaggaa atcgccagcc atgactttgg tcttttgacc 6420
   aagagtgacg aggaaactga agatatacct gtcaaaggtc cggccttagc ggtgagttct 6480
   tagggcgcgc cctcgaggga tccgaattcg agctccgtcg acaagcttgc ggccgcactc 6540
   gagcaccacc accaccacca ctgagatccg gctgctaaca aagcccgaaa ggaagctgag 6600
   ttggctgctg ccaccgctga gcaataacta gcataacccc ttggggcctc taaacgggtc 6660
   ttgaggggtt ttttgctgaa aggaggaact atatccggat 6700
<210> 79
   <211> 51
   <212> DNA
   <213> Artificial
<220>
   <223> Primer
<400> 79
   aaacgtctca gatgcaccac caccaccacc acatggccat cgagaaacca g 51
<210> 80
   <211> 36
   <212> DNA
   <213> Artificial
<220>
   <223> Primer
<400> 80
   aaaggcgcgc cttaagaagc taattcacta attgcc 36
<210> 81
   <211> 6607
   <212> DNA
   <213> Artificial
<220>
   <223> Vector
<400> 81
   tggcgaatgg gacgcgccct gtagcggcgc attaagcgcg gcgggtgtgg tggttacgcg 60
   cagcgtgacc gctacacttg ccagcgccct agcgcccgct cctttcgctt tcttcccttc 120
   ctttctcgcc acgttcgccg gctttccccg tcaagctcta aatcgggggc tccctttagg 180
   gttccgattt agtgctttac ggcacctcga ccccaaaaaa cttgattagg gtgatggttc 240
   acgtagtggg ccatcgccct gatagacggt ttttcgccct ttgacgttgg agtccacgtt 300
   ctttaatagt ggactcttgt tccaaactgg aacaacactc aaccctatct cggtctattc 360
   ttttgattta taagggattt tgccgatttc ggcctattgg ttaaaaaatg agctgattta 420
   acaaaaattt aacgcgaatt ttaacaaaat attaacgttt acaatttcag gtggcacttt 480
   tcggggaaat gtgcgcggaa cccctatttg tttatttttc taaatacatt caaatatgta 540
   tccgctcatg aattaattct tagaaaaact catcgagcat caaatgaaac tgcaatttat 600
   tcatatcagg attatcaata ccatattttt gaaaaagccg tttctgtaat gaaggagaaa 660
   actcaccgag gcagttccat aggatggcaa gatcctggta tcggtctgcg attccgactc 720
   gtccaacatc aatacaacct attaatttcc cctcgtcaaa aataaggtta tcaagtgaga 780
   aatcaccatg agtgacgact gaatccggtg agaatggcaa aagtttatgc atttctttcc 840
   agacttgttc aacaggccag ccattacgct cgtcatcaaa atcactcgca tcaaccaaac 900
   cgttattcat tcgtgattgc gcctgagcga gacgaaatac gcgatcgctg ttaaaaggac 960
   aattacaaac aggaatcgaa tgcaaccggc gcaggaacac tgccagcgca tcaacaatat 1020
   tttcacctga atcaggatat tcttctaata cctggaatgc tgttttcccg gggatcgcag 1080
   tggtgagtaa ccatgcatca tcaggagtac ggataaaatg cttgatggtc ggaagaggca 1140
   taaattccgt cagccagttt agtctgacca tctcatctgt aacatcattg gcaacgctac 1200
   ctttgccatg tttcagaaac aactctggcg catcgggctt cccatacaat cgatagattg 1260
   tcgcacctga ttgcccgaca ttatcgcgag cccatttata cccatataaa tcagcatcca 1320
   tgttggaatt taatcgcggc ctagagcaag acgtttcccg ttgaatatgg ctcataacac 1380
   cccttgtatt actgtttatg taagcagaca gttttattgt tcatgaccaa aatcccttaa 1440
   cgtgagtttt cgttccactg agcgtcagac cccgtagaaa agatcaaagg atcttcttga 1500
   gatccttttt ttctgcgcgt aatctgctgc ttgcaaacaa aaaaaccacc gctaccagcg 1560
   gtggtttgtt tgccggatca agagctacca actctttttc cgaaggtaac tggcttcagc 1620
   agagcgcaga taccaaatac tgtccttcta gtgtagccgt agttaggcca ccacttcaag 1680
   aactctgtag caccgcctac atacctcgct ctgctaatcc tgttaccagt ggctgctgcc 1740
   agtggcgata agtcgtgtct taccgggttg gactcaagac gatagttacc ggataaggcg 1800
   cagcggtcgg gctgaacggg gggttcgtgc acacagccca gcttggagcg aacgacctac 1860
   accgaactga gatacctaca gcgtgagcta tgagaaagcg ccacgcttcc cgaagggaga 1920
   aaggcggaca ggtatccggt aagcggcagg gtcggaacag gagagcgcac gagggagctt 1980
   ccagggggaa acgcctggta tctttatagt cctgtcgggt ttcgccacct ctgacttgag 2040
   cgtcgatttt tgtgatgctc gtcagggggg cggagcctat ggaaaaacgc cagcaacgcg 2100
   gcctttttac ggttcctggc cttttgctgg ccttttgctc acatgttctt tcctgcgtta 2160
   tcccctgatt ctgtggataa ccgtattacc gcctttgagt gagctgatac cgctcgccgc 2220
   agccgaacga ccgagcgcag cgagtcagtg agcgaggaag cggaagagcg cctgatgcgg 2280
   tattttctcc ttacgcatct gtgcggtatt tcacaccgca tatatggtgc actctcagta 2340
   caatctgctc tgatgccgca tagttaagcc agtatacact ccgctatcgc tacgtgactg 2400
   ggtcatggct gcgccccgac acccgccaac acccgctgac gcgccctgac gggcttgtct 2460
   gctcccggca tccgcttaca gacaagctgt gaccgtctcc gggagctgca tgtgtcagag 2520
   gttttcaccg tcatcaccga aacgcgcgag gcagctgcgg taaagctcat cagcgtggtc 2580
   gtgaagcgat tcacagatgt ctgcctgttc atccgcgtcc agctcgttga gtttctccag 2640
   aagcgttaat gtctggcttc tgataaagcg ggccatgtta agggcggttt tttcctgttt 2700
   ggtcactgat gcctccgtgt aagggggatt tctgttcatg ggggtaatga taccgatgaa 2760
   acgagagagg atgctcacga tacgggttac tgatgatgaa catgcccggt tactggaacg 2820
   ttgtgagggt aaacaactgg cggtatggat gcggcgggac cagagaaaaa tcactcaggg 2880
   tcaatgccag cgcttcgtta atacagatgt aggtgttcca cagggtagcc agcagcatcc 2940
   tgcgatgcag atccggaaca taatggtgca gggcgctgac ttccgcgttt ccagacttta 3000
   cgaaacacgg aaaccgaaga ccattcatgt tgttgctcag gtcgcagacg ttttgcagca 3060
   gcagtcgctt cacgttcgct cgcgtatcgg tgattcattc tgctaaccag taaggcaacc 3120
   ccgccagcct agccgggtcc tcaacgacag gagcacgatc atgcgcaccc gtggggccgc 3180
   catgccggcg ataatggcct gcttctcgcc gaaacgtttg gtggcgggac cagtgacgaa 3240
   ggcttgagcg agggcgtgca agattccgaa taccgcaagc gacaggccga tcatcgtcgc 3300
   gctccagcga aagcggtcct cgccgaaaat gacccagagc gctgccggca cctgtcctac 3360
   gagttgcatg ataaagaaga cagtcataag tgcggcgacg atagtcatgc cccgcgccca 3420
   ccggaaggag ctgactgggt tgaaggctct caagggcatc ggtcgagatc ccggtgccta 3480
   atgagtgagc taacttacat taattgcgtt gcgctcactg cccgctttcc agtcgggaaa 3540
   cctgtcgtgc cagctgcatt aatgaatcgg ccaacgcgcg gggagaggcg gtttgcgtat 3600
   tgggcgccag ggtggttttt cttttcacca gtgagacggg caacagctga ttgcccttca 3660
   ccgcctggcc ctgagagagt tgcagcaagc ggtccacgct ggtttgcccc agcaggcgaa 3720
   aatcctgttt gatggtggtt aacggcggga tataacatga gctgtcttcg gtatcgtcgt 3780
   atcccactac cgagatatcc gcaccaacgc gcagcccgga ctcggtaatg gcgcgcattg 3840
   cgcccagcgc catctgatcg ttggcaacca gcatcgcagt gggaacgatg ccctcattca 3900
   gcatttgcat ggtttgttga aaaccggaca tggcactcca gtcgccttcc cgttccgcta 3960
   tcggctgaat ttgattgcga gtgagatatt tatgccagcc agccagacgc agacgcgccg 4020
   agacagaact taatgggccc gctaacagcg cgatttgctg gtgacccaat gcgaccagat 4080
   gctccacgcc cagtcgcgta ccgtcttcat gggagaaaat aatactgttg atgggtgtct 4140
   ggtcagagac atcaagaaat aacgccggaa cattagtgca ggcagcttcc acagcaatgg 4200
   catcctggtc atccagcgga tagttaatga tcagcccact gacgcgttgc gcgagaagat 4260
   tgtgcaccgc cgctttacag gcttcgacgc cgcttcgttc taccatcgac accaccacgc 4320
   tggcacccag ttgatcggcg cgagatttaa tcgccgcgac aatttgcgac ggcgcgtgca 4380
   gggccagact ggaggtggca acgccaatca gcaacgactg tttgcccgcc agttgttgtg 4440
   ccacgcggtt gggaatgtaa ttcagctccg ccatcgccgc ttccactttt tcccgcgttt 4500
   tcgcagaaac gtggctggcc tggttcacca cgcgggaaac ggtctgataa gagacaccgg 4560
   catactctgc gacatcgtat aacgttactg gtttcacatt caccaccctg aattgactct 4620
   cttccgggcg ctatcatgcc ataccgcgaa aggttttgcg ccattcgatg gtgtccggga 4680
   tctcgacgct ctcccttatg cgactcctgc attaggaagc agcccagtag taggttgagg 4740
   ccgttgagca ccgccgccgc aaggaatggt gcatgcaagg agatggcgcc caacagtccc 4800
   ccggccacgg ggcctgccac catacccacg ccgaaacaag cgctcatgag cccgaagtgg 4860
   cgagcccgat cttccccatc ggtgatgtcg gcgatatagg cgccagcaac cgcacctgtg 4920
   gcgccggtga tgccggccac gatgcgtccg gcgtagagga tcgagatctc gatcccgcga 4980
   aattaatacg actcactata ggggaattgt gagcggataa caattcccct ctagaaataa 5040
   ttttgtttaa ctttaagaag gagatatacg atgcaccacc accaccacca catggccatc 5100
   gagaaaccag tgatagttgc ttgtgcctgc ccactagcgg ggcacgtggg cccagtgctc 5160
   agcctggtcc gcggtctact caatagagga tatgaggtga ctttcgtaac agggaacgca 5220
   ttcaaggaga aagttattga ggcaggatgc actttcgtcc ctctccaagg acgagctgac 5280
   taccatgaat acaatctccc tgaaatcgct ccaggattgc tcacgattcc tccaggcctt 5340
   gagcagaccg gttactcaat gaatgagatt tttgtgaagg cgattcctga gcagtacgat 5400
   gcacttcaaa ctgctctaaa acaggttgag gctgaaaata aatcagctgt ggtgattggc 5460
   gagaccatgt ttctaggggt gcatccgata tcactgggtg ccccaggtct caagccccaa 5520
   ggcgtaatca cgttaggaac tattccgtgc atgctgaaag cagagaaggc gcctggagtt 5580
   cctagtcttg agccaatgat tgatacttta gtgcggcaac aagtatttca accaggaact 5640
   gactctgaga aggagatcat gaagacgctc ggggccacga aggagcccga atttctcctg 5700
   gagaatatat acagcagccc tgacagattt ttgcaactgt gccctccatc tcttgaattt 5760
   cacttgactt cgcctcctcc tggcttctcg ttcgctggta gtgcaccgca tgtaaagtct 5820
   gctggattag caactccacc tcacctgccg tcttggtggc ctgatgtgct gagtgcgaag 5880
   cgtctgattg ttgttacaca aggaacagca gccatcaact atgaagatct gctcattcca 5940
   gcattgcagg cctttgctga cgaagaagac actctcgtag ttggtatatt gggcgtcaaa 6000
   ggggcgtcac ttcctgatag cgttaaagtt cctgcaaacg ctcgaattgt tgattatttt 6060
   ccttacgatg agctactacc gcatgcctct gttttcatat acaacggtgg atacggaggt 6120
   ctgcagcaca gtttgagcca tggcgttccc gtcatcatcg gaggaggaat gttggtagac 6180
   aagccagctg ttgcttcacg agctgtatgg gctggtgttg gttatgatct tcaaaccttg 6240
   caggcaactt ctgagctagt ctccacggcc gttaaggagg tgttggctac tccctcgtat 6300
   cacgagaaag ccatggcagt caagaaagag cttgaaaaat acaagtctct tgatattcta 6360
   gagtcggcaa ttagtgaatt agcttcttaa ggcgcgccct cgagggatcc gaattcgagc 6420
   tccgtcgaca agcttgcggc cgcactcgag caccaccacc accaccactg agatccggct 6480
   gctaacaaag cccgaaagga agctgagttg gctgctgcca ccgctgagca ataactagca 6540
   taaccccttg gggcctctaa acgggtcttg aggggttttt tgctgaaagg aggaactata 6600
   tccggat 6607
<210> 82
   <211> 50
   <212> DNA
   <213> Artificial
<220>
   <223> Primer
<400> 82
   aaacgtctca gatgcaccac caccaccacc acatggttgt aaactcctcg 50
<210> 83
   <211> 30
   <212> DNA
   <213> Artificial
<220>
   <223> Primer
<400> 83
   aaaggcgcgc cctagacctt ctggttagcg 30
<210> 84
   <211> 6088
   <212> DNA
   <213> Artificial
<220>
   <223> Vector
<400> 84
   tggcgaatgg gacgcgccct gtagcggcgc attaagcgcg gcgggtgtgg tggttacgcg     60
   cagcgtgacc gctacacttg ccagcgccct agcgcccgct cctttcgctt tcttcccttc    120
   ctttctcgcc acgttcgccg gctttccccg tcaagctcta aatcgggggc tccctttagg    180
   gttccgattt agtgctttac ggcacctcga ccccaaaaaa cttgattagg gtgatggttc    240
   acgtagtggg ccatcgccct gatagacggt ttttcgccct ttgacgttgg agtccacgtt    300
   ctttaatagt ggactcttgt tccaaactgg aacaacactc aaccctatct cggtctattc    360
   ttttgattta taagggattt tgccgatttc ggcctattgg ttaaaaaatg agctgattta    420
   acaaaaattt aacgcgaatt ttaacaaaat attaacgttt acaatttcag gtggcacttt    480
   tcggggaaat gtgcgcggaa cccctatttg tttatttttc taaatacatt caaatatgta 540
   tccgctcatg aattaattct tagaaaaact catcgagcat caaatgaaac tgcaatttat 600
   tcatatcagg attatcaata ccatattttt gaaaaagccg tttctgtaat gaaggagaaa 660
   actcaccgag gcagttccat aggatggcaa gatcctggta tcggtctgcg attccgactc 720
   gtccaacatc aatacaacct attaatttcc cctcgtcaaa aataaggtta tcaagtgaga 780
   aatcaccatg agtgacgact gaatccggtg agaatggcaa aagtttatgc atttctttcc 840
   agacttgttc aacaggccag ccattacgct cgtcatcaaa atcactcgca tcaaccaaac 900
   cgttattcat tcgtgattgc gcctgagcga gacgaaatac gcgatcgctg ttaaaaggac 960
   aattacaaac aggaatcgaa tgcaaccggc gcaggaacac tgccagcgca tcaacaatat 1020
   tttcacctga atcaggatat tcttctaata cctggaatgc tgttttcccg gggatcgcag 1080
   tggtgagtaa ccatgcatca tcaggagtac ggataaaatg cttgatggtc ggaagaggca 1140
   taaattccgt cagccagttt agtctgacca tctcatctgt aacatcattg gcaacgctac 1200
   ctttgccatg tttcagaaac aactctggcg catcgggctt cccatacaat cgatagattg 1260
   tcgcacctga ttgcccgaca ttatcgcgag cccatttata cccatataaa tcagcatcca 1320
   tgttggaatt taatcgcggc ctagagcaag acgtttcccg ttgaatatgg ctcataacac 1380
   cccttgtatt actgtttatg taagcagaca gttttattgt tcatgaccaa aatcccttaa 1440
   cgtgagtttt cgttccactg agcgtcagac cccgtagaaa agatcaaagg atcttcttga 1500
   gatccttttt ttctgcgcgt aatctgctgc ttgcaaacaa aaaaaccacc gctaccagcg 1560
   gtggtttgtt tgccggatca agagctacca actctttttc cgaaggtaac tggcttcagc 1620
   agagcgcaga taccaaatac tgtccttcta gtgtagccgt agttaggcca ccacttcaag 1680
   aactctgtag caccgcctac atacctcgct ctgctaatcc tgttaccagt ggctgctgcc 1740
   agtggcgata agtcgtgtct taccgggttg gactcaagac gatagttacc ggataaggcg 1800
   cagcggtcgg gctgaacggg gggttcgtgc acacagccca gcttggagcg aacgacctac 1860
   accgaactga gatacctaca gcgtgagcta tgagaaagcg ccacgcttcc cgaagggaga 1920
   aaggcggaca ggtatccggt aagcggcagg gtcggaacag gagagcgcac gagggagctt 1980
   ccagggggaa acgcctggta tctttatagt cctgtcgggt ttcgccacct ctgacttgag 2040
   cgtcgatttt tgtgatgctc gtcagggggg cggagcctat ggaaaaacgc cagcaacgcg 2100
   gcctttttac ggttcctggc cttttgctgg ccttttgctc acatgttctt tcctgcgtta 2160
   tcccctgatt ctgtggataa ccgtattacc gcctttgagt gagctgatac cgctcgccgc 2220
   agccgaacga ccgagcgcag cgagtcagtg agcgaggaag cggaagagcg cctgatgcgg 2280
   tattttctcc ttacgcatct gtgcggtatt tcacaccgca tatatggtgc actctcagta 2340
   caatctgctc tgatgccgca tagttaagcc agtatacact ccgctatcgc tacgtgactg 2400
   ggtcatggct gcgccccgac acccgccaac acccgctgac gcgccctgac gggcttgtct 2460
   gctcccggca tccgcttaca gacaagctgt gaccgtctcc gggagctgca tgtgtcagag 2520
   gttttcaccg tcatcaccga aacgcgcgag gcagctgcgg taaagctcat cagcgtggtc 2580
   gtgaagcgat tcacagatgt ctgcctgttc atccgcgtcc agctcgttga gtttctccag 2640
   aagcgttaat gtctggcttc tgataaagcg ggccatgtta agggcggttt tttcctgttt 2700
   ggtcactgat gcctccgtgt aagggggatt tctgttcatg ggggtaatga taccgatgaa 2760
   acgagagagg atgctcacga tacgggttac tgatgatgaa catgcccggt tactggaacg 2820
   ttgtgagggt aaacaactgg cggtatggat gcggcgggac cagagaaaaa tcactcaggg 2880
   tcaatgccag cgcttcgtta atacagatgt aggtgttcca cagggtagcc agcagcatcc 2940
   tgcgatgcag atccggaaca taatggtgca gggcgctgac ttccgcgttt ccagacttta 3000
   cgaaacacgg aaaccgaaga ccattcatgt tgttgctcag gtcgcagacg ttttgcagca 3060
   gcagtcgctt cacgttcgct cgcgtatcgg tgattcattc tgctaaccag taaggcaacc 3120
   ccgccagcct agccgggtcc tcaacgacag gagcacgatc atgcgcaccc gtggggccgc 3180
   catgccggcg ataatggcct gcttctcgcc gaaacgtttg gtggcgggac cagtgacgaa 3240
   ggcttgagcg agggcgtgca agattccgaa taccgcaagc gacaggccga tcatcgtcgc 3300
   gctccagcga aagcggtcct cgccgaaaat gacccagagc gctgccggca cctgtcctac 3360
   gagttgcatg ataaagaaga cagtcataag tgcggcgacg atagtcatgc cccgcgccca 3420
   ccggaaggag ctgactgggt tgaaggctct caagggcatc ggtcgagatc ccggtgccta 3480
   atgagtgagc taacttacat taattgcgtt gcgctcactg cccgctttcc agtcgggaaa 3540
   cctgtcgtgc cagctgcatt aatgaatcgg ccaacgcgcg gggagaggcg gtttgcgtat 3600
   tgggcgccag ggtggttttt cttttcacca gtgagacggg caacagctga ttgcccttca 3660
   ccgcctggcc ctgagagagt tgcagcaagc ggtccacgct ggtttgcccc agcaggcgaa 3720
   aatcctgttt gatggtggtt aacggcggga tataacatga gctgtcttcg gtatcgtcgt 3780
   atcccactac cgagatatcc gcaccaacgc gcagcccgga ctcggtaatg gcgcgcattg 3840
   cgcccagcgc catctgatcg ttggcaacca gcatcgcagt gggaacgatg ccctcattca 3900
   gcatttgcat ggtttgttga aaaccggaca tggcactcca gtcgccttcc cgttccgcta 3960
   tcggctgaat ttgattgcga gtgagatatt tatgccagcc agccagacgc agacgcgccg 4020
   agacagaact taatgggccc gctaacagcg cgatttgctg gtgacccaat gcgaccagat 4080
   gctccacgcc cagtcgcgta ccgtcttcat gggagaaaat aatactgttg atgggtgtct 4140
   ggtcagagac atcaagaaat aacgccggaa cattagtgca ggcagcttcc acagcaatgg 4200
   catcctggtc atccagcgga tagttaatga tcagcccact gacgcgttgc gcgagaagat 4260
   tgtgcaccgc cgctttacag gcttcgacgc cgcttcgttc taccatcgac accaccacgc 4320
   tggcacccag ttgatcggcg cgagatttaa tcgccgcgac aatttgcgac ggcgcgtgca 4380
   gggccagact ggaggtggca acgccaatca gcaacgactg tttgcccgcc agttgttgtg 4440
   ccacgcggtt gggaatgtaa ttcagctccg ccatcgccgc ttccactttt tcccgcgttt 4500
   tcgcagaaac gtggctggcc tggttcacca cgcgggaaac ggtctgataa gagacaccgg 4560
   catactctgc gacatcgtat aacgttactg gtttcacatt caccaccctg aattgactct 4620
   cttccgggcg ctatcatgcc ataccgcgaa aggttttgcg ccattcgatg gtgtccggga 4680
   tctcgacgct ctcccttatg cgactcctgc attaggaagc agcccagtag taggttgagg 4740
   ccgttgagca ccgccgccgc aaggaatggt gcatgcaagg agatggcgcc caacagtccc 4800
   ccggccacgg ggcctgccac catacccacg ccgaaacaag cgctcatgag cccgaagtgg 4860
   cgagcccgat cttccccatc ggtgatgtcg gcgatatagg cgccagcaac cgcacctgtg 4920
   gcgccggtga tgccggccac gatgcgtccg gcgtagagga tcgagatctc gatcccgcga 4980
   aattaatacg actcactata ggggaattgt gagcggataa caattcccct ctagaaataa 5040
   ttttgtttaa ctttaagaag gagatatacg atgcaccacc accaccacca catggttgta 5100
   aactcctcga aggaccctca aaacaaagga atgactccta gaaaagaaat tgaccaggaa 5160
   atggtctctt gggccaaaaa aaacctcaaa aacacccctg gcaatgaaaa ctatgagaag 5220
   atggtctcag gagttcctta caatccatac gatccagatc ttatgtttag agccctggct 5280
   actagtgaga aagttaggga gttcaatacc attgcaagtg aaagtcgtac ttttgagtca 5340
   aatcacgctg cttatatcaa gaaggtcgag attctcaaag acacttttgg tcaaacaaag 5400
   gatattgtct ggctgaccgc tccattctca gttgattttg gattcaacat cagcgtaggc 5460
   gagcactttt acgccaactt caacgtttgc ttcttggact cggctccaat aatctttggt 5520
   gatgaggtga ttgtagggcc caatacaacg ttcgtgactg cgactcatcc tattagcccc 5580
   gagaaacgtg cgaggagaat tgtgtatgct cttcctatca aggtggggaa taatgtatgg 5640
   attggtgcga atgtgactgt cctgccgggt gttacgattg gagatggctc aacaattgcg 5700
   gctggtgctg tcgttcgaga agatgttcct cctcgtactg tggtgggagg agtccctgcg 5760
   cgaatcctca agcatattcc agaggaggat cccgacgagg ctgaaggaga ggaactggaa 5820
   ttccttcttc cagttgaaat gaacgtcaat accgctaacc agaaggtcta gggcgcgccc 5880
   tcgagggatc cgaattcgag ctccgtcgac aagcttgcgg ccgcactcga gcaccaccac 5940
   caccaccact gagatccggc tgctaacaaa gcccgaaagg aagctgagtt ggctgctgcc 6000
   accgctgagc aataactagc ataacccctt ggggcctcta aacgggtctt gaggggtttt 6060
   ttgctgaaag gaggaactat atccggat 6088
<210> 85
   <211> 10065
   <212> DNA
   <213> Artificial
<220>
   <223> Vector
<220>
   <221> misc_feature
   <222> (3998) .. (3998)
   <223> n is a, c, g, or t
<400> 85
   gaattctcat gtttgacagc ttatcatcga taagctttaa tgcggtagtt tatcacagtt 60
   aaattgctaa cgcagtcagg caccgtgtat gaaatctaac aatgcgctca tcgtcatcct 120
   cggcaccgtc accctggatg ctgtaggcat aggcttggtt atgccggtac tgccgggcct 180
   cttgcgggat atcgtccatt ccgacagcat cgccagtcac tatggcgtgc tgctagcgct 240
   atatgcgttg atgcaatttc tatgcgcacc cgttctcgga gcactgtccg accgctttgg 300
   ccgccgccca gtcctgctcg cttcgctact tggagccact atcgactacg cgatcatggc 360
   gaccacaccc gtcctgtgga tccaggccgt tgagcaccgc cgccgcaagg aatggtgcat 420
   gctgaggtgt ctcacaagtg ccgtgcagtc ccgcccccac ttgcttctct ttgtgtgtag 480
   tgtacgtaca ttatcgagac cgttgttccc gcccacctcg atccggcatg ctgaggtgtc 540
   tcacaagtgc cgtgcagtcc cgcccccact tgcttctctt tgtgtgtagt gtacgtacat 600
   tatcgagacc gttgttcccg cccacctcga tccggcatgc tgaggtgtct cacaagtgcc 660
   gtgcagtccc gcccccactt gcttctcttt gtgtgtagtg tacgtacatt atcgagaccg 720
   ttgttcccgc ccacctcgat ccggcatgct gaggtgtctc acaagtgccg tgcagtcccg 780
   cccccacttg cttctctttg tgtgtagtgt acgtacatta tcgagaccgt tgttcccgcc 840
   cacctcgatc cggcatgcac tgatcacggg caaaagtgcg tatatataca agagcgtttg 900
   ccagccacag attttcactc cacacaccac atcacacata caaccacaca catccacaat 960
   gaaaaagcct gaactcaccg cgacgagcgt cgagaagttt ctgatcgaaa agttcgacag 1020
   cgtctccgac ctgatgcagc tctcggaggg cgaagaatct cgtgctttca gcttcgatgt 1080
   aggagggcgt ggatatgtcc tgcgggtaaa tagctgcgcc gatggtttct acaaagatcg 1140
   ttatgtttat cggcactttg catcggccgc gctcccgatt ccggaagtgc ttgacattgg 1200
   ggagttcagc gagagcctga cctattgcat ctcccgccgt gcacagggtg tcacgttgca 1260
   agacctgcct gaaaccgaac tgcccgctgt tctgcagccg gtcgcggagg ccatggatgc 1320
   gatcgctgcg gccgatctta gccagacgag cgggttcggc ccattcggac cgcaaggaat 1380
   cggtcaatac actacatggc gtgatttcat atgcgcgatt gctgatcccc atgtgtatca 1440
   ctggcaaact gtgatggacg acaccgtcag tgcgtccgtc gcgcaggctc tcgatgagct 1500
   gatgctttgg gccgaggact gccccgaagt ccggcacctc gtgcacgcgg atttcggctc 1560
   caacaatgtc ctgacggaca atggccgcat aacagcggtc attgactgga gcgaggcgat 1620
   gttcggggat tcccaatacg aggtcgccaa catcttcttc tggaggccgt ggttggcttg 1680
   tatggagcag cagacgcgct acttcgagcg gaggcatccg gagcttgcag gatcgccgcg 1740
   gctccgggcg tatatgctcc gcattggtct tgaccaactc tatcagagct tggttgacgg 1800
   caatttcgat gatgcagctt gggcgcaggg tcgatgcgac gcaatcgtcc gatccggagc 1860
   cgggactgtc gggcgtacac aaatcgcccg cagaagcgcg gccgtctgga ccgatggctg 1920
   tgtagaagta ctcgccgata gtggaaaccg acgccccagc actcgtccga gggcaaagga 1980
   atagtcgacg ctctccctta tgcgactcct gcattaggaa gcagcccagt agtaggttga 2040
   ggccgttgag caccgccgcc gcaaggaatg gtgcatgctg aggtgtctca caagtgccgt 2100
   gcagtcccgc ccccacttgc ttctctttgt gtgtagtgta cgtacattat cgagaccgtt 2160
   gttcccgccc acctcgatcc ggcatgctga ggtgtctcac aagtgccgtg cagtcccgcc 2220
   cccacttgct tctctttgtg tgtagtgtac gtacattatc gagaccgttg ttcccgccca 2280
   cctcgatccg gcatgctgag gtgtctcaca agtgccgtgc agtcccgccc ccacttgctt 2340
   ctctttgtgt gtagtgtacg tacattatcg agaccgttgt tcccgcccac ctcgatccgg 2400
   catgctgagg tgtctcacaa gtgccgtgca gtcccgcccc cacttgcttc tctttgtgtg 2460
   tagtgtacgt acattatcga gaccgttgtt cccgcccacc tcgatccggc atgcactgat 2520
   cacgggcaaa agtgcgtata tatacaagag cgtttgccag ccacagattt tcactccaca 2580
   caccacatca cacatacaac cacacacatc cacgggctgc aggaattcga tatcaagctt 2640
   atcgataccg tcgaggggca gagccgatcc tgtacacttt acttaaaacc attatctgag 2700
   tgttaaatgt ccaatttact gaccgtacac caaaatttgc ctgcattacc ggtcgatgca 2760
   acgagtgatg aggttcgcaa gaacctgatg gacatgttca gggatcgcca ggcgttttct 2820
   gagcatacct ggaaaatgct tctgtccgtt tgccggtcgt gggcggcatg gtgcaagttg 2880
   aataaccgga aatggtttcc cgcagaacct gaagatgttc gcgattatct tctatatctt 2940
   caggcgcgcg gtctggcagt aaaaactatc cagcaacatt tgggccagct aaacatgctt 3000
   catcgtcggt ccgggctgcc acgaccaagt gacagcaatg ctgtttcact ggttatgcgg 3060
   cggatccgaa aagaaaacgt tgatgccggt gaacgtgcaa aacaggctct agcgttcgaa 3120
   cgcactgatt tcgaccaggt tcgttcactc atggaaaata gcgatcgctg ccaggatata 3180
   cgtaatctgg catttctggg gattgcttat aacaccctgt tacgtatagc cgaaattgcc 3240
   aggatcaggg ttaaagatat ctcacgtact gacggtggga gaatgttaat ccatattggc 3300
   agaacgaaaa cgctggttag caccgcaggt gtagagaagg cacttagcct gggggtaact 3360
   aaactggtcg agcgatggat ttccgtctct ggtgtagctg atgatccgaa taactacctg 3420
   ttttgccggg tcagaaaaaa tggtgttgcc gcgccatctg ccaccagcca gctatcaact 3480
   cgcgccctgg aagggatttt tgaagcaact catcgattga tttacggcgc taaggatgac 3540
   tctggtcaga gatacctggc ctggtctgga cacagtgccc gtgtcggagc cgcgcgagat 3600
   atggcccgcg ctggagtttc aataccggag atcatgcaag ctggtggctg gaccaatgta 3660
   aatattgtca tgaactatat ccgtaccctg gatagtgaaa caggggcaat ggtgcgcctg 3720
   ctggaagatg gcgattagcc attaacgcgt aaatgattgc tataattatt tgatatttat 3780
   ggtgacatat gagaaaggat ttcaacatcg acggaaaata tgtagtgctg tctgtaagca 3840
   ctaatattca gtcgccagcc gtcattgtca ctgtaaagct gagcgataga atgcctgata 3900
   ttgactcaat atccgttgcg tttcctgtca aaagtatgcg tagtgctgaa catttcgtga 3960
   tgaatgccac cgaggaagaa gcacggcgcg gttttgcnta aagtgatgtc tgagtttggc 4020
   gaactcttgg gtaaggttgg aattgtcgac cgatgccctt gagagccttc aacccagtca 4080
   gctccttccg gtgggcgcgg ggcatgacta tcgtcgccgc acttatgact gtcttcttta 4140
   tcatgcaact cgtaggacag gtgccggcag cgctctgggt cattttcggc gaggaccgct 4200
   ttcgctggag cgcgacgatg atcggcctgt cgcttgcggt attcggaatc ttgcacgccc 4260
   tcgctcaagc cttcgtcact ggtcccgcca ccaaacgttt cggcgagaag caggccatta 4320
   tcgccggcat ggcggccgac gcgctgggct acgtcttgct ggcgttcgcg acgcgaggct 4380
   ggatggcctt ccccattatg attcttctcg cttccggcgg catcgggatg cccgcgttgc 4440
   aggccatgct gtccaggcag gtagatgacg accatcaggg acagcttcaa ggatcgctcg 4500
   cggctcttac cagcctaact tcgatcactg gaccgctgat cgtcacggcg atttatgccg 4560
   cctcggcgag cacatggaac gggttggcat ggattgtagg cgccgcccta taccttgtct 4620
   gcctccccgc gttgcgtcgc ggtgcatgga gccgggccac ctcgacctga atggaagccg 4680
   gcggcacctc gctaacggat tcaccactcc aagaattgga gccaatcaat tcttgcggag 4740
   aactgtgaat gcgcaaacca acccttggca gaacatatcc atcgcgtccg ccatctccag 4800
   cagccgcacg cggcgcatct cgggcagcgt tgggtcctgg ccacgggtgc gcatgatcgt 4860
   gctcctgtcg ttgaggaccc ggctaggctg gcggggttgc cttactggtt agcagaatga 4920
   atcaccgata cgcgagcgaa cgtgaagcga ctgctgctgc aaaacgtctg cgacctgagc 4980
   aacaacatga atggtcttcg gtttccgtgt ttcgtaaagt ctggaaacgc ggaagtcagc 5040
   gccctgcacc attatgttcc ggatctgcat cgcaggatgc tgctggctac cctgtggaac 5100
   acctacatct gtattaacga agcgctggca ttgaccctga gtgatttttc tctggtcccg 5160
   ccgcatccat accgccagtt gtttaccctc acaacgttcc agtaaccggg catgttcatc 5220
   atcagtaacc cgtatcgtga gcatcctctc tcgtttcatc ggtatcatta cccccatgaa 5280
   cagaaattcc cccttacacg gaggcatcaa gtgaccaaac aggaaaaaac cgcccttaac 5340
   atggcccgct ttatcagaag ccagacatta acgcttctgg agaaactcaa cgagctggac 5400
   gcggatgaac aggcagacat ctgtgaatcg cttcacgacc acgctgatga gctttaccgc 5460
   agcagatctg tatatatata tatatatgca agccattttt tttctctcac catctatttt 5520
   aatatataaa attagatcat ctatctaaac tttttcatta aataaattag atggcgaaaa 5580
   taatggagac gtattccatt ataatatata aaaacctaaa actatgtttc attataacaa 5640
   tttacttcct aatttggaaa attcgaagtt ggttattata tgtgcatata tactgaatgt 5700
   tcataacttc tagtcaacag atataattta ttcctcgtag taacttgccc gcaaacattt 5760
   tatatctaaa ttaatttcaa gggaagttct tgtaaatata tatttatctc aagtaaacag 5820
   ttagaaatat cagccatgat gacattttcc aggatggcaa tgactcatga tcacactgag 5880
   atttttaata gatatttcgt tagagatgat ggtatctcaa aacaaaacga ctgtagctct 5940
   tttaccacct catttacaat ttcatctttc atcaaattta gggatgccat caactttcag 6000
   ttcataatta atatcttacc aaattaggta atctgcaaaa gttcagactg tgaaatgtaa 6060
   cattttatat atcaagctct atttaatgcc tcacagtagt taacataaag agatacagaa 6120
   ttgtcgtgtc agtgtatact atccatgtgt atactctgga tatccatttg tattccatta 6180
   tctacgaaaa gcacttagat aaatactaaa ttgttatttg gtatgtatcg tataagttga 6240
   aagttttgag cccatcttgt tgttttcttt tattaaataa aataaaataa ctaacgttat 6300
   gatactttga tgtgtttttt aatttaatta taccagtact tgtttgaaat ttttttctgc 6360
   agaattttgg ccggctcatt tctatttgtt gtaagtacga gtatttgaac ttttagtcag 6420
   atactggtag ttatatattt attttgtttt tgtttatttt gttgggtttt ttgtttgttg 6480
   ttttttttcg gggggttgtg ttccaacttc gtttttggaa ttttaattta gtttctcgat 6540
   cttcgctttt ggaatttatt taatttatcc ctccccttga ggtgtgaata acttaaaaat 6600
   gctagaagga gctacacagg tgtttgtaca gtaaaaacta tcagcaggat accatcgcaa 6660
   gatgttcata tcgctttgtt gagtcactgc aggggaccgc tgaggtattc gctggttcgg 6720
   tgagggcggc cgtccctgtg attcgtacga ataaattctt tgtacaagta ccagtgctac 6780
   aattgtaggt ggtgctcata caggtacacc ccgtgtgtaa gtaaactcca attatgttat 6840
   gtctgataaa aggatgtaac ataggcaagc tgctcgtgag tgttgagtac gaaccttaga 6900
   tccaaatcac ccgcacccta cggatatact tgcttgaata tacttgtaat aaggctgtct 6960
   gctgacatcg gtgcgcgtat gttctgggcg gcgactctct ccgaaccatc gaacagttcc 7020
   tgaacacgac gagctagcta caacatgact cgcaagagct ctgtgcgtgt acacaacgag 7080
   ccgtgcccgt gtaacagtct tcggttccga cccccaaaaa acccaccata caccgaaata 7140
   gcacatcctt acgaccagta gcagcagagt gcgctacagt aagtattcgt caatacaagt 7200
   aaatcacgag tacgacagtt gccgacacgg acagaaagga actacagatt taaatatacc 7260
   aaacaataat tcattactaa tgtcaatcct tacagctgga taaaaaaact gggggatttt 7320
   gttaacgagc tcattcgcaa atgaaacggg aaaagttctt cgatttagtg ttaaatctcc 7380
   gttaaaaacc gcttatttgg atcgagctcg gaccttgcgg cgctttcgct tgagtcgtct 7440
   gactctcttc tttctccact tagctctcat tctgggttag ttccatgttc tccgctggcg 7500
   ggggcgacca ccgctaatcg agccgacttg tattgaaagg caggcaagaa ggtatcgaag 7560
   gggaagaacc gttttgtggt tgctgcacca cggcttccaa tgctctccca atgaagaacc 7620
   aaggtcggta attaatactc acttgaaaga tcaagacaag aacctgatga atgtgaggaa 7680
   aaaaagacaa gaaggggaaa gtttgaccat ttttaagctg tgcgagccac aggccgggta 7740
   acagataaat taggttctga aaattcggat ctgctgcctc gcgcgtttcg gtgatgacgg 7800
   tgaaaacctc tgacacatgc agctcccgga gacggtcaca gcttgtctgt aagcggatgc 7860
   cgggagcaga caagcccgtc agggcgcgtc agcgggtgtt ggcgggtgtc ggggcgcagc 7920
   catgacccag tcacgtagcg atagcggagt gtatactggc ttaactatgc ggcatcagag 7980
   cagattgtac tgagagtgca ccatatgcgg tgtgaaatac cgcacagatg cgtaaggaga 8040
   aaataccgca tcaggcgctc ttccgcttcc tcgctcactg actcgctgcg ctcggtcgtt 8100
   cggctgcggc gagcggtatc agctcactca aaggcggtaa tacggttatc cacagaatca 8160
   ggggataacg caggaaagaa catgtgagca aaaggccagc aaaaggccag gaaccgtaaa 8220
   aaggccgcgt tgctggcgtt tttccatagg ctccgccccc ctgacgagca tcacaaaaat 8280
   cgacgctcaa gtcagaggtg gcgaaacccg acaggactat aaagatacca ggcgtttccc 8340
   cctggaagct ccctcgtgcg ctctcctgtt ccgaccctgc cgcttaccgg atacctgtcc 8400
   gcctttctcc cttcgggaag cgtggcgctt tctcatagct cacgctgtag gtatctcagt 8460
   tcggtgtagg tcgttcgctc caagctgggc tgtgtgcacg aaccccccgt tcagcccgac 8520
   cgctgcgcct tatccggtaa ctatcgtctt gagtccaacc cggtaagaca cgacttatcg 8580
   ccactggcag cagccactgg taacaggatt agcagagcga ggtatgtagg cggtgctaca 8640
   gagttcttga agtggtggcc taactacggc tacactagaa ggacagtatt tggtatctgc 8700
   gctctgctga agccagttac cttcggaaaa agagttggta gctcttgatc cggcaaacaa 8760
   accaccgctg gtagcggtgg tttttttgtt tgcaagcagc agattacgcg cagaaaaaaa 8820
   ggatctcaag aagatccttt gatcttttct acggggtctg acgctcagtg gaacgaaaac 8880
   tcacgttaag ggattttggt catgagatta tcaaaaagga tcttcaccta gatcctttta 8940
   aattaaaaat gaagttttaa atcaatctaa agtatatatg agtaaacttg gtctgacagt 9000
   taccaatgct taatcagtga ggcacctatc tcagcgatct gtctatttcg ttcatccata 9060
   gttgcctgac tccccgtcgt gtagataact acgatacggg agggcttacc atctggcccc 9120
   agtgctgcaa tgataccgcg agacccacgc tcaccggctc cagatttatc agcaataaac 9180
   cagccagccg gaagggccga gcgcagaagt ggtcctgcaa ctttatccgc ctccatccag 9240
   tctattaatt gttgccggga agctagagta agtagttcgc cagttaatag tttgcgcaac 9300
   gttgttgcca ttgctgcagg catcgtggtg tcacgctcgt cgtttggtat ggcttcattc 9360
   agctccggtt cccaacgatc aaggcgagtt acatgatccc ccatgttgtg caaaaaagcg 9420
   gttagctcct tcggtcctcc gatcgttgtc agaagtaagt tggccgcagt gttatcactc 9480
   atggttatgg cagcactgca taattctctt actgtcatgc catccgtaag atgcttttct 9540
   gtgactggtg agtactcaac caagtcattc tgagaatagt gtatgcggcg accgagttgc 9600
   tcttgcccgg cgtcaacacg ggataatacc gcgccacata gcagaacttt aaaagtgctc 9660
   atcattggaa aacgttcttc ggggcgaaaa ctctcaagga tcttaccgct gttgagatcc 9720
   agttcgatgt aacccactcg tgcacccaac tgatcttcag catcttttac tttcaccagc 9780
   gtttctgggt gagcaaaaac aggaaggcaa aatgccgcaa aaaagggaat aagggcgaca 9840
   cggaaatgtt gaatactcat actcttcctt tttcaatatt attgaagcat ttatcagggt 9900
   tattgtctca tgagcggata catatttgaa tgtatttaga aaaataaaca aataggggtt 9960
   ccgcgcacat ttccccgaaa agtgccacct gacgtctaag aaaccattat tatcatgaca 10020
   ttaacctata aaaataggcg tatcacgagg ccctttcgtc ttcaa 10065
<210> 86
   <211> 28
   <212> DNA
   <213> Artificial
<220>
   <223> Primer
<400> 86
   aaagatatct ctatgcgcac ccgttctc 28
<210> 87
   <211> 39
   <212> DNA
   <213> Artificial
<220>
   <223> Primer
<400> 87
   tttagatcta agcttgagac acctcagcat gcaccattc 39
<210> 88
   <211> 8114
   <212> DNA
   <213> Artificial
<220>
   <223> Vector
<400> 88
   agcgcccaat acgcaaaccg cctctccccg cgcgttggcc gattcattaa tgcagctggc 60
   acgacaggtt tcccgactgg aaagcgggca gtgagcgcaa cgcaattaat gtgagttagc 120
   tcactcatta ggcaccccag gctttacact ttatgcttcc ggctcgtatg ttgtgtggaa 180
   ttgtgagcgg ataacaattt cacacaggaa acagctatga ccatgattac gccaagctca 240
   gaattaaccc tcactaaagg gactagtcct gcaggtttaa acgaattcgc cctttcatct 300
   cgagatgctt tattcaggca cgctacgtga gaatattcta atgggatggt ctggccctga 360
   gtctgaagta acgcaggaga tgattgagga tgccgctcgc aaagcgaaca ttcacgaatt 420
   catcatgtcg ttgcctgatg gctacgaaac gctcagcgga tctaggggat cgttgctatc 480
   tggggggcaa aagcagcgaa ttgcaattgc aagggccctg atcagaaatc caaaggtact 540
   cctcctcgat gaggccacct cagctctgga ttccgaatct gagaaagtag ttcaagcagc 600
   actcgacgca gcagcgaagg gccgtactac aatcgccgtt gcgcatagat tatcaacaat 660
   tcagaaagca gatgtcatat atgtgttctc aggagggcgc atcgtggagc agggcgacca 720
   tcagagcctc cttgaactca atggatggta cgctgaattg gtgaacttgc aaggtctcgg 780
   agagatttga cgttcattta tttttggcca ctgcttgcat acattatttg attaaaggca 840
   ctcattaatt gaaatagcat atcgaatttc tctagttatg gcccctgagt caccatacat 900
   tgtctgatta aagggactcg ttaattgaaa tagcacattg gattcctctg attatgaccc 960
   ctgagtcacc tatcctgcat aattcactcg tgacgataat ctgtagatat agggaactgt 1020
   cgtagtactt gaagagacag caacaatcta tctctgggat ttcgtgctga ttttgggctt 1080
   ttgctttgac gggctatgac tgaggtaatg tagaccaata ataaccctca cgcgaattag 1140
   atatgccctg agggttagct tgcatcacct tacccatatg cacactgact tgcattaccc 1200
   ggagcatatt ccggtagtcg gagataagca ctttgagata tcttaaggta caactcaata 1260
   cgttcctcct tccttgcctc attccacctc acattctaga attcaataac ttcgtatagc 1320
   atacattata cgaagttatt aattaacatc atcgtcacta tacacatcgt catcaactcc 1380
   atggcgtgag gacttccgag actgctgggc ccttcgtttc tttaatgcct caagagatga 1440
   cttcgtaccc gaagagacgc ctgttgtacc ccgttgacgc ttggcggagg gggcttcgtc 1500
   ctcgtcagca acccgcgtca tctgcttcct tcgctgagca agataccttc tctcctcgta 1560
   ccgctgcatc tcctgagctc ggtcatacaa gatctcttct cgctcaatct ctggcagcgc 1620
   gtccaacttc gccctgtctt cagcatcgag atatttgcct tctagaggat agggattgac 1680
   gacctcattg cttggcggcg acggcagcga gatttcctct tcggagtcgg agccaacgtc 1740
   ggccaatgcc agcagatcat catcactgtc actcatagta ggaaggttga agtgtgctga 1800
   cgaatcagaa tcgcgaagga tgccattgaa ggcatatata ttttaatctg taccttttat 1860
   ggtaatttaa tcagatttta taggtattca tgtgcaagtt gcattgaagg aactgtttga 1920
   gaaaatcatc ttgactgaac ttttctcaga tatgcattcc agcccgcctt ttggtaacgc 1980
   tgagcttcgt gcacaggatc tcgtcccttg ctatagagcc cgcgtccgac gataataacg 2040
   tctgtgccgg tctctatgac gtcgtccaca gtacgatact gctgccccaa tccatcacct 2100
   ttgtcgtcca ggcccacccc aggagtcata atgacccagt cttcctctgg ctttccgact 2160
   ttttgctgag cgatgaaacc aaacacaaat gcgcggttac tgcgagcgat gtctactgtc 2220
   gcttgcgagt attcgccgtg agccagtgtg cccttcgaac tcagttctgc aagcatgaca 2280
   aggccgcgag gttcatccgt agtttccttc gcagcctctt ctagtccgct cacaattccc 2340
   ggcccaggaa caccgtgagc atttgttata tcagcccatt gagcgatctt aaacactcca 2400
   cctgcatatt gggccttaac agtggaaccg atgtctgcga actttcggtc ttcaaaaatg 2460
   agaaaattgt gcttcgttga aagctgtttc aaaccgctga cagttgtgtc gtattcgaag 2520
   tcgtcaatta tgtcaatgtg ggtcttaacc atacaaatgt aaggtccaat gcggtccagg 2580
   atactcagta actcagaggt agttcgcaca tccaagcttg cgcaaagatt tgtttgcttg 2640
   ctcacaatga tgtcgaatag ccgggctgct acagccggca gcctctctcg gcgctcctca 2700
   tagctcagct tcatattatt tctctacagt agtgcccgtg ccctcgatca gctaggactt 2760
   ttcaaattaa tcgggctgtt tgatgtaagt aagatgaagt cacgcgcgtg caggagactg 2820
   cgtcccgcga tattctgcag gcttgaaaaa tttaccctaa cggtaggcat caagtgagtg 2880
   agtctcagcg tcgatatggg tcaaaaaagg ggaaaactag ccgagatcgt tgcgagctgt 2940
   ttcgaaaatt atgccctatg gcaattatca cgtggagtat ccgaatttct ccaggctgtc 3000
   aagcggcaat tataaccgag actgagatcg agaagtatat aaccgcagca gtagtggata 3060
   aataattgcg aagtcttccc agcagagcgg gctgtttttt ggagttggtt actgtaaaat 3120
   gctaaaatga ctgacaacaa tggagcgtct acagcattgg caacagtggg aacagtatgc 3180
   tggtgcatcc agttgatacc ccaggttctg cgaaactggt atgttcggga ttgcgagggc 3240
   gttcctcctc tgatgttctt tttgttcgcc gtttcgggga ttcccttcgc agtgtacttc 3300
   attgatcaga attcgaacac tgccatcatg gttcaacctc acttgtttac tttctttagc 3360
   cttataggct tttggcaaag cctgtactat ccgcccgtca gttaattaat aacttcgtat 3420
   agcatacatt atacgaagtt attaggtaaa ctaaattcat gacagccttt tcttctttct 3480
   ttccacaaaa caattaaaaa aaataacaga attagaagaa ggtaaatata ttggcaaact 3540
   cctctcttcc ttttacttat ttttttgaaa gttgcagtgt gtgtgtgtgt tgttgtttgt 3600
   tcaaattaat ttgatggttg ttgtattgta aatttcaatc aataaaaaca aagacataaa 3660
   taaaaaaaac cctacctctc ttccctgatc tgatttgatc gtacgattct aagaactcac 3720
   cgctaaggcc ggccctttga caggtatatc ttcagtttcc tcgtcactct tggtcaaaag 3780
   accaaagtca tggctggcga tttcctcgat gctttcctca agaattttca aggagttgtg 3840
   gctttccaac tccatttgaa ccttcttcga ggcttcgtgg aatttcggat ttccaattat 3900
   cgaatcaaca gcttctttga tttgctccac tgtaggcaag ccagttttca aatcaattgc 3960
   cacgccagcg gcctcagctc tcgatgccac cattggcttg tcttcagagt caccagcaat 4020
   aacaactgga acagagtggc ttaagctgtg ctgaagtccg ccatatccac cattgtagac 4080
   aagagcatca acgtgaggaa gtagagcatc gtagttgaag tagtcgatca cgcgagcatt 4140
   ctcaggaacc acaacatcat ccggtagctt ggcaccgcgg cggcccaata tggctactgt 4200
   taaagtgtca ggctcgtcct tcaaggcctc aagagtaggc acaataagat gcttgtaact 4260
   gacagcaaaa gttccttgag tgaccatgat gactcgcttg gcactcagaa catcccccca 4320
   ccaggaagga ggggtgaatt gagttcggtg cttgggcgtt gagccggcga atttgaagtt 4380
   gctaggcaga tggtctctgc tgaactcaag agaaggcggg cacagctgca ggaacttgtc 4440
   tgcaggtacc tcaagggcga attcgcggcc gctaaattca attcgcccta tagtgagtcg 4500
   tattacaatt cactggccgt cgttttacaa cgtcgtgact gggaaaaccc tggcgttacc 4560
   caacttaatc gccttgcagc acatccccct ttcgccagct ggcgtaatag cgaagaggcc 4620
   cgcaccgatc gcccttccca acagttgcgc agcctatacg tacggcagtt taaggtttac 4680
   acctataaaa gagagagccg ttatcgtctg tttgtggatg tacagagtga tattattgac 4740
   acgccggggc gacggatggt gatccccctg gccagtgcac gtctgctgtc agataaagtc 4800
   tcccgtgaac tttacccggt ggtgcatatc ggggatgaaa gctggcgcat gatgaccacc 4860
   gatatggcca gtgtgccggt ctccgttatc ggggaagaag tggctgatct cagccaccgc 4920
   gaaaatgaca tcaaaaacgc cattaacctg atgttctggg gaatataaat gtcaggcatg 4980
   agattatcaa aaaggatctt cacctagatc cttttcacgt agaaagccag tccgcagaaa 5040
   cggtgctgac cccggatgaa tgtcagctac tgggctatct ggacaaggga aaacgcaagc 5100
   gcaaagagaa agcaggtagc ttgcagtggg cttacatggc gatagctaga ctgggcggtt 5160
   ttatggacag caagcgaacc ggaattgcca gctggggcgc cctctggtaa ggttgggaag 5220
   ccctgcaaag taaactggat ggctttcttg ccgccaagga tctgatggcg caggggatca 5280
   agctctgatc aagagacagg atgaggatcg tttcgcatga ttgaacaaga tggattgcac 5340
   gcaggttctc cggccgcttg ggtggagagg ctattcggct atgactgggc acaacagaca 5400
   atcggctgct ctgatgccgc cgtgttccgg ctgtcagcgc aggggcgccc ggttcttttt 5460
   gtcaagaccg acctgtccgg tgccctgaat gaactgcaag acgaggcagc gcggctatcg 5520
   tggctggcca cgacgggcgt tccttgcgca gctgtgctcg acgttgtcac tgaagcggga 5580
   agggactggc tgctattggg cgaagtgccg gggcaggatc tcctgtcatc tcaccttgct 5640
   cctgccgaga aagtatccat catggctgat gcaatgcggc ggctgcatac gcttgatccg 5700
   gctacctgcc cattcgacca ccaagcgaaa catcgcatcg agcgagcacg tactcggatg 5760
   gaagccggtc ttgtcgatca ggatgatctg gacgaagagc atcaggggct cgcgccagcc 5820
   gaactgttcg ccaggctcaa ggcgagcatg cccgacggcg aggatctcgt cgtgacccat 5880
   ggcgatgcct gcttgccgaa tatcatggtg gaaaatggcc gcttttctgg attcatcgac 5940
   tgtggccggc tgggtgtggc ggaccgctat caggacatag cgttggctac ccgtgatatt 6000
   gctgaagagc ttggcggcga atgggctgac cgcttcctcg tgctttacgg tatcgccgct 6060
   cccgattcgc agcgcatcgc cttctatcgc cttcttgacg agttcttctg aattattaac 6120
   gcttacaatt tcctgatgcg gtattttctc cttacgcatc tgtgcggtat ttcacaccgc 6180
   atcaggtggc acttttcggg gaaatgtgcg cggaacccct atttgtttat ttttctaaat 6240
   acattcaaat atgtatccgc tcatgagatt atcaaaaagg atcttcacct agatcctttt 6300
   aaattaaaaa tgaagtttta aatcaatcta aagtatatat gagtaaactt ggtctgacag 6360
   ttaccaatgc ttaatcagtg aggcacctat ctcagcgatc tgtctatttc gttcatccat 6420
   agttgcctga ctccccgtcg tgtagataac tacgatacgg gagggcttac catctggccc 6480
   cagtgctgca atgataccgc gagacccacg ctcaccggct ccagatttat cagcaataaa 6540
   ccagccagcc ggaagggccg agcgcagaag tggtcctgca actttatccg cctccatcca 6600
   gtctattaat tgttgccggg aagctagagt aagtagttcg ccagttaata gtttgcgcaa 6660
   cgttgttgcc attgctacag gcatcgtggt gtcacgctcg tcgtttggta tggcttcatt 6720
   cagctccggt tcccaacgat caaggcgagt tacatgatcc cccatgttgt gcaaaaaagc 6780
   ggttagctcc ttcggtcctc cgatcgttgt cagaagtaag ttggccgcag tgttatcact 6840
   catggttatg gcagcactgc ataattctct tactgtcatg ccatccgtaa gatgcttttc 6900
   tgtgactggt gagtactcaa ccaagtcatt ctgagaatag tgtatgcggc gaccgagttg 6960
   ctcttgcccg gcgtcaatac gggataatac cgcgccacat agcagaactt taaaagtgct 7020
   catcattgga aaacgttctt cggggcgaaa actctcaagg atcttaccgc tgttgagatc 7080
   cagttcgatg taacccactc gtgcacccaa ctgatcttca gcatctttta ctttcaccag 7140
   cgtttctggg tgagcaaaaa caggaaggca aaatgccgca aaaaagggaa taagggcgac 7200
   acggaaatgt tgaatactca tactcttcct ttttcaatat tattgaagca tttatcaggg 7260
   ttattgtctc atgaccaaaa tcccttaacg tgagttttcg ttccactgag cgtcagaccc 7320
   cgtagaaaag atcaaaggat cttcttgaga tccttttttt ctgcgcgtaa tctgctgctt 7380
   gcaaacaaaa aaaccaccgc taccagcggt ggtttgtttg ccggatcaag agctaccaac 7440
   tctttttccg aaggtaactg gcttcagcag agcgcagata ccaaatactg ttcttctagt 7500
   gtagccgtag ttaggccacc acttcaagaa ctctgtagca ccgcctacat acctcgctct 7560
   gctaatcctg ttaccagtgg ctgctgccag tggcgataag tcgtgtctta ccgggttgga 7620
   ctcaagacga tagttaccgg ataaggcgca gcggtcgggc tgaacggggg gttcgtgcac 7680
   acagcccagc ttggagcgaa cgacctacac cgaactgaga tacctacagc gtgagctatg 7740
   agaaagcgcc acgcttcccg aagggagaaa ggcggacagg tatccggtaa gcggcagggt 7800
   cggaacagga gagcgcacga gggagcttcc agggggaaac gcctggtatc tttatagtcc 7860
   tgtcgggttt cgccacctct gacttgagcg tcgatttttg tgatgctcgt caggggggcg 7920
   gagcctatgg aaaaacgcca gcaacgcggc ctttttacgg ttcctggcct tttgctggcc 7980
   ttttgctcac atgttctttc ctgcgttatc ccctgattct gtggataacc gtattaccgc 8040
   ctttgagtga gctgataccg ctcgccgcag ccgaacgacc gagcgcagcg agtcagtgag 8100
   cgaggaagcg gaag 8114
<210> 89
   <211> 8578
   <212> DNA
   <213> Artificial
<220>
   <223> Vector
<400> 89
   agcgcccaat acgcaaaccg cctctccccg cgcgttggcc gattcattaa tgcagctggc 60
   acgacaggtt tcccgactgg aaagcgggca gtgagcgcaa cgcaattaat gtgagttagc 120
   tcactcatta ggcaccccag gctttacact ttatgcttcc ggctcgtatg ttgtgtggaa 180
   ttgtgagcgg ataacaattt cacacaggaa acagctatga ccatgattac gccaagctca 240
   gaattaaccc tcactaaagg gactagtcct gcaggtttaa acgaattcgc cctttcatct 300
   cgagatgctt tattcaggca cgctacgtga gaatattcta atgggatggt ctggccctga 360
   gtctgaagta acgcaggaga tgattgagga tgccgctcgc aaagcgaaca ttcacgaatt 420
   catcatgtcg ttgcctgatg gctacgaaac gctcagcgga tctaggggat cgttgctatc 480
   tggggggcaa aagcagcgaa ttgcaattgc aagggccctg atcagaaatc caaaggtact 540
   cctcctcgat gaggccacct cagctctgga ttccgaatct gagaaagtag ttcaagcagc 600
   actcgacgca gcagcgaagg gccgtactac aatcgccgtt gcgcatagat tatcaacaat 660
   tcagaaagca gatgtcatat atgtgttctc aggagggcgc atcgtggagc agggcgacca 720
   tcagagcctc cttgaactca atggatggta cgctgaattg gtgaacttgc aaggtctcgg 780
   agagatttga cgttcattta tttttggcca ctgcttgcat acattatttg attaaaggca 840
   ctcattaatt gaaatagcat atcgaatttc tctagttatg gcccctgagt caccatacat 900
   tgtctgatta aagggactcg ttaattgaaa tagcacattg gattcctctg attatgaccc 960
   ctgagtcacc tatcctgcat aattcactcg tgacgataat ctgtagatat agggaactgt 1020
   cgtagtactt gaagagacag caacaatcta tctctgggat ttcgtgctga ttttgggctt 1080
   ttgctttgac gggctatgac tgaggtaatg tagaccaata ataaccctca cgcgaattag 1140
   atatgccctg agggttagct tgcatcacct tacccatatg cacactgact tgcattaccc 1200
   ggagcatatt ccggtagtcg gagataagca ctttgagata tcttaaggta caactcaata 1260
   cgttcctcct tccttgcctc attccacctc acattctaga attcaataac ttcgtatagc 1320
   atacattata cgaagttatt aattaacatc atcgtcacta tacacatcgt catcaactcc 1380
   atggcgtgag gacttccgag actgctgggc ccttcgtttc tttaatgcct caagagatga 1440
   cttcgtaccc gaagagacgc ctgttgtacc ccgttgacgc ttggcggagg gggcttcgtc 1500
   ctcgtcagca acccgcgtca tctgcttcct tcgctgagca agataccttc tctcctcgta 1560
   ccgctgcatc tcctgagctc ggtcatacaa gatctaagct tgagacacct cagcatgcac 1620
   cattccttgc ggcggcggtg ctcaacggcc tcaacctact actgggctgc ttcctaatgc 1680
   aggagtcgca taagggagag cgtcgactat tcctttgccc tcggacgagt gctggggcgt 1740
   cggtttccac tatcggcgag tacttctaca cagccatcgg tccagacggc cgcgcttctg 1800
   cgggcgattt gtgtacgccc gacagtcccg gctccggatc ggacgattgc gtcgcatcga 1860
   ccctgcgccc aagctgcatc atcgaaattg ccgtcaacca agctctgata gagttggtca 1920
   agaccaatgc ggagcatata cgcccggagc cgcggcgatc ctgcaagctc cggatgcctc 1980
   cgctcgaagt agcgcgtctg ctgctccata caagccaacc acggcctcca gaagaagatg 2040
   ttggcgacct cgtattggga atccccgaac atcgcctcgc tccagtcaat gaccgctgtt 2100
   atgcggccat tgtccgtcag gacattgttg gagccgaaat ccgcgtgcac gaggtgccgg 2160
   acttcggggc agtcctcggc ccaaagcatc agctcatcga gagcctgcgc gacggacgca 2220
   ctgacggtgt cgtccatcac agtttgccag tgatacacat ggggatcagc aatcgcgcat 2280
   atgaaatcac gccatgtagt gtattgaccg attccttgcg gtccgaatgg gccgaacccg 2340
   ctcgtctggc taagatcggc cgcagcgatc gcatccatgg cctccgcgac cggctgcaga 2400
   acagcgggca gttcggtttc aggcaggtct tgcaacgtga caccctgtgc acggcgggag 2460
   atgcaatagg tcaggctctc gctaaattcc ccaatgtcaa gcacttccgg aatcgggagc 2520
   gcggccgatg caaagtgccg ataaacataa cgatctttgt agaaaccatc ggcgcagcta 2580
   tttacccgca ggacatatcc acgccctcct acatcgaagc tgaaagcacg agattcttcg 2640
   ccctccgaga gctgcatcag gtcggagacg ctgtcgaact tttcgatcag aaacttctcg 2700
   acagacgtgg cggtgagttc aggctttttc attgtggatg tgtgtggttg tatgtgtgat 2760
   gtggtgtgtg gagtgaaaat ctgtggctgg caaacgctct tgtatatata cgcacttttg 2820
   cccgtgatca gtgcatgccg gatcgaggtg ggcgggaaca acggtctcga taatgtacgt 2880
   acactacaca caaagagaag caagtggggg cgggactgca cggcacttgt gagacacctc 2940
   agcatgccgg atcgaggtgg gcgggaacaa cggtctcgat aatgtacgta cactacacac 3000
   aaagagaagc aagtgggggc gggactgcac ggcacttgtg agacacctca gcatgccgga 3060
   tcgaggtggg cgggaacaac ggtctcgata atgtacgtac actacacaca aagagaagca 3120
   agtgggggcg ggactgcacg gcacttgtga gacacctcag catgccggat cgaggtgggc 3180
   gggaacaacg gtctcgataa tgtacgtaca ctacacacaa agagaagcaa gtgggggcgg 3240
   gactgcacgg cacttgtgag acacctcagc atgcaccatt ccttgcggcg gcggtgctca 3300
   acggcctgga tccacaggac gggtgtggtc gccatgatcg cgtagtcgat agtggctcca 3360
   agtagcgaag cgagcaggac tgggcggcgg ccaaagcggt cggacagtgc tccgagaacg 3420
   ggtgcgcata gagatgtgga gtatccgaat ttctccaggc tgtcaagcgg caattataac 3480
   cgagactgag atcgagaagt atataaccgc agcagtagtg gataaataat tgcgaagtct 3540
   tcccagcaga gcgggctgtt ttttggagtt ggttactgta aaatgctaaa atgactgaca 3600
   acaatggagc gtctacagca ttggcaacag tgggaacagt atgctggtgc atccagttga 3660
   taccccaggt tctgcgaaac tggtatgttc gggattgcga gggcgttcct cctctgatgt 3720
   tctttttgtt cgccgtttcg gggattccct tcgcagtgta cttcattgat cagaattcga 3780
   acactgccat catggttcaa cctcacttgt ttactttctt tagccttata ggcttttggc 3840
   aaagcctgta ctatccgccc gtcagttaat taataacttc gtatagcata cattatacga 3900
   agttattagg taaactaaat tcatgacagc cttttcttct ttctttccac aaaacaatta 3960
   aaaaaaataa cagaattaga agaaggtaaa tatattggca aactcctctc ttccttttac 4020
   ttattttttt gaaagttgca gtgtgtgtgt gtgttgttgt ttgttcaaat taatttgatg 4080
   gttgttgtat tgtaaatttc aatcaataaa aacaaagaca taaataaaaa aaaccctacc 4140
   tctcttccct gatctgattt gatcgtacga ttctaagaac tcaccgctaa ggccggccct 4200
   ttgacaggta tatcttcagt ttcctcgtca ctcttggtca aaagaccaaa gtcatggctg 4260
   gcgatttcct cgatgctttc ctcaagaatt ttcaaggagt tgtggctttc caactccatt 4320
   tgaaccttct tcgaggcttc gtggaatttc ggatttccaa ttatcgaatc aacagcttct 4380
   ttgatttgct ccactgtagg caagccagtt ttcaaatcaa ttgccacgcc agcggcctca 4440
   gctctcgatg ccaccattgg cttgtcttca gagtcaccag caataacaac tggaacagag 4500
   tggcttaagc tgtgctgaag tccgccatat ccaccattgt agacaagagc atcaacgtga 4560
   ggaagtagag catcgtagtt gaagtagtcg atcacgcgag cattctcagg aaccacaaca 4620
   tcatccggta gcttggcacc gcggcggccc aatatggcta ctgttaaagt gtcaggctcg 4680
   tccttcaagg cctcaagagt aggcacaata agatgcttgt aactgacagc aaaagttcct 4740
   tgagtgacca tgatgactcg cttggcactc agaacatccc cccaccagga aggaggggtg 4800
   aattgagttc ggtgcttggg cgttgagccg gcgaatttga agttgctagg cagatggtct 4860
   ctgctgaact caagagaagg cgggcacagc tgcaggaact tgtctgcagg tacctcaagg 4920
   gcgaattcgc ggccgctaaa ttcaattcgc cctatagtga gtcgtattac aattcactgg 4980
   ccgtcgtttt acaacgtcgt gactgggaaa accctggcgt tacccaactt aatcgccttg 5040
   cagcacatcc ccctttcgcc agctggcgta atagcgaaga ggcccgcacc gatcgccctt 5100
   cccaacagtt gcgcagccta tacgtacggc agtttaaggt ttacacctat aaaagagaga 5160
   gccgttatcg tctgtttgtg gatgtacaga gtgatattat tgacacgccg gggcgacgga 5220
   tggtgatccc cctggccagt gcacgtctgc tgtcagataa agtctcccgt gaactttacc 5280
   cggtggtgca tatcggggat gaaagctggc gcatgatgac caccgatatg gccagtgtgc 5340
   cggtctccgt tatcggggaa gaagtggctg atctcagcca ccgcgaaaat gacatcaaaa 5400
   acgccattaa cctgatgttc tggggaatat aaatgtcagg catgagatta tcaaaaagga 5460
   tcttcaccta gatccttttc acgtagaaag ccagtccgca gaaacggtgc tgaccccgga 5520
   tgaatgtcag ctactgggct atctggacaa gggaaaacgc aagcgcaaag agaaagcagg 5580
   tagcttgcag tgggcttaca tggcgatagc tagactgggc ggttttatgg acagcaagcg 5640
   aaccggaatt gccagctggg gcgccctctg gtaaggttgg gaagccctgc aaagtaaact 5700
   ggatggcttt cttgccgcca aggatctgat ggcgcagggg atcaagctct gatcaagaga 5760
   caggatgagg atcgtttcgc atgattgaac aagatggatt gcacgcaggt tctccggccg 5820
   cttgggtgga gaggctattc ggctatgact gggcacaaca gacaatcggc tgctctgatg 5880
   ccgccgtgtt ccggctgtca gcgcaggggc gcccggttct ttttgtcaag accgacctgt 5940
   ccggtgccct gaatgaactg caagacgagg cagcgcggct atcgtggctg gccacgacgg 6000
   gcgttccttg cgcagctgtg ctcgacgttg tcactgaagc gggaagggac tggctgctat 6060
   tgggcgaagt gccggggcag gatctcctgt catctcacct tgctcctgcc gagaaagtat 6120
   ccatcatggc tgatgcaatg cggcggctgc atacgcttga tccggctacc tgcccattcg 6180
   accaccaagc gaaacatcgc atcgagcgag cacgtactcg gatggaagcc ggtcttgtcg 6240
   atcaggatga tctggacgaa gagcatcagg ggctcgcgcc agccgaactg ttcgccaggc 6300
   tcaaggcgag catgcccgac ggcgaggatc tcgtcgtgac ccatggcgat gcctgcttgc 6360
   cgaatatcat ggtggaaaat ggccgctttt ctggattcat cgactgtggc cggctgggtg 6420
   tggcggaccg ctatcaggac atagcgttgg ctacccgtga tattgctgaa gagcttggcg 6480
   gcgaatgggc tgaccgcttc ctcgtgcttt acggtatcgc cgctcccgat tcgcagcgca 6540
   tcgccttcta tcgccttctt gacgagttct tctgaattat taacgcttac aatttcctga 6600
   tgcggtattt tctccttacg catctgtgcg gtatttcaca ccgcatcagg tggcactttt 6660
   cggggaaatg tgcgcggaac ccctatttgt ttatttttct aaatacattc aaatatgtat 6720
   ccgctcatga gattatcaaa aaggatcttc acctagatcc ttttaaatta aaaatgaagt 6780
   tttaaatcaa tctaaagtat atatgagtaa acttggtctg acagttacca atgcttaatc 6840
   agtgaggcac ctatctcagc gatctgtcta tttcgttcat ccatagttgc ctgactcccc 6900
   gtcgtgtaga taactacgat acgggagggc ttaccatctg gccccagtgc tgcaatgata 6960
   ccgcgagacc cacgctcacc ggctccagat ttatcagcaa taaaccagcc agccggaagg 7020
   gccgagcgca gaagtggtcc tgcaacttta tccgcctcca tccagtctat taattgttgc 7080
   cgggaagcta gagtaagtag ttcgccagtt aatagtttgc gcaacgttgt tgccattgct 7140
   acaggcatcg tggtgtcacg ctcgtcgttt ggtatggctt cattcagctc cggttcccaa 7200
   cgatcaaggc gagttacatg atcccccatg ttgtgcaaaa aagcggttag ctccttcggt 7260
   cctccgatcg ttgtcagaag taagttggcc gcagtgttat cactcatggt tatggcagca 7320
   ctgcataatt ctcttactgt catgccatcc gtaagatgct tttctgtgac tggtgagtac 7380
   tcaaccaagt cattctgaga atagtgtatg cggcgaccga gttgctcttg cccggcgtca 7440
   atacgggata ataccgcgcc acatagcaga actttaaaag tgctcatcat tggaaaacgt 7500
   tcttcggggc gaaaactctc aaggatctta ccgctgttga gatccagttc gatgtaaccc 7560
   actcgtgcac ccaactgatc ttcagcatct tttactttca ccagcgtttc tgggtgagca 7620
   aaaacaggaa ggcaaaatgc cgcaaaaaag ggaataaggg cgacacggaa atgttgaata 7680
   ctcatactct tcctttttca atattattga agcatttatc agggttattg tctcatgacc 7740
   aaaatccctt aacgtgagtt ttcgttccac tgagcgtcag accccgtaga aaagatcaaa 7800
   ggatcttctt gagatccttt ttttctgcgc gtaatctgct gcttgcaaac aaaaaaacca 7860
   ccgctaccag cggtggtttg tttgccggat caagagctac caactctttt tccgaaggta 7920
   actggcttca gcagagcgca gataccaaat actgttcttc tagtgtagcc gtagttaggc 7980
   caccacttca agaactctgt agcaccgcct acatacctcg ctctgctaat cctgttacca 8040
   gtggctgctg ccagtggcga taagtcgtgt cttaccgggt tggactcaag acgatagtta 8100
   ccggataagg cgcagcggtc gggctgaacg gggggttcgt gcacacagcc cagcttggag 8160
   cgaacgacct acaccgaact gagataccta cagcgtgagc tatgagaaag cgccacgctt 8220
   cccgaaggga gaaaggcgga caggtatccg gtaagcggca gggtcggaac aggagagcgc 8280
   acgagggagc ttccaggggg aaacgcctgg tatctttata gtcctgtcgg gtttcgccac 8340
   ctctgacttg agcgtcgatt tttgtgatgc tcgtcagggg ggcggagcct atggaaaaac 8400
   gccagcaacg cggccttttt acggttcctg gccttttgct ggccttttgc tcacatgttc 8460
   tttcctgcgt tatcccctga ttctgtggat aaccgtatta ccgcctttga gtgagctgat 8520
   accgctcgcc gcagccgaac gaccgagcgc agcgagtcag tgagcgagga agcggaag 8578
<210> 90
   <211> 4657
   <212> DNA
   <213> Artificial
<220>
   <223> Vector
<400> 90
   ggtttaaacg aattcgccct ttcatctcga gatgctttat tcaggcacgc tacgtgagaa    60
   tattctaatg ggatggtctg gccctgagtc tgaagtaacg caggagatga ttgaggatgc   120
   cgctcgcaaa gcgaacattc acgaattcat catgtcgttg cctgatggct acgaaacgct   180
   cagcggatct aggggatcgt tgctatctgg ggggcaaaag cagcgaattg caattgcaag   240
   ggccctgatc agaaatccaa aggtactcct cctcgatgag gccacctcag ctctggattc   300
   cgaatctgag aaagtagttc aagcagcact cgacgcagca gcgaagggcc gtactacaat   360
   cgccgttgcg catagattat caacaattca gaaagcagat gtcatatatg tgttctcagg   420
   agggcgcatc gtggagcagg gcgaccatca gagcctcctt gaactcaatg gatggtacgc   480
   tgaattggtg aacttgcaag gtctcggaga gatttgacgt tcatttattt ttggccactg   540
   cttgcataca ttatttgatt aaaggcactc attaattgaa atagcatatc gaatttctct 600
   agttatggcc cctgagtcac catacattgt ctgattaaag ggactcgtta attgaaatag 660
   cacattggat tcctctgatt atgacccctg agtcacctat cctgcataat tcactcgtga 720
   cgataatctg tagatatagg gaactgtcgt agtacttgaa gagacagcaa caatctatct 780
   ctgggatttc gtgctgattt tgggcttttg ctttgacggg ctatgactga ggtaatgtag 840
   accaataata accctcacgc gaattagata tgccctgagg gttagcttgc atcaccttac 900
   ccatatgcac actgacttgc attacccgga gcatattccg gtagtcggag ataagcactt 960
   tgagatatct taaggtacaa ctcaatacgt tcctccttcc ttgcctcatt ccacctcaca 1020
   ttctagaatt caataacttc gtatagcata cattatacga agttattaat taacatcatc 1080
   gtcactatac acatcgtcat caactccatg gcgtgaggac ttccgagact gctgggccct 1140
   tcgtttcttt aatgcctcaa gagatgactt cgtacccgaa gagacgcctg ttgtaccccg 1200
   ttgacgcttg gcggaggggg cttcgtcctc gtcagcaacc cgcgtcatct gcttccttcg 1260
   ctgagcaaga taccttctct cctcgtaccg ctgcatctcc tgagctcggt catacaagat 1320
   ctaagcttga gacacctcag catgcaccat tccttgcggc ggcggtgctc aacggcctca 1380
   acctactact gggctgcttc ctaatgcagg agtcgcataa gggagagcgt cgactattcc 1440
   tttgccctcg gacgagtgct ggggcgtcgg tttccactat cggcgagtac ttctacacag 1500
   ccatcggtcc agacggccgc gcttctgcgg gcgatttgtg tacgcccgac agtcccggct 1560
   ccggatcgga cgattgcgtc gcatcgaccc tgcgcccaag ctgcatcatc gaaattgccg 1620
   tcaaccaagc tctgatagag ttggtcaaga ccaatgcgga gcatatacgc ccggagccgc 1680
   ggcgatcctg caagctccgg atgcctccgc tcgaagtagc gcgtctgctg ctccatacaa 1740
   gccaaccacg gcctccagaa gaagatgttg gcgacctcgt attgggaatc cccgaacatc 1800
   gcctcgctcc agtcaatgac cgctgttatg cggccattgt ccgtcaggac attgttggag 1860
   ccgaaatccg cgtgcacgag gtgccggact tcggggcagt cctcggccca aagcatcagc 1920
   tcatcgagag cctgcgcgac ggacgcactg acggtgtcgt ccatcacagt ttgccagtga 1980
   tacacatggg gatcagcaat cgcgcatatg aaatcacgcc atgtagtgta ttgaccgatt 2040
   ccttgcggtc cgaatgggcc gaacccgctc gtctggctaa gatcggccgc agcgatcgca 2100
   tccatggcct ccgcgaccgg ctgcagaaca gcgggcagtt cggtttcagg caggtcttgc 2160
   aacgtgacac cctgtgcacg gcgggagatg caataggtca ggctctcgct aaattcccca 2220
   atgtcaagca cttccggaat cgggagcgcg gccgatgcaa agtgccgata aacataacga 2280
   tctttgtaga aaccatcggc gcagctattt acccgcagga catatccacg ccctcctaca 2340
   tcgaagctga aagcacgaga ttcttcgccc tccgagagct gcatcaggtc ggagacgctg 2400
   tcgaactttt cgatcagaaa cttctcgaca gacgtggcgg tgagttcagg ctttttcatt 2460
   gtggatgtgt gtggttgtat gtgtgatgtg gtgtgtggag tgaaaatctg tggctggcaa 2520
   acgctcttgt atatatacgc acttttgccc gtgatcagtg catgccggat cgaggtgggc 2580
   gggaacaacg gtctcgataa tgtacgtaca ctacacacaa agagaagcaa gtgggggcgg 2640
   gactgcacgg cacttgtgag acacctcagc atgccggatc gaggtgggcg ggaacaacgg 2700
   tctcgataat gtacgtacac tacacacaaa gagaagcaag tgggggcggg actgcacggc 2760
   acttgtgaga cacctcagca tgccggatcg aggtgggcgg gaacaacggt ctcgataatg 2820
   tacgtacact acacacaaag agaagcaagt gggggcggga ctgcacggca cttgtgagac 2880
   acctcagcat gccggatcga ggtgggcggg aacaacggtc tcgataatgt acgtacacta 2940
   cacacaaaga gaagcaagtg ggggcgggac tgcacggcac ttgtgagaca cctcagcatg 3000
   caccattcct tgcggcggcg gtgctcaacg gcctggatcc acaggacggg tgtggtcgcc 3060
   atgatcgcgt agtcgatagt ggctccaagt agcgaagcga gcaggactgg gcggcggcca 3120
   aagcggtcgg acagtgctcc gagaacgggt gcgcatagag atgtggagta tccgaatttc 3180
   tccaggctgt caagcggcaa ttataaccga gactgagatc gagaagtata taaccgcagc 3240
   agtagtggat aaataattgc gaagtcttcc cagcagagcg ggctgttttt tggagttggt 3300
   tactgtaaaa tgctaaaatg actgacaaca atggagcgtc tacagcattg gcaacagtgg 3360
   gaacagtatg ctggtgcatc cagttgatac cccaggttct gcgaaactgg tatgttcggg 3420
   attgcgaggg cgttcctcct ctgatgttct tttgttcgcc cgtttcgggg attcccttcg 3480
   cagtgtactt cattgatcag aattcgaaca ctgccatcat ggttcaacct cacttgttta 3540
   ctttctttag ccttataggc ttttggcaaa gcctgtacta tccgcccgtc agttaattaa 3600
   taacttcgta tagcatacat tatacgaagt tattaggtaa actaaattca tgacagcctt 3660
   ttcttctttc tttccacaaa acaattaaaa aaaataacag aattagaaga aggtaaatat 3720
   attggcaaac tcctctcttc cttttactta tttttttgaa agttgcagtg tgtgtgtgtg 3780
   ttgttgtttg ttcaaattaa tttgatggtt gttgtattgt aaatttcaat caataaaaac 3840
   aaagacataa ataaaaaaaa ccctacctct cttccctgat ctgatttgat cgtacgattc 3900
   taagaactca ccgctaaggc cggccctttg acaggtatat cttcagtttc ctcgtcactc 3960
   ttggtcaaaa gaccaaagtc atggctggcg atttcctcga tgctttcctc aagaattttc 4020
   aaggagttgt ggctttccaa ctccatttga accttcttcg aggcttcgtg gaatttcgga 4080
   tttccaatta tcgaatcaac agcttctttg atttgctcca ctgtaggcaa gccagttttc 4140
   aaatcaattg ccacgccagc ggcctcagct ctcgatgcca ccattggctt gtcttcagag 4200
   tcaccagcaa taacaactgg aacagagtgg cttaagctgt gctgaagtcc gccatatcca 4260
   ccattgtaga caagagcatc aacgtgagga agtagagcat cgtagttgaa gtagtcgatc 4320
   acgcgagcat tctcaggaac cacaacatca tccggtagct tggcaccgcg gcggcccaat 4380
   atggctactg ttaaagtgtc aggctcgtcc ttcaaggcct caagagtagg cacaataaga 4440
   tgcttgtaac tgacagcaaa agttccttga gtgaccatga tgactcgctt ggcactcaga   4500
   acatcccccc accaggaagg aggggtgaat tgagttcggt gcttgggcgt tgagccggcg   4560
   aatttgaagt tgctaggcag atggtctctg ctgaactcaa gagaaggcgg gcacagctgc   4620
   aggaacttgt ctgcaggtac ctcaagggcg aattcgc                            4657